# EUROPEAN PATENT APPLICATION

(11) **EP 2 292 800 A1**
(43) Date of publication of application: **09.03.2011**
(21) Application number: 09305792.5
(22) Date of filing: 27.08.2009
(51) Int. Cl.: C12Q 1/68

(54) **Combination of DNA markers for characterizing and fingerprinting a Lactobacillus sakei isolate**

(71) Applicant: Institut National De La Recherche Agronomique (INRA), 75338 Paris Cedex 07 (FR)
(72) Inventor: Chaillou, Stéphane, 78910 Orgerus (FR); Champomier-Verges, Marie-Christine, 92330 Sceaux (FR); Lucquin, Isabelle, 92140 Clamart (FR); Zagorec, Monique, 91400 Orsay (FR)
(74) Representative: Jacobson, Claude

(57) **Abstract**

The present invention relates to a method for characterizing a *Lactobacillus sakei* isolate which comprises the step consisting of determining the presence or absence of 46 specific DNA markers in said *Lactobacillus sakei* isolate.

SEQUENCE LISTING

<110> INRA

<120> Combination of DNA markers for characterizing and fingerprinting
 a Lactobacillus sakei isolate

<130> BET09P1037

<160> 213

<170> PatentIn version 3.5

<210> 1
<211> 2927
<212> DNA
<213> Lactobacillus sakei


<220>
<221> allele
<222> (530)..(530)
<223> G is inserted in some strains

<220>
<221> allele
<222> (1480)..(1565)
<223> the sequence is deleted in some strains

<400> 1
atgttgatac tgatattaat aagttctgta attattaatg agaacgttgt caacgcagtc 60

caaagacagg ataattcatc agaaatcaag gtgccaacag attcaaaaac aagtagtcac 120

tcagaacaat cagtgagwac ccaatccaaa tctgaatcta agtctgaatc taaaacgacg 180

tcatctgaaa agymttcttc tgagamgaca amrcagtcmt cragtagtca tsrrgrtaag 240

yctgtgattc agaatcaatc aygagataca ataatgccga cagctactat tgatgatggc 300

gtgacaggtt ggcaaccggc aaagcaaaca acgattgcaa gyattgatat tgataaagcg 360

gctaataaaa gccttaatta tacttttggt aatagtgatg ggaccttaaa gtttgatcaa 420

ggaaatggtg ggataatarc gaataacatg ccgccaagaa tgaacgtttt cattaaggat 480

aatggcaaga ttgtcgagtc ttcattcaaa tcaacatctg ggatgggggc tcgtaygact 540

attcactctt aaccactaga agttccacgg caccaacgat taaaaccaca tcgacatcgg 600

cacaggratc actgacaggt gtgcaatatt atcaagccaa agacgccaaa gggcagacaa 660

ttttaaaaat ggtggggcaa tatacaacca tagtggggcg tgtccccagt ttcacacttg 720

agtatctatt acggccgtca ctagaaaaac catctgttca acaggaacta tatatttata 780

atgcgacatc ggcagcaatt gatggcggga tcttttttgc taaagatact tcgttaaatg 840

gtaacgatgg crtaccagtt tctgcgcaag ggaataacgc tgggatgtac attgccgatg 900

gtaaatataa actattcctt aatatgggtg ttgaggatgg tccagccaaa tttaacgcga 960

ygaattatca ccgaaatggt ggatataact tatctgatta tgcaatgaat gattattcgc 1020

cagccaactt tgatggtacg ggtatcraag ttaaaaatct aaaagaaggg gctactgttt 1080

attcaggaag tgattctgcg tatagcgcta agtggaattr gcaaactttt cagccagata 1140

ccgtttatca ttttagaaat gatttaggga ttgcgactgc gggcttrgtt gtcccagaag 1200

cggcagagam ctataaaaat aaaacgacaa gtgatcaaaa aaatcacgtt caagataata 1260

ttacyatcga aatgaaarcg cayaattyag gytatagtag tgaatggaaa gatattaacg 1320

taacgtcaaa aataycagat gaactggata ttgatcctag tacaattaaa gtaactctga 1380

ataatggcga tcaagtagca attgatgcta gtaagtataa ccaaacgact agaatgttaa 1440

cggttagatt acyyaaaaat ttgkccgaaa atgartggtc atcgatcagt ttcgatgcca 1500

aaattaatgg taaagcttct ggaaaaacaa ttaatagtag catggttgct aagactgaag 1560

gtgggactgg cartttygat acrgctacrg cgaryacgaa aatcccwgty gargttgaac 1620

ctgtcgaaat tgaaaagaaa gttttaaatg aaactgaaaa agaaacgacg taccaaaarg 1680

cgacggtcgg gcatgtaaat gatgttcttg attaccaagt gaagttaaca gtcagcagta 1740

acggagctga tatgtctaac ggtgtcttgg ccgatgacct agctaaggaa gggttaraat 1800

tagtgccggg ctctgttaaa ttgacatata gcgataagac agttgaaacc ccagcagatg 1860

ttaaacagat taatttgaaa aaaatgacac ctggacagaa tgtagtatta acctataagg 1920

ytaaagwcaa agaaggtgtt gtgataggca cagtgcttaa aaatattgtt ttatattctg 1980

gggaacaagc taatcagggt gttatgaacg ggcaagctga cgcgtcagta acgattgaaa 2040

agacgaagaa tagtgacgtt cactttagtt atattgatcg tgaaacgggg cagcagattr 2100

ctaaygaagt cgttgcaact gggccaattr atgccaaaat atcagcatta aaagcaactg 2160

atattagcga tgggcaagat cctaataaga ttcggcccgc ctacattgaa ggttatacgc 2220

cagttgattt tacgacggca actgatttaa acgctgcggt ctatgcggat attaaagatg 2280

ttgayccagt aattgaagaa aaatcggtga cctatacytt tagatatgaa aaaacgcgct 2340

tagcgattac ggcgttacca agtaaactga attttggtaa attcgacgat acgcaaagtg 2400

aacggacgtt ttatttgccg gcacaaattg agaaaccarc tgatgaaaaa acaccatatg 2460

gcattgaaat ctctgattat tggggcatta agggctggac gttgagtgtt rcacaggaac 2520

aacaatttca tggcagtgcc acgatagatt yaaaggaaca agyagttgag ctgtccggcg 2580

cacaactcca gtttaataay gcaacattga gcacgmatac ggaaggtaat aagtctgttt 2640

cgaattygca ggataaagta gtaactaaat caaactttaa cttggtaccc ggrgctgagc 2700

cgttagaatt agttgaatac gaacgaaaag gccagtacct taatcaagac ggcgacaata 2760

aaggcggggt cagttatgat atccctggct attcagttca taaatatcaa tttggtgacg 2820

caagaacggc tgattayagt attggcctgc atgtcccaga aacgactgaa cgttaccgta 2880

cagaatacac atcaacattg aagtggcatt taacggytgc gccataa 2927

<210> 2
<211> 4698
<212> DNA
<213> Lactobacillus sakei

<220>
<221> allele
<222> (873)..(2015)

<223>  the sequence is deleted in some strains

<220>
<221>  allele
<222>  (2012)..(2012)
<223>  A is inserted in some strains

<220>
<221>  allele
<222>  (2029)..(2029)
<223>  G is inserted in some strains

<220>
<221>  allele
<222>  (2681)..(3698)
<223>  the sequence is deleted in some strains

<400>  2
atgaagggta ttattttagc aggtgggtca ggtactcgcy tgtatccaat tacaaaggca      60

gtwtcaaarc aacttattcc rgtttatgat aaaccaatga tttattatcc aatgtcaryt     120

ttaatgttag ctggcattca ggayattatg attatttcma caccaaryga tacaccacgt     180

ttcgaagaat tatttggtga tggycatgat ytgggyttaa atattgaata tgccgtgcag     240

gataatccca acggtttggc cgaagcattc attattgggg ccgattttat cggtgatgat     300

tcagtctgtt taatcctagg tgataacatc tattatggtg gtggcctatc agaaatgcta     360

caaagagcgg ctcaaaagga atctggtgcg acggtctttg gytaycawgt taatgaccca     420

gaacgttttg gggtcgttga ttttgatgaa aatatgcatg cgaaatcaat tatcgaaaaa     480

ccggaacatc cagcaagtaa ctacgcggts ackggtttrt ayttctayga caaycaagtt     540

gtygagattg ctaaaaatat caaaccatca ccacgrggtg aactcgaaat tacmgatatt     600

aacaaggctt atctygaaaa aggycaatta gatgttgaat taatgggccg ggctttgcck     660

tggttagata ctggcactca cgattcattr catgaggcya gcagttttat tgcaaccgtt     720

caaaagcgsc aaaayttaaa rgttgcttgt ttrgaagaaa ttgcttaccg aatgaactat     780

atcgataaag mraaagtgat tgaattggcr caaccgctta agaaaaatga ttatggccaa     840

tatttattga gattagttgg tgaaaaaaat gattagatta ttattcttag ggacacagga     900

tattgatgat aaattatcgt ggtctggcac catgtaccac atgacacaag caattaaaaa     960

agatgaaaac tatactatat cttctttaaa agttccggag aaaagttttt tggataagtt    1020

aagaattaaa atgcttgcta taccaagaaa aatttttagc ccagggaaaa aatatgttag    1080

taatgtagac gtacaccaag ttcgaattaa tggaaaaaga ttaacaaaac tattggcaga    1140

taaagttgaa aaggaatcag tagatattat atttgcacca gccatgagca catacttagc    1200

taatttaaca actaatattc caattgtata tttgagtgat gctacttttg cactcatgaa    1260

taattactac tggtttggat taagcaataa agaagttagg gatggaaata gagtagagtt    1320

ggctgctcaa gaaaattcca cagcacttat tttttcaagc gagtgggcgg ctaatagtgc    1380

tattaatgat tataaaagtt caatagacaa ggttaacata gtaccgtttg gagcaaattt    1440

agcgactagt aatttaataa aaaataaaag gattaattca acaagagagg ttaaaattct    1500

tttggtcggt aaggattatg aacgaaaagg aatttttatt gctgaacaga taattcaaaa    1560

gttgaatatt aattctgata tccgatacaa gctaattgta gttggagttg accgagaaga 1620
tactgataat attcattatg ctggagtatt ggataaaggc aatgaaacgg attataaaaa 1680
attgatgaac tattaccatg aatcagtatt gttcttacta ccgacaaaag cagaggcagc 1740
cggaattgtt tttgctgaag ctagtatgtt tggactacca actctgactt ttgatactgg 1800
tggtattggt gaatatatca ttaacggcgt aaacgggtac cgaatatcac ttgatagtca 1860
agatattgta gcttcattta cggataaaat tgatgaaatt gtacatggtg aagtattata 1920
tgagcgttta tccgtaggtg cggtggaaca atatcagacg cgattgaact gggatcattg 1980
gatgacagaa tttaatagga ttgttrasaw tgtamwmrrw gakkagrrya akmaathatg 2040
ggaaaattaa aagtaacaac aactaaacta caagacgtta aaattattga accagccgtt 2100
tttggtgatc accgwggvtt ctttacrgaa tcatattcag aycgtgactt taaagaagcy 2160
ggrattgata tcrattttat tcaagayaay cattcaytwt caackcaagc mggtgtytta 2220
cgtggyttrc atttccaacg kggcaaagcy gctcaaacga aaytaatycg rgttgtkacw 2280
ggygcygttt tagacgtcat ygtrgatgtg cgtgcyggyt cmccaactta yaagcaatgg 2340
gaaggytata tyctttcagc aagtaaycac cgtcaattat tagtrccaaa gggyttygcy 2400
cacggytttg trackttaac mgataaygtt aayttcttat ayaaatgtga yggytaytay 2460
gatgcmgaag ctgatggtgg gatttcattt aaraccccag aattaaayat tgactggcca 2520
atygattttg accaagcrat tacatcagaa aaagatgcra accaaccaac ctttayagaa 2580
ttcgaaaaag acaacccatt tgtttacggt gaaatttaag gagactctaa cttatgaaaa 2640
atattattgt taccggcggc gccggtttta tcggttctaa ttttgtccac tatgttgtta 2700
acaatcatcc agaggttcac gtaacggttt tggataagtt aacttatgca gggaatcgcg 2760
ctaacttagc tggtttacca gcagaccgtg tagaattagt ggttggcgat atctgtgacg 2820
ctgaattagt cgataggttg gttcaaaaga cggacgcggt tgtgcattac gctgcagaaa 2880
gtcacaatga taactcctta aaggatccat cacctttcat tcaaactaat attattggct 2940
catatacttt aattgaagct tgtcgtaaat ataacgtgcg ttatcatcac gcttcaactg 3000
atgaagttta tggtgattta ccattgcggg aagacttacc aggtcagggt gaaggtgctg 3060
gtgaaaaatt tacacctgaa acacgttaca atccaagtag cccttattca tcatcaaaag 3120
ccagttcaga tttattagtg cgtgcttggg tccgttcgtt cggtctacaa gcaactattt 3180
caaactgttc aaacaactat gggccttacc aacacattga gaaatttatt ccccgtcaaa 3240
ttactaacgt gttaagtggc attcgtccta agctttacgg ttcaggtcaa aacgtgcgtg 3300
attggattca tacgaatgac cactcaagcg ctgtttgggc catcttaact aaaggtaaga 3360
ttggtgaaac ttatttaatc ggtgccgacg gcgaacaaaa caacaaagat gtcttggaat 3420
tactcttaga attaatgggt caacctaagg atacttacga ccaagttaaa gatcgtcccg 3480
gtcatgattt acgttatgcg attgattcaa cgaagttacg tgaagaatta ggctggcaac 3540
cagaatttac tgatttcaaa tcaggattac aacatacgat tgactggtat cgtgataacg 3600

aagactggtg gcaagcagaa aaagcagcag ttgaagccaa ctatgctaaa aatggtcaat 3660

aaatataact aaatggaagt aggcgagcgc ctactttttt kaaggagaak artwaaydat 3720

gaaaatttta atyacbggtg cyaatggtca attaggracy gaaytacgtc ayytattaga 3780

tgaaaatcaa atygartayg trggmacdga tgcygctgaa ttrgatatya csaatgcaga 3840

agcrgttaat rcttattttg aagcyaacaa accrgaatta gtttatcact gtgctgctta 3900

tacagccgtk gatgcwgcyg aagargaacc tggcaagtca atcaaytaya aggtmaacgt 3960

sgttggkacr caaaatattg ctaaygcygc wgaagyagtt ggsgcaacmt tggtttacat 4020

cagtacagay tatgtcttcg atggcactaa yraycaaatg tayacagaag aaacaccwkc 4080

wgcacckaag aacgaatatg gtcggacaaa attrrckggt gaagaagcyg tkgctaayac 4140

gatgagcaag tattayatya tccggacatc atgggtyttt ggtgaatayg gtaagaactt 4200

tgtttayacs atgttraacc tkgckaarac acaygatcat ttaacrgttg ttagtgatca 4260

agtcggtcgg ccaacttgga cycgsacrtt ggctgaattc atgttatatt cagttaaaaa 4320

yraygtmcct tayggtttat aycwactttc aaaygayaat tcatgtactt ggtatgaatt 4380

ygcgagygaa atyttgargg ayaargmygt wgaagtragt cchgttacrt cagaygaata 4440

yccmcaaaaa gchttccgtc caagacattc aatyatggat ttgacgaaga ttaaagcaac 4500

yggytttgaa gtaccaacat ggcaagaagc attacaaaaa tttatggcaa agattgacta 4560

atatagatag tatataaaaa ggataaagtc gactgacttt atccttyttt attagattaa 4620

caayytacta aactytttct gcaccctaaa tttaatatta ttagcygttc gccgtgtgat 4680

gtttaagtcc gcggcgat 4698

<210> 3
<211> 1081
<212> DNA
<213> Lactobacillus sakei

<400> 3
tacttatctt ttggaggaga taaaatgtct aacttttttg cattactatt ggtcatttct 60

ttttttgccc tatcctatta tggcattaag cggtatatga ccaagaaaaa gaatcccgag 120

gtttacgcca agatcaagaa acggatctgg tatgcactta ttatttttgt aatcagtttt 180

attggtgtgg aggcaactca acctgctgcc gctaaaacaa aagctccaca gcatgacaaa 240

gtatcaactg tcgataacaa actaacagcg gcaaaatctg cggtaaatgc gctttttaca 300

aatagcaagc acactaaatt aaaacaagat gtgaccaagc atgatatcac tgctgctgac 360

aagcaagtca aacagctaaa agattcaaaa actaaaaagc aactacaaac atcaatcacg 420

accgcttcag atttactaaa aaaagctgaa aaagaagctg cattagcaac ctcaaaatcc 480

gaaagtgctc gcgctgcttc taaatcagaa tccaaagctc aaaaagaatc ggaagaacgt 540

gctgaaaaag aatcttcaga gaaattagcc gcctcgattt caaactcaga aagcgtatca 600

agtgtccaag ctgcttctac tagcgctcgt caagcaagtg aacaagccgc ttcaagcgct 660

tgcttctgaa agtattcgca ttgctaatga acggctgcta gagctgcaag tgaatcaact 720

gaacgagcag caacagttgc cgcccaaagt acacaagcaa caggcgctga tacaactacc 780
aatcaaggtg tgatcgtcgg taacacaaga tctcacatct atcatgtacc cggacaagca 840
acctatcata tgagttctac taatatgatt acattcccta acgaacaagc tgcgattgtt 900
gcaggttatc gcaagtcaga acgttaatca ccagctaggt aaccctttat ggttaacaag 960
aataggttca attcctatta gagttgttat tgaagaaaaa aataaaaagc acataagaaa 1020
gagctattaa accgcggttt aatagctctt ttcattctag gatatgatca atatcacccg 1080
t 1081

<210> 4
<211> 3534
<212> DNA
<213> Lactobacillus sakei

<400> 4
gtgactttaa ataaacgaaa tatcatattc agtttgttgt tgttactggt aatgagcatt 60
caaatgatag ttactactgg ggtgagtctt gctgaaagca acatccaaat agattcagac 120
ctagcgttaa caccagacca agaattatct aatgagaaac aaatcgtgat taaagtaagt 180
gaccaagcaa aagcacagcg tgatttaaag cttaatgtac cggcaggcac aacgtttgat 240
gaaaaggcca ctgataaatt aaatccagag ccaacatatt cggttgcgtt caatccaaaa 300
gataaaacga atcagttaat acttaaagcg ttggtaaaaa agacagttga aaacgaacca 360
gtcgaaacgc caaccaatac cgaaataagt gaaagtcaca cagtaaccga atcaaccgcc 420
actagcgggt tggcaccagt cgctaagaag gattacgtat tagtttttaa gattaaagat 480
atcaagaccc ttgttaatca acaattaaaa ctagcgaccg tgctagaagg taaacgctat 540
gaatctaaac cgttaatttt taaagaattc attacgctag aagatgccgt tgctgctcag 600
tctactgaaa gtgcaagcaa ggcaccagat ttagtgaaag atacaatttc tctaaaagaa 660
aaacttccag cgtcaccttc aaagtctaac tcagttcttg aaacaccatc aatcgatgac 720
cctggtgaac tagatggctg ggttcaatct gataagaata ttatggtacc aattaaagtt 780
gatcagtcag caaatgaagc aaatcgtgaa tattttatgt attttggtgg gtactacacg 840
gggagtacaa cgcggccaaa tggtagtagt agttggggat cggggcaata cgaggtacct 900
attggcgcct cgaccaaaga aaatgtgcct gatgcagcgt tctttcaact tttaagtaaa 960
acccaggctg gtaaaatatt ttccgccgtt tatgaccatg ggaccaacag taaccccact 1020
gatagtaaat ttggtgaaac ggggctctct ttgttgacga ctaacaccga ttatgtggat 1080
ccgaaacagt ataatcccag acgaacaaat accaaggtga atgcgcttgg aactgatgcc 1140
cctaaaaata cttttccaat ttataaatta atgtataaaa aagacagtgc ccatcctgga 1200
ttacgagcat acgcgatgac gaatggtgct gatggtcaac cggaaggtat gttgcgcgtt 1260
actttggaac caatcaatca aaaaggccgg attagagcgc agattcggta catttcttta 1320
gataagaaac cacgtttgat ggcgggagtt actcaatgca tatggatttg gatacattac 1380
acacaagaac accgacctac tcactaggcg gaaccacagg gttatatact tatacggata 1440

aaacgccatc tgataaacaa ctgtttagac tgacttactg gaaagatggg taccccgatg 1500
atcctgaata cgaaatggtc aattctcaag atgatagtaa atattatggc aaacaatggg 1560
gacaatatac aggtggtggg gtctctatcc tagcaccggg atgggaaatc aatgggacgg 1620
ataagaatat tacggatcca ggcaaatatc agctttataa aggccatggt tacaatcacc 1680
ccgtttggca ctttaaatgg gcttatcagg aagtgcaacc aagagagcga tatcaagtga 1740
atatggattt tggcctatcg agtgaagtgg actacaacaa cgttgaagcc actaaatcgt 1800
atgagaattt gacctctaaa gatggctata accgagttgg tgatcgactg aaattcaaac 1860
tactagggcg gaacacggaa tgggatacta atttaaccaa tatcatgatt attgatgcta 1920
ttcctaagga ttttatggtg gatactgcca cctttaagtt agtagacagt acgggtaagg 1980
aacaagtctt aagcccaagt gtttatgatc cccaaacagg gcttttaaag gttgacgtat 2040
caggatcaat gggctttaac aaaacagttt atatcacctt tgaggtcaat attgctccgt 2100
ctgcatccgg taagacagtc acaaattcaa tggatattta taccgataaa gcttccaaac 2160
atgattcatc cgatgttcag gtcaaagttg aaaaggcgca agtcttatta cacaaacaag 2220
ttaagaattt gacgacacca gatgagtcct atgcaactga aacaaaagga aaattagggg 2280
atacccttag ttatcagatt catgctgtta atgtttcaac tgagcgtgag gttaagccag 2340
gcgctgatat tgaagatgtt ttagattcag ccttaacaag tgatcgggaa gtgatgattg 2400
attatttcga tgtcaacggg acaaccattc aacaagaaac cggtcagttt gataataatg 2460
gacacctaaa attggcatac gctattccta aagcaggtca tgccacacta acttttacag 2520
ccaatattac tgataacagt caagaagtta tcaataatag cgctcgagta acctacgatg 2580
atggcgaaag tacgcagtct aatattacgg ttgttaactt accaaagccg attatcccaa 2640
ttatgcaaaa agatgtgcgg aatgtgacaa ataacgatgc tgaatttgtt aaagaaacaa 2700
caggtgcaat tggtgatgaa gttgaatatc gtatcatggc cgttaatgca tcaactgaag 2760
agcgtattcc taaagagacg atgttcagtg atcaattaga tgaggattta gtcaatgata 2820
cgccggtgac agttgaatac tatagcgtac taggaactaa aacggcgacg caagaagtct 2880
atttccaaaa tggtaaattc aaattgaatt atgaaattga acctagtggt tatattatgg 2940
tctacttcaa ggcaaaaatt agcaagacaa ctaaatcagt tgtgaataat acagcgacat 3000
tagccgaggg aaaatcgaat gtcgctaagg ttaatatcat caaaaagaca ccaccaatgc 3060
ttattaaaga cgttaaaaat ctaactaaag ctgatgaaga ttacgcgtta gaaacagaag 3120
ctgaagttga tgatcaagtt gaatatcgga tccaactggt gaatatgact tcaattgata 3180
caaagatagg tgctgtttta aaagatgttt tagatccgga cttgggcgat attcaacaag 3240
ttaagattga ttacctcgat aaagatgaaa agattacagc cacccaaact gcggatttaa 3300
ctgatgatca ggtgacattg gatcacgtgc taccggctgg tggccaggga atggcaattg 3360
cctatatcaa agcgaaggtc aaagaaactg acaagacagt tattaataat caggccagtt 3420
taaccactga atttggtaaa ggtacttctg atcgggctaa attaaacatt aaacaaagca 3480
aaggtcgaat cgtggttcgt tatcgggacc gaaaagatga aagccacaag ttgg 3534

<210>  5
<211>  1260
<212>  DNA
<213>  Lactobacillus sakei

<400>  5
atgaaacaag taccagaaac aaaaatcaaa ctaacggatg taaaggagtt gattaataaa      60

gggtataacc ttttaggtga gcttcgtgaa gaagcagatg ctccagtagc aaaagcagaa     120

tttctgacag aagaaattac aaccgtttat ggagaagaag cagcgcgtaa attttataat     180

cctgaaaact tcaagcggga agggtctatg ccaaaagtag tattgaaaac tttatttggt     240

gaagacggcg tacagacgat agatggaaag aaacaccatc aacgtaaaaa ttatttcatg     300

gatttgatga ctccagaacg tatggaagac tatagagcta ttctagatca aaatcttgca     360

accgaactcg atcagcaaca tggcactttt gagttattcg acctttctaa aagagttctc     420

tttaactcta tttgtgaatg ggctggaatt aatctagcac agtatgatcc gaaagaaatc     480

gataagcttg ctagtaatca aatatccatg attagcggag ccatcacttc tccaacgaac     540

cacttaaaag gagtaaaaga ccgtaacgaa tcagagggat gggctcaatc attaattaaa     600

gaagctcgca aaaatcctgt accaggcaaa gaaaatcttg ccctctatac attcgcacaa     660

gcagaagacc tagaaggaga gttattacca gtagaggttg cagcagttga attgctaaac     720

atcattcgtc ctactgtagc actgacggtt tggatggcct taatggggca tgcgcttttc     780

agtaaaacga acttgtatga ccaattaaaa gaagactttg ataccctaca agattccttt     840

attcaagaaa tgcgtcgcta ctatccattc tttcctatgt taccagcgat cgctcttcgt     900

gatgtagaaa ttgatggtta cgaaatacca aaagatagct gggttgtctt agatatttat     960

ggtacaaacc atgacgctcg tacgattgat catccagaaa aatttgatat caagagatat    1020

attggaaaaa caaaagaaat ttcttatgaa gaagaatacg aaatgattgc gcaaggtgga    1080

ggagaattta gaaatatgca ccgttgtgca ggagaatgga ttactcttca cagtatgcgt    1140

gtcttttctg accaattagt aaataaatat aactttagta ttcccgaaca agactggacc    1200

gttccaatga accaatttcc tacgtatcca aatagtaaag cattgttgtt taaagaataa    1260


<210>  6
<211>  2581
<212>  DNA
<213>  Lactobacillus sakei


<220>
<221>  allele
<222>  (2051)..(2052)
<223>  the sequence is deleted in some strains

<220>
<221>  allele
<222>  (2276)..(2276)
<223>  G is inserted in some strains

<400>  6
gttaatgcgg cgagtcgtcc taaatctttt tatgaattag catttgatgc gcaagatttt      60

gaaccaatca cagtacccca acatatcgaa ttggcgkgtt atgagcaatt gcactacatc 120
aacacaatgt atccgtggga aggkcattat tatcggcgtc ccgctttytc aacryccgat 180
gacaagcaac atctcggyat gtttagygaa gccgattata atcccgtygg ttcatacctg 240
caycattttg atttaacgcc agcyttgaga aaycaacggg tcatcattcg ctttgaaggg 300
gtcgaacaag cgatgtaygt ttggctmaat ggycaattta tcggctatgc tgaagatagt 360
tttacaccgt cygaatttga cttaacccct tatcttaarg aaactgataa ttgcttggcg 420
gttgaagtgc ataagcgcag tagtgcggmt ttcatcgaag atcaagattt cttccgcttc 480
ttcgggattt tccgggatrt taagttrttr gctaaacccc gcacgcattt ggaagattta 540
tgggtrattc ccgaatatga ygtggyacar caaacrggac aggtgaaact tcgycttcaa 600
ttttcaggrg atgaaaaccg agtycatcta cgcattaggg accaacatca aatcatytta 660
acggccgatt taacaagtgs cgctcaggtr aacgrccttt ataagatgcc cgaattggtt 720
caagcrtggt ccaaccaaac yccyaactta tacacattmg agctagaagt tgtggatcaa 780
gctggggaaa cgattgaaat tagtcagcaa ccatytggct tccgtaaaat tgagattaaa 840
gataaagtga tgttgctcaa cggaaaacgg ttagtcatta acggcgtyaa tcggcatgaa 900
tggcaycctg aaactggtcg gacaattacg gccgaagatg aagcgtggga tattgcctgc 960
atgcagckya aycayatcaa tgccgtccga acatcgcatt atccagatcg gttatccttt 1020
tataatggtt gcgatcaagc tggcatctat atgatggctg aaaccaattt ggaatcgcat 1080
ggttcttggy aaaaaatggg ggcggtcgaa ccttcttgga acgtcccagg ttcatatgat 1140
gaatgggaag cggcgacgct cgatcgagca cgaaccaatt ttgaaacgtt taaaaatcac 1200
gtctcaattc tcttttggtc attaggsaat gaatcttayg ctggtagtgt tcttgagaag 1260
atgaaygcct attacaagca acaagatcca acgcgcttag tgcattatga aggggtcttc 1320
cgggcgcccg agtataaggc gacgatttcg gatgttgaaa gtcggatgta tgcgacaccg 1380
gctgagatta aggcttattt agataacgmc ccacaaaaac catttattct ctgcgaatat 1440
atgcatgata tggggaattc gctgggtggy atgcaatcgt atatcgactt attrtcacaa 1500
taygacatgt accaaggcgg ctttatctgg gatttcattg atcaagcact cttggtgaca 1560
gatccagtga ctggtcaacg tgaattacgc tacggtggcg attttgatga ccgcccatca 1620
gactatgaat tttcrgggga tgggttagtc tttgctaccc gcgatgaaaa accagcaatg 1680
caggaggtta gatactatta tggcgaacac aaataaacgc ttagcagtga tcttcggaga 1740
tgtgacttta ggacttaaag gccctgactt tcactaccwc ttttcgtacc aaacgggtgg 1800
gccagaatca ttgcggattc aaggtaaaga atggctatat cgctcaccaa aaccgacttt 1860
ttggcgggca actaccgata atgatcgcgg caaccaattc ccattgaaat cgggaatgtg 1920
gctagctgct gatcagttca ttgcttgtca gtcgattaca gtggcgattg acgggcaaac 1980
gattccgctg ccaattgcgc ccgagaacaa ccgctatagt ggtcrggaaa cggcccaaga 2040
agtgacggyt acgtatacct atcaaacaat tacgacgccg cagacgacgg tagaagtcag 2100

ttatacgatt caagccagtg gtaaaatccg tgtagcggtg acttatcatg gkcaagctgg 2160

attaccatca ttrccagtct ttggyttacg ctttgtcatg ccraccccag cgacgcgctt 2220

tatttatcaa gggttgtcag gggaractta tccagatcga atggctggtg gratkgctgg 2280

ygartacgaa gtcaccgggc tacctgtgac gccttatctr gtrccacaag attgtggtgt 2340

gcayatggca acygattggg tracgattta tcggcaagcg gtkctggata atygtttgcr 2400

cgaaccagtc gaaaccggct tgaagttyaa gryggtcgat caaccatttg cyttttcatg 2460

yttgccatat accgctgaag aattggaaaa tgcgacccat cattcagaac tacccgcacc 2520

acaccgaaca gtcttaartc tcttaggggc cgttcgtggc gtgggcggta tcgatagttg 2580

g 2581

<210> 7
<211> 2157
<212> DNA
<213> Lactobacillus sakei

<400> 7
atgatagaty gtaaaatwta tatwccrcar gtwgatgara tggattgtgg cgtagcagcy 60

ttagcgatga ttcttaarca ttatggttct tcagyttcgc tggcttattt acgtaatgag 120

gctaaaactg atttagaagg aacaacggcg cttggactag tgaagacagc tgagaattta 180

ggtttcgaaa cgaaggcrat tcaagctgay atgagtttgt ttgaagtcgc agatttacca 240

tttcctttta ttgcacatgt tctaaaaaat ggcgagctat tacattatta tgttgtacta 300

gggataaaaa aagatgccat tataatygcy gatcctgatc caacagtkgg yrtyrctaar 360

rtgrckcaat cacagtttaa akcggaatgg acrggygttg cwttatttat tgcwccaaar 420

ccgarctatc aaccagttaa gcarcaaaca aaagcgagtt tattytcatt tgtyccaacy 480

ttgatrmarc aaaagcgrtt ggtrattaat attacwttrg crgckytact catmacsatw 540

attagtatty trggwtckta yttcttacar actgtmattg atacstatat yccaaataay 600

atgcakagya crytrgcgat wgtygcratt ggkttaatya tmttttatgg yttycagtca 660

atytttacat atgcwcaram ytttttaytr rcrrtyttrg grcarcgrtt atcaatwgar 720

attattttra gttacttrag rcaygttttt gaattaccaa tgtcwttttt tgcaacacgw 780

aaracaggkg aaataatttc amgattyaat gaygckaata aaattattga tgcrctagcr 840

agygcratta tttcaatttt tttagatatt acwgtsgtyg ttattatggg saktatyytg 900

atsattcaaa atacaacttt attytggtta acyttagcrg tkattccaat ytatrttgcm 960

rtsattactt tatttgcraa rccwtttgaa mrrttraatc aaaaggaaat ggaaagtaat 1020

gcagtattga attcrgcgat yattgaagat ytacatgrya ttgaaacrat taaagcrtta 1080

acwagtgaam rkgaacgwta tcaaaaaatw gatacrgcrt ttrttgatta yytraaraag 1140

akttttrrct atgtwaargc tratacaytg carcargcra ttaagttrtt trtycaatta 1200

ggrytraayg tctttgtkyt gtgggtwggy gckmtwttag tyacscataa ysrgttatca 1260

atyggscart tratgacmta yaatgcsytr ytggcktatt ttgtaaatcc rttacagaat 1320

atyatyaaty tacaractaa rttacaragy gcgmgggtwg cyaayaatcg kttgaaygaa 1380

gtktatctrg twgaytctga rttyaagrya gawcgaccrr ttaaatcyga gwcacarttg 1440

rtaggmccaa ttgarttaca raatgtgagt taycaatayg gttatggkca rarrgtgytw 1500

gaakcgrtta rtytaarrat trwgccraat gaaaaaytra caattgtkgg catgagyggw 1560

tcggggaaat caacattrgt taagttaytm gttrattttt ttgaaccaac ggagggaaag 1620

gttcttttta atcaacaaga tacrcagaca attgataaaa agacattgcg gcagtttktt 1680

aaytatatts crcagacwcc ktayatttty tcaggaasya tytgggataa tcttaagtta 1740

ggtaatcgkc cmggratyac acaagckgay atcatkcatg crtgtgaryt agcagaratw 1800

aagamygata ttgaaaaaat gcckctccaa tttgaaacat tattggatga agatggwaay 1860

acwttrtcag gtggtcaaaa acagcgatta acrattgcgc grgcactmyt rtcaccagct 1920

crrgtaytaa twtttgatga gtcwactagt gggttagata cgataacaga gggrctattr 1980

gttgatcaty taatggcrtt agaagaaara acaatcattt tcatagcgca tcgwttagcg 2040

attgctaaga aaacgaayaa tattctkgtt ttagaaaatg gkagwgtkgt tgaaactggr 2100

acacatgawr ygttrgtgkm rgcacarggr cgrtattayc aattgctyaa tcattag 2157

<210> 8
<211> 2454
<212> DNA
<213> Lactobacillus sakei

<400> 8
agtactttga agataaacac ggtgttcgaa ttagtaatga aaatacttac tatgcagatt 60

caacagctgg tgcttcaagt gagcgattca ctaattttgc ttttgaattc gttaaccgaa 120

actatgaaac caatgaagac atttatttag ttatcaagaa cgaagaaacg aaggttgagt 180

tagatcgtgt tgatttcgtg attgataatc catttgccgg tggatttgga tttgatattt 240

aggaggtgag atttgtggat caagttattt tagataaact cgtcgcaaat ttccctggga 300

agtttgttcg taaggatctg actaagaaaa ttaaggaagg ggctaatgtt ccagtatacg 360

tattagagta tttattggga atgtatgcct cttcgttgga cgaagaatta gtcgaaaaag 420

gtgttgaaaa tgttaagaat atcttagcgc gtaattatgt tcgcccggat gaagcggaaa 480

aagttaaagc taagattcgt gaaaatggtg gctacacagt tattgacaaa gtaactgttt 540

ccctaaatga gcacaaggat caatatgaag ctgaattctc taacttaggg ttagctggcg 600

tgcagattag tgatgaatat cctaaaaaat atgatcgctt attaggcggt ggtatttggt 660

gtattgttca aatggagtac tggtttgacg aaaatgatca aaggaattcg ccatttattg 720

ctaaaaaagt agatccaatt caaatgccag gattagatat tgaggaatta aaaagaggac 780

gtgctgcatt cacagaagac gaatggatcg atttagtttt gcggtcaatt ggaatggaac 840

caacacagtt tgatgaacgc gtaaaatggc ttcatttagc aaggttggtt cccttaattg 900

aaaataattt taacctatgt gaattgggtc cgcgtggaac aggaaaatca catgtttata 960

aagaaatatc acccaattct attttagttt ctggtggtca aactacagtc gctaatcttt 1020

tttacaatat ggcacgtcga tcaatggggc tagtgggtat gtgggatact gtggccttcg 1080

acgaggttgc tggcattaat gtcaaagacg gtgatcttgt tcaaattatg aaggattata 1140

tggcctcggg ttcatttgct cgtggtaaag aagaaaagaa tgcgtcggcg tccatggtat 1200

ttgtcgggaa tattaaccaa agtgttgatg tgattttaaa aacatccaac ctttttgaac 1260

catttcccga aaagatggga actgattcag cgttcttaga tcggatgcat tgctatttac 1320

cagggtggga gattcctaaa tatcgtccgg aattctttac aaatgattat ggctttatta 1380

ctgattattt ttcggaggta atgcgagaac taagaaaaac aacctatagt gacgcgattg 1440

ataagtattt tagattagga gcacaattga atcaacggga tacgattgca gttaagaaga 1500

tggtttctgg cttaattaaa ttagtttacc cagatggaaa ctttggtaaa atcgaagttg 1560

agaaaattct acgattttca atcgaaatgc gccgtcgagt taaagaacaa ttgaaaaaaa 1620

ttggtggtat ggaatttttt gatgtcaatc tttcttatat tgatttggaa gagaatcgtg 1680

aagagtatgt gtctgtacct gaacaaggaa gtggcacatt aattccagaa ggaattggaa 1740

aaccagggca tatctattct gtttcaaccg gggctagcgg cagaatcggg acctttacat 1800

ttgaaaccca aatggtggct ggaaatggta agtttaaagc gactggactt ggtggggatg 1860

gacaagctaa agaaaaagca gattcagctt ataagtatct aaatgcgaat gcgagctcta 1920

taagtgctag catctcaaca acaacccacg attatgtgtt taatacacaa gacttaaacg 1980

gtgttggaaa tccggaaatg ctatcgctac ctgcattgat agcactttgt tcagtaactt 2040

taaatcgccc acttgttgct agcactgtca ttttaggaga cgtaacaatt ggcggaactt 2100

tagtcaaagt gacgaattta gctgatatga tgcaagtagc aagagattca ggtgctaaaa 2160

agattttagt accgatgatg aatgctgcgg atttatcgac cgtaccgcca gaattaatgg 2220

caacgtttaa catcattttt tatcaaacac cggaagatgc agtttataag gcattaggcg 2280

ttgaatgaga ttcaatatat agaaatccag tgttttgcgg gggatattta gatggcaatt 2340

acaactaatt ataaagaact aaaactatgt aaacatgggt tacctacatg ggatgcgatg 2400

ttaccagttg ttttattggt agcaggtgag catgccgagc caatcaaaag aagt 2454

<210> 9
<211> 777
<212> DNA
<213> Lactobacillus sakei

<400> 9
actgataata gagtaacgta tgatcgccct ggatatgcag tttataagta tcagtttggt 60

gatgaaaaaa cagcggatta cagtattgga tagtttgtac caggagcaac gaagcgatat 120

aacacgactt attcgacaac cttattatgg aatctaacgg ttgccccata aagcacattt 180

tataaagaag aggatgttga actgtgaaaa tgaaaagcat agcatttggc cttttagccg 240

gtttagtcgg cttaggctta caaatacatt ctgtatcagc ggcgactgat gaacaatctg 300

attttgaaat ccaaagaatt ccggtcgaaa cgcaagttaa taatgacgtt aattacttcg 360

atatgcagat ccaaccgggt aagaaacaag tcattatgat gaaggttaag aattatacgg 420

atcataaagt gaaggttaaa tcagaactca gaaatagtat gacgcaagtc ggtgggggcg 480

ttaacttcgt ttctacagct aagggtttgg atgaaagctt gaaatatcca ctaacaagta 540

ttgggcatgt tgataagaag gatcgtgtca tcacccttaa cccattagaa acaagaacag 600

tttcagcaac gattaagatg ccaagtgatg gtttccgcgg gttagtctat ggggactggc 660

atttccttga agaaacgaca agtaagagta aagattctca cgtttctagt aattacgcct 720

actcaatggg gattgcctta cgtggcgctc attacaaggt ttatccagag ttgaagt 777

<210> 10
<211> 1024
<212> DNA
<213> Lactobacillus sakei

<400> 10
atggagagaa taagtgaata caaagtattg aataataatg ttttagctgg agtacaaggt 60

ggtaaaaaga aaaagggtgg cttcttttgg cattattttg gagatcctat tgttagtttt 120

ggtaaaggat ttattggata ttaaataaaa ttaaggagta ttcttatgaa taaaaaattg 180

gatagtttca gcagtattga agatgataaa ttaggactag ttattggggg acgaaataat 240

ctagcatatg gtcttggtaa gttagttcgt gccggtgttg atataggaat agcgattggg 300

agcaaaggtc gctacaaacc aagacactaa attaatgaaa tcataggtaa gataattgaa 360

tacgttagaa gtgattagcc agagaagggc tgttggtggg tttgtattat taataatagg 420

ttatttattg agtagtcgta ttagtctaga aatttctttt tttaatatat cttgcttatt 480

tttaatctta ttttcgtata gtgcactgat tgtattattt ggttatcaat caacaaaaga 540

tttattaaaa ccgctaaaac gaccgtttgt gagaaaaata ttatcagcag ttggattaag 600

ttttctaatt gtctttggaa taagtattgt gttaatgatg ttttttggag aagtggcggt 660

cactcagaat ggcaatattc cattcctgaa gtcaattact gtaaagtggc aattagtgag 720

cttgctaatt atctttttaa acataatagg tgaagaatta tgggtcgctg ggattgtttt 780

accaatagcc acttggctaa cagaatataa atttaattgg ctattggcta atttaattgg 840

ctgtttaatt tttgcattaa tccatttaga gatttataag ttcaatattg caatatgtct 900

aattgtagga ttttctaggt atggattttc gatggcttgg aaatcgaatg atacattaag 960

aggtgggata tatgcgcatc tattatatga ctccttattg ttagctgtga atatactatt 1020

ataa 1024

<210> 11
<211> 1041
<212> DNA
<213> Lactobacillus sakei

<400> 11
atggcaacaa ataatttagt tttgaagaca gctgacattt gtgatattcg tgatattgaa 60

gaggcgcggg caacaatcaa accttacatc cgggaaacac cattaattca atcgatgttt 120

ttaagccgga atgtcgctaa gggcaatgtt tatctgaaat tagaaaacat gcaattaaca 180

ggctcgttca aatttagagg tgccaataac aaaattaatc atttaactga agaacaacgt 240

caacgtggga ttgtaacagc atcagctggt aaccatgcac aaggtgtcgc attaaccgct 300

aagttattgg gaattgatgc aacagttgtt atgccagaag aagccccaat tgctaaacaa 360

gaagcaacgg ctggttacgg tgctaacgtt gtcttacacg gtgcaacttt taacgatgca 420

cgtttataca tggaacaatt agcagaagaa aaaggcatga caatcgttca tccttacgat 480

gatcgtgaag tcatggctgg ccaaggcaca attggtcttg aaatcttaga tgaaatttgg 540

aatgtcgaca cagtaattat ccctgttggt gggggcggct taatttcagg tgtggcaaca 600

gctttgaaat ctttcaaccc atcaattcac attatcgggg ttcaatctga aaacgtgcat 660

gggatggcgg cttcaatcga tgctggcaaa attacaagtc accatgatga cttcacatta 720

gcagatggta ccgatgttgc tattccagga gacttaactt acccagttgt tcaaaaccta 780

gtagacgaat tcatcttagt cactgaagac gaaatcgcaa tggcaatgac cgacttgatg 840

caacggacta agattgtcac agaaggtgcg ggtgcattac caacagctgc cttattaagt 900

ggcaagattg atcctaagtg gcttgaagat aagaacattg ttgcgatggt ttcaggcggt 960

aatgttgatt taacacgggt ttcaggtatt attgaacacc tcttcaaacc agcagacaca 1020

agcaagggtg ttgttggata g 1041

<210> 12
<211> 7542
<212> DNA
<213> Lactobacillus sakei

<400> 12
ttacttttta agtttgtggt gcataatcgt atccactgtt tgcttcattc gccaattgta 60

ttgctcatct tctaaaagta acatcgccaa aatatcgata acataggtca gcgcaaattg 120

tgagttcaca aaccgcgcac tggcaaagcc aatgctatct tcaacaaaca acgtcaagtc 180

actcgcagct gccaatggac tatcagtccc actggtaatt gcagccgttt tagcaccatt 240

ttcccgcgca ttttggaccg cttgattgac ctcttccgta ttaccagaat ttgatagacc 300

aatcactaaa tccgttttgt taataatccg agaggtcatc aacatcatat gactctccgt 360

gacgacagtc gcttgaatgc ccattcgggt taatcgctgc ccaaactccg cgcccgtata 420

gcctgaacta cccaaaccat agataaaaag ctgctggctg gttttgatcc aactaaccaa 480

ctgatcaagg tccgcttgtt taatccgtgc ctgtgtctta gtgatgacat cttgatagaa 540

ttgataaatt tcaacggacg tttgtgattc atgatcaacc tgtccttgaa cccccgtcat 600

cgttggttgg gggccctcac taaattgtaa tttaaaatca acgaagtttt gacagccaat 660

cttacgaaca aaccgcgaaa tggttgaatt actaacttgc agttcagcgg ctagttcatc 720

aatcccaatc cgctgaatgc tcgccccttt ttgaatcacc gctagggcca ccttttgttc 780

ttgtggcgat aaaaaagaat attgttgttt gatatgctcg ataattgcca ttgtaactcc 840

tttaaaaacc gagattgtga ctttcctact attatattgg atatgaagca ctttagaaag 900

cacataaaaa gacgcccttc ttgcaggccg tctttttcga tagattaata ggatctctcc 960

gttatactaa ttaaaaatta taatatcaac cgattacaat ttgtatccaa ataccagata 1020

aaaaatctag caatattttt caattgcagt cgtaatcatc gttgctgatt ctttagcaat 1080
ttctaaatca gcttcattta acggtgctaa cggttctcta acagatccta agtctaattg 1140
ttcattaatt cgtagaattt ccttaatcat tgcatacata ttgccatgac ccgaacacat 1200
cttatatata atttcatcaa tagcgtattg aagatcttta gcttcttcta acttcagttc 1260
actaatcaat gtatttaatt ttaagaataa tgccggcatt gccccatacg taccaccaat 1320
tcctgctgca gcccccatta aacgaccacc taagaattgc tcatctggtc cgttgaaaac 1380
gatatgatct ttaccaccgg cagcacagaa catttgaatg tcctgaacgg gcattgatga 1440
attcttaaca ccgatgacgt taggattccg gcgcatttct gcgtataatt ctgttgtcaa 1500
actaacacca gctagttgag gaatgttgta gataataaaa tcagtattcg gtgcacctgc 1560
actgattgtg ttccaatagt tggcaattga atattctggc aatttaaaat agattggcgg 1620
aattgcggca atggcatcaa cctgcagact ttctgcatgc ttagcaagtt caacactatc 1680
ttttgtattg ttgcaagcaa cgtgtgcaat aactgttaat ttgcctttgg ctaccgccat 1740
cacattttca aggacaattt tacgatcttc aacgctttga tagatacatt ctcctgaaga 1800
gccattaaca taaacacctt tgacaccctt tttaatgtaa tattctgtta aagcacgtac 1860
tctttctgga ctaatttctc ctgcttcatc ataacatgca taaaatgctg gaataatacc 1920
tttatacttc gttaaatcct tcattataaa tccctctttc tttttatagt tcattaacgg 1980
catcaatata agttttagca atcaattgtg gccgtgtgat acttgatccc acaacgacac 2040
tatgacaacc taattttaga gcagtgactg ctttttcagg tgaatcgatt tttccctctg 2100
caattacggg cgccttggac atcgctaaaa cctgttttaa atattcaaaa tcattatcgg 2160
caatattgca gccttccgta gccggtgtat aaccatacat tgtcgtacca ataatgtcga 2220
aacctaatcg gttggcttct ttcacatcat caagactggc cgtatcggcc attaataatg 2280
tatcaggata ctcagtccgc attgtttcga caatcgttgc taattgttca ccgtgaggtc 2340
gtggacgccc cgtaacatcg caagcaacaa ctgctgcacc agtggcagca atgccacgca 2400
tttctttgat tgtaggcgtg atataaatgg gtgcatcaga ataatctact ttatccaacc 2460
caattaccgg tagctcaacc gtatccataa ttgcctgaac atctactacg gaattagccc 2520
tgattgcact agcgcctgct tcataagccg ctcgtgccat tcgtgccatg ataaatgagc 2580
tgtgtaatgg ttcatccgct aatgcctgac atgatatgat taatcgatct ttaaccttat 2640
ccaaaaaatt atttttcaaa ttcatcaacc ttctattctt tccctaaata acaacctata 2700
aaactttaaa actaattcca tctaaattgt attaaccgct tacacataag atacggcagt 2760
tattaatttt catcaacaat tagatatgaa aaaaatttcc aatttactaa aataataaaa 2820
cgacgtagtt acattacgcc gcttcattat tttatccctt ataagtttca gagattgtct 2880
gaactgttaa cccagtgata tctttattgc tcttcttgaa gaccaagcta actaaataac 2940
taattacaaa cgctgagata aatgaaacgc aactaacaac aaacggtgaa atggaagcca 3000
tctgtgcaaa ataagttaga acagcactag caattaagcc aatcaaggca cctgttgcat 3060

tagctctttt tgtaaaaatg ccaactgcaa agattcctgc caatgggaca ccgaaaatac 3120
cagtaatcgt catgaataaa tcgagcgttt ttgattgatc tgaaagcatc aaataaatag 3180
cgactgctaa actaattaat cccgaaataa taataataac tctggcccat gtcacatccg 3240
aatattgctt gaatttatca ttaaagaaac gaactttgaa atcagcaatc gcacatgatg 3300
agattgcgtt taaactggaa gcaactgtcg actgcgctgc tgcaaaaata gcggcaatca 3360
ctaatccagc gatacctgct ggtagtgttt taataataaa ataaggcacc aatgctgttg 3420
tattaaaacc cttaggtaac gattctgcac gactataaaa actatataag acagtaccca 3480
taccatagaa aattggtact gtaataattg ccaatagtcc attcgtccat agggatttaa 3540
cggtttcttt catcgtactt gtcgtttgat accgttgaat aacatcttga ctacctgtgt 3600
attgataaag tgtattaaaa aactgacctg caaaaattag tggtataaat cgtgagagaa 3660
cacttgcgtt gaaatcaagt ccggaaatta ttttatgatc agccatggca tcattgaaaa 3720
cagtccccca tcccccctta atgtaatgtg cgcccaacat gctaaccaat aatgcgccac 3780
ctaacaacag aatgccttga ataacatcgc tccagataac cccttcgata ccaccaagga 3840
atgtataaat aatacataac ccaccaacaa taaccgcaat taaaacagga ttaatatcag 3900
taaccgatgt gacagccagg atcggtaagt aaataacaat cgcaactcga ccaatatgat 3960
agagcataaa tagaatgctc ccaacaattc gcatcaatgg attaaaacga tgttcgagat 4020
attcataagc cgttgttaca gataattttc taaaaaaagg cacgtagaat ttgatcaaaa 4080
taggaattaa aataataatc gctaaactcc caaccgaata ggaccaatcc tgtaagaatg 4140
cttgttctgg tgttgacata aacgtaattg cgcttaacgt cgttgcataa atactaaagc 4200
cagcggccca tgctggaatc tttccttctg ctttaaaaaa ggcatccgta ctcttgctag 4260
ctttcttcgt aaaataaaca ccaacgagta acattgctag taagtaaaca cataaaacaa 4320
tccagttaaa tgttccgaaa ccaatttttt gcattttctt cacctctaaa atttattgtg 4380
cggtcaatac attttaggat gattggattt gcactaattt ttaattaatg ctaaactcca 4440
actgaaaatt aaaaattatg aatggttcaa ctataaacac caacttacaa agttgtcaag 4500
acttttttga taacgcttac acgaaaggat tgttttttat gttaactcaa gcttcctatc 4560
tgacctttga tattggtggc accaccatca aatatggctt aattgatgaa aatttatctc 4620
tcacccattt aggccaatta gaaacccagc aaaatcaaaa tggcatgata atgcaacaac 4680
ttatccagac tactcggaaa gtgatgacca cttataaaat cattggcatc ggtgtaagta 4740
ctgccggtat tgtcaatcgt gaattaggta ctattatcta tgccggacca actatcccaa 4800
actacatcgg tactgaaata aaagccattc tcagtcatga atttacgctc ccagtctatg 4860
ttgaaaatga tgttaacgct gctttattag gcgaaaaact agtaggtgct gctcaagatg 4920
ctaataatat ttattgtgtc gcactaggta cagggattgg tggcgctcat ttattaaaca 4980
accacttaca agatggcggt agtaggtgct gctcaagatg ctaataatat ttattgtgtc 5040
gcactaggta cagggattgg tggcgctcat ttattaaaca accacttaca agatggcggt 5100
acggctcaag ccaactcaat tggctatctc ttgtatgatt ctgccacgca aaccaactat 5160

gaacaacgcg cctcaacctt aacactacaa gctcaacttt cagcacaatt acatcttagt 5220
gtccctgaag catttgatgc tgctaaacgt ggcgaatctt tgccattaga gattattaaa 5280
aattggagcc gttcagttgc acaaggtctc gctcaaatca ttttgattgt tgatccagaa 5340
ctcttgatta ttggcggtgg tgtctcaaaa caggaaaaat ttctacttga tctattaaat 5400
caaagtatcc ctgcctatct tccgcctaat ttctatcgga caaaactagt gactgctcat 5460
aattttaatg atgccgcctt gtttggagct gtttatcgtt ttttcaatta aagcgcttgc 5520
aacaaattaa atacgtgtta tactttaatc aatttcatct taatatttaa ccgctaaatt 5580
taagacgatt ccctacatag ggagtagtct gtcaatttag ctttttttat ggggtgaaaa 5640
catgacaaaa attttaatca ataccatcgt ttttcaaaag gaaattcaca gtggccaatc 5700
acaatatgca ctcttaaatc aattaaccgc aaaaatcgct ggtttcgaag ttcgaggaga 5760
attatttaag ccagaaacga aacacgatga actagtccaa cttcaacaac ttgctgagca 5820
gcgtcattgg gaatttcgct attcaatccc agaatgtctt tttattaaat ctgagattaa 5880
tcccaattta gaatcacact ttaaattagc acaacagttt ggaattactg cattaaaaat 5940
tagtttaggt gaattggaac acataacggc tcaacaagtt agcttattaa accaactcat 6000
tacaacctat caagttcaac tcaatatcga aaatgaaccc aatcaaaatg gtattttaaa 6060
gcatatggca atgatttgcc tagaacttac caactataac tcacttactg gctatacctt 6120
tgattcaggc aattggtatt ggattaatga agcaccatta accgcttggc atcagcttgc 6180
accttacaca actgttttac atctcaaaaa tattcaagca caagaaactg ttttactcga 6240
taacggttta acaccatggc ggcaactttg tgaatcctta tcaccaaaca taccaattat 6300
tcttgaatat ccaatgtcta attccgaatt agaatcagaa ctaacattgg ttgctagtgc 6360
attggaggcc taatatggct tatctactac cacttgcatt catcatttta ttgactaagt 6420
tgttctctca cctatttgcc cggcttaatc taccaactgt tattggagaa ctagcagtcg 6480
gtgtcctatt tggtccagct tgctttaact tgattcaatc taacgatttt attacaagtt 6540
tcgcgcaact tggcgttatc tttttaatgt ttatggccgg aatcgaaggt gatctcagtc 6600
agttattaaa atattggaaa ccttccctaa ttgtcgcaac gttaggtgtc attttaccaa 6660
cattaggtgc ctttttcatc tgtgtaacac tctttcattt tacagtcagc accgctattt 6720
ttatcggctt aattctaagt gcaacttccg ttagcatatc aatccaggtc ttcaaagaaa 6780
tggattatct caatactgaa gaaggaatga tcattcttgg cgcagctgtt gcggacgata 6840
ttatctgtgt cattctatta gggatttttt ctagtatcat gcaaccagca tctggtcagt 6900
cgctcaatct ggttaaccta attttaccta aactattatt ttttattcta gccttcgttt 6960
taggtaaatg gttcgtccca ctaattttaa atctagccaa acgcttaaca gcttctgaaa 7020
acattactag tatcgccatt acactctgtt tactgtttgc tagcttagca acttatttag 7080
gtatgagcga tgtcttgggt gcctattttg taggactagc tatttcacag actgactata 7140
aaaatgttct aacagaaaag attgaaacaa ttggctactc tgtttttatc cccgtatttt 7200

tcgtcagcat cggacttaat attcgtctgg cccatcttca agataatctt agttttatcg 7260

tattaacaat cattatcgcg attattggta aacaattagg tggatggctt gggactcgta 7320

tttgtggtct ttcctcaagt gctggtcata ttgttggtgc agggatggtt tcccgtggcg 7380

aaatggcatt ggttgtcgca caggccgcaa ttaacgccca tttaatcgac gacagtcatt 7440

acacagcgat gattattgtc accgttgtta caactctaat tgcaccacta ttacttaaaa 7500

atctttataa tgcatcaagc acaaaaaatg tctactaatt aa 7542

<210> 13
<211> 3162
<212> DNA
<213> Lactobacillus sakei

<400> 13
gtggaagaac agatattaca atatcgagat gccgtttatg agcaagtcgt caaggtaggg 60

aaatcattag ggaatgtgac gcgcttgcgg atactagatt tattggtgca gggtcccaaa 120

acggttgaaa atttagccaa tacgattggg ttaagcgttg cgacaacgtc acgcaattta 180

cagattttga agaaggcgcg cttggttgaa attgaacgac ataaaaattt tgtaacctac 240

cgcttaacgt cagatcagat taagcagctt atatcgttat tagtcacggt cgcagaacag 300

tcacagcctg aattggcagc cattcagaca acgttcaagc aacaaactgg ttcaccacag 360

gcgttaacga tttcggcgtt aaaagcgaaa ttaaccgctg aatcaacgta cttagttgat 420

cttagaccca atgatgagta tgagacgcaa cacttgccgg gagcgcataa tatcccatat 480

gatgagttac cacaacattt agccgaatta ccgcaagata aggaaattgt tgtctactgt 540

cgcggtcggc tatgtgctta tgccaatgtc gctagtcagg cgttgcatga tgcgggtttc 600

aaagtggcga cctttaatca atcggtgtgg gaatggaatc agttgatgag ttcctaaagt 660

tgacttacaa aaagtaagat gatatattat gagtatcaat tcaacggaaa cgttgattaa 720

cttttgaaga ggaaggcgat tgtgatgaca gcacaaattt taaatgaagc caattttaac 780

gatgagatta aagaaggggt cacactagtc gatttttggg caacgtggtg tccaccatgt 840

aagatgcaag gaccaattgt tgaacagcta gcagatgatt atgcaggaaa ggctaaaatt 900

gccaaagttg atgttgatca aaatcaaaat ttggcagcgc aatttggcat tcaagccatt 960

ccaaccttat taatcatgaa agacggtcaa ttacaagaac aactagttgg gttgcaacgt 1020

aaagaagcat tagcgacaaa actagatgcc acattagcat aaataaacat ttaaaaagga 1080

tgacggcgaa ggtactttgc tgtcatcctt ttttatttag tagtaagttc taaaattaat 1140

tcgagcagaa aaaacttaaa gcagtctctt ttaatgctag tagctttttg gggaaggtat 1200

tggatcagca ataatcctac ctaataggac tattgctaac taatttataa gaatatatat 1260

tttattataa aaatgtctat tataaatata acagagtata atatatataa actaaggagc 1320

ttagttaagc attttagcca aaaattaata taatggggat aattactatg agttcaaata 1380

ataatatagt taataatatc ggagaacaaa ttaaacaatt aaggcaaagt aaaaaaataa 1440

gtgtagaaaa attatcagaa ttatcaggac taagttcact aactataaca aagatagaga 1500

atggaaaatc gaatccaaca ataaatactt tatggaagtt aacaaaatgt ctagaagccc 1560
cactaatgtc tctatttaaa aatagaggaa gtaatgtcgt tttttcaact gaaaatcatg 1620
gttatttttt agaagatgaa aatcacaagt gggaagtcga accaattgta ggtcaatttg 1680
atttcgaggt atataaagtt cggctaaaag ctaatagtaa gtattatcta gatagccaga 1740
cttctaatac tttagaggtg ataactgtac tatcagggac attaaatctt agagttgagc 1800
atgatagtta tattgttcat gaacacaata caatttactt cataaataat aattcgcatg 1860
agtatattaa tgatactaat gaagatattt tgatgaacat agttgtaaaa tatgagaacc 1920
tataatataa tatgcaatta tatactataa tatataattg catattttgg agggttcata 1980
tgagagagag tacaagagaa gagtgtagag gtaactgtat tacttctagg aattatattt 2040
gtcgcaacaa atttaagagc accattaact gcagttggcc ctttaacagg tttaattaag 2100
gacaatattg gtatttctgg aactattgcg ggatttttaa cgacaattcc gttattggct 2160
tttgctattt tatcaccagt ggcacccaaa atagctgaaa aattgagtct agaatatact 2220
attggttatt ctacaatcgt attagttata gggttattta ttagatcaat tccccaaaca 2280
agcactttat tatttggaac tattttagta ggtagtgcaa ttgccatgtg taacgtatta 2340
attccaagtt taataaaaag agaatttaat catcgcttag gcctaattac tggtatttat 2400
tcaattagta tgaatttatg tggggcaatt gcatctggag ttagttttcc attagctaat 2460
aatctaaatc taggttggaa taattcactt aggatttggc ttattttggc tttaattgct 2520
tgtatcgcat ggattccaca attgaaaaga catgataaac cagataagat agaggaatta 2580
cctatagaga atagtatttg gcattcaaaa actgcgtggc aagttactat atttatgggc 2640
ctacagtcac tagtattcta cgtgttagtc gcgtggttac ctgaaatgct tattcaaaag 2700
ggattcactg cagaacaagc tggttattta ttgtcaatga tgcaattatt tttattacca 2760
tttacattta taattccaat tattgctggg agggtcaaaa gacagagtaa aattgccata 2820
aacacatcaa tcctaatgtg tttaggaatt agtgggcttt ttataaataa tactattatt 2880
attattatag gaattgcttt tattgggatc agtggtggct gtgcatttgg actatcaatg 2940
atgtttttta acctaaaaac aagaaatgca agagaagggg cagaactatc aggcatggct 3000
caatcaattg gttacttgtt agctgcaatt ggcccaactc tctttggctt tatgcatgat 3060
atgactcata attggaatgt atcattaatt attttaattg tgactgctat tttattaggg 3120
ggatttggat tctttgctgg tgaagataat tatataaatt aa 3162

<210> 14
<211> 2202
<212> DNA
<213> Lactobacillus sakei

<400> 14
ttgaatgcgt ctaatgcgca aggaaaagcg ctrgctggcg ttcaactaaa gctcaaactg 60
atgggttcta aagaaagtta tgagcgaaca gttacgacaa cggcaacagg taaagctact 120
ttggcagctg tyccagtagg cgactatcaa gtgacgcaag aatcaacggt grygggttat 180

cagccagcca ctgctgttca gtcattaaca attagtaacg accatcagaa tacygtagat 240
tggcaattgc aacgaayggt agcggatgtt acttttagag ttcgcgatgc cgcaacgcat 300
caaccgytag ccaawgctgc ttttacttta acgacagcaa cgccggctga taacggtcaa 360
actgtttttg tttckcaaaa gacgaatcaa gatggtgaag kgaccattaa gcaaktacca 420
acaggcraaa taacttacga gcaaatkgcc aatgctgcgg gctacgagcc actagcagcg 480
attcaaacgg cgattgtggg tgctgatggg gcacaaaaca ctgaagtaac agttgataat 540
cagcggctaa aagtgactga aaaacagcaa attatcgtgc ataaaaccaa tcaacaaggg 600
caagggytag acgatgcggc cwttaaatta actaatctgg caactgggcr gacacaaacg 660
caaaagacgg tgtccgggca actacacttt actgagttga acccaggacg gtatsagatt 720
caggaaacaa aagcmccaac tggctatcag ttggatcaaa cacctcagtt tgtgacgrtt 780
aaagyccatg aaaaacggtt ataccaagtc cgctttgctg ataaacgtga agtgactgcg 840
ccaatcagtc cattgygcat ccagatttta gataatcact tgcaaggcgt tgctggtgtt 900
ttactgcgac taacygccga tcaaccrgat gaccaaggtc agaccgtttg ggaattgaca 960
actgatcgat tcggacaggc tatactcccc aatgcaagta cggggcacta tcgggttgag 1020
gttttacaag taccgaccgg gtatcaattg tcgtttgatc aaagtcaatt agatgtcagc 1080
cgctatggtg aaaatcagtt acaattacaa gctaatcgaa ttgagatgcc attgcaaacc 1140
ttaacgatta ayaaaaccaa cttaaaaggg caacctttat caggtgctgt ctttaaagtc 1200
gagtcactga aaactggtca gatgactaaa gttgaaacgg atcaaaccgg tcgggctgag 1260
ttgcsggacc aaaaaccagg gcgctatcgc gtaactgaag tggaagcacc acttggttac 1320
cggttagcaa ctgaacctag aattgtagcg ttgtctgaaa aatcaccgcg agcaacacaa 1380
ctaacggtaa cagatgaagc acaaatgggg cagttactga ttaagcatac aactaaaaag 1440
ggcgcaccga ttgcaaaggc ctactttgag gttaaggatc aaagtggtcg agaagtcggt 1500
tattatcaaa ctgatagtca aggacagatt aaattaaccc agctagcagt ggggcaatat 1560
actgttcaag aaatcaaggc accaactggg tatgaaatta atccggcagt gacaaaggtg 1620
gccattacgg atcgcaaaac rgcaacggtc gccattaagg ctgagcaaca agcacccgat 1680
gttaaattgg ggagttttgt attgatcgat agggatcaga aaacgcagtt agcgattgct 1740
rgtgcaactt atcgattggr aacgttagct ggtcaggygg ttcggccaga aattgtagtg 1800
ggtgcarctg gtcaagtcgt agttracgac ctcacacctg gtcaatatcg actcgtacaa 1860
ttgacggcgg cacctrgcta tcaaaaacaa actaacratc aaatcatcga rattaaacag 1920
gatgcgcaac ttaaacargt gacgattgaa agtcatcagt cacaagggac agtcattgta 1980
aatcaagtag atggcaatac gaaccaagct ttggttggag cgaaatatga attacaaaat 2040
caaagtggta argtgcttgt cgaaaacttg aagagygatg aacatggtca agttcgcatc 2100
acacatctcg atcctgatac gtaccgtttg gtgcaagtat cggcaactaa gggctatgac 2160
ccgcttaaaa agccgattgt ctttacgata accaatcgct aa 2202

<210> 15
<211> 3046
<212> DNA
<213> Lactobacillus sakei

<400> 15
gtgcaaaaat ttatgaacgg ctttgtaaag ggtgtttata cagtactttt ggtaagcatt 60

tttgctttta ttacgtatta ctcaatcagt aatttagtca ccggtaatta cctatctaat 120

tctaaattaa tgattgttgc tattgtcttt tacatattaa ttatcttggc attggtttta 180

ttgaccaaga taaccagtaa aaaaatattc tttatgattg taacgggaat ggcgatatta 240

gtcagaattg gttggttgat taaggtcccg acagcaccga gttctgattt tcaaatgatg 300

catcatgccg cgatactagc gacgcaaggt gacctctcat ttctaaagga aagttacttt 360

caaagttggc cttatcagtt aggctttgtt tattttcaac attgattatc aaaatatttg 420

gtcaaaatgt gcttattcta cagataatta acatccttct aaattgcgga attgcatttg 480

ttgggtataa gattattaat ctacacttta aagaaataac agggcgcatt gtgtatacgt 540

tattgctatt ttatccagcg tatatttata tgacaggtgt tttaacgaat caatttctag 600

caactttttt aatttattta gcaatctatc tatacttaaa gcatgatcaa ttatgggtta 660

aagcaactgc tggtgtttta ttagcactcg ggaatatgat gcgaccattg ggcattttgt 720

tgattattgc acttgtctgt tttgaaatta caaaatggtt attggcaccg gatcggaaaa 780

atattttaaa atcactcgga agagttacca ctagtgtttt ggcctatttt ctcatgttgt 840

tcttagtgaa tagcgctttg caagtaacgc atttatccga atatccgctg gaaaatagaa 900

atccaacttg gaaatttgta ttagggttga atgatgaaac cgttgggagc tactctgcaa 960

cggacttaag cttaatggat cggtatccac ttggcgcaaa acgagataag ctgggcaaag 1020

agattattaa ggaaagaatc caagataaac ctaagctcgt taatttaatg ttcaataaaa 1080

gtaagaagat gtggactgct cgggatgatg ccctaatgtg ggggatggca gataatgtta 1140

agttatcttt aaagattaaa aattggttga atgcggttca gtttttgttc tatattttta 1200

ttgtagcaaa tgccttatta gcagtattca aatggcgtga aacatggacg gacggttatt 1260

tattaaaatt gatgatcatc ggttatttta tggtccattt attaattgag attcagtcta 1320

gatatcgatt tttcattgtc ccggcattta tcatgttaac tgccataggt tgggtcactg 1380

tctatgaaaa gatccactca aggcaagcaa atgataaaaa gactttaaaa gaggatatag 1440

taataaggta ggattctaag acgactttat tatctatgtt tattactcga aaggacgctt 1500

taaaatgaca gttcgtaaca atgaggcaat aaaaaaatat ggtttctttt ttctggtttt 1560

tattttcttt ggttatctat caattaactt tccgttgact ggagatgatt taaactgggg 1620

gataacgact ttaaaaaatt attttggatc aggtcagttc ttaaattatg atggacgtta 1680

cgctggtaat agtttaatta tcattgctag tcatagtgcg gtatttaagg ttttaagtta 1740

cgcaggtatt acaacactag ttgtctatct ggctgcccat ttgattaaca acgttaggca 1800

attagaaaga aatacgttat taattcttat tttgatgtta acgatgagta cagagttgtt 1860

tgcgcaagtt ttagggtgga atgccggctt ctttaactat atggcttcat tggtatatcc 1920

cttaattatt attaacttag tgaagtatca ctattctgat tggcaagcat caaagtattt 1980

acgttatagt attttaatag cggttctttc aataatcagt tgcttttttg ttgaacacgt 2040

tacactatta aatttagcag ttgccacaat tttaatgggc tatatgctat accaaaaaaa 2100

gaaaaactac ttagtaattg ctaatttcat tggcacatat attggtggca ttttaatgtt 2160

ttccaataaa gcttatttaa atatattatt acatcatgac agttaccggg aaacaagttt 2220

ttcattgact aaggtttatc atattatttc acaacaaatg gatttttatc tcttgattga 2280

taatccaatt attacagtgt tacttgctgt cattctgggt tacttaatta ttaaaaatct 2340

ccaaaagcgt tcgggtaata agttggggac tgtggtttct tatctagaat tgagtatcct 2400

gattgctttc atagaatatc attatatttt gtttaatacc tttttaaagg acttcggcca 2460

tcgctattta gtatcatgtt tattatcaat tttattctta ttagtaattg taattgaaat 2520

ggctaagtta tcactggaaa cccacaatat cgaatatatt gcattaattg cagctgctat 2580

tgttttaatt gtgccattct ttgtagttac accgtttggt cctcgaggag cgtttgcaag 2640

ttatttctgt ttgtgtttgc tagtagttgc cttatgcaac gaattgaatg ctgttaaatt 2700

tagcttacca attatcagaa taatgatggt aatggttgtt atcttttatg cgggcttagc 2760

aacacgaatt ggtcatgcga gtcgtatcaa aaatgatttt ataacctatc aggaaaaaca 2820

taacacgcct aaagattatc gtttatatct agaggtaccg tataatcaat attattgggc 2880

ggttcatcca caggctactg atagtagtta cagagggtat tataagatta aaaccatagg 2940

gggcaagttg gtcccatatg ctaaatggcg tttaattgaa aagaatacga atagtaacgc 3000

agaaatgttt tctcaagttt taaaagttga tagtcaaaat aaatag 3046

<210> 16
<211> 849
<212> DNA
<213> Lactobacillus sakei

<400> 16
atgaagaagt ggggcttttt acttagcggt ggttttctag tagcaattat tgtaggaata 60

ttactgataa atcagcgtcc aaaccaagca agacaacaag caaatcgtgt ccatgacagt 120

gcaccaactt tttatttaca tgggtatggt ggctctggtc gttcgagcga tagtatgatt 180

gcggcggcag aagagcgagg tagggctact aaggtattaa ccgcaattgt cagtcggacg 240

ggacaagttg aattagaggg gcattggaca ggcaatacaa cacgaccgat tatccaggtg 300

atttataaga ataatcgcaa tgccaattac cggcaaaatg gtgaatggtt caaacgggta 360

ttgattgcgg ttaaccggca gcatcatttt aaacaattta atgtagtcgc tcattcgatg 420

ggtaatttaa cattagcttt ctatctggca aataatgctc agaataaaaa aatgccacaa 480

ttaactaagt tcgtttcgat tgcggggcac tatgctggta ttatcgggat ggatgatcgt 540

gcaaatcaaa atcatctggc taaaaatggc cgcccacagc agatcaacgc cacttaccgg 600

caactaatgg gattgagaca cagattaccc aaaaatcaaa ttcaatactt aaatatttat 660

ggggacttat cagatggtag tgattctgat gggcgtgtta gcaatgtctc ttcacaatcg 720

ttgcgatatt tagtggcacc aagagcaaaa tcatatcaag aagttcaatt taagggctcg 780

aatgcccaac atagcaaact atatgaaaac gaagctgtta ataaagcggt aatcgatttc 840

ctatggtaa 849

<210> 17
<211> 5307
<212> DNA
<213> Lactobacillus sakei

<400> 17
atgaagaagc gaataatcta tagtttatta ataattttgt tattaatgac aactgttttg 60

cagtcactag gtactgtatt tgcggcagca actgccaata atagtagtga aacaacatct 120

aaaataatag atactagtaa atcgattgca caagtgagtg agtcttcgca acttcgaagt 180

agcagccaga atgttgtgca atcttcgagt acaagtcaga aaaaagaaag taagaagagc 240

tctagttcga attcaacagg taagtcggtc atgcaaagtg catcattgtc ggcggctgat 300

gatactggac taacattcca tcaaggtaag ttagcacttg gtggtgggaa ctacttagcc 360

tatacaaatg ggacgaacta tgatgggatt cgaggtaaga caccgactat cactgatcag 420

tcgatttcct atgctaattt tgtagcgggt aaagttcaag ggtaccaatt tactaagacc 480

ttgatggaac acgctggacg gaataatatt ggtttatggg cacaaggtgc gaaagatttt 540

ggacaataca cacgttggat gtcaattcat aaagatgatg taaataaatt tgatattgat 600

cttggtgcag atacaccgat tgcccccaat caagctgatt tgagttttac aaatttaagc 660

tcctcgacaa ctttactttt tggcttagag gctgataatg cgattggaac aagtggaaca 720

ataaaagtta cggcatcatc attagaagca caaggaacta ttttgaagca gactttagaa 780

tatacacgta cggtcggtgg tgctgcgact acaattactg atgatgtaac gtaccaacca 840

actgattgga agaccgtcta cattaaggag cacatcacca atacttctgg tcgtgattta 900

aaaggcctct tctttggtcg aacaattgat actgatttac ggaatttttg gactagtgac 960

caagggtcag gtgccattgg agataagcca aaacttattg ctgatggtga tcgcggtatg 1020

tacgagaaac aagatatttc agcaacagaa ggtccaacta agtatcagga agggaatgct 1080

tctttatttt atgattttaa tcttttcaat ggttctggtc ctgatggttg gcaaggcttt 1140

aatctcacaa atgcgcccac aagtatccca tcaactttta ttggtaaacg atttgcgaca 1200

ccacgtgcaa aaggagatcg aacgacgaag gtaaacatcg ataccgaact cgacatgctc 1260

tggtggccta aagatttaaa acaaggtgaa tcgcgggata ttggctatga agttggttta 1320

aatggtgggg gtcaaccaag tccaccggaa atcactttga atcaaaagcc gcgagtaatg 1380

tatgttgatg gtagtcaaaa ggatgttgtc ttagacggga cagtgacgaa caaaaaccca 1440

actgattcag ctgatcctaa atacaagaag atgaatgaat taatctatta tgaagtgcaa 1500

cataaagatg ctagtggcaa tgtcacgacc ggtggcccta aagttctgac atcagtcgca 1560

gatgttgcgg ctggacaaac caagaattat tctggtctag ctaaaaggat tgaagatgac 1620

ctacacgtcg gtgatcggat tgttgtctgg gctgtcgata gtggagggat gacttccgtt 1680

aaccatgaac acactgactt agtagaacgt gcggctgaga ttagcaaaaa ggttaagaat 1740
atcacgaatg gagattcaag tttcaagacc gaaaccgact ataaaccggg tgatacggtt 1800
gaataccaag ggacgttgaa gaatcttaaa acgaaagtag ccttacctaa gggtgcaatt 1860
attactgatg aattagactc aaacttgacc gtcaaaggtg atgtaacgtt aacttactat 1920
gatgcgaatg acacgcagat tggcgcagct caaaccactt cgtttgatag taatacgcat 1980
actgtaaaaa ctgctaatga aattccagtc ggcggttacg tcgtgattga cttcaaggct 2040
aatgttgcta agaccgcaac aggtgatatt gataatatca tgcggtctaa ggttactgtt 2100
actgaaaaag atacacaagg taatgaccaa acatatgaag ttaatacaga tccatcgaat 2160
aaagcgataa tccatccagg tgaagaccgt gaagttcaag atttaaaaca gtacattaaa 2220
cttgatacca acactgattg gacattaggg acaaccgggg ttactgggca tcgtaaggat 2280
aaagttaact acaaatttgc atttactgct aaaaagagca attcggctgg aattagcgat 2340
gctgaaatca gcgacattaa gatggcgcct gatgaattaa cagcaccaac gaatgtaaaa 2400
gttaagatta taacgccttc tacgactaaa gccgatacat cagaaacagt tgatgggaca 2460
gctacaccaa gtagtgatgg tgagacttat tccattaaga ttgataagtc aatcaaagct 2520
ggtcaaaagg ttgaagtaac attcgatcga acagttaatg atgatgcaac aatcgatacg 2580
acaacaacag aacatgatca aacagggaag ttaacagcag cttcgttaag tacaccgatt 2640
gccgataagg ataactttaa ccttgcaaaa ttaaagattg aagaccaacc aataacggta 2700
actgatctaa aacaaacgat tagtaataca accacaccag caaatgataa gaatgatgca 2760
acaacgcatg ttgctgttga aacgtcaggt aaacaaaatg acatcattca atatacgttt 2820
actggtaaag ctggggataa cacagacgct ataaaggatc ttctgctttc ttcttttaac 2880
atgaataaac caaacgaaat gtcttatatt gatgattcat tggaaattac gattggctca 2940
gaggtacagg aaaaaacacc gcaggttcca ggtaatggta acaccgttaa gattacaagc 3000
gacttgccta agaatacgac ttttaaagta acctatcaga tgaaggttgt aaatgatgtc 3060
gcgaatacaa ttaccaacga tgcaaagtta tctgccacaa acttaaaagc aacaccattt 3120
aatacaacga ccttgaatac agccgcggct tcaaatacgg cgacaatcaa gcaattcatt 3180
aagaaccgga atacggctgg tgaaacttgg aaaggtccta atgtgactgg tgataaagct 3240
gaaacaagtg gtgttccagg aaacattatc gattacaaat ttgctattgc accaggtgcg 3300
aagaatagcg ctgatttact agatactgct ttgaaagata ttgcgatgaa agaatcgagt 3360
gggatgactt tagttaaccc agaaggttct atggataata caaagcaggt gaaagtcaca 3420
gttgatggtg tgacaccaca atatattagt ggtaatccca tcagtgcgaa tgcagaatta 3480
aacgatgtct tcacgccatt aacaaaaggc aaagggatga caattgagta cagcgctaag 3540
atcaatgata acgctggtac gcaaaatgtg acgaacgatg ctaacttcta cgcatcaaac 3600
ttaacgggtg atatgccagc aacctcaaca gtagctgaca aggcacataa aacaccagct 3660
aacaaatcaa ttttacacat tgtgcgtaaa gataacgtca caatcaaaca agaattgaaa 3720

caagattcgg caaccacctt taagacgacc gaaactggtg ttaaaggcga tacaatcgac 3780

tatcgtttca tggttactgc tggcgatgat aatagtaccg atattaagaa tattgtcatc 3840

gataacattg tgatggatcc agccggcaag ttggattacc aagctggcgt cacagcaacg 3900

cttcagggga cagcgctccc tgctggggat gtcgtcatga gtgatggcac aacagccggc 3960

acgaagaaga ttacggttaa gaatgtttca ttgtcgaaaa cacaattatt aattgtgaac 4020

tacaagatga agattacagc ggatatggat ggcgacgtaa acaacgatgg gctgttaaca 4080

gctgatagtt tcacggatca gaccaacacc gttgccgctg ataaagggaa atttaataca 4140

actgttttaa cccttaaaaa gatatccaat aaggcaaaga ttagccaaag tattaatctt 4200

aaagataatg tagcgaatac aaattcagga tggattgggc cggatgaacc ggcgcctaat 4260

aataaacctg cggaagcaac tgttaaccct ggggatacag ttacgtacaa gttttcgatt 4320

aaatttccaa cgaccgatcc gaagaataac gctgatctat tgaatagtgc gttacaagat 4380

attgcgatga atgtaccgga tgatttagag ctagcgaatg ttgtaggtac gactcataag 4440

attcaaatta gctatcggac gggtactgaa ggtcaagttt ctgagtataa cgatactatt 4500

aattcgatta aggacattaa aaaccttgac cttaagagtc cactttctaa gaatgtgaca 4560

gaggctcttg tttactacgg cgctaacatc atgccggatg ctaaaacaca agacgttacc 4620

aacgctgcga acttctacgc atcaaactta acgggtgatt taaaagatac gactgttgct 4680

aattatcaaa acaagacaaa agcgaaccaa tcaattttac acattgtccg taaagataag 4740

gtgacaatca gtcaacaatt gaaacaagat aaagcagcag aaacagcttt tggtccaaaa 4800

gcatctggta ctaaaggcga cacaattgat tatcgtttca aggttactgc tgataaagac 4860

aacaatgctg atgttaagga tatcgtcatc gataacattg tgatggatcc agccggcaag 4920

ttggactacc taacaggcat taccgcaaca gcgggtggaa cagccgtccc tactgctaat 4980

gtcactatga gtgatggcac aacggcgggc acgaagaaga ttacgcttaa gaatgtcgcg 5040

ttgaagaaat cacaagtatt aatggtgaac tacaagatga aggttacagc ggatatggat 5100

ggcgacgtaa acaacgatgg gcttttgaca gctaacgggt ttacagatca gaccaacacc 5160

gctgccgctg ataaaggcaa gtttaataca accacattga cattgaagaa acaaaagaac 5220

acggcaacca ttcaacaatt tattagaaat ccagcaacgg ctatttcggg acaagcgtgg 5280

tttggtcctg gatatggggg atggtaa 5307

<210> 18
<211> 3398
<212> DNA
<213> Lactobacillus sakei

<400> 18
atgacgactg aagtaaatgc tgttgaagcg cctgctaatg cgtcttcgga aacgatcact 60

aaaatagctc aggacgctat ttcaggttca acaccaacta aacaatcaaa tagtgtcatt 120

caaaaaaagg cggcttcaga tgcggaaaca caaaccataa cgccagcaac gtcaggtgaa 180

aaagcgacgc tacttgaagc accgcagact aaaaaactag ccaaccccaa aagcagtaac 240

ttgatcaacg agcaggcacc agtttgggca cagtcacctg gtatatcgat gatacaacgg 300
cgaccttaca tttatcaggc ggtgtattgc cggataaagt ggctaatgat actactaata 360
caccttggta tttccctaac tcgccttata ttgcgcgaat cgcgcatatc agcattgatg 420
gtgaaattac agctaaagat gtctcctata tgttttgggg gttgaccaac ttaacaacgg 480
ttcaggggtt agctaatcta aaaggggcca ctaattttac gatgttattt gccagtgaca 540
gtgcgctgca atcagttgac gcgactaact tagatttttc aaaagtgaca gcaatgaatt 600
cgatgtttag tgattgtgcc aacctagtat ctgttggcga cacagcgaat tggcagcttg 660
ggcaagtcac cacaatggta agatgcttca gtggtgataa aaaattaagt cagttaaata 720
gtacgaattg ggacacttct aatattcaaa atatgaataa tacatttttc aattgtacgg 780
ccttaacgaa cttagatgtt tcaaaatggc agacggcaaa aatgactaat ctggggagta 840
ccttttcaca atcgggcatt actgttttag atgtttcaaa ttgggatacc agtcacgtta 900
ctaatttaag tagtactttt ctgaatacta gtattgccga attagatgtt tcgaattggg 960
atacaagcca agtgacgaca atggcttata ctttttcggg atgttcaaga cttgaaacct 1020
tagatgtctc aaaatggcag ctcggcaaaa atacatcgtt gtcttacacg ttcagcggtg 1080
atcaaagatt aacgcagtta gacgtttcta agtggcaaac cgctaatgtg acgaatatgg 1140
cttcaacatt ttccgggact gtcggtgtta agactttagc ggttaaagat tggcagacca 1200
ctaaagtgat aaccatggcg ggcatgtttg ccaagagtgg cgttgatcag ttagaaattg 1260
ctgattggga tacgagcaac gtccaaagta tgcgactcat gtttgatgcg acgaagttaa 1320
caacgcttga ttatcctgat tggaataccg ctagtgtaac cgatatgagt tatatgctgc 1380
gtgggatgac gaaattaacg gatgcttatt tcacgaattg ggatacgagt caggtaacga 1440
atatgggcgg catgtttttg aatgatcaac aattagcgca cctgcggttg ggatccaaat 1500
ttaaattctt aacgagtacc tcaactggac catcattagc agaacccagc actgaaacac 1560
cctattttgg gaagtggcaa cgacatgatg caaatgataa tcaagttggg aatacctata 1620
catcagcagc attaatggcc cagtacgatg ggacgactgt tccaactggt gattattatt 1680
gggccgtcgc aacaccccca acgattacca agttggtacg taatgtgaca gctgatggta 1740
ataatgcccc atttaagacg gcaacaacgg ctcaaaaggg tgacacagtt gattaccaag 1800
tcaatatcac ccagccaagt ggtcagcaat tagatcgggg tgcggtcttt gaagatgtac 1860
ttgattcaca tttgaaattt aataataata aggaactatc aatttcatat tctgatgaaa 1920
atggtgattt tcatggagaa cagctcatta aatttaacga tcaatggcag attgcactcg 1980
gcgaaaattt agcaatcgga caaaaagcac aagtcctaat taaggcgatt gtcaacgacg 2040
atagtgcgcc agaaatcgat aatcttttca aattggtttc tggaagttac ggtgccggta 2100
ccacttcaaa cactgctatc gtacacgtca agaaaccatt gacgttaacc gaagcgataa 2160
aaaatgaaac gacggatacg gattgggaca ccaaacagga tgtgagtccg ggtgatcgag 2220
tcggctttaa gctcgattat caaaacacga cgggcgccac tagcaaccag ataacgttca 2280
atgatccgtt ggtcactaat gaactgacgt atcaaacggg ttcactcaaa gtgacctatc 2340

aagatgggac gactgaaact gtcagtgatg cggcgcaaac gcaatttgca acgaccggaa 2400

aattaacgtt aaccaaatcg ttagcgaatt atgaagccgt taagctcagt ttttcagcaa 2460

cggttaatcc gttggttacc tcaggaacga ccttacacaa taaggcaaca ttgaccgcag 2520

ataacgtgag tcaaccagtt acctcgaata cagttgatat gaacgtggtc caatcagagc 2580

atcaattaac cattcggtat gttgatttag atgaggatct tagccagcct gctagtgcgg 2640

ggactcaaat tgccgcgccg attacggtgt ctggtaaaac tggcacagct ttatcaaccc 2700

ttttaccagg gcagcaagtc gcccctaagg tgattgaggg ttacacgatt tattcagtca 2760

gtgaagatcc agatttaaaa ccggagaatt ggcaaaaagc gtatcgcgat gatcctcaaa 2820

ttggcgacca agatcgaatc attacttacg gttataaaaa ggcaatgtta tcgattgatg 2880

cgcctagcag ttgggaattt ggtgattata ataataaacc catggaccgg acgtattatt 2940

taaatcataa tcagggcacg ccacaagcag tgacggtcac cgataattat ggcgttcaaa 3000

attggcaatt acaggttagc caagcaaaac cgtttgttga tagtgaacaa catgagttaa 3060

ccgatgcaaa atgggccttt tcaaatggca acgttaagac gttatcgaac accgatgtgg 3120

ggactgtcac taataatagt gagcacttca cattggcatc aggccagagt gccactttaa 3180

tgacgatgac taagagtggt catttccaat ccgattcacc ggataccagt gatcccgaaa 3240

atccctatac ccaagttgga caagggcagt gggcttatcg ctttggtgac gaaaaaagtg 3300

ccgattatag tattggttta aatgtaccgg caactactaa acgatatacc ggtcattatc 3360

gaacgaaatt gacgtggtca ctatcagttg gcccatag 3398

<210> 19
<211> 3912
<212> DNA
<213> Lactobacillus sakei

<220>
<221> allele
<222> (321)..(321)
<223> the sequence ACAAACAGC is inserted in some strains

<400> 19
atgttcagkt tgkygttryt agcagtaatg ggtatacaac trrttgttac taccggaacg 60

akycttgctg aaartagtat caaagtagat tcaratcttg crttaacatc ggatcaggaa 120

ttatctaatg aaaaacaaat tgtaattagr gygagcgatc agtcaaaagc gcaacgtgat 180

ttagsgytaa ctataccaga gggggccrca tttgatcgca caactagtaa tcaattaaac 240

aaaaaatcag cgtatacggc tayccttgat caaaaaaata attccaatca agttatttta 300

aaacaaacag cacaaacagc tgaaaatatt gagaaygatt yggtaaatag acaagcaaca 360

acgttcaaaa aagaatactt attagtyttt aaactaaaag atactrgtat atycgctaaa 420

caacagttga aagtgacaac aataatgaac ggsrttcrct atgaatctaa accactattg 480

ctgaaagakr tcgttggaac agatggtgtt caacgacaaa ctagtttgwc gaaggaggrt 540

rmacctcaaa taacctttaa tctaaaycaa acagaatata tcgttggaga taccatccag 600

ttaaaagttg atcttratgc tagygaaaty aatgarccty tctatattaa tcttggcaat 660
aaagcaatac taacgccgat aaaartatct agcgataacr ggrrtattaa ctttgaatca 720
caaaataatc agaatacagg gaagaacatt ttaaagattt ccagtaacga accgataaag 780
aaagatttta cgctactagt aaaagcgaca acagttggga aggtcgatat tackgcttct 840
accgaaaytg atcatttaat ttcgaagaca gctactgtta aagtgrttga taacccaaca 900
tcaaaagyat tagcaattaa tcccaatact tttgatgacg gtattcctaa ttgggttcaa 960
aagacaaaaa cagaggtcgg aagtaagkct tttcaargaa gtacaagtcg atttcttaat 1020
tataccttcg gtggtgtgaa tggyggaatt gaygcagaag cagattcaaa aatttctagc 1080
tacaatrttc agggtagcca gattaatatc tttattaaag ataacaataa aacaatcggc 1140
gcttttttta agggagaaga cactagtagt aaagcagtaa gaacatcaac cggttttggg 1200
attgttttcg aaccaaatga aagtaatgat ggacctaaac aagctatctt agctaacaag 1260
ttaacaaata aggcatattk tgtcggrcac gacagcgagg gtaaccttgt tagcaaaata 1320
atrgggcagt ttgaacgtaa tgggaagaca ttaattgctg aaattctatt gcggccttca 1380
ctatcaggta ctactgcggt tcaacaagag ctttatttga aaaatgacac aactcaatcc 1440
gtttcttatg ggacctttat tggtcaagat acaatgttaa acggtaatga taaagtaccg 1500
atgtcatcaa tgggcaataa cgcaggatta tatatcactc aaaatcccta taaattggcc 1560
atcaatatga aggttcctga tggaccaatt aattatgcgg cacaaacttg gactaggaga 1620
aatccttggt ttgatgggtt cacaccacgt aatttttctg gaacgggctt ggaacaaaag 1680
aatttagaag agggttacac agttttaaaa aattccgata cttcatatac agccaagtgg 1740
ccgtttgcta ccttggcccc tggtgaatca caacattatc gtcaagatat agggattact 1800
aaagcccctg acgttgcacc agaagcttat aaagaatatc aaaatgaaac tagtactgat 1860
gggagtaata gaccaggcga taatataaag tttacattac gtgctcataa ttcgggattg 1920
gattcttctt ggagtgatgt ttcgtttagt gatattattc cgtccgaatt tcaaattaat 1980
acaaattcaa taaggttaat aaatmaaagc ggtcaagaaa taaccatttc accgtcagct 2040
tacaatgaag taactcgaga gttgaaagtt acggttccta acagtgttaa agataaccag 2100
tgggttagtg tcacatttga agcgaaggct ttaactacag cttctgggcg aactgttcgt 2160
aacactgtca atgttgttgg tatagacagt aatgttaatc aacaagaacg ttcagcaagt 2220
gcgatagtcg atgtaccgtt tatcaaagta caattaccag aattaacgaa aaaagtaaaa 2280
aatatttcta gattagatgc gaagtatgct actgaaacag aagcaagtat tggcgatgaa 2340
attggttata aattagtttt cactaacact aattctgagg aagtaccgga ggccattatt 2400
gaagatccgc tcgattctga tctagatgaa ccaagcgctg ttcatgttag ttataaagat 2460
tcaaacggga gtgaggtgag aagcgaagac ttaaccttta ctggtaatca attaatttta 2520
aaagctatcc cagcggatgg aagtgtggtg ctaacttttg agactattct caaagacact 2580
aaaaagaccg ttattaataa tatagctatg gttgctgatg ttaaatcaaa tgtcgctaaa 2640

gtcaatgtta tcaaaaaaac accaccaacc ctaattaagg aagttaaaaa tataacgaag 2700

tccgacgcag attatatgac agaaacaacg gctgaagttg acgatgagat tgaatatcgg 2760

atacaaattg ttaacactgc ctcaggcgat attaaaccgg gtgcaatatt aaaggatgtt 2820

tttgatgctg acttaggtga tattcaacaa gttagaattg attatttgga caaagatgaa 2880

aaaattattg cgaagcaaac aactgattgg actgataacc aggttatctt ggatcatggt 2940

attccgattg cgggtcaagg tatggcgatt gcttatgtca aagccaaagt aaaagagact 3000

aataaatcag tgattaataa tagtgttagt ctggacaccg attttggagc tggggcttct 3060

gaaaaagcca aattaaatat taaacagtct aaaggccgga ttgttgttcg ctatcgggac 3120

cgaaaagatg aaagccacaa gttggccgaa gatgagacct ttgatggcaa aattgggaac 3180

actagactag tgcaacccaa agttattcca gaaacagacg gcaattggac cgtggttgat 3240

tcttctaata tggtagatcc agattggggc tcaaccacga aacctgattg gacattggct 3300

catgatcata ctgtcactta cgccaaagat gaacaagtca ttacttatcg atatgaagaa 3360

tcccacattg gcattattgc cgataaacgc tgggattttg gtaaacatga tacgactggc 3420

actgaccgaa actattatct aaaagctaaa actaaagaca atcaaaaaca gccatacgcc 3480

gtcagcgttg aggattacta cacctctaag ggttggactt taaatgttaa acaagatgat 3540

cagttccata ccaatgctaa tgaaaagatt gctggagatc aaaaattctt agataatgcc 3600

gtcttaaatt tccataatgg gcagattgtc ttaaaagaaa gtgatgacgt aggggcaacc 3660

gccccagttt ctaaggtaac ttctgagttt gagttaacac caaaaggcgc ggcagttaat 3720

ttaatgacgc acaccaataa gacacctaat cctggttatt atgccgcaca tggctttggg 3780

atttgggctt atcaatttgg ggatgcccaa caggctgatt acagcattgg tttgaaagtg 3840

cctaaagcaa caaaacgatt cccaagacaa tatacaagcc aattaacttg gtcattagta 3900

atcgcagaat aa 3912

<210> 20
<211> 918
<212> DNA
<213> Lactobacillus sakei

<400> 20
atgaatacaa ttttcggtat cattatgacg ttttcgtttt ttgccattcc ctactatggc 60

attcggcgaa caatattaaa aaagaataaa cctgagtttt actcaagaat caaaaaaagg 120

atttggtacg ctactgcagt atttgttgtc agtgtcattg gggtcctggc aacacaatca 180

ccaaccaaaa tgaccgctca tagtcaccat cctgaaatag ccaaatctga aaaatcagat 240

gcggccaaaa agttagacga cgccaaatta aatgtcgatg gactattctc ggatgcaaaa 300

cacaccaaat tagttcacgg tatatcccac aaagatatta agagcgtttc tgaagatgtc 360

gacgccttaa aagattcaaa aactaaagat acgctcaaaa aagatatttt aaaagctgaa 420

aaattatgga ctgttctcaa gaaaaataaa gcgcacgaat cagccgtcgc ctcttctaaa 480

ttagcaagcg aaagtcatac aaaagcgatt gaatcaagta aagaagcgtc aaacaaagct 540

gttgaatcaa gtaaagctga ggaatcaaaa aaagcatcag aatcagtggc tgctagtgaa 600

tcagctgcaa aagctagttc agaatcagca caacggattg ctgattctaa ctcagtcgcc 660

gcaagcaact cagccgcaca agcagcttcg gaaagcaccc gacaagcaca ggaaaactct 720

gtttcacaag ccaatgctca agcggctgca tcccaggcag ctgccacaac tacttataat 780

gcaccaacaa ggaccaatag tggcagacgc tgggctattc aagacggata cgattgggcc 840

acacgaaagg gtcactctcg tatattaaat gctggtgagc aattaccagc aggatatcat 900

tggcaaacag gaaattaa 918

<210> 21
<211> 5938
<212> DNA
<213> Lactobacillus sakei

<220>
<221> allele
<222> (2400)..(2402)
<223> the nucleotides are deleted in some strains

<400> 21
tacaagcgct tgaatggatt gtaatyggya acggtgtygg ttttggcaaa caccctggcg 60

acttggtcga tgaatcaaag attgaacgcc gttttatcgc aaccggttca aaggggttcg 120

atgcaaccca rgttcaggca ctaacagaaa tcaatgcaaa aatgctaacc attatgtccg 180

aaatcgttac gttagttgag ccagcattag atactaagat taacgatcgc caatatctga 240

ttctagcgga ccatctccaa ttcgccgtta aacgcatcaa cgcccatgtc acgttaaacg 300

atcaatcatt actctgggaa ttacgtaatt tatttccaac tgaatataaa actgcgcagc 360

agactattca actgatcaat caaaaattag rcttcaaact accygatggt gaagcagtgc 420

cyctcactta tcattttgtr aatatcaaaa ayggcgacag tgaaatcgaa gayacgatga 480

ccatggccaa gttggtcgct aacatcattr gtatcgtcca aaaraactac ggcattgtgy 540

tagaygaaga gtcgttcaat tacagtcgyt tcatctccca tcttcgctac ttcatcattc 600

grcgtctcaa aaagacgcaa gaaacagaca cacaacttga yccsgcactr cttgcyatga 660

tgatcacacg ttatccacar gcgtatcaag ttgtccaaca aatyacggcm tttttraaac 720

ttgagatrga ttggrcctta caatcygacg aagaagttta cttattattg catatctggc 780

gtgtcaccca gcatcaacaa actgaataaa atggtgtgtt actgattcga tcaggcatta 840

acccagttaa cgaatgcgac ctatttttag gctgtattca ttagctgggt ttttgtttta 900

acaattatta atctaaggag gtactatcat ggcttacgaa caacttgcaa cagacatctt 960

ggcaaacgtg ggcggacccg aaaatatccg cagcgtcgtc cactgtacga cgcgcttgcg 1020

cttcaaattg aaggacgaaa aaaaggcgca cactgatatt ytaaaaaata tggacggcgt 1080

tgtgacggtt gtccaaagtg gtggtcaata ccaagtcgtt atcggtaatc aagtcgctga 1140

cgtttatgac gcacttttga aaatcggyaa tatyrcyggt gaaggcatcg tccctgatga 1200

tgacgaccaa caacctaara tgagtttact cgataaattc atcgatttaa tttctggcat 1260

cttcgcacca acscttggyg tcttagcsgc aacygggatg attaaaggrc tcgtcgcsct 1320
ctttgtcgya gtsggytggc tcacsayyaa atctggcact tatcaaatty tattygcrct 1380
cggygattca ttcttctatt tctttccaat tatgttaggc tacagygcya gcaagaartt 1440
caargtcaat ccgttyctcg gtatggygat tggsgcctcc ctcrtttacc crtcaatcgt 1500
gggmykagtc ccttcagcgc tggcgcaaac wggcgctaaa ccactttaca ctttgtttgc 1560
cggtacgcta ttygartcac caatytacac aacattccty ggtattccag trattatgat 1620
gagttacgcg tcatccgtca tcccaatcgt ggttgcygtt tggttcacct caaaagtyga 1680
acccttcttc aaaaaagtct tacccgaagt gattcggaca ttcttagtac cgttttgtac 1740
ggccctcgtc gttgtgccgg tgaccttctt agtgattggt ccactgtcaa cttggcttgg 1800
tgatctatta ggcgctggta gtaatgcgct ctatcaattc agtccagtag ttactggggc 1860
agtgcttggt ggcctctggc aagtgctcgt tatcttcggc cttcattggg ggattgtccc 1920
actggcaatt ctcaayattt ccacaytrgg ttacgacacg atyttaacrc ttgtctttgc 1980
cgcatccttt gcacaaatcg gatctgtttt agccatcaca ttacgaacga aaaataaaaa 2040
gctcaaaggc ttaggcyggt catcyttcat ctctgggatt ttyggtgtca cagaacccgy 2100
aatttacggg attacactgc crcgtaaaaa gccattcatc ttcagttgta tcggtggtgc 2160
agtcggygga ttgctactcg gcttattygg cactcgcggy tacctcgtgg gtggactygg 2220
tgtcttcggt attccgaact ttatcaatcc aaagaccggt ctcgatatga ccgtttaygg 2280
tgcgytaatt gctgccrctg ttgccttcgt aattgcatta ttattaacac ttttcttcgg 2340
catttctaam gaagatcaag tggcaacsgt tgaacccgcw rctcctatgr caccwactcc 2400
tccgrcagaa rcscctgcca aagtaacrcc ygaaatcatt actagtcccc ttagyggcac 2460
cstaryacct ctttcagaac twaaagatcc tgttttctca tcagcwgtca tgggyaaagg 2520
rgtggcaatc aytccttctg atggccaact tttcgcaccc gttgccggaa cagtcagyct 2580
cgtctttcca actggacacg caattggtat taattctgaa cacggtgctg aaattttaat 2640
gcatgtcggg atggataccg tccaacttga gggcaaacca tttacggtac atrtgaaaca 2700
gggkcaagca gtcctccccg gtgatttatt attaaccttc gatattgaag ccattcaagc 2760
ggccggttac gaaatcgcga caccggtcat cattactaat agtaatttgt acgattcagt 2820
ggcacctctg atttcaaccg gaactgtgtc tactggcgat caactcttaa ctttaaaata 2880
acattcaaag gagactaaag atgactaatc acaccccttt cccaaaagat tttctctggg 2940
gcggcgcaac tgccgctaac caatatgaag gtgcttacca aaccgacggc aaaggtttgt 3000
cgctcgttga tattttgcca gatgctcacc atggccgctg ggaagcccta atgaatccta 3060
aaaaagcgca agcgactcaa tacgacttct atccaagtca caaatccgtt gatttctatc 3120
atcattatga agaagacctg cgcttgrtgg ctgagatggg ctttaaaacc tatcggctat 3180
caattagctg gcctcgtatt ttcccaaatg gtgatgattt aacacctaac gaagccggtc 3240
tcgcctttta cgatcgcgtg attgatctgt gtcgcgagct caacattgaa ccactcgtga 3300
cyatcaacca tttcgatacy ccactagcga tgatggataa gtgtaacggc tgggccratc 3360

gccrgatgat cgaccactay ytgcgctttt gttcggtgct ttttaaacgc tatagaggta 3420
aagttcacta ttggttaacg tttaacgaaa tcaacatgat tctccacatt ccgttcttag 3480
gcggtggcat ggacrtttcc gatrcygctc atccagatca aattaaatac caagcttccc 3540
atcaccaatt agtggcttca gcgttagcaa ccaagcttgc acatgaaatc gatccagata 3600
atcaaattgg ctgcatgcta gccgctggcg aaacttatcc gaatacgcca aatccagcgg 3660
atgtcttcaa agcacttgaa accaatcgcg aaggctactt cttcatcgat gtgcaatcac 3720
gtrgtgctta tccgaactat gccaagcggt tatttaaagc caaaaatatc gtattagcaa 3780
tgggacctga tgatgaggcg attctcaaag ccaatacagt ygacttcatt tcatttagyt 3840
attactcatc tcgtttagtt agtgctgatc cagyggtgaa tgaacaaact gaaggtaacg 3900
tcttcgcctc gcttaaaaat ccatatctca aaacaagcga atggggctgg caaactgacg 3960
gtcttggctt acggacgaca atgaatcaac tctatgatcg atatcaaaaa ccactgttya 4020
tcgttgaaaa cggcctaggt gcacgtgata cagtcactgc tgatggycaa rttcacgacg 4080
actaccgtat cagttatctg caagaaaata tcaccgctat gggccaagca attgccgacg 4140
gcgtaccytg tatcggttac acttcctggg gctgtatcga tttagtcagc gccggttcag 4200
gtgaaatgga caagcgttac ggttacgtat acgttgaccg cgacaatgat ggtaacggca 4260
cattagcccg tacaccaaag gattctttct actggtatca aaatgtcatt aaaaccaayg 4320
gtagcgtgtt atataacggc caaacatttt aatttttgga gggacaatta tgtattcaaa 4380
acaaccaatt accggcttta aaccagattt cttatggggt ggtgcaaccg cggctaatca 4440
aattgaaggt gcctggaata tcgacggcaa ggggctcacg accgccgaag tcgtccaaaa 4500
agcgaccgat cgccagaatt tttcaatgaa caccgtgact aaagacagca ttcaagtcgc 4560
aattgacgat ccaaccgata csctttatcc aaaacggcrc ggaatcgatt tttaycacca 4620
ctacaaagaa gacatcgcat tattcgccga aatgggcttc aaggctttyc grrtragtat 4680
cgcrtgggct cgtatttttc caaacggcga tgacgcgcam cccaatgaaa taggcttagc 4740
cttctacgat aacgtcttcg ctgaactcaa aaaatacaat atcgagccag tcgtgaccct 4800
ctcccattay gaaatgccgc ttgcactgac actgaaatat aacggttggr ccagtcgcga 4860
aaccatcact gcgtttaacc gytataccga aacggtcttc aaacgttatc aacatcaagt 4920
taaatactgg atgactttta acgaaattaa tgccgcaact tggggattca ctggtaccgg 4980
cgcggttgac gataccctct cyccaaatga gcaattacaa ctgcgctatc aagcgctgca 5040
tcatcaattt gttgcgagya gyatcgccgt taaacaatgt caygaattra tgccmraygc 5100
saaaattggt tcaatgttgg cgcggatgca aagttatcct aaaacaycga acccmrycga 5160
tgtccgtcaa gcgcaagaag atgatgaatt gaatctcttt ttcacagatg ttcaggtccg 5220
tggcgaatat cccaactata tgaaccgtta cttcaacgac cacgrcatcg aacttgaaat 5280
ggcacctgac gacttacaaw taatcaaaga ttatccggtc gattatttaa gcttcagcta 5340
ttacatgtca atggtcagct cagctaracc cgccggtgaa aaaacagctg gcaacttaat 5400

yctcggtgaa aaraatccgt atctcgaatc aagcgactgg ggttggcaaa ttgatcccgt 5460

cggtttacgg attaccctca agaatctctg ggaacgctat ggtgtaccac tattcatcgt 5520

cgaaaatggc ttaggcgcaa tcgataaggt tgcagctgac ggtcaaattc atgaccctta 5580

tcgtattgac tacatgcgca aacacatcgc acagatgaaa gaggccgttc aagacggcgt 5640

ggatctaatg ggctacacca tgtggggmcc aatcgactta attagtgcct ctacctctga 5700

aatgtcmaar cgctacggct tyatytacgt cgaccaagat gatgacggya acggtacgct 5760

cgaacgrmgc cgtaaagact cgttctactg gttcaaaaay gtyattgcya rcaatggyga 5820

agayctctar aacatagaaa aagcccccga cttttrtcmg gggctttttc ttatactgta 5880

aaaataaatc grtcgaatgc ttgcgyaatc ccawcakaat ygttatcggc rgtaataa 5938

<210> 22
<211> 1327
<212> DNA
<213> Lactobacillus sakei

<400> 22
taacgttggg atcttagcgt ttaattgacc aagaccaccg ttcaatgttt cagaacctgt 60

tgccaatcga ctaacgccac ttgttaattg tgggacttgg ctattcaatt gaccagtacc 120

agttgctaat tgttttgaac cagcagctaa ttgaccaaca ccgcttgtca attgtggcac 180

ttgaccattt aattgtgttg taccagcata caaggctttt gaacctgttg ctaattgacc 240

aacaccgctt gtcaattgtg gcacttgacc gtttagttgt gctgtaccag catataaagc 300

ttttgaacct gttgctaatt gaccaatacc acctgtcaat tgtgggactt gaccgtttaa 360

ttggctagta ccagcatata aagcttttga gcctgttgct aattgactaa taccacctgt 420

taaggctggg atttgaccat ttaattggtt tgttccggca gctaattgtg taccacctgc 480

aactaattca ccgttcttac ttactaatgc ttcagaacca ttagcagcag ttgcgacacc 540

tgttgtatat ttacctaaac cagcgatcac tgcttgtgac cctgtttcta attgactaat 600

accagcatct aacttagctg caccagatgc taaaccagga ttgccctgtg catcatcgtt 660

caagccagtt tgaattgctt taagactacc taacattgaa acagaacctt taacagctgc 720

tgttaactgt gcgtattgtg cagctgttgg tttcttagca tcaattttag cattgatacc 780

agaaacaatt tgttgtgctt catccttagc tgaagcggag agttgtgcac tcaacttagc 840

tttaatgcct gcttcttgtt gtgcactgaa gacaatacct gcttgttttt cagcatcaat 900

aattgcttgt gcttttgcat tgaaatcagt tgttgcaaca acttgatcag gaatcgcatt 960

taaatctgtt tgtaatgcag gaatcgtact ttcaacttga gcaatcatac catttaatgc 1020

tgtatattgc gcaacggctg cattataact tgcatcatct ttgaatgttg ggttgctgtt 1080

aagttgtgat tgcatctttt gtaacccagc agaaatcttt tgactacctg cttttaagtc 1140

tttaacacca gatgctagac ttgttgcacc gttatttaaa tctttgttgt ttttagcaag 1200

ttcttgtaaa ccatcattta gactacttgt gccatcagta taagctgtta cacccttact 1260

taagctagca gcaccattat ttaaatcttt tgcacctgag gctaaatcgc ctgttttacc 1320

atttaat                                                              1327

<210>  23
<211>  500
<212>  DNA
<213>  Lactobacillus sakei

<400>  23
ttaataacaa taaattttaa agtatagtca aggaggcgat tagaatgtta caaccaacaa      60

ttcagctatc ggcacaagct tatgtgatcc aatggaatcg gtttttagag cggtttattg     120

tcttcgaaac ggtttttagt gaatatgcgc aaactagtaa aaatgttatg cggggtaaag     180

gattggtaga tgaaaattta caattactta atcactattc cagacagaat ttaactgtcc     240

tcgatcaatt tgttgatcaa attgcgcgta ttcaaccgcc aacggaattt gtcgcattac     300

atgagcaatt acagatggtt attaagcatt atgtgactgc tgcaaaggat tttgtcaatg     360

cgatgcgatt gactgaagag tcggaagtgg aacaacaatt aaatcaatgt cgaacgattc     420

aaaaacagca acgactaaaa ttaaatcaac tcttaaatag cattggtgca ctaaacagtt     480

agtggcgtca tcaaaaagga                                                 500

<210>  24
<211>  141
<212>  DNA
<213>  Lactobacillus sakei

<400>  24
attgaatacc acgttggtca accggaacca caaggaaata atgcgagctt accaggtggt      60

ggtattatac ttaaacagta ttacggtgca caaggaataa agatttttaa actaacgcct     120

aaaccaggaa acaaggtcaa t                                               141

<210>  25
<211>  1476
<212>  DNA
<213>  Lactobacillus sakei

<400>  25
atgaaggtta atgagttatt taaaaagcca atagatcgtg agattcaagg agtcgtaaaa      60

attggccaag atcaagaatc aaatacatat caggaattag atgaatatgt gattactagc     120

gaaatacgta agaatatggg aacttttttt gaggcgtacg taaagtcgtt agagcaacca     180

actgatcaaa tgggtgtctg gatatctgga ttctttggtt ctggtaaatc tcatttctta     240

aagatattaa gctacctaat tgatagtcaa aagacagtta aaggacgacg acctatatcg     300

tttatcgaag ataagtttga agaaaatgat gaaacattag cgctaataaa acgcgctagt     360

gaacaaccta atcaggttgc attatttaat attgaatcta aggctgaggc tgacagtatg     420

aatagtaaat cggctgttgt taaagtcttt aataaagttt tgaatgaatt acgaggcttc     480

tcaggtgcta atgcatggat tgcggagatg gaagagacac ttgccaataa cggacagtat     540

gaacagttta aaactgcttt tgagcgtatt gctgacttag attgggtgga aggccgggag     600

gaaatttttt ataatgagga ctcggtaatt gaggccttaa gtgaagtaac agaaatgacg     660

attgagagcg cacgacactt catagaatct ggggaagcta attatgaaat tagtgatgag 720

tcatttgcta aaaaagtgaa gcgatacgtt gatcaacagc cagataatta tcatttagtt 780

tttttagcag atgagatggg gcaatatatt gctgataatg gtcaattaat gttggattta 840

cagatggttg ttgaagatct agcgcaatat tgtcaaggga aggtctgggt agttgtaaca 900

agtcagcagg atattgattc actaaatcgt ggtggtatgt cctctaatga tttttctaag 960

attcaggggc gttttaacac gcgattgcca ttgagttctg ctaatgcgga tgaagttatt 1020

aagaagcgat tattagcgaa gacagaagaa gccagcgcga tattaacaac tgaatataca 1080

gaaaacgaag ctgttctaaa aaataaaatt ttatttacta atgatacggc agaaatgaaa 1140

tcttttgata atgtagatga atttattgat acttatcctt ttgtgcctta tcagtttaat 1200

ctattacaaa aagtatttat gggaattcga ggacatggtt tcgctgggaa acatttggca 1260

aatggtgagc gtagtctgct atctgcagca cagtatgcgg cacaaatgtt tagtgaacaa 1320

tcgaccgatt ttttggtacc attctcggta ttctatgaaa atattgaaaa tgcactagaa 1380

tattctgtgc aaacacctat tattcaggct aaaagtaata aacggttgga taaatttgat 1440

gtcgaagttc tgaagttatt gttcttaata cgctat 1476

<210> 26
<211> 1840
<212> DNA
<213> Lactobacillus sakei

<400> 26
ttatggccaa tttgctcgta ttgtttctag aatggaggcg atttggcgtt tattatgggt 60

taaatagttt gcgtaaattg gcatcttggc ctgttcatca ctaattggra ccgtrattct 120

rtctaattga tgtggtgata rttcgttgaa gaaagcatcc ttttgtgaga grttggaagt 180

gaaaaayggt aggcttgart gyttratrat ctcaatgaag gcggcgcggt cgtcttgata 240

cataaattgy gcgttgggaa tatcttgttt aatcatatcg ctccaggggc cgattgcgcg 300

catgacgacg aaactcatgc cagctaaatc ttgaaarcta acsgttgatt ggctagcaaa 360

cgtcgtgaat tgatcgagat taatttgrag ctgttcytga ccgaggtatt grgattcgat 420

ggyygtatca aggatttctt gatctgrara gattagggca acttggtctg tctttagtgc 480

attactaatt tgatccaccg cratgaattc tttggtgatt tgcacatcat cacggttagg 540

cacgagggtt agcggtccgg gaatcgttgc gccaattatc gtaaagtgyt gaccgcgrtc 600

gaaattttga attttagtca gcatcgtttg rctagyygtc aaaagttgtt cagcttctcg 660

agcsgcaagc tcgccwgttg gtgtcagttt aatgcgatta gcytgacggt caaacagctg 720

gacaccaaaa tcctcttcca acttttgcat gccgcgtgtg acggatggct gggtgatgtt 780

caaagcagca gcagttttcg tgagggkgcc agtcttggcg aaggtcgtca attgttctaa 840

taaatattaa tctaacattc gctgttctcc ttagtatacg tttaatgtat actagcatgt 900

tttttaagta attttcaagt gagccactgc gcactatact aaagtcatca attagaaaaa 960

raggttatca acgatggata tggtgcaatt aaacaatggc gtacaaatgc cacaattagg 1020

ttttggtgtt ttccaaatga cggatttagc agaatgtgaa caagcagtgg ttgatgcrat 1080

tgcatcaggg taccgtttga ttgatacagc ggcagcttac cataatgaag ccgcagttgg 1140

ccgggccatt aaacgyagcg gcgtggcgcg cgaagawyta ttcattacyt ckaaactctg 1200

ggtatcagat gcsagytatg aacgtgctaa aaaagggatt gaccaatcac tccaaaaywt 1260

aggsgttgat tatttagayc tytacttact tcaccaaccw ttyggcgatg tgattggggc 1320

atggcgggct ctagaagaag catacaaagc cggcaagatt cgcgcaatcg gcgtttctaa 1380

tttctatgcc gatcaactta aaaatttaat yttatcaacc ratgtcaaac cagtgattaa 1440

ccaaattgaa gttaaycctt ggtatcaaga acaytcagaa gtkgcttttg cacaagcgga 1500

agagattcaa gttgaagctt gggcaccatt tgcagaaggc aaayacaata tytttactga 1560

tgaacgaatt gcsgcaattg gyrrkcgtta yggtaaatca aacggacaag tsatctyaag 1620

atggttactt caacgcggca ttgtggtgat tccgaagtct gtgcaccaaa atcggatggc 1680

cgaaaatatc aatgtctttg attttgaatt aaccacagcy gaaatggcaa cgatgaatga 1740

tttggacaaa ggcgaaagyc aattyttcga tcaycgtgat ccagcrgcaa tygaacaaat 1800

ttttgggacg agcttagcgg ctttaaagga gaataaataa 1840

<210> 27
<211> 866
<212> DNA
<213> Lactobacillus sakei

<400> 27
atgaaagcaa tcgttgtctc aaaggctggt gggccagaag tccttacata taccgacgtt 60

ccaaaaccaa caattaagtc aggctggtca ctcgttaaag tgatgggttt tggcattaac 120

cgctccgaaa ttttcacgcg ggaaggcaag tctccctcgg ttcaattccc cagaatcctc 180

ggcattgagg ctgttgggat gattgctgaa tcttccgatc cggaacaact gccagttggt 240

caaaaagtga tttcaattat gggcgagatg ggccgcgcct ttgacggtag ctatgctgaa 300

tacgtcttac tacccaatga acaaatctat ccggttgaat cgactttaag ctgggctaat 360

ctcgccgcga ttcccgaaac cttctataca gccttcggaa ttttcaaaag tttacaaatt 420

actaaaagcg accatgtttt agtcagagca gccactagcg gtgtcggtat tgcagtgatg 480

aaattaatca aaggatatgc tgccagtatc tccgtaactg ggaccacgcg ctctgcaaat 540

aaatctgatc aattattagc agccggcttt gatgatgtac tcgtcacacc cgcacccaac 600

accctcccaa gtgagtccgg ccaattcgat aaaatcgctg accttattgg acccgcagct 660

actcgtgact cattacgaca tctaaatgag tatgggattt taagtgccac cggcgaactt 720

ggtggcgtct ggacgattga tgccttagac cctattatcg atattccgaa taatcgttac 780

ctaactggct tttattccgg cgatgtttct agcagcaaaa ttcaggaaat gttagccttt 840

atcgagaaat atcatattga tgtgac 866

<210> 28
<211> 1587
<212> DNA

<213>  Lactobacillus sakei

<400>  28
atgtgcggat ttttagctgt tgattctaag gaatttgatt taacgacctt ttgcgacgcc      60

ctggagaaaa acgttgaccg cggcccggac atgaccgaaa ccgttgaaga agacgcggtg     120

atgtttggtt tcaaccgcct ggcgatcatg gacctgtccg atgacgggat gcaacccttc     180

aagggcgaag attgcaccct ggtctgcaac ggggaaatct ataacttctt acagctcaag     240

gagaacgtaa aagacgactt cagcttccaa agctctagtg actgcgaagt tttgatcccg     300

ctctaccgta agtacgggct ggacaccatg tgcaagatgc tcgacgccga gttcgccttc     360

gtcttatacg acaaggtggc caagaaggtc gtggccggac gtgacccgat cgggatccgg     420

ccaatgtttt acggctacac caaggagaag ggcgaaatcg cctttggttc cacggccaag     480

accctgatgg acttgtgcga ccaaatcttc ccgttcccac cgggccacta ctacgacggc     540

gaaaagttcg ttacctaccg cgacccggcc atggttaccc ggatgcacac gccgagcttt     600

gaagaagcca cgaccggcat ccgtgattac ctggtcaagg gggtggaaaa gcggctccac     660

gctgacgctc cggtgggcta cctgttgtcc gggggcctgg attcttccct ggtttgctcg     720

atcgcatcta agttaatgcc gggcaagaag ttacggacct ttgcgatcgg gatggaccgc     780

aacccgatcg atctcaagta cgcccgcgaa gttgccgact acttaggcac tgaccacacc     840

gaattcatca tgactcgcga cgatgtttta ggggcgctgc gtaaggttat ttataccctg     900

gaaacctggg acattacgac catccgggcc tcgatcggaa tgtacctatt gtgcaagaag     960

atccacgaaa cgaccgacct gaaggtgatc ctgaccgggg aatgttccga cgaaattttt    1020

ggctacaggt acaccgacta cgcgccaaac gccgaggcct tccaaagcga atcgatgaag    1080

cgggttcgtg aactgtacat gtacgacgtg ctgcgggccg accgttgcat ctcggctaac    1140

tcccttgaag gccgggtgcc gtttgccgac ctcgacttcg tggaatacgt gatgtcctta    1200

gacccggaca tgaagatgaa ccactacgac aagggtaagt acctgttgcg gatggccttt    1260

gccggacaag actacttgcc agacgacatc ttgatgcggg aaaaggcggc cttctccgat    1320

gccgttggtc actccctggt cgacgactta aaggaatacg ccgacgccaa gtacacggat    1380

gaagacgtgg ccaaggccag cgaaaagtac gcctacaaga cgccgtttac caaggaatcc    1440

ttgctctacc gcgacatctt tgaagaattc ttcccgggca aggccgaatg gatcaaggac    1500

tactggatgc caaacagcga ctgggaaggt tgcgacgttg acgacccatc cgctcgcgtt    1560

ctctccaact acggggactc gggcaag                                        1587


<210>  29
<211>  2462
<212>  DNA
<213>  Lactobacillus sakei

<400>  29
acggcatggt ctatgtcaac cgcacgaatc acgatttgaa ggacatgcaa cgtgtgccaa      60

agaagagttt ttattggttt aaagacttga ttgctaagta tcagtgttaa atgtttttta     120

aaaggagtgg ggttttcgcc actctttttt atttacctat ttcatttcaa acttattctt     180

gttataaccg ataccagttg tccgtgttat aatacagcgg actaaatgtt ataacagagg 240
agagtacaga tgagtattgg cgatatttta acgaagacgt ttacgaacat ggatattgtg 300
agttcgatca cgtcgacagt gtttattatt ttattaggat tctactgccg caaacgcgag 360
atcttcacgg ataaatttgg gaagattctg tcaaaagtcg ttttaacggt tgctttacca 420
gctttggcat tcaactcatt catgcaagat attaatggga agactttaaa agaaggtatc 480
aacgttttag tttggggctt cttgatctat attattttaa ttttaatttc aaaaccatta 540
tacttacgct ttaagggtga tcaacaagat acattgcgcg tgttgaccat ctttggttca 600
acaacgttct tcgggattcc aattgttgga gcagttttcg gagctaaagg ggttatgtat 660
agctcaattt tcaacattgg ttatcgcgtg ttcttatact catatgggta catcaagatg 720
agtggcttga agatggaatc gaagaacttg aaaaacatgt tcttaaatcc aatcgtgatc 780
gccacatttg cggggttaat catctggtta atccaaggcg cattaccaca agtggcggtt 840
gctgggcatt cagtggcctt cttacgaatt gatcaaacag cgccatggtt gttcaaacca 900
atgacgtact tagctggttt atgttcacca ttagcttggt tagcaattgg gacaacgctt 960
ggtgaaatca gtttcaaaga agctgcttcg gacaagacat cgtggattta tagtattaac 1020
aaagtgattt tagtacctgc tttaaacatc gtgttattgg ccatcttaac aatgacccac 1080
attttaccag ttagctatat cagtttggca acgattgtca tcatgatggc aacgccaacg 1140
gcaacagtcg ccgctgctta cgcgattagt tttgacaagg atgcggtctt agcatcgaac 1200
gcgtcattac tatcaacggt cttggctgtt gtgatgacgc cagtctggtt agtaatttta 1260
gaagtaatca accaaatcgg tattttctag gaagagggaa attaaatgtt taaaattgct 1320
tgttacggcg tacgccccaa tgaagtggcg acgtttaatg aatataataa ctatgattat 1380
gatttaacct taattgaaga actattaaca cacgctaata tcgaaacagc taaaggccat 1440
gacgcggtat tgttacgcgc taactgtgcc gctgatgcgc aaaatatggc gaagttgaat 1500
gaatatggca tccattatgt ctttacgcgg acagtcggtt acgggcatat cgatttagcc 1560
gcagcaggca agtacaacat ggaagttgct cgcgtaccag cctactcacc aaacgcgatt 1620
gctgaattag ctttgacgtt agggatgtca ttattacgcc acaccactta cacgacgaat 1680
cggacagcac atcatgattt tcgtgtgagc ccagtgatgt ttagtaaaga agtccgcaac 1740
tgtacggttg ggattatcgg tactggtaag attggctata ccgaagcaaa gctatacaaa 1800
ggcttaggtg ccaaggtagt ggggtatgac ttgtacccat ccgaaattgc taaggaagtc 1860
cttgattttg ttagcttaga tgaattattg gcgcaaagtg atattgtcag cttgcatatt 1920
ccatactttg ctggtcaaaa cgatcaattt atcaatgctg atttaatcgc gaagatgaag 1980
aatgatgcca tcttggtcaa tacagctcgg ggtcaattgc aagataaccg tgcgattatc 2040
gatgctttga aagctgatca acttggcgca tttgccgctg acgtattacc agctgaaggc 2100
gaaatcttct tcaaacaatt cccagcagac caaccagtcc ctgatgcgac aacccaagaa 2160
ttaattgatt tatacccacg cgtcttgttg acaccacacg ttggttcgaa tactgacgaa 2220

gccctcaaga acatggtcga aattagcttt gaaaacttcc atgaaatctt gacgacaggt 2280

aagacgcaaa atagcgtcgc aaccactgaa aattaaagcg gttatttgta ttttcatgaa 2340

aattatggta tattgttatc agtaattaga tacttcagag cgagtgagca gaaatgctca 2400

ctccttttat tggagcacgt ctaatctgaa tatataaatg aattggaggg aatcatttga 2460

gt 2462

<210> 30
<211> 930
<212> DNA
<213> Lactobacillus sakei

<220>
<221> allele
<222> (535)..(535)
<223> T is inserted in some strains

<400> 30
accacttata acgatgcctt atattgcccg tgttttaggc ccaacgggtg ttgggattaa 60

ctcatttacc aattcaaata cacagtattt tattttaryw ggtagtatyg gdgtttcayt 120

rtatggtaat cgycarattg cwtattaycg mgatrataaa rcsaaarctt ctcaaatttt 180

ttgggaagtt tttctaatgm gaatgattac sattytwgyw kcaytadywg cwttytttrt 240

bttyttaggg ttygaraaak mhtrtcayca agcwtatctv atgcaakcwa tyttaatyrt 300

tgckgcwgcw tttgayatyt catggttytt tatgggbttt garaayttya argtaackgt 360

wytwagaaay atkatyrtta arttratatc rttrgcwtgt attttyatwt ttgtbcgwgr 420

taarggwgat ttracattrt ayattgctgt wttrtcwatt tcwcaattaa tyggbaatrt 480

tacgttatty ccwyatttrm rwcgrtayat taayttaych rattggcgag aattaaraat 540

wtggcgycat ttyaaaccyt cwytagtwtt rtttgtkccs caaathgcaa cvcaakttta 600

yttratwcty aataaaacra tggttgggaa ratggtwtcr gttgaagcag cwggmttcta 660

ygataattcb gataarrttr thaaratggt mttrgcratt gtaachgcka crgghacdgt 720

vatgytaccw cgagtbgcya atacrttygc taagggdgat caygaacarg ttaaraarta 780

yttatatcaa tcbttygatt ttgtgagygc brtwtcrath ccratgatgt ttggvatkgc 840

rgcsatygck cctmaatttk cwaykatgtt ytttggamaa gcwtttrrtk cmgywggmsm 900

aytratgatg gttgartckw twgtwattat 930

<210> 31
<211> 576
<212> DNA
<213> Lactobacillus sakei

<400> 31
atggaaaagt ttattgaatt atctttaaaa gaagtaacag caattacagg tggaaaatat 60

tatggtaacg gtgtacactg tggaaaacat tcatgtaccg tagactgggg aacagctatt 120

ggaaatatcg gaaataatgc agctgcaaac tgggccacag gcggaaacgc tggctggaat 180

aaataataag caaaacttaa tttagattta aggactctct atcatttgat ggggattttt 240

tagcttatag taacagaata aaataagagg tgtcagatta tgaaaatatt gaagtggtat 300

tcaggtggta aggatcgcgg agaacgggca aatgatatta taggccaatt gttgctagat 360

ctgaaccatg atccaaaaaa tgaacattta gaagcaatat taataaatta tcagaatgaa 420

attaaaagga aagaaagttc ggtgccattt attttgagtc ggatgaatat atcaatagcc 480

aatacaatca gaagagatag gctcatttta accgattttc aagaagataa attgaaattg 540

ttaactgcgt tgtctaatat aagatatggc tattag 576

<210> 32
<211> 3260
<212> DNA
<213> Lactobacillus sakei

<220>
<221> allele
<222> (3010)..(3010)
<223> the nucleotide is deleted in some strains

<400> 32
ccgctattaa acgratgaat ttaactgcca agattaagtt aattaaattt gatttggktg 60

ttgatgggca gcgggatgag gatgarcgac aactcttaaa tttagccgga gatgactatt 120

tttatcaact gcaatataaa tattayggtg acggtgaatt aatggctttc caaaargtat 180

caatattatt tgatcaattt ccaytaatta gagtatccga gataaaagat aagtcgttat 240

atccgatgct ggccaaaaaa tatgggttta gtaatgtaga ttttgtacag gagacgacaa 300

cagggttagt tacaaataag gaacggaaat ttttagagat aaatacggga tcaccgtttg 360

taaaaattga aaaacaaggt atctcagaac atcgcacagt attgtttgat gaaatctata 420

tgattgattc attaccaatg tayttaattg ataaytagaa aayyggagga attagatatg 480

tcagaaccta atattgtatt aacaagaatt gataacagat tggtccaygg acaggtaggg 540

gttgtttgga cgtcatcaat tggcgctaat ttgttaattg ttgcyaatga ygaaacagcg 600

gaaaatgaat tagaacaaca attaatgtca gcaactgcgg aaaattcggg tgttggtatw 660

cgctttttta acttacaaaa aacaattgat gttatccaca aagcagcagc tcgtcaaaaa 720

atattyttag ttgttcgcaa tccacaagat gtattgaygt tagttaaggg cggrgttcct 780

gttaaagatt taaatatcgg taacatgcac ttttcagaag gcaagactga aattactaaa 840

aargtttata tggatgataa tgataaagaa acattaaagg aaattgctga tttaggggtt 900

aacgtcttta ttcaagacgt accagaagat aagcgattaa tgttgggaga tgctattaaa 960

tgacagtaac cttaactcaa gggattctaa ttgctgtatt tgccttgatt gcaggttttg 1020

atttttggtt agaaggcctt tatatcttca gaccaatgat tgtttgtaca gtaacaggat 1080

ttcttttagg cgacttaaca atgggaatta tggctggtgg tttaactgaa ctagcctttg 1140

cwggtttaac accggtcggc ggracacagc cacctaaccc tgtgatggct ggtattatga 1200

ctgttgtgat tgcycataca acaggrcaca gtccagctgc agcaattggt ttggctttac 1260

catttagtat tttaatgcaa tatattatct tgttttatta ttcaattttc cctgttttca 1320

tgacaaaatt agaccggtat gccaaggaag ctaatacaag tgcttatacg cgggtagctt 1380

tattgccaac aggaattgtt atgatcacgt acgctgtttt cacattcctt tgtgtttatg 1440

cagctcaagg accaatgcgc gatttagtga atgcaatgcc trtytggttg agtcatgggt 1500

ttgaaattgc cggsggtaty ttgccagcag tcggatttgg tttattactt aagagcatgy 1560

tgaagagcga tttgattcca tatctkttra tcggttttac ggcagcttgc tttattaaat 1620

tcggcaactt aatgccagtt gcygtyatyg gtgttgcatt tgctttaatc gaatttaata 1680

gaactaagaa acaaaatgat aatgatcaaa aaatyaagaa aatgatcgaa gaatcaagat 1740

taggaggtga cgaagaagat ggcatctaat aaagaaaaag aaacgacaag tacgattact 1800

aaaaaagata tcaacaaaat ggcactscgt tcaattttta tgcaaacatc aatgaactat 1860

gaacgtatgc agtctgcggg ttggttattg gcaatgctac ctgtattaaa gaaaatctat 1920

aaagatgata aagaaggttt atctagcgca atgcaagata atctagaatt tatcaayact 1980

aataatacgg tagcrccttt attgatggga ytattracat ccytagaaga aaaycatgaa 2040

aarcgttcaa ctatcaaagg tttgagratt gccttatttg gtcctttrgc tggtattggg 2100

gatgccattt tctggttcac cgttttacca atcgttgcgg ggataackgc ttcttttgct 2160

aaacaaggta gtataytagg kccattarty ytcttcttag trtatgtcgg gattttcttt 2220

agtcgaattg ggttygctag attagggtac tatactggtt caaaagcgat tactaaaatt 2280

cgagacaact caagtatgat ttcraaggca gctagcatct taggtgtcac agtaattggt 2340

ggtttaattg caacttaygt taacatcaat gtactttcta aaattaaagt ggcggctggt 2400

catacaatct caatycaaac acaattctta gataaratat tccccaatat tttagcttta 2460

ggmtatacmt tcttattgta ttggttattg aaaaagaaaa atgttagtcc agttgtwctc 2520

attttgataa cgtttgtctt agcaattggt ctatcatggt tgggagtgtt gtaacatgga 2580

tattattgtg acaggacatg ggcattttgc gacaggttta tcrtcaacya ttcaattrtt 2640

agctggtgaa ttaycgcatg ttcattaygt tgattttact aaagaaatgt cagaagaaaa 2700

tttggtggat cagttaaaat caataatggt taaggataaa tcktatgtat tcttttgcga 2760

tttrrttggt ggracaccct ataargaggc cgctcgattg aktgccgctg atgmcaaaag 2820

aataggtgta gttgctggkt gyaatctagc rtctttatta gaggtcggaa tttcattarc 2880

agatagygat aatgatgtrg rytatttaac ygaagaatta gtaactatca gtcaaaaagg 2940

aacccagrta tttaaggtta aaccaatyaa acmggaagtt attgaagayg gtatttagak 3000

wttaaaaagg attcyrytgt gratrwwwta ttcacaatag rrtcyttttt trtryaatat 3060

wrtcgktagy ratyaataat taaagcgctt agactaccgr aaagwrgggc atcaatggta 3120

gaattgaatt agaatctgat tgaggaggat gacaatgaca aacgttcgaa tgcaagatga 3180

tttttayrcg gcagtgaatc argaatggtt aaaaacrgcc gaaattccag ctgataaacc 3240

agcaacyggt ggctttgtgg 3260

<210> 33

<211>  8265
<212>  DNA
<213>  Lactobacillus sakei

<400>  33
tggtcgcggc cgaggtaacg gaatcaccgt aatatgatgt tgtttaattt cttctggtgt      60

taattgaatt gatgaatccg ttacgatttt aatattagcc actactactt cagccacact     120

ttctatctcg aaacaggccc atatgcggac cgctatttct atgattatat ctataagaca     180

gtatatcaaa ctacgcccat tttttcagca aaagttactg agaaaaaatg acagtctaac     240

taaaaaagat tacgtcgttc gtttttaaac gaataacgta atccttttta acaatgttta     300

tttctaatat caacagacac taatctttgc gtcaactgca agcacaaaca gactagctgc     360

caataaatct gcactaccac ctggcgaaat atggtgttct tggcagtatt ggatcagcgc     420

cgtaaaacta tcattgggga gtgcgttttg accagcctga atggctaatt gttgcatctt     480

aataagtgtt tgttgatcac tacggtgtag cacgcaagta tccgcgactg atcccattaa     540

cgtcaatagc atctgaagtt gagcttgttt tggcgtattt gtttgccact tttgataagt     600

ctgaacggct aacaaggtat gtggataccc caagacggct tcttcataag cgccatttac     660

acgataccgt ttttttacaa cggcgccgtg tgtaacattt tgagatgtat actgttcatc     720

caggaattgg gctaactgac tcactttttt taaaacagtc tcaatttgcg tagactgacg     780

catactaatt tggtgcgcat aaacgctcac caagagaccc atcacccaaa tagcaccctt     840

atgtgtatta accccaccag ttgcttcaaa catagcttgt tcagcggatc gaccaatctg     900

tgcgattgtc tctcgcaatg tctgatcgac aggatgacac caagcagctt gtgccatttg     960

ttcaaaaaaa ggcgtcaaag cccgtgccga cttgataaat aaagacgcgt ccatatccgt    1020

atgtgcacca ttatcgcgcg catcaactaa accaggtttg gggctcaact gaacctcgtc    1080

gactaaagct tgctcaacta aacaagccaa ttgtttagtc atcatttgtt gtgccttatt    1140

atttatcaaa gtctaaacac tccttaacta aattgctttt taatccctga ccatcctaaa    1200

tgaaggagcc agctgagcat aaacgcgccg gttgccgtac tcaagtatgc gaccacgatc    1260

atcgtcccca ttggtaatcg taaccaagtt gacttaaaga ctagtaataa ggcttggaaa    1320

acaaaaacat tggctaaata cgtccgataa gcataaaccg ctagccattt gaacaagggt    1380

aacttaggtg attgattaac aatcattctt gaggcaaacg taaagacggt cagaataata    1440

atcaaactgt ataagctttg caaggtattc aaataaggcg cgtggccaaa gttaacatcg    1500

ccaggataag ctaataaggc gtgtactgat aatccaccca ttactaagcc aacgggtagt    1560

aatgcaaaac gaatccgatt taactgctta aaaattgctt catgatagat aaaaccggca    1620

acgccataaa tcccataaat taggaaacta acgacaaatc ggtctaataa ataccagctt    1680

tcagctaatg ggccgtttaa aacctgcata ctataaaaga gataccatcc gatataaaga    1740

acagtggcac ctatgagtac cggccaaacg agtttacgat tggcaaaaac atgatagccc    1800

aaccaaataa agaacggcat caataattga atctgtaaca tcatgaccgt ataccataga    1860

tgcggtgcac catcacctgt gataactttc aataaaaagg ttcccacatt agtgtatggt    1920

gttttttgtt gcacacctgg gaataccaat aaataagcga ttgtccatag taagtatggt 1980
acaacgagtt caaaaaattt atcctttaaa aattcaaaat aagatgtttt ctcactggtt 2040
ttaaccatat tgtagacaat ggcaaaaata aacatcggtg ctgtgtattt agcaaataca 2100
taaagcgttc caacaaaagc cgccgtcgtc tgattgtgac aattagccaa tgcaaaactc 2160
aatatggttt gtaacatgac cgccgtcacg gctgatactt tttgaaaatc actaacgttt 2220
attttagtct tctgatcttc gaacattttt tccaccctca tttatgacca aagttctttg 2280
gttaatagcg ggcctgcatc ggtttttaat aaaatctttt tagccggtgt tcgcaaatat 2340
tcctttaatg caaaaccacc cttggcggtt atcacttgcg tatcaaactg ttcgaaaaaa 2400
gaatgatcgg cagcaattac cgtaagcggt aattctgccg gcgcgttagc atatagcaat 2460
aaatcaaaat cactcgtcgg cttaatagcg ctaatgccag atgataattc aaaccctaag 2520
ctgccaccaa caccccattt gaatcctgat aatattttcc tagcagcatg atattgcgca 2580
taaaccggaa attgtgcgac ttcagtgact ctgaaagatt cacgcgtcgt aatttcccag 2640
ggatgaataa cagcttgaat tgcactatca ggaatttcga gagcaaaccg ctgattccga 2700
gctactccgc gaaggccgac tggtaccaaa tctaactgac tagcaattcc ccgacgaata 2760
acgccgaaaa cctgtttagt gagcgctaat tgggccacta aagctggttg ttgaataatt 2820
tcacgatcaa agcgaacaat atcatgtgca gatttcattt aattaagcat cccattcttc 2880
atccattttt gcttggacct tattagtttc aacacggccc attttgacag caatgtcatt 2940
tgtgtaacgg ttcgtcaaca tcgtatcgct cgtgtgacga acgtcttcaa tggcatcatt 3000
aacggcttca atcacagttg caacgttatc agtcgtcgtt gtttgatctg tcacaccatc 3060
taagaaacga tacaaggcac ctaatttatt ataattacgg atatcgtagg caattgaagg 3120
tactttttct gctgctgctt caatttcagc aattgatcgt ttagtaattc ggctagcact 3180
tgctttagac atcgcttgaa cagtaattgc atcgctgttt aaagcaatta aacggctaga 3240
ttgtaaccca tgtgctaaga aagcacctga aatagcatta ccaacgacca ccccaattac 3300
tgggtgccct gcttgccgta atttggcata agccgctgca ctattcgcta gcgccacatg 3360
aatcccgatt aattcttcgt tataaccgta agcttgacta gggacatcaa tgacgacaac 3420
gattggtcgt ttaacggtct tattttgatc ttcttcatag atgtgattta agatttgtgc 3480
catccggaag ccttctacca agccaacttc gccatctcgt acacggtaca ttggattttt 3540
accatccggt acaattgtcg ttacgacagc atcttcacta aaacgatctg aatcaccata 3600
ataaacagta ctaattgttg aacttggatt ctttaaacca gttagcgctt ggaaccagtc 3660
gtacccaaca ctggctgttg ccggtgttgc ttgttcagta atcacaggta gttcaactgc 3720
ttgtgtttgg tgtttggcaa gtgcttgatt atagtcttca atggccattg gttttgaaag 3780
atcaagtttt gccaataatg acaagtaaaa gtccgcattt tcactacggt ggctatcttt 3840
atgttcaacc attgcagcca cgattgcagc cttaacattt tcgacgctat cagtcaccaa 3900
ttcatcaata atttgagctt caacacgttg acgggcacca attgtattcc aaattaaatc 3960
tttgtcgctt gaatcaaact cacggacgcc ggcttcttgt tcgattactt ctgggccatt 4020

caagccaacc cgcgcttttt tagtcgtaat taaataactc attaaagctg aagtaatgga 4080
catcccacca aatgaaccaa ctcgcccagg gacaacccca atgacaggga cgtacttttt 4140
aagtgccacg attaagttgc caatttcaga aattgaaagt agaccataat tggcctcttg 4200
tagccgcacg ccaccagtat ctaacacgat aattgggaaa ttttcattac cattttgatt 4260
ttcagtcaag gcatgtgtta gtgcagcgat aatctttgca cctgaaactt cgccaattcc 4320
accaccttgg aaactgcctt ccattgcgat tacgacaact tctttattac caatcgtccc 4380
acgtgacacg ataatgccat catcactttc tgggacaatt ccttgagcaa tcaagttagg 4440
agctgtcatt tggtcaaaag gaccaactaa ttcgcgtcca gtaggtgtgt ccaataaagc 4500
attaacccgt tcgcgggctt ttaattctac aaaactattt ctcatcagct agttcctcca 4560
ttgcttgtgt taatcgaaga taaacgacac cgggtgttgc accgaagtca ttaatcacaa 4620
tgtcggctga aattggataa cgatcaaaga aacgtgttaa aacatttccc catacctcat 4680
caaaaccgtc actaccggta cagactttaa ctgttgattc agctttattg gttggaaaca 4740
ttaaaatttc taaatccccc gatccaacga ccccgacgtg cactcgttta ttaatgggtt 4800
ggcttgtttg atattgataa gctaattgtt ccatgataat ctccctttcc tatgaccaag 4860
ttctgaactt cattggtggt tgatataggc catctgacca ttcaaccaaa tcatcaattg 4920
tttgcgctgc taataatgaa cgttttgcat cgcttcgctt gatacccaag tcttcaggaa 4980
gttttacaat cccagcttga cgtaattgat ccaatgattc tttagtactt gtcatgccaa 5040
gtggtgttac gccaccgatt gcagcaattg cggcttaacg ctgttctttg ctagttgcct 5100
tgtaaagata ggcaatccct tcttcagtca caatatgcgt tgtatcttcg ctataaatca 5160
taacgggggc gtttgctaga ctcgcttgtt cttgaacagc aagtgcatct aaggtttcaa 5220
caaaaactgg tttcttatta gaaccatatg tttcaaccat ttggacgact aatttttggc 5280
ctttgcctaa aggtttttct tcatcgcgta atgattgcca tgccggtgtt gaatgacgac 5340
gtccaccagg attatgcccc atgttaggtg cgccaccaaa actatggata ctttcaaccc 5400
aaccagattc aatcgctgga ataattgttg gatgtggatt taaagcccaa tttttagcaa 5460
ttttaccctt aaggcctagt gattcaccgt aggttggtaa gagaagttca atggcagctg 5520
tgttaaaacc aatcccatga ttaagtgatt ggacaccatg ttctgcataa atccctttaa 5580
tcgccatcat tgccattaga atctgaagtt ccgtaatatt ttgtggatca cgggtaaaga 5640
gcgcttctaa ttcataaggt ttgtccgctg gaacaacaac atccacccag ccagcaggaa 5700
tatcaatccg tggtaattca tcaacgactt cattgacttg aacgatcaca atcccatctt 5760
taaatgcggc tgcttcgaca atcgttggtg tttcttcggt attaaagccg gtatataaat 5820
taccagcttt atcagcctta tctgcagcaa ccaatacgac attaggaatt aaatcaatga 5880
ataagcgacc atatagttct agataagtat gaatatcgcc taacttcatt ttgccgtctt 5940
caatcatttg agcaacacga acactttgtg cacctgcata tgaaaaatca atttttgaag 6000
caatttggcg ttcaaagata ttcaaatgtt cgggacgtga aatactcgac ataatcatgt 6060

gtaaattatt tactttttca gggttaactt gctctaagct ttgtgacaag aaactggctt 6120
gcttttgatt atccccttct aaaacgacct tatcaccagg ggcaattaag gcctctagaa 6180
ctgtgataat ttggtctgtt tcaacatatt taccagtcat taactgactg attttggcta 6240
atcgttgtgc tttttgaact cgtttcgtgt cccaattgcg ttgtggtttt tccatctagt 6300
tcatctccat tttcgatata gaaacgttgt atcatcaatg ccatgttggc taatattaat 6360
cgtcctgatt aactgctctc caaaattagc atgaatcaac tttgttaacg tattgcctgg 6420
cggaaattca atcgtcagat ccattcccat ttccttagca acggccatca tttgtcgcca 6480
ttgtactggt tgttcaatat tggccaacaa atcctgacga atggcttctg catcctgtgt 6540
gagccgacca tccgtattct ttaagtacgg acaagtcgcc ggttgcaaag tcaattggtt 6600
aatgtaaggc gttaattttt caacggttgc ttgcatcaac ttacaatgtg atggcaccgg 6660
aacctttaat aatttagcgg ttcgtgcttg tttctgctta gctaccacca aagtcttctc 6720
aaggccaatt acggcacccg agattgtatg ctgcattggg cagttctcat ttgaaagata 6780
gaccggataa tcgggatcaa atgtttcttg aactacttga gccgcctcat cccgggtcaa 6840
tccaacaata acgcccatcg cataaccgct tggataagca gccttcatta atactgctcg 6900
ttgataaacc aaccacacag catcatcaaa cgataacgtt tgacaagcga cagcggcggc 6960
aaaagccccc aacgaatgtc cagcaaccaa gtcaggcacg atatcattct tttggatttc 7020
gtctaaagca aataatgcct tcactaacaa cgccaattga ataaagacgg tatcctcata 7080
attctgaggg tcatcaatta attgatagcc tgtcacttta gcgacccgct caaggtattc 7140
aacgggaata tcttgtaaca tcccgacttg ttgactgcct tgccctggaa ataaaaatgc 7200
tgtactcatt aaatctcctc ctttcgcaat taccgattac cctttaatct taagggagtt 7260
ctgttataat aaaaaatact taaaaaatgg agaacgataa ctaataggta tcaattaagg 7320
tggtcaataa tgaaattaat tcatttacaa tactttaaaa aagtctttga aactcaaaat 7380
attacacagg cagccggggt aacactgttt tatcacaccg gtcgtaatat tgaagcaaca 7440
ccatatgccc ataccttcta cccctacgtt accaagacac tagccatact tgatgacgga 7500
tttcgcgcag ttactgcctt aaataaggag attatcacct cagtgacgct ttgtttagaa 7560
gttgcctctg tgtcggtgcc aaacctaatt cgaatttttt cagaaaaaca tcctgatatt 7620
aaattaacaa ttatccaaca tcatctccca gaaaatatta atgaatcggt tttatttatt 7680
acatcggatt caaagccgga tttaaataat gtcccgatta taaaagagcc cgtcttagtc 7740
gctatcccga aatcacatcc attagctcac aaagcaattt tgcaaacaag tgatattgaa 7800
caaacaccca ttattatgtt gagtcccagc aacgcttttc gtaatacgat tgatatggcg 7860
cttgaagcta atcatattca ccctaaaatt agttcaacaa cggatgatcc tggcacattg 7920
cgctccatca ttaatcaggg gttaggaatc accttcttcc cgcaaatttc ttggtcatat 7980
cgagctaacg atcacttcgt cctcaaagaa attgcggaca tctcaattga acgaactatc 8040
tatctttctt caacactgcg aaaagatcac ccgcttgttg aaacaattgt gacgacactc 8100
cgagaatggt tcctctgcca caattaaaaa aggaacggtt actatcaatc aatgatagta 8160

accgttcctt tttgttgttt attcagttat tctttgataa atctcaaatg tatgcgggta 8220

ttgattacgt tcatcaaccg tccctgcttg ttgcgaaatt aattt 8265

<210> 34
<211> 1609
<212> DNA
<213> Lactobacillus sakei

<400> 34
actgggctta gtgggcaaca tgaggaagct tttgtagata tagatagtaa tggttattta 60

cagaatattt caaaagatat acgacaaatg cgaaatttgt cggttgaatt tattggtatc 120

agtaaatatt catacagttt tttcaaaaag cttgtagacg aatataagaa ttctgaaaac 180

gaatggttaa attacgaata cttagttaat gcaatttcta gatatgatgc tgttaaatgt 240

attctagtag ataatctaat ttggatagat attgatgatg aatctcgtta ccacaaatta 300

atgaatgcag tatatccagc cattaaaatt agggaggata aggagaacac acgaaatata 360

cagaacataa ttagttcagt tctaaaatta cctgaagata ctgacattat agtcagagat 420

gctggtggaa tgactaacga taattataga gtaaaagtaa ataatcaata ctattttgtg 480

cgaatgccag gcgcaggaac gaatgaaata attattcgag aaaatgagga aattaattct 540

aaattagcaa gtactattgg tgttacgcca acaatttttt attttaatgt ggattctgga 600

attaaaatcg ctgaagaaat taatggagca acgactttga ctagtcgaag cataaaaaca 660

agagtaaact taaaaaaagt atcggaaatt ctacttaaat tgcatagttc aaatattcaa 720

tttgcgaata gatatgatta tttaagtgaa cttagaaatt atgaggaaat attaaagaaa 780

cgattcggac atttctatga tggttatgag ttaacaaaga caaaattaat gttggcaatg 840

gattctttag agtttaatag tgatagtcca tgtcacgtag acttagttcc tgagaatttt 900

gttctagata agaatgagag catgtacctt atcgactggg agtactctgg aatgacagat 960

caattttggg atcttgcatc attctgcaat gagtcgcatc ttacaattga ggagattagg 1020

acatttttag aattgtattt taaccggaca attgaagaaa aagaaatgca gaaaatattt 1080

tggtttatgg cttttcaaaa cttactatgg agtgtatgga ctttaaataa agcacttgaa 1140

ggcgaagatt ttggaagtta tggtatttct cggtttaaga atgctgagaa gatagttgag 1200

gaatatttaa atgcttaaaa aaaataatgg gattttttgg ggcttactag gggctttgtt 1260

ttggggaata gacactgttc tgcttgtgtt tgttagtaag ggggatgctg gaacaaacag 1320

tataagaact cctttgatga ttgcatttct acatgatttt ttttcatgta tttgggtgtt 1380

aataattaga ttcgttaatg acaaaggtgt taacgtatgg cattatagta tgaaatcaat 1440

agttttgcag agctttgcaa gtgtattagg tgcacctttg ggtatgacga gttatgtaat 1500

agctatattc tttttaacat cgagtttgac atcaattatg tcttcaatat atcccgtcgt 1560

aagtgcatta ctagcaattg tgatacttaa ggaaaaagtt tcttttaca 1609

<210> 35
<211> 336

<212>  DNA
<213>  Lactobacillus sakei

<400>  35
atgaagagat tatgcatatt agcttatcaa gggctgccat ttctgattga taatttggtt      60

atctttaata agaaggaacg gaattttgtg aagaagttaa acaatagaat cgatgaacta     120

tcgatgagtt ggcaagtaga acttgatagc tcatttggaa atattagtga tattgaagtc     180

aaagcaccgc aagccgtgtt attaaaaaat ggcttgcgat atcgttttaa tactggcagc     240

ttccctaaaa atgatatcta tcaattggga gctttagaat tacaagaagg tgatattgat     300

gccgttattt cttttttgaa gcatttagag aggtag                                336

<210>  36
<211>  482
<212>  DNA
<213>  Lactobacillus sakei

<400>  36
taaaatgcgt aaaataccct cggtggcagt tgtgtcgctg ttcgggtcat tggaaaaagc      60

gggtgatgct acacttagca cgtagtcgat gttctgcatg gctgtcgccc agccagcatc     120

ttgagtgaga tcagcggcga caaaacttaa attttcgcgg tgttgcgcct gattttcttt     180

tagggcggtg cggacggtgg tttgtttgtc gagttggcgt aaggtggcac ggacgtgata     240

accttgattt aaaagttgta agataatgtg cagacccaga taaccgttgc cgtcggtaac     300

taatacgttt tttgtcatga tgttatgctc ctattcccta atgagggtta atttaatcag     360

tcatgtatgt tatgccgtta agtagcatga tccttgttta tggcttcttt ttatttataa     420

tagtccctag agtatactct agggcaagca aaagaacact atttcaacgc agcgattttt     480

ac                                                                    482

<210>  37
<211>  3211
<212>  DNA
<213>  Lactobacillus sakei

<400>  37
tatttaagaa tattatgttg catatgatct ctcccttaat tttagttcga ctaaagtgta      60

ttagcttttg ggagatataa acttgacgtc catcaagttt ataaaatagt tctagatact     120

taaaatgctc gtattgacta atataataat ggaatataca atagaaggcg agatattgtg     180

aattattaaa ctaaaagggg tggcacggaa tggaaaacca agaaaaaatg gcggcttcaa     240

tgttgcatag aatggtccgt gaatttaccg aaaatgaact agcaccgctg gatatggaaa     300

tcgacgccca gggtgattat ccgcaaggct tattccaaaa agtgatcgat aatggcttgt     360

taacaatgac actaccacgt gaatatggcg gagcaggcat tagctatgac agcgttgcca     420

aggcgattca cctgatggcc attggtaatg ccagcatggc agttacactg gaaggccact     480

ttaagacgtt ggaacagctc ttaaagtatg gtgatcagcg cttaaaggat gagtatctga     540

cgagtgcggc ccatcgaatt tttgccttct cgcagactga atcgtctggg ggctcaaacc     600

cgcgtgggat tgctacgcgg gctgtgaaga gtggcgatga ttggatcatt accggcgata     660

aaattatgat cacgaacggc ggcctcgccc aggtatactg tgtgttggca aaaacggaag 720
atgatcaaat ggccgtgttt gtcgtggatc aggatatgcc tggtttttca tttggaaagc 780
gtgaagactt cattggtcta cgcgggacac ccgtcggcga aattgtgatg gatcatatca 840
aggtgccggg ttaccaccta cttggtcaga tcggccaagg cctcgaaatc ggcaataacg 900
cccacgacga cgcgcggatc ttgatgggcg ccgtcctcac gggcattatg gaacacgaat 960
tacagattgc ggtcgactat tcgaagcagc gtaaagcact cgataccccg ctttatgaat 1020
tacaaaccat tcaagaaaaa atttcagaaa tcgcgattgc ccgtcagaac acggagctac 1080
tgtatcagca tgcggcacag ttgaagatca cgggacagtc gtatgccaaa gttgccgcga 1140
tggcaaaatc gtacggaagt cgttcggcgg tccggtgcgg cgatatggcg cttcaagtac 1200
tggccggcta cgggtatagt cgcgagtatc cgatcgagca cctcattcgt gatgcgcgga 1260
ccatggaaat tgctgaaggt acggtagaac ggatgagcgg tgaaattgca ctaaaacagg 1320
ttaatcagta aggggtgaac aacatgaaaa ttgtggtctg cattaaacag gtaccaagaa 1380
gtgattcggt caaaatcgat cccaagacga acaatatggt gcgggataat gtcgaagggg 1440
tcatcaatcc atttgataaa aatgcgatcg aagaagcact ccggattaaa gatcaacttg 1500
gtggagaagt cattttgctg agcatggggc cggacggatt catgagttcg ctgcgcgacg 1560
gtctcgcaat gggggctgat caggccattc tcctcagctc acgggccttt gggggcgccg 1620
ataccctcgc aaccggctat ctaatcagtc aggcaattaa aaaaattggt ggcgtggatt 1680
tggtattgtt gggacgccaa gcagtcgatg cggataccgg ccaagtcgga ccgatcgtgg 1740
ccgaatactt aaacgtgccc caggtcacct ttgctgaaga actgcggttt gtcgatgaac 1800
acaccttgta cggccaacgc ttattggatg gcgcaacgca aagtgtccaa gtgacattgc 1860
cagccgttgt aacagtgcgc agtgaattaa acaccccacg ctacgaaaca ccggttaaca 1920
ttcaaaccag ttttgaaaaa ccaatcgacg tgtggacgga acacgatctc gacttagatg 1980
caacacgcat tggtcaggcc ggttcgccaa ccacagtaca aaaggtgtac gcaccaaaac 2040
gtgagagccg tcaaacggtt caactcagtg atgacagtgc aacggcagtc aaagaactgc 2100
tggcgcggat tcgaaattag cggggtgaat aaaatggcac aatcaatgat tgcagtttat 2160
gtgcaaacgg acggttcgat ggtggagccg accagtttac aactgattac caaggccaga 2220
gcaattgcga acggtcagcg tgtggtggct ctattgccga ccagcgacgc ggaccaaacc 2280
gttgcgacgg tcaagcagta cggtcccgat gaaatttttg tgttagccga cgaccggttt 2340
gcggcagcga cggacaccga gattgcggat ggcctctatc aggtcgtgaa acaggttcaa 2400
cctaacagca tgctgattcc agcaacggtg gttgggcggt cggtggcccc acggttacag 2460
gcaaaactcc aaacagggtt aacggcggac tgtttagacg tctactttga tggtgaaaca 2520
ctggttcaag tgaagccaac ttatggtgat gacatcatgt gtgaaatcat ttgtgcggat 2580
cgccggccac aaatggcaac ggtccggccc aatgtatttc aggctgaacc agcgcaagtt 2640
gctacgcaag taacgaacgt ggactttgaa tttcatccag aagcacacat taagatggaa 2700

aaaccgacac caattatcag tagttcggcc aacattagtg atgccggtgt ggtgattgcc 2760

ttaggacggg gggctgataa cccgcaactg atcgaaaaag cgcagaaatt agcggctcgt 2820

ctgggcggaa tggtcggtgt ttctcggcca ttaaccgatc acgcagaatt tggacatgaa 2880

agccaaattg gacaaagcgg ccagtctatt gcacccgatt tattgattaa cttcggtatc 2940

tcgggggcca cccagtacct agtgggaatc gaaaatgcga agacgattgt ttcggtaaat 3000

accgatccgg atgcgcccat ttttaccaaa tcagattatg cgtacgttgg ggattccaat 3060

gatttcatgg ataccctgtt aagtagcaca agagtttaaa aatcaacgcc agcgactctt 3120

agatagggat cgttggcgtt ttgttgtgcg aagaacttta caccctgtca gtgtgctctt 3180

atactaaaaa aatggactat ttttattcag t 3211

<210> 38
<211> 4520
<212> DNA
<213> Lactobacillus sakei

<400> 38
tccgagatgg ctgatcgcct tcaaattcaa cacgttgacc tcaaagtccc cgaaacggaa 60

ccaatcaccc ttgaattagt acaaaccttc tttaaaaagc atcccgaaat cacccatttc 120

gcaatgattc actgcgaaac aaccaccggg attttaaatc ccatcgatac tattttccca 180

tgggtcaaag ctcaaaacgt caccacgatt gttgatgcca tgagtagttt tggcggtatt 240

cccattgatg tgccaacctt aggcattgat tttctaatca gcagtgccaa taaatgtatc 300

caaggtgtcc ccggtttttc attcgtgatt gccaactgtc gccaattaga aaattgtcaa 360

aatcgttcca cttctgtgtc acttgattta tacgcccaat atatcagtct caaagaacat 420

cccggtaaat ggcgcttcac atcccccacc cataccgtac tagccttcca agaagccctt 480

cacgaactcg ccctcgaagg tggaattgaa gcccgccact gccgttatcg ccaaaatcaa 540

acaaccttag ttgctggcat gcaatcccta ggttataaaa ccatcttacc aactgactac 600

caatcaccga ttattaccac tttcaaaatg cctagcgact catttgattt ccaaactttc 660

tatgacatat taaaacgccg gcacttcgtt atttatcctg gtaaaacaac taaagtccca 720

tcattccgga tcggaacgat tggcaacgtt gacggcactc aaatcgctac tttattagaa 780

accatcaaaa cagttcaaca atacttgtaa tgcattggag gtttcaccct tgaaaaaaaa 840

gttatctgcc ctattcgcct tattatcaat cctattgatt gcgacattgg ccggttgttc 900

ttcaaaaaaa tcagccagta gtaatcaaac cttaacaatg gtcttttatc ccaacgaatc 960

tgctaaagac ttcaacgctt cgcgaactgc cattaaacaa caactcaaaa aggctactgg 1020

taaaaaaatt aagattcaaa caaccactga ttacaacatt gcgactgaag ccatcgcttc 1080

tggcaaagcc caattagctt tcatggggcc tgaaggttac gcccaatcac atcttaagaa 1140

caaagccgtt caacccttat ttgccctttc cggcgcttct ggtactttaa aagacgctgc 1200

ttaccactca tatttcatgg tccccaaagg caaagaagcc caataccaaa ataatggtca 1260

atacagcatc gacaacatta aaggtaagaa gatttcattt gtttccgtca gctcaacttc 1320

gggtcacttg attccagttg ccgctatcca aagcaagttc aacttaaaaa atagtgacga 1380
actttctcaa aacggtcact tcttcaaatc cgtcctcttc ggtaactcgc accaaggttc 1440
tgccgttaac ctcttcaacg gcgatgctga tgtcgctgtt ttcgacgata ttgataccaa 1500
cgcttatatc aacgtcgcta aaggttcatg gactaaagtc ggttcagaat tccgcgtcaa 1560
agacaatgcc acggcgccat tcatcagcgt tcgtggtaaa gaaagtgttg cgattgccat 1620
cagccccgtt caaaatggcc cattcgttgc aaacactgat aaattaagca aaaaagatca 1680
agaaaacatc accaaagcat ttacaagcaa agcagtcact gacaatccta agattctttc 1740
cccaaaagac gcccaagtgc ctgccatctg gaagaaagaa tcaagcaaat cacaactttt 1800
aaaggtaaca aacgactggt atgcacctac caataaactc gttgctaaaa attaatccta 1860
acaatccttg aaaggcggtt ttgacgcatg ttatcggtca accacatcaa taaaactttt 1920
gccactggac gccaagcgct tacagatatt tcattcacag caaacaaagg tgaatttatc 1980
gcgattattg ggccttccgg tgccggcaaa tcaaccattc tacgtagtat caaccgacta 2040
atcaacaccg atagtggcga aattagcctc gacggacaag atatccgcca cgcaaagaaa 2100
aaagaactcc ggctaatccg ccgccagatt ggcatgattt tccaaagcgc taacttagtc 2160
cctcatttga cggtcatcga aaatgtcctt cacggtcgcc ttgggtacca atcaacgctt 2220
cgcggcgttt ttggttggta ctcccaagtt gaaaaggaag aagccgtcgc attattaacc 2280
gcagttggct tggctgattt tatgtataac cggtgtgatg aattgagtgg tggccagatg 2340
caacgggtcg gaatcgcacg cgctttaatg caacatccca acctattact ttgtgatgaa 2400
ccaattgcct cactcgatcc ccagtctgcc aaaacggtaa tggattatct ccatcgtttg 2460
actaaaaaag ccaatatcac ttgcatcgtt aacttacatc aaatcgatgc tgctcgagaa 2520
tacgcagatc ggattctagg catcaatcaa ggtcacctcg tcttctcagg aatccaggat 2580
gaattaaccc caacggttct tgaacaactc taccaagata gcgaggatta gctaatgact 2640
gttcaaaaat tctttagaaa ccgaaactta gcgctgattg ccatcgcggt attgcttagt 2700
ggcctctatt tttcaacagc cacactaatc gactatcatc tagtcagttc agtcaaggcg 2760
atcccagccg ctatcatttg gatggtcacc aactttatgc ctaccagccg cgccattgcg 2820
gcactccccg agattgtgac taaatttacc caaaccgtca ctctttcaat tgccgccact 2880
ttagcggccg ctttcttggc cctaattgtc gctctgaccg gttcagaaac gactggtatc 2940
aatcgtttta cgaagttatt cgcacgcata ttcgcatcgt tttgtcgcaa tattccgcta 3000
atcgcttggg cgatgatttt actcctcgcc ttcaagcaaa gcgatttaac cggtttgttg 3060
gccctttttc tagggtcctt cggttattta acccgggcct tcatggcatt aattgatacc 3120
aaagcaacta atatctacga aacactcagc acaactggcg ccaactactt acaaattgtg 3180
tgccaaggca ttctccctag tatctcgtct tctttgttaa gttggattct ctacatgatt 3240
gaaaacaaca ttcgtgatgc gaccttagtg gggattttga ctgggaccgg gattggtttt 3300
tcatttgatc tttactacaa gagtttccgc tttgacctcg ctgggatgac gacactcgtc 3360
atcattgttg gtgttgtctg tgttgaacta ttatctaatc aagtcaggag attaatgcga 3420

tgattgaaac accccgtaaa atcgtaacac aacccgaaat cacgccacct acggctaata 3480

aaattttagt ccatccttgg aatcagcgtc gtaaaaccgt cgtcattacc ttctttcttt 3540

tggccgtcat tagcttgtac gaactaatga ccctttcaac aggtaacgtt caattaccga 3600

ccgccttcaa aacgttgggt caaaatatgg ccttaatctt cttgcaaccg agcttaaatg 3660

ggagtgatac gtttccccaa ttactccgcg ctttactcga tagtgtcgcg ctcacgacac 3720

tcacgacctt actgggtgcc atctgttctt tcttcgtcgg cctattagcc gctagcaacc 3780

tttctaaccc gaaaattggg accgccattc gaaccctaat ggcaattatc cgcgcgatcc 3840

cgaccatttt atgggtgcta atcttctcga ttgtgattgg acttggtgct gacgcggccg 3900

taatcggcct aagctttcat agttttgcct atctcacaaa agcctattca gaaagtttcg 3960

aaacattgga caaaggaact atcgaaagtc tcaaagcgat gggcgctagt tggtggcaaa 4020

ttgttttcca agcgatctta cctaccagct ttgccgcttt actctcctgg acattcattc 4080

gttttgaaat caactttact aacgcaattg ccgtgggtgc ggcggccggt gccggtggta 4140

tcggttatca actcttcacc gccagtggct tttactacga tttccacgaa gtcggcgtga 4200

ttgtttacct ctgtttagca gtcactgcct gtttggaatt catttccatt aagcttcaaa 4260

aacgttacct ctcaaattag ccccccatta aaaaacggat tcgctcttca attgaaaagc 4320

gaacccgttt ttatttggtg ctcatgatta aggtgccacg gtcagatgcc agactaattt 4380

agtcttataa gtcgtctttt gtctgacggt ttttcgaggc acatgaagtg caatactgta 4440

atcttgattg ctctgattac caaaccgata ctcgaactcg gaatatcctg gcacatcata 4500

ttgatgatct gcgttgttct 4520

<210> 39
<211> 390
<212> DNA
<213> Lactobacillus sakei

<400> 39
attgtaaaat tacgtttctt acaccagcta aacagttcaa cagctataat ggaaccgaac 60

attgatacga ataacccttt agtcccaaaa tacatcgtat caaccatacc atctttagtg 120

aaatcaactg ctagaattaa aaatcccatt actgccattg tgacacaccc aggaacatcc 180

aatttcttag actgagctag atgataccca ataattgcag cgacatataa cgccatcatc 240

cccatcccga cagtatacgg gatactgata acactagaat attgggcaat ccattgagac 300

acaggattat tcttaccaat catattagga atagcatctg gaattaatgc aaaactacca 360

ataattaaaa taggtgtcat cgccaccatt 390

<210> 40
<211> 1851
<212> DNA
<213> Lactobacillus sakei

<400> 40
gaaaccgtta acgttttaac gttaaccgtt cttatctaga aagttaattt tcgtagggtt 60

cgggtcgttt tttgtttatc gtgatcattc ccgcgcagga tgatcgccgc cattactaaa 120
gtcggcttag caatggcaac actcatgatt taaacgtggg gcaacagacg cttagtcttt 180
gaaccacatt tccacaacct gcaaaattta aggagtgctg cttatgttag aggaacgtaa 240
actcactaaa cgcgattatt tcgtggttag ttcaatgctt tttggtctat tctttggtgc 300
tggtaacttg atttttccat tacatttagg ccagctagcg ggacagaatt gggtgcctgc 360
cgcgcttggc ttcttagtca ctggtgtgtt attgccatta ttatccgttt tagcaatcag 420
tatcactcgt tccgaaggtg tctatgatgt tgggcggcct ttaggtcccg tattcgcgct 480
cttattcatg atcttgatcc acggcaccat cggcccactt tttggtacac cacggacagc 540
caccgtttcc tacacggttg gcttagcgcc actagtacca aagaatttcc aaacagttgg 600
tttattagtt ttctccgctg ttttcttcgg cttagccctc tttttctcat tagaggaaaa 660
taaaatcgtc aacaatatcg gtaagttatt aaatccagtt ttcttagtct tattagctgg 720
cgttttcttc ttagctttta gttcaccaat gggccacgcc ggtgttcaac acgccaccgc 780
tgcttataca tcaggctctt tcgtcaatgg tttcttgcaa ggttataaca cgatggacgc 840
cttagctggc ttagccttcg gggtcaccgt agttaccgcc gttcgcacct taggcaaaaa 900
acgcgctaac agtgtcgcgc tggtcactgc ccgtgccggc gtcttcgcaa tggcaatgat 960
cgccgtcatc tacgttggtt tgatcttcgt tggcgcgatg tccttaggta aactcaaagt 1020
ttccgctaat ggcggcatcg cttttgacca aattgtcaat cattatatgg gcgtcgctgg 1080
tcaggccatc ttagccactt tgatcaccgt cacttgctta accacggcga tcggtttagt 1140
tgcggctttt gcccaagact ttcataaaca ttttcccaaa gttagctacc gcacttggtt 1200
agttctaact actctggcat cattcttgac cgctaatatc ggtttaaatc aaatcatcgc 1260
ttggtcaacg ccaacgttga tgttcttata cccactatca atggtcttga ttttattgtc 1320
agtcgcttca ccactgttcc acaaagaccc agtcgtgtat aaattcgttg ttctctttac 1380
cgttgttcct gcttttttcg acatggtaac tgcttttcca gcggtcgtca gccaaagtag 1440
cttcggtaaa gcgatgactg gcttcgaaca ggcatatttg ccattcgcca actacggctt 1500
agcttggtta gtccctgcct tagtcggtgc cgttttaggg gttgccgtcc acgtcttcaa 1560
acgtgttagc gcccgcaaca ccgtttcaca aactaaatag gtaagtcaaa aaaacgttca 1620
cttgcggtga acgtttttta ttgccttgaa tttatacttt aagtgcaaca aaagagactc 1680
atgatatgaa tccctgtaaa atggtgtttg cgaagacaac catcgaaggg agcacataat 1740
catgagccca tacaaacatc ttaccatgag tgaacgcgaa acgatattcc tgatgcataa 1800
taatcaggct tcactgactt caattgccca aacaattggt cgagcaacgt c 1851

<210> 41
<211> 269
<212> DNA
<213> Lactobacillus sakei

<220>
<221> allele

<222>  (236)..(236)
<223>  A is inserted in some strains

<220>
<221>  allele
<222>  (269)..(269)
<223>  T is deleted in some strains

<400>  41
tttatgggtt cacgatttag aagcaatgaa ggctttttat caaacgatgt ttcaagcgac      60

tgcgggtgag ttgtatgaga aycacaaaaa aggrttccaa tcatactttt taactttygc     120

agatggtgyg cgtttagaaa ttatgcacca agatagtgaa ctagycggct tagcaactag     180

gggccaattt gcgcacttag cgatggctgt tggyagtgaa actgcrgttg aycaattagt     240

gactaagttt caagcacaag gcttaccat                                       269


<210>  42
<211>  5790
<212>  DNA
<213>  Lactobacillus sakei

<400>  42
gtggtattag gccaacgaaa ttgagtgagg atttgcgtat ctcaagagcg gccgtgagcc      60

gaaaattgac gcagctctat tatagcaatt atttgtctaa agaacgcagt ggcgttaatg     120

aagatcagcg cgtcgttaca attgcgttaa cgtcaactgg agaagaggtt gttaagaaga     180

tagataaatt ctataaagct aagctagcaa cactcacaga cccagtagaa gaagtgcagt     240

caactgcaaa aatttttgaa acgttgagtc aaataggtta aattttaata tcatgaaaaa     300

acacccaatt aatgggtgtt ttttatttta gcaaggaaga atggtttaag aagctttttc     360

gatagattaa atcagcatca attaaaattg tttttcgcat tttagtagga aaaataatgt     420

tttctttgag tgtatcaatt gctactttgg cgagttggtc agtatcaatt ctaaacgttg     480

ttagaggtgg tgaaacatac ttaacgagat cgatatcgtt gatactaata attgaagtat     540

ctgtcggtac ggtaatttta ttttcattaa aagcttgtaa aacaccaact gacaataaat     600

cagatgcaat caagaaaccg ttaggtagag gctgactgtc aaggttagca actatcattt     660

tacctagttc atagccagtt ttaacggaaa aatcatcacc aatatagatg gcttctggat     720

gatagatgcc tagttcgcgg gtataacttt caaagtactt ctgacgggga tcctttaatt     780

gggtcagtgc ttggttagca tgccaaaagg aaccgccaat aaaaccaatc agtttaaagt     840

tagttttaat aaataaatca attgcttgtt cagtgattgt ttctagatta ggttgaactg     900

cgttgaagcg atggggatct ggattggcat ctataaaaag gcagttacta gattgatttg     960

caagaacgtc caattcctct gagttaaatt ggccaatggc aattaaacca tctattttat    1020

cggggatatc agtgacattt aaataaaaat caagaataat attagtttga ttactgtaat    1080

gaacaatact ttgccgaaga ttactgtaat aaacgtcttc cagctctttt tgaggagaaa    1140

tagcaaaaat gacacctatg ttataaggat tcttcttaat attactaatt tgttgatagt    1200

taagattgcg agcagtttct agtattttat tacgggcagt gtctgaaatt gaaaatgtcg    1260

ggtcatcgtt taataatctg gagaccgtag caggtgaata accagcttta gcagcaattt    1320

cgcgaattgt cgtcatgaaa atcaactcct gtaaagtaag atgtgtacat aattatgtaa 1380
gcggtaacga taactaaatc atagcatact tttacgaaag ataaactact atattattga 1440
tttgtagcgg tataaacacg gtaaacaaga gttaattttt gtttactatg ttttagtaat 1500
taatttatga aaacgcttgc tgatgatatt tataatcatt atgctaggga tagattaaaa 1560
atgactttca aaaaaggtag gtaaaaatca tatggtaagg gacttaaaaa caaccgtttt 1620
tttacacggt ggggattata atccggaaca atggcaatct cagccagatg ttgtgaaaaa 1680
tgattttaag gcttttgagc aagctaaaat taatacattc tcattaggga tattctcgtg 1740
ggctaagtta gaaccgaaag aagggcagtt tgatttttct tggttggatc aaatatttaa 1800
acaagtagct gatcaaaaag gaaaaataat tctagcaacg cccagtggtg ctagacccag 1860
atggttagcc gagaaatatc ccgaggtact tagggttaac gaacagggcc aacgattaca 1920
atttggggaa agacataatc attgttatac gtcgccgatt tatcgcgaga aagtccaaat 1980
tattaatcgt aagttggcgg aacgctatgg tcatagtgga ttagttattt tatggcatat 2040
ttcaaatgaa tatggtggtc aatgttattg cgaactgtgt cagtcagctt ttagagaatg 2100
gttaaaacgt aagtatcaaa cattagataa tctaaataat gcttattgga atacattttg 2160
gagtcatacg tatacagatt ggcagcaagt gcaagcgcca atgccaaatg gtgatactgg 2220
tgtattaggg ctaaatttag attggcgtaa atttgtaaca gaccaaacaa ttgattttta 2280
tgagtctgaa attcaaccgc taagagagat aaccccagaa atgccagtaa cgactaattt 2340
catgggtggt aatccaccag aatcacacgt gttctatgac ctggattatc agaaatttgc 2400
caaacatgtc gatgttatca gttgggattc ttatcctaat tgggctaatg gctacgaaag 2460
tacggcgcac ttagccatga aaacagcatt gatgaatgat gtgatgcgcg gtcttaaaca 2520
atcaaattat ttaattatgg aaagtacacc ctcacaagta aattggcatc cttacaatcg 2580
ttcaaaacgc ccaggcatgc atgaaatggc tagtttacag gaaattgcgc atggcgcaga 2640
ttcaatacta tactttcaac ttcaccaatc attaggggct tcggaaatgt ttcatggtgc 2700
tgtgattagt aatcaagtgg aaacaaaagg tagagttttc cgggatgtta tgaaagtagg 2760
acaggattta gaacaattaa aaacagctaa aaatacaaaa tatcaagcag caaaagtagc 2820
aattgttttt gattacgcta atatgtgggc gttagatgat gctcgtaatt acgcggatga 2880
aacgaagaaa tattggcgga caatcagcga acattatcgc tatttctggg aaaatgatat 2940
tgccgtggac attatctcga ctgaacagga tttaacggat tatcaattgg tcattgatcc 3000
aatgcacttt ttaatgagta atgactatgc tgaaaaatta gcagcatatg tgaagcaagg 3060
tggacagctt gtcggaactt atataacaag tgttgtggat gtgtcctttt taacacacca 3120
aggaggctgg ccacaagcct tgaaagaaag ttatgggatt acaattcaag agactgatac 3180
actctatcca caacaatcaa atcatgtatt gattggaaat aaaagttatc aagcaaagga 3240
ttattgtgaa gttgtatcag tagtggatgc agagactatt ggcacttatg aaaatgattt 3300
ttataaagga acaccggcgc ttacgaaaaa cagctttggt aaaggaatag cctattatca 3360

agcttgtcgc ctagaggatg attttttaga tgatttctac gccaaaataa cacgaaaatt 3420
aggactcaaa gcgcaattac cattaaaaga aacgaaccct accgtttcta ttcaagttag 3480
agaagacaag catgatcggt attattttat gattaatttt agccatgaac ctattacatt 3540
agaagtaacg agagacttgg tggacgtatt gacgtctgat cataaaatta caaacgggcc 3600
tattaaacta agcgcttatg aggccagagt cttttcagag gcaatcaaat aaaaaataga 3660
ttagggaggt attaagatgg aaaaacagca aacggctaag aaaaagttaa cgtggccaca 3720
gttaaaggaa cgaattgcat ttgcattggg gaatttgggg cattccgcct tttatggggc 3780
attaagtacg tattttattg tttatgtcac tagtgggatg tttacggatc ttagtcccaa 3840
agtggctaat cggttgattg gtttaatcac cacactagtc gtgattatcc gactggcaga 3900
agtcgtcgtt gatccaatcc tggggaatat tgttgataat acagagactc gttggggaaa 3960
gtttaaacct tggcaagtga tagggagcgt tgtcagtgca attttacttg ttattatttt 4020
tacagggatt tttggattag ctaaggtaaa ttggctcctc tttgcaatag cgtttgtaat 4080
catttttatt attttagatg tattctattc ctttgccgat gtggcctatt ggggcatggt 4140
gcccgctatc agtggagata gtaaagaacg tggtattttt acagcattgg gtagttttac 4200
aggctctatt ggttggaacg ggttaacgat gattgttgtt ccgattacga catactttac 4260
ttacctggca acaggaaagc atgatcaagg gcctcaaggc tggttagcct ttgcgattat 4320
tgtttcaata attgcagtcc tttcagcgtt agcagttgcg tttggcacaa cggaaaaaaa 4380
tgacgttatt cggcaggcgg ctagtcaaaa aacaacaatt aaggatgttt ttgtaggaat 4440
cgccaaaaat gatcagattc tttggattag ctgtgcttat ctactatatt cacttgctta 4500
cgttgttaca aatggggttt tattctatct atttaaattt gtgattggac aacctggtga 4560
attctggata gctggcgcag tggcaacaat tgtgggcttc tcaacagcgc cattatatcc 4620
acttttaaat aaatttattc cacgtaaaat gttattctta gttggacaaa tttcgatgat 4680
tccatcatat attatcttta tttttgctag tacaaatatg gcattattaa ttgtcggctt 4740
agtcctcttt aatttcactt ttgcacagct cgtaattgtt ttatcgttaa cagatgcgat 4800
tgaatatggt caattaagaa atggtaatcg taatgaagca gttgtcttgg ctgtacgtcc 4860
aatgctggat aagattacag gtgcattttc aaatgggatt gttggaacga tagcgattat 4920
ggcaggaatg actgggagtg caacggccgc tgatatgacg gcaaaaaata ttcacacttt 4980
tgagctattc gccttctaca caccattgat ttcatcaatt ttagcgttaa ttgtatttat 5040
gtttaaggtt aaaattactg aaaagaaaca tgctgaaatt gtgactgagc tagagaatcg 5100
tttagcaaaa ggccacttac ctagtgataa tgataaggtt ttgacgggca ttacgactaa 5160
aattctagcg cctgttgatg gacagttatt aacgttagat gatgttccaa atggttttga 5220
ggggaaaggc tttgcaattc aaccaacaga tggacatatt tacgcaccat ttgacggaca 5280
tattaaattt actttttcga cgcggcatac attaggaatt gtctcggata atggcctgga 5340
gatgattatc catgttggtt tggagaccgt taatatgcga ggtgagggat ttgtcactca 5400
ttatgttgac ggccaaaaga tctcggcagg ggatttgcta ctcagttttg atcaacaact 5460

aattcacgaa gcaggttatg atgacattgt gattaatttc tttacacaac ctaaacaaat 5520

gagaacgttg gagaacttac aggctaaatc ggtcactcat ggggaatcga ttttagaagt 5580

aatcatgatg gaaaaatagt gataataaaa acgagttaac ctattagggt taactcgttt 5640

ttttaatact gatttatttt tgtttagcaa gattcgccca aaatcaactt aggcgtaata 5700

tctggtagtg aggtttgaag gctttgcttt tctaaagtag caatcaataa ctgtgcggct 5760

gagttactga gggcttcaat attatggtca 5790

<210> 43
<211> 6346
<212> DNA
<213> Lactobacillus sakei

<400> 43
attgttcttt atatttttta tagaaaaaag ttttatttgt aatgccaact tgctccataa 60

tatctcggat aggttggtta gtagacgtta gtagtaatcg ggcgttaatt aaacgttggt 120

aggttaataa acctgaaaat gatttccctg ttttattttt taaaaggttg cttagataat 180

ttttattata acctagcttg gcagcctctt tagttagtgt tatttcacga taatggtgtt 240

caatatcatg taaaacctca gtgactgtct tttgagagga gcttagtgta ctagttggtt 300

taatctgata atttctaaca agttcggtta ataaaatttg aaggtaagat gaaattactt 360

cgttatagaa aggacgtttt ttgacgtatt cattaactag attttcgatc acagagttta 420

tttgggagtg attattatgt ttgaagagga tgaaatggtt aatattttct tccccgtaac 480

tacgattaag gagtagtgaa taaagtaggc tttggctttt tttaagatta tttaaccagt 540

caattgaaat attagtattt tgaaaaagaa tattaatcat aatatcgtta gtgtttagtt 600

tttttatagt gtgtgtacag ccaatatcaa ttaataataa atcgtgttgg tgtaagagaa 660

tctcacgacc atcaacagtt tgatgacagt taccagatac catgtaattt atctctaaaa 720

attgatgtgt atgtgcggga taatctgcat aacgattatg tttactaata taaatatcct 780

tattagcgaa aaaatattta ttttgaagcg taaaatcaga agatttatta agacgtgcat 840

atgaaggaat atccgcaata taatggttat tttttttctg ctctttttcg atggttgttt 900

cagccgttaa aagattaaga atactagagt tcataatatt tccccctgtg aaaatgatac 960

tttagttctc tttaaatgac attgtaacat tttgagcaat gaagttaaat ataacttgta 1020

agcgatttct aaaatgatat taggagatga gatctatgcc agaaaaaagt aagcttaaat 1080

ccgtaatagc cgtttcgtta acaaactatt tagattcagg ctcaattgtt gctggtgcaa 1140

gtggacttgc attatggaca aaagcattag gacttagtag ttttgaagtc ggattgttag 1200

gggctctaag tgcgaatgct tttggtgccg ctgtgggtgc tttaattggc ggtcgattat 1260

ctgataaata tggtcgtaaa tttatctata cgtacaatat gttggtttat atgttaggcg 1320

tcttaattgt tatggtgtca tttaactttc cgatgttatt gacaggcttt atggtaactg 1380

ggatatctgt gggtgctggt gtaccagcat catggacgta tattgcagaa acctctcaat 1440

cgagcaatcg ggcacataat atcgggatat cacaatttgc ttggtcgatg ggaccagcta 1500

ttatttttgt actaggtgta gttttatcac ctcttggcct tttaggtaat cgaattattt 1560
ttggctcgtt gtttgtagtt gcgctcattg catggatgat gcaaagggga ttggaagagt 1620
cttccgattg gcaagaacaa aagcaaaaag aaaatgctag tggtattaaa ccgcatcctt 1680
ataaagaatt attctctaat atcgttaacg ttaaaacgat tttatattta atcggggttt 1740
atgcattgtg gaatttagca gcaggtgttc aaggattttt catgccatac atctattcaa 1800
ctgttggtgg tttaacaaat atgcaagcta acttattatc agcggtcgtt tggattttaa 1860
cttcaatgac tggatatttc atctttgcta aattgggcga tcgcgttagt catcgattaa 1920
tgtacggtat aggtgcctta atggcaatgt tagcttgggt tttaattacc tttggcggta 1980
tgggatggac tactctatgg ggatttgtat tactttgggg actatctgca ggatttggtg 2040
cacaagcttt ctacgcatta tgggcaacag agttattccc aacacgttat cgtgcaggtt 2100
ctcaaggtgt aatgtttttt gttgttagag cttcagctgg tatttggtcg attattttcc 2160
caccaataat ggcttctcta ggatttaaat acgctggcac tatgattgtt atctttttag 2220
caatttcatt agtgattggt gttatctggg caccacatac acgtggtaaa tcattagacg 2280
aaataacaga agaacggtat ggttcaaaaa aataatgtga atcaaatact caagagtatt 2340
atgcgttagg agtgatttat ttgaaaaatt atattgcagt tgatattggg gcttctagtg 2400
gacgattgat gttgggtcaa ctagtagatc aaaaattaga acttaaagag ttgcatcggt 2460
tttcgaatgg attcatcaaa gaaaatggcc atgatcggtg ggatattgac cgattaattg 2520
atgaaatatt taaaggctta gaaatcgcaa aaaaacaagg catcgaaaac gcagttttag 2580
gaattgatac atgggcagtc gattatgtat taattggcga tgatggacgg aaactacaag 2640
atccatgtag ttatcgtgat agtcgtactg ctaatagtat tcaagaatta acatctgagt 2700
attctaaaga atatatttat aaaaaaacgg gcattcaatt tttggatttt aatacactat 2760
accagttata tgaagaagat aatgagttac ttgataaaac tgatcgaatt atgatgatac 2820
cagattatat tggttatgtt ttaacgggaa acgcagttac ggaagtaacg aatgcctcta 2880
caacacaaat gatgaatctt cgtcaaggat tatttgatga tgatttatta aagaaggtaa 2940
atgtaactga tgatcaattt ccaaaattag tagatgcagg aacaaaatta ggaaaattgt 3000
cacataaatg gtatcgacaa tatgatatac caaaatgtga agttgtaacg acaacaacac 3060
acgatacagc ttctgccgtc gttggaacac ccggaaaagg tgatgactgg gcatttttga 3120
gttcaggaac atggtcactg cttggtcgag agttggatgt acctgaaaat ggtgaagaag 3180
catttaaaaa aaattataca aatgaatggg gagcatacgg tacttatcga tttttaaaaa 3240
atatcatggg actttggatt attcagagta ttcgaaaaga acaaaataat aaatttagtt 3300
ttgcagaaat ggctgaagca gctgaaaagg ttgaaccatt tcaacaattt attgatatta 3360
atgatgaacg ttttaaaaat ccaagcagca tgattgatga aatcaaggca tattgtcagg 3420
aaacgaaaca aaaggtccct gatacaacag gtgaattagt aaacactgtt tactcaaatc 3480
tagcgctatt ttacgcgaac gaactacaaa aattagcgcg gattactaag aaaccaatta 3540

acgttctaaa tattgttggt ggtggtagca atgtagcatt cttaaaccaa ctaacaagtg 3600
atttggcgtc agtgagagtc gaagctggcc ctggtgaggc aactgcaatt ggtaatattc 3660
tagttcagat gattgcgcta ggtgatataa aaaatgtatc tgctgcaagg tcaattgtaa 3720
gcgcttctaa tggaattaga cattttgttc ctcgtgaaaa ttattatgga gaagtgttag 3780
aagcatatca aaattatatt aatcgaaaaa gtgagggaac aggcgaatga aacgaattgc 3840
acagattatg tatgtttatc cggatcaata tgatgaatat gaacgacgtc ataatgaact 3900
atggccagaa atgaaggaag cattaaagaa atatggtgcg accaattatt ctatcttttt 3960
aaatcgtgaa aatggtgagt tatttgctta tcttgaagtt tcgaatgtag atcaatataa 4020
caaaatagct aatacagata tttgcaaaaa atggtgggtt tacatggaac cacttatgaa 4080
aacaaaccca aacagtagtc ctgtagcact agacttaaat gaagtattcc acttagcgta 4140
aaaatatagg aggtttttga aatgaataaa gaagaaaatg ttaaaaagag ctatgaatta 4200
gcaaaagaac gttatgctgc attaggtgtt gatacagaaa aggcattaga agacttagct 4260
aaggtaaaat tatcaattca ttgttggcaa ggtgatgata ttcatggatt cctattccct 4320
aaccaagaat tgacaggcgg tattggtgtt agcgggaatt atccaggatt agcaacaacc 4380
ccagatcaat tgagttcaga tttatctgaa ggcctatcat tgattccagg gaagcataaa 4440
gtacaattac acgctactta tgctgtaacg gataagccaa aagatttaga tgaattagaa 4500
ccagaagatt ccaaatattg ggttgactgg gctaaaaaag agggtgttgg tcttgacatg 4560
aacgggacat tcttctcaca cccaatgttg aaagatggct tcactttagc aagcccagat 4620
aaaaatgtac gcgatttttg gattgaacat ggtaaacgaa gccgtagaat tgctgaatat 4680
ttcggtaaag aattaggaca acaatctgtt aataattttt gggtgccaga tggttataaa 4740
gataatccaa ttgataaaat atcaccacgt cgccgtttaa tggcttcatt agacgaaatc 4800
attaaagaac caatggatga aaaaaataca attgaagctt tcgaaggtaa attgtttgga 4860
actggtgttg agtcatacac tgttggctca catgaattct atgaaaatta tgctttaaca 4920
cgtcaaaaat tatggacaat tgatgctggt cacttccatc caacagaaga tgtttcagat 4980
aagttttcgg cattcttacc atttggtaaa ggtttaatgc ttcatgtttc acgtccagta 5040
cgttgggata gtgatcatgt agttatcttt gatgatgctt taatccgaat tatgcgttca 5100
ctggttagag atgacttatt agggcgtact aatattgggt tagatttctt tgatgcaact 5160
attaatcgtg tttctgcatg ggtcgttggt gcgcgtgctt ctcaaaaagc tattctacaa 5220
gcaatgttgg caccaattga tgatttaaag aaagcagaag aacaaggcga ctatacaaaa 5280
cgtctagcag tgacagaaga attaaaatca tatccatttg gtgctgtttg ggatgaattc 5340
tgtgttagaa ataatgtacc agttggagaa gattggttgg ataatattaa agattatgaa 5400
aataacgttc agttcaaacg ctaatcaaag gggattataa aatgacgaag tttatagatt 5460
caccatttgt tgaagaagta aaaaaaatta cgtatcgtct atttgatcat ggatgggacg 5520
agcgcaacgg tggtaatgtt agctatcgaa ttttacctga agaattaaca ggatacacag 5580
atattgaaac cgttaaacgg accatcccaa ttgattttaa tggtaaagat ttagcaggca 5640

tgtatttctt agtaacagca acaggaagat attttaaaaa tattattgat tttccagaac 5700

gtgatttagg actagttaga attaatgaag atggtactag tgttgactta atgtggggat 5760

attcagatgg tgctgcacct actagtgaat ttccaacaca tctaatggga catcaagtac 5820

gtttaaaaat ggataataat caaagagttg tcatgcactg tcatccaaca aacctaatag 5880

ccatgacatt tacacaaaag ttagatgaac gtgaaatgag tcgtatctta tggaagatgc 5940

aaacagagtc attagttgtt ttcccagaag gcattggtat tattccttgg atggtaccag 6000

gaacggttaa gataggggaa gaaacagcgc ataaattaga aacttatcga accgttattt 6060

ggccacaaca tggtattttt gcgacaggta gcacaatcga tgaagcattt ggattaattg 6120

aaacaattga aaaagctgct caaatttgga ccattattca aacccaaggc ggacatattg 6180

aacaaacaat cacggatcaa gatttaaaag acttagcaca ggcgtttaac gttacccctc 6240

atgaaggtta tttggactaa atttattggg taggaaggta tttaaaatga aaattaaagc 6300

tgcagttgtt gaagaaaaag gtggaccctt tgtcattaaa gataat 6346

<210> 44
<211> 1984
<212> DNA
<213> Lactobacillus sakei

<400> 44
tacatctatt ttgcagcaac cgatacgacc gatattgtaa acggtttatt cacacataga 60

atgtattgta ttgaatgcgc tgcagataat cctatgaact ccgaagatga ttgggttgaa 120

aaaggacaaa tcgtcactga taaagataca ttttgcttag atgccacaac atttgaacat 180

aacaacaagc tttattatgt ctgggcacaa aaggacaacg atatccgcgg taattctaat 240

ttatatattg ctgaaatggc aaacccctgg accctaaaaa caaaaccagt catgctttca 300

aaaccagagt tcgattggga aacacggggg ttctgggtta atgaaggacc tgccattttg 360

aaacgtaatg gcaaaatttt cttaacttat tcagcaagtg ctactgatga aaattattgt 420

atgggtatgc taactgttga tgattctgcc gacctactcg atcctactaa ctggcataaa 480

gaagctgaac ccgtgtttgc tagtgattta acaactgacc aatttggccc tggccataac 540

tcattcacta tttcagaaga cggcttaacc gatattcttg tttatcattg ccgcgactac 600

actgatatca aaggtgaccc actttatgat ccaaatcgcc atactaaagt tcagccattt 660

tcatgggata aatcaggcat gccacgtttc gataaacccg ttccttacaa ttacaaataa 720

agggagatta ttagtatgaa tgcaattcat aaagaaaata aacacttttg gagtttcgca 780

agtactcatt tcacctactt ctttatctgg gctattgttt acggctattt aacactttgg 840

ttagaacaag tcgctcattt aaatgggact gaatcaggct ttatcttttc atttatggcc 900

ggtatttcgt tgttcttcca gccagtattc ggaattttat ctgataaact agtcttcaaa 960

aagaacttat tagtgaccat tacattagcc gctattttgg tcggaccttt cttccaatgg 1020

ttattcttgc caatcgtcca taccagttca ttcttagctg ctattattgc tggtttatat 1080

ctcagtttta tcttaaatgg tggcgttggc gttgttgaac aatacgttga acgtgctagt 1140

ttagtcaata aatttgaata cggccactca cgaatgggtg gttcactagc tggtatgaca 1200

gcatccctta ttgccggtcg cttattcatc tggcaaccta actcaatttt ctgggcatgt 1260

tctgtttccg ccgttatcct aacttgtctt gttctattca gtgataaagt caacgttagt 1320

gatttacaat ccaatccaga tactgattca gacaatgaat cagctccgtt agataaaaaa 1380

acaattcttt ctatctttaa acttaaaaat ttctgggtac tttcattatt ctacatgggt 1440

gccgctgcaa tctatgatgt ttttgatcaa cagttcattg tttacttcac aacgttcttt 1500

aaatctgcta gtcaagggac cattacctat agttacatgg cttcagccca aactgctatt 1560

gaattactat tgatgattcc aatgccattt attatcaaca aaatcggtgc taaaaaaggc 1620

ctaattgcat ttggttttat tacattcatc cgtattttcg ggagtgcaat tgcaccaaac 1680

tgggaattat tagtcttctt tagattatta gctggtttag aaatgccatt actcttggtt 1740

tcaatcatga aatatatcac cggcgcattt gatattcgtt tgtatgcaac ggtttactta 1800

atggcttcca attttgctaa acaaatttca gttttcatct tctcaacagt tgccggtaaa 1860

ttatacgatg tcgttggttt tcaaaaaaca tacttcatcc tttcagctat cgtttttgtc 1920

attactttag tcgcagcgtt catgctcgaa aaagaacacc cacttgttga acaaactact 1980

ggtc 1984

<210> 45
<211> 2511
<212> DNA
<213> Lactobacillus sakei

<400> 45
atgaaaaaaa taatgaagtt agtaatattc ggtattttga tagcgctatt attggtgatt 60

tatgtagaga atgtgacggc cggacagaga gaaaccgcca cgacccagaa gcaatttacg 120

aaaacacaaa cgattttaaa aaatacaacg agccaagcaa atcagaaaag taatactcag 180

aaaatgtcgc cggaaacggc tgaagcagtc ggcgataaaa agctcgccaa agctatgaca 240

attgatgaag gtgttcagaa ttgggcggta gtaccgcagt caacgattgc ctatgtcgac 300

ttgggtaacg gtcgtcgatt aggctacact tttggtaaag cgagtgtgag tcggtcagat 360

cgttcgattt taaaattggg gcaaacacct aaggccaata tctttataaa taataatggt 420

cggatagatt ctgccttctt tggggctaaa caaaatttga ttacgtcatt tgattatgca 480

attcatcccg atcggtcaaa taaaaatcca tccatgataa atgaatcaat tggtgagcgg 540

ttaaaatcat atagcatcta taaagggtca accagtgatg gtagtattgc actcaaagcc 600

gttggtgcag ttgaagttaa taagaaaaat tatgaagcaa cgttattaat gcgtcccaat 660

cctgggaaag ccgttattca acaagagcta tatctggcga gtaaagagga tacgaaacat 720

gaggccggca tttatttagc gaaggattta ttggtggatt atgacgataa tattccggtt 780

tattcacaag gtaataatgc tggattatat ctgcaaagta agaaatacaa agtttttatc 840

aatgttaaag tgccggatgg tccgcaacac tacggtactg gtagttatct tgattcatat 900

agttatgcct tatcgccata taaaggaacg gattttgata gccctggact agaaagcgat 960

aatctaaaag agggtactgc tgtttttaaa aagctggatt ctacgtacag taataagtgg 1020

tcttggcaaa cttttgagcc gaacactgta tatcattatc gttctgatat tggcttgacc 1080

gaatcaggtg tggttagccc ggatgcggtt attaaatata caaataagac cagtgacagc 1140

ggaaagaatc atgtgaacga tcagctgaca gttgaatcga tggcgcataa taatggttat 1200

aacagtgact ggcaaggcgt gacggttagg gtcgacgttc ctaaaggctt aacaatcgat 1260

tcggactcga ttaagttgaa attagctgat gagacaatcc aggcggttga tgcgagtcat 1320

tatgatgcac agactggtca agtgagtgtt aaattgccga aagctttggc ggaaaatgaa 1380

tggacaagcg ttctgtacga tgcccaaatc aataaaagtg cggcgggtac gacgatgacg 1440

ccgaaaatga tcgctaaggg tgaaaatggc tttcctaacg aagcatcaga tgacgttcag 1500

attccggttc aagatcaacc aaccgccatc gaaaaaatgg tacgtaatgc agcagatccg 1560

gatggtgatt acggacaaac ggcggaaagt gcggtcaagg gagtacttga ttaccgtgtt 1620

gttctgaagg ttaaccggtc tgatggtggt cttgtagaag gaacgctcag cgatgaactg 1680

ccaaagggat taacgctagt tcctaattcg gcaactataa aatacggaca gcagacaact 1740

acgaaatcag ttagtgacat cgggaaaatt agtctggaat caatggtagc aggtgaaaca 1800

gcaaccttaa cttatcgggc acaggtcgct gatacggttg atgacggagc cttattaaag 1860

aatacggcac aggtgactgg taaagaagtc aatggcactg cgctaacgat tgatgccagt 1920

gcttctgtga agattaccaa acctaaaatc ggaaaggtaa tcttcaaata tcttgatcgg 1980

gctactggac agccaatcgg cgatcagcag attacaatta ccggaccaat cggtaaacgt 2040

ctttcagaag ttaagtcgga tgaaattcaa gtcagtggtg ggcaaaatat taatcgaatc 2100

cgaccagctt atatcgatag ttatgtgccg gtcgacttta ccgccgatgg taatgccagt 2160

gtgccgactt ttgataacca tgttcgggat gttgatccgg tctttgatga gaccgagaca 2220

gtctatcttt tcaggtatga gaagcagcga ctcgaaataa tggcccaccc agtgaaattc 2280

tcatttggaa aattctcgaa tacacaggct gaacgtacgt attatctacc aacggctaag 2340

gatcaaaatg ggcagaaaaa gccatattcg attggaattg cggattactg gggaatcaat 2400

agttggcaat taacggtgag ccaacctgag caatttgaag gtacctatct cgatcaagat 2460

aagaaaccac agcgcacagc tctcgacgac gcccgactga tttttaataa t 2511

<210> 46
<211> 2757
<212> DNA
<213> Lactobacillus sakei

<400> 46
tacattgcca aaaggaactg ctagtccgat tggtatgctg atttacaacg ttttaggttt 60

aattatcttt atgggatttg cttggatatt ctgggcacgt tacgaaaaaa aacaaaaaaa 120

ggtggctgaa tcataatgaa attatgggaa aattatcaac tcgatagtat taatcgtgaa 180

ccggcgcgtg cacacttttt aacatttcca tctaaagaag cggcgcaact caatcaaaat 240

aagtacacac atgctttcaa aaatcttaac ggcaattggc aattcttatt cttacaggcc 300

cctgaatata gccctaacaa cttcttcaaa cctgactttg atacttcagg gatgacaact 360
atcaccgttc caggttcatg gcaagtacaa ggatttggta agatgcacta ttctgatttg 420
tggtataact tccccatcaa tccgccgttt gtaccaacga ttaacccaac tggcatttac 480
aaacggtctt ttacaattga tgaatccttc agcaatcaaa aaatcatttt gcgttttaac 540
ggtgttgatt cagcctttga ggtctgggtc aatggcgaat cggttggtta tagtaaaggt 600
gcccgcaatg aggcagagtt tgatatcact gatcttgcta aaatcaatca aacaaacgac 660
gttactgtcc gcgtttatca atggtctgat ggtacttatt tggaagatca agatatgtgg 720
tggctcagtg gtatttatcg ggatgttgaa ttactaggtg tccctcaaaa cggtatcaat 780
gacctaaccg tcgttgcgga tttagatgct agttaccaaa atggcttatt atctgtggaa 840
acgacattcc gccaacaaac cgatcaaact gttacttttg aacttcggaa tcctgctggt 900
aaagtcgttt tagaggctag ccaacaagtt actaatgcaa aagcgaccct cgctgctact 960
attgatgccg ttgatgcctg gactgctgaa acgccaaatt gttatgattt aacgattagt 1020
atttatcaag ctgatcaatt aattgaagtc atcccacaaa cagttggctt tagaaatatt 1080
catgtcgaag gtgaaacgtt cctagtcaac ggtgtcgcta ttaaattcaa aggtgttaat 1140
cgccatgact ataaccccca taatggccgt gttgtttcta aggaagaaat tgaacaagat 1200
attattctca tgaagcaatt taatatcaat gcgattcgca ctagccacta ccctaatgcc 1260
tattatctct atgatttatg cgaccaatat ggcatgtatc tgattgctga agctgacgtt 1320
gagtgtcatg gctttgagtt aactcagaaa tacgattgga tcactgatga tccacaatgg 1380
gaaacagctt atgtatcacg tttaacacgt atgattagcc gtgaaaaaaa tcatccttca 1440
attctgttct ggtcacttgg caatgaatcg gattttggtc ataattttaa aagaatggcc 1500
gctgtcgcaa aagaaatgga cccaactcgc ttagtgcatt atgaaggcga ttttgaagct 1560
gaagttgttg atgtgtattc cacaatgtat acctggattg aacatcctac taaaaagcta 1620
ctcatgaaag acatcatcga aaatagtaaa aaacctcatc tcttatgcga atatgcccat 1680
gcaatgggca atggccccgg taacttaaag gattatcaag atttattcta cgctcacgat 1740
aaactccaag gcggttttat ctgggaatgg ttcgatcacg gtattgagtc gcagactgca 1800
tctggtgaga cctattaccg ctatggtggt gattttggtg atgaccccag caatcgtgac 1860
ttctgtattg atgggctcct aatgcctgat agaaccccat ctcctggctt atatgaatac 1920
aaaaaagtca ttgaacctat cactacatct gttattaatt tagatgctgg agaattcgag 1980
ttacttagtc gatttgattt tgctaacctc aatcaattcc aactcgttta tactgtgatg 2040
gcagatgata gtattcttca aagcggcacc ttagcactcc catcaattcc agcccgccaa 2100
aaagcaacga ttcaagtacc aattgatctc gactttactg ctcatcccgg cacaacctac 2160
ctattgaatt tctcttatca attagcaagc aatacccctt acgcaccagc tggtcacgaa 2220
gttgcaacgg ctcaatttga actaccaatt catcaaatcg gacttcctat taaaccggtt 2280
ggaacactca caactcagga agatggtgca tttcttcaag taactgggac caacttctcc 2340

ctcacttttg ataaggtact aggtgccatt acgagcttaa cacgcgacgg tatcgaactc 2400

atgtcaaaag ggccacacct cactttctgg cgagcaccaa tcagcaatga tatggaaatc 2460

gtggatgaga tgaagaaaaa acaattcctt cacttagaac atgaaaccgt tcaaaatttt 2520

gactaccatc aaaatggcca acaactgatt gtgactgttg agacattaaa cggaacaacc 2580

aatagcgctt ggcattacga ttgccgttat gaatacacca tctatccaag tggcgatatt 2640

caattcaagc tcagtggcat accgggtggc aaattagata tggcacctga aatgcttcca 2700

agattaggcg ttgtcttaaa tctgaaccaa tcactcgaac aagtccgcta cttcgga 2757

<210> 47
<211> 2927
<212> DNA
<213> Lactobacillus sakei

<400> 47
atgttgatac tgatattaat aagttctgta attattaatg agaacgttgt caacgcagtc 60

caaagacagg ataattcatc agaaatcaag gtgccaacag attcaaaaac aagtagtcac 120

tcagaacaat cagtgagaac ccaatccaaa tctgaatcta agtctgaatc taaaacgacg 180

tcatctgaaa agcattcttc tgagaagaca acacagtcct caagtagtca tgagggtaag 240

tctgtgattc agaatcaatc acgagataca ataatgccga cagctactat tgatgatggc 300

gtgacaggtt ggcaaccggc aaagcaaaca acgattgcaa gcattgatat tgataaagcg 360

gctaataaaa gccttaatta tacttttggt aatagtgatg ggaccttaaa gtttgatcaa 420

ggaaatggtg ggataataac gaataacatg ccgccaagaa tgaacgtttt cattaaggat 480

aatggcaaga ttgtcgagtc ttcattcaaa tcaacatctg ggatgggggc tcgtatgact 540

attcactctt aaccactaga agttccacgg caccaacgat taaaaccaca tcgacatcgg 600

cacagggatc actgacaggt gtgcaatatt atcaagccaa agacgccaaa gggcagacaa 660

ttttaaaaat ggtggggcaa tatacaacca tagtggggcg tgtccccagt ttcacacttg 720

agtatctatt acggccgtca ctagaaaaac catctgttca acaggaacta tatatttata 780

atgcgacatc ggcagcaatt gatggcggga tcttttttgc taaagatact tcgttaaatg 840

gtaacgatgg cataccagtt tctgcgcaag ggaataacgc tgggatgtac attgccgatg 900

gtaaatataa actattcctt aatatgggtg ttgaggatgg tccagccaaa tttaacgcga 960

tgaattatca ccgaaatggt ggatataact tatctgatta tgcaatgaat gattattcgc 1020

cagccaactt tgatggtacg ggtatcgaag ttaaaaatct aaaagaaggg gctactgttt 1080

attcaggaag tgattctgcg tatagcgcta agtggaatta gcaaactttt cagccagata 1140

ccgtttatca ttttagaaat gatttaggga ttgcgactgc gggcttggtt gtcccagaag 1200

cggcagagaa ctataaaaat aaaacgacaa gtgatcaaaa aaatcacgtt caagataata 1260

ttactatcga aatgaaagcg cataatttag gctatagtag tgaatggaaa gatattaacg 1320

taacgtcaaa aataccagat gaactggata ttgatcctag tacaattaaa gtaactctga 1380

ataatggcga tcaagtagca attgatgcta gtaagtataa ccaaacgact agaatgttaa 1440

cggttagatt acttaaaaat ttgtccgaaa atgagtggtc atcgatcagt ttcgatgcca 1500

aaattaatgg taaagcttct ggaaaaacaa ttaatagtag catggttgct aagactgaag 1560

gtgggactgg cagttttgat acggctacgg cgagcacgaa aatcccagtc gaagttgaac 1620

ctgtcgaaat tgaaaagaaa gttttaaatg aaactgaaaa agaaacgacg taccaaaaag 1680

cgacggtcgg gcatgtaaat gatgttcttg attaccaagt gaagttaaca gtcagcagta 1740

acggagctga tatgtctaac ggtgtcttgg ccgatgacct agctaaggaa gggttagaat 1800

tagtgccggg ctctgttaaa ttgacatata gcgataagac agttgaaacc ccagcagatg 1860

ttaaacagat taatttgaaa aaaatgacac ctggacagaa tgtagtatta acctataagg 1920

ctaaagtcaa agaaggtgtt gtgataggca cagtgcttaa aaatattgtt ttatattctg 1980

gggaacaagc taatcagggt gttatgaacg ggcaagctga cgcgtcagta acgattgaaa 2040

agacgaagaa tagtgacgtt cactttagtt atattgatcg tgaaacgggg cagcagattg 2100

ctaatgaagt cgttgcaact gggccaatta atgccaaaat atcagcatta aaagcaactg 2160

atattagcga tgggcaagat cctaataaga ttcggcccgc ctacattgaa ggttatacgc 2220

cagttgattt tacgacggca actgatttaa acgctgcggt ctatgcggat attaaagatg 2280

ttgatccagt aattgaagaa aaatcggtga cctatacttt tagatatgaa aaaacgcgct 2340

tagcgattac ggcgttacca agtaaactga attttggtaa attcgacgat acgcaaagtg 2400

aacggacgtt ttatttgccg gcacaaattg agaaaccagc tgatgaaaaa acaccatatg 2460

gcattgaaat ctctgattat tggggcatta agggctggac gttgagtgtt gcacaggaac 2520

aacaatttca tggcagtgcc acgatagatt taaaggaaca agtagttgag ctgtccggcg 2580

cacaactcca gtttaataac gcaacattga gcacgcatac ggaaggtaat aagtctgttt 2640

cgaatttgca ggataaagta gtaactaaat caaactttaa cttggtaccc ggggctgagc 2700

cgttagaatt agttgaatac gaacgaaaag gccagtacct taatcaagac ggcgacaata 2760

aaggcggggt cagttatgat atccctggct attcagttca taaatatcaa tttggtgacg 2820

caagaacggc tgattacagt attggcctgc atgtcccaga aacgactgaa cgttaccgta 2880

cagaatacac atcaacattg aagtggcatt taacggttgc gccataa 2927

<210> 48
<211> 2927
<212> DNA
<213> Lactobacillus sakei

<400> 48
atgttgatac tgatattaat aagttctgta attattaatg agaacgttgt caacgcagtc 60

caaagacagg ataattcatc agaaatcaag gtgccaacag attcaaaaac aagtagtcac 120

tcagaacaat cagtgagtac ccaatccaaa tctgaatcta agtctgaatc taaaacgacg 180

tcatctgaaa agtcttcttc tgagacgaca aagcagtcat cgagtagtca tcgagataag 240

tctgtgattc agaatcaatc acgagataca ataatgccga cagctactat tgatgatggc 300

gtgacaggtt ggcaaccggc aaagcaaaca acgattgcaa gtattgatat tgataaagcg 360

gctaataaaa gccttaatta tacttttggt aatagtgatg ggaccttaaa gtttgatcaa 420
ggaaatggtg ggataataac gaataacatg ccgccaagaa tgaacgtttt cattaaggat 480
aatggcaaga ttgtcgagtc ttcattcaaa tcaacatctg ggatgggggc tcgtatgact 540
attcactctt aaccactaga agttccacgg caccaacgat taaaaccaca tcgacatcgg 600
cacagggatc actgacaggt gtgcaatatt atcaagccaa agacgccaaa gggcagacaa 660
ttttaaaaat ggtggggcaa tatacaacca tagtggggcg tgtccccagt ttcacacttg 720
agtatctatt acggccgtca ctagaaaaac catctgttca acaggaacta tatatttata 780
atgcgacatc ggcagcaatt gatggcggga tcttttttgc taaagatact tcgttaaatg 840
gtaacgatgg cgtaccagtt tctgcgcaag ggaataacgc tgggatgtac attgccgatg 900
gtaaatataa actattcctt aatatgggtg ttgaggatgg tccagccaaa tttaacgcga 960
tgaattatca ccgaaatggt ggatataact tatctgatta tgcaatgaat gattattcgc 1020
cagccaactt tgatggtacg ggtatcaaag ttaaaaatct aaaagaaggg gctactgttt 1080
attcaggaag tgattctgcg tatagcgcta agtggaattg gcaaactttt cagccagata 1140
ccgtttatca ttttagaaat gatttaggga ttgcgactgc gggcttagtt gtcccagaag 1200
cggcagagac ctataaaaat aaaacgacaa gtgatcaaaa aaatcacgtt caagataata 1260
ttaccatcga aatgaaaacg cataatttag gttatagtag tgaatggaaa gatattaacg 1320
taacgtcaaa aataccagat gaactggata ttgatcctag tacaattaaa gtaactctga 1380
ataatggcga tcaagtagca attgatgcta gtaagtataa ccaaacgact agaatgttaa 1440
cggttagatt acccaaaaat ttggccgaaa atgaatggtc atcgatcagt ttcgatgcca 1500
aaattaatgg taaagcttct ggaaaaacaa ttaatagtag catggttgct aagactgaag 1560
gtgggactgg cagttttgat acggctacgg cgagcacgaa aatcccagtc gaagttgaac 1620
ctgtcgaaat tgaaaagaaa gttttaaatg aaactgaaaa agaaacgacg taccaaaaag 1680
cgacggtcgg gcatgtaaat gatgttcttg attaccaagt gaagttaaca gtcagcagta 1740
acggagctga tatgtctaac ggtgtcttgg ccgatgacct agctaaggaa gggttagaat 1800
tagtgccggg ctctgttaaa ttgacatata gcgataagac agttgaaacc ccagcagatg 1860
ttaaacagat taatttgaaa aaaatgacac ctggacagaa tgtagtatta acctataagg 1920
ctaaagtcaa agaaggtgtt gtgataggca cagtgcttaa aaatattgtt ttatattctg 1980
gggaacaagc taatcagggt gttatgaacg ggcaagctga cgcgtcagta acgattgaaa 2040
agacgaagaa tagtgacgtt cactttagtt atattgatcg tgaaacgggg cagcagattg 2100
ctaatgaagt cgttgcaact gggccaatta atgccaaaat atcagcatta aaagcaactg 2160
atattagcga tgggcaagat cctaataaga ttcggcccgc ctacattgaa ggttatacgc 2220
cagttgattt tacgacggca actgatttaa acgctgcggt ctatgcggat attaaagatg 2280
ttgatccagt aattgaagaa aaatcggtga cctatacttt tagatatgaa aaaacgcgct 2340
tagcgattac ggcgttacca agtaaactga attttggtaa attcgacgat acgcaaagtg 2400
aacggacgtt ttatttgccg gcacaaattg agaaaccagc tgatgaaaaa acaccatatg 2460

gcattgaaat ctctgattat tggggcatta agggctggac gttgagtgtt gcacaggaac 2520

aacaatttca tggcagtgcc acgatagatt taaaggaaca agtagttgag ctgtccggcg 2580

cacaactcca gtttaataac gcaacattga gcacgcatac ggaaggtaat aagtctgttt 2640

cgaatttgca ggataaagta gtaactaaat caaactttaa cttggtaccc ggggctgagc 2700

cgttagaatt agttgaatac gaacgaaaag gccagtacct taatcaagac ggcgacaata 2760

aaggcggggt cagttatgat atccctggct attcagttca taaatatcaa tttggtgacg 2820

caagaacggc tgattacagt attggcctgc atgtcccaga aacgactgaa cgttaccgta 2880

cagaatacac atcaacattg aagtggcatt taacggttgc gccataa 2927

<210> 49
<211> 2842
<212> DNA
<213> Lactobacillus sakei

<400> 49
atgttgatac tgatattaat aagttctgta attattaatg agaacgttgt caacgcagtc 60

caaagacagg ataattcatc agaaatcaag gtgccaacag attcaaaaac aagtagtcac 120

tcagaacaat cagtgagtac ccaatccaaa tctgaatcta agtctgaatc taaaacgacg 180

tcatctgaaa agtcttcttc tgagacgaca acgcagtcat cgagtagtca tcgagataag 240

tctgtgattc agaatcaatc acgagataca ataatgccga cagctactat tgatgatggc 300

gtgacaggtt ggcaaccggc aaagcaaaca acgattgcaa gtattgatat tgataaagcg 360

gctaataaaa gccttaatta tacttttggt aatagtgatg ggaccttaaa gtttgatcaa 420

ggaaatggtg ggataataac gaataacatg ccgccaagaa tgaacgtttt cattaaggat 480

aatggcaaga ttgtcgagtc ttcattcaaa tcaacatctg ggatgggggg ctcgtacgac 540

tattcactct taaccaccag aagttccacg gcaccaacga ttaaaaccac atcgacatcg 600

gcacagggat cactgacagg tgtgcaatat tatcaagcca aagacgccaa agggcagaca 660

attttaaaaa tggtggggca atatacaacc atagtggggc gtgtccccag tttcacactt 720

gagtatctat tacggccgtc actagaaaaa ccatctgttc aacaggaact atatatttat 780

aatgcgacat cggcagcaat tgatggcggg atcttttttg ctaaagatac ttcgttaaat 840

ggtaacgatg gcgtaccagt ttctgcgcaa gggaataacg ctgggatgta cattgccgat 900

ggtaaatata aactattcct taatatgggt gttgaggatg gtccagccaa atttaacgcg 960

atgaattatc accgaaatgg tggatataac ttatctgatt atgcaatgaa tgattattcg 1020

ccagccaact ttgatggtac gggtatcgaa gttaaaaatc taaaagaagg ggctactgtt 1080

tattcaggaa gtgattctgc gtatagcgct aagtggaatt ggcaaacttt tcagccagat 1140

accgtttatc attttagaaa tgatttaggg attgcgactg cgggcttggt tgtcccagaa 1200

gcggcagaga actataaaaa taaaacgaca agtgatcaaa aaaatcacgt tcaagataat 1260

attactatcg aaatgaaagc gcataattca ggctatagta gtgaatggaa agatattaac 1320

gtaacgtcaa aaataccaga tgaactggat attgatccta gtacaattaa agtaactctg 1380

aataatggcg atcaagtagc aattgatgct agtaagtata accaaacgac tagaatgtta 1440

acggttagat tacttaaaaa tttgtccgaa aatgagtggt actggcaatt tcgatacagc 1500

tacagcgaat acgaaaatcc ctgttgaggt tgaacctgtc gaaattgaaa agaaagtttt 1560

aaatgaaact gaaaaagaaa cgacgtacca aaaagcgacg gtcgggcatg taaatgatgt 1620

tcttgattac caagtgaagt taacagtcag cagtaacgga gctgatatgt ctaacggtgt 1680

cttggccgat gacctagcta aggaagggtt aaaattagtg ccgggctctg ttaaattgac 1740

atatagcgat aagacagttg aaaccccagc agatgttaaa cagattaatt tgaaaaaaat 1800

gacacctgga cagaatgtag tattaaccta taaggctaaa gacaaagaag gtgttgtgat 1860

aggcacagtg cttaaaaata ttgttttata ttctggggaa caagctaatc agggtgttat 1920

gaacgggcaa gctgacgcgt cagtaacgat tgaaaagacg aagaatagtg acgttcactt 1980

tagctatatt gatcgtgaaa cggggcagca gattactaac gaagtcgttg caactgggcc 2040

aattgatgcc aaaatatcag cattaaaagc aactgatatt agcgatgggc aagatcctaa 2100

taagattcgg cccgcctaca ttgaaggtta tacgccagtt gattttacga cggcaactga 2160

tttaaacgct gcggtctatg cggatattaa agatgttgat ccagtaattg aagaaaaatc 2220

ggtgacctat acctttagat atgaaaaaac gcgcttagcg attacggcgt taccaagtaa 2280

actgaatttt ggtaaattcg acgatacgca aagtgaacgg acgttttatt tgccggcaca 2340

aattgagaaa ccaactgatg aaaaaacacc atatggcatt gaaatctctg attattgggg 2400

cattaagggc tggacgttga gtgttacaca ggaacaacaa tttcatggca gtgccacgat 2460

agattcaaag gaacaagcag ttgagctgtc cggcgcacaa ctccagttta ataacgcaac 2520

attgagcacg aatacggaag gtaataagtc tgtttcgaat tcgcaggata aagtagtaac 2580

taaatcaaac tttaacttgg tacccggagc tgagccgtta gaattagttg aatacgaacg 2640

aaaaggccag taccttaatc aagacggcga caataaaggc ggggtcagtt atgatatccc 2700

tggctattca gttcataaat atcaatttgg tgacgcaaga acggctgatt acagtattgg 2760

cctgcatgtc ccagaaacga ctgaacgtta ccgtacagaa tacacatcaa cattgaagtg 2820

gcatttaacg gctgcgccat aa 2842

<210> 50
<211> 4698
<212> DNA
<213> Lactobacillus sakei

<400> 50
atgaagggta ttattttagc aggtgggtca ggtactcgcc tgtatccaat tacaaaggca 60

gtatcaaaac aacttattcc ggtttatgat aaaccaatga tttattatcc aatgtcagtt 120

ttaatgttag ctggcattca ggatattatg attatttcaa caccaagcga tacaccacgt 180

ttcgaagaat tatttggtga tggccatgat ttgggcttaa atattgaata tgccgtgcag 240

gataatccca acggtttggc cgaagcattc attattgggg ccgattttat cggtgatgat 300

tcagtctgtt taatcctagg tgataacatc tattatggtg gtggcctatc agaaatgcta 360

caaagagcgg ctcaaaagga atctggtgcg acggtctttg gctaccatgt taatgaccca 420
gaacgttttg gggtcgttga ttttgatgaa aatatgcatg cgaaatcaat tatcgaaaaa 480
ccggaacatc cagcaagtaa ctacgcggtg acgggtttgt acttctatga caatcaagtt 540
gttgagattg ctaaaaatat caaaccatca ccacgaggtg aactcgaaat taccgatatt 600
aacaaggctt atctcgaaaa aggtcaatta gatgttgaat taatgggccg gggctttgct 660
tggttagata ctggcactca cgattcattg catgaggcca gcagttttat tgcaaccgtt 720
caaaagcggc aaaatttaaa ggttgcttgt ttagaagaaa ttgcttaccg aatgaactat 780
atcgataaag agaaagtgat tgaattggcg caaccgctta agaaaaatga ttatggccaa 840
tatttattga gattagttgg tgaaaaaaat gattagatta ttattcttag ggacacagga 900
tattgatgat aaattatcgt ggtctggcac catgtaccac atgacacaag caattaaaaa 960
agatgaaaac tatactatat cttctttaaa agttccggag aaaagttttt tggataagtt 1020
aagaattaaa atgcttgcta taccaagaaa aatttttagc ccagggaaaa aatatgttag 1080
taatgtagac gtacaccaag ttcgaattaa tggaaaaaga ttaacaaaac tattggcaga 1140
taaagttgaa aaggaatcag tagatattat atttgcacca gccatgagca catacttagc 1200
taatttaaca actaatattc caattgtata tttgagtgat gctacttttg cactcatgaa 1260
taattactac tggtttggat taagcaataa agaagttagg gatggaaata gagtagagtt 1320
ggctgctcaa gaaaattcca cagcacttat tttttcaagc gagtgggcgg ctaatagtgc 1380
tattaatgat tataaaagtt caatagacaa ggttaacata gtaccgtttg gagcaaattt 1440
agcgactagt aatttaataa aaaataaaag gattaattca acaagagagg ttaaaattct 1500
tttggtcggt aaggattatg aacgaaaagg aatttttatt gctgaacaga taattcaaaa 1560
gttgaatatt aattctgata tccgatacaa gctaattgta gttggagttg accgagaaga 1620
tactgataat attcattatg ctggagtatt ggataaaggc aatgaaacgg attataaaaa 1680
attgatgaac tattaccatg aatcagtatt gttcttacta ccgacaaaag cagaggcagc 1740
cggaattgtt tttgctgaag ctagtatgtt tggactacca actctgactt ttgatactgg 1800
tggtattggt gaatatatca ttaacggcgt aaacgggtac cgaatatcac ttgatagtca 1860
agatattgta gcttcattta cggataaaat tgatgaaatt gtacatggtg aagtattata 1920
tgagcgttta tccgtaggtg cggtggaaca atatcagacg cgattgaact gggatcattg 1980
gatgacagaa tttaatagga ttgttaacaa tgtactaagt gaggagaaca ataaataatg 2040
ggaaaattaa aagtaacaac aactaaacta caagacgtta aaattattga accagccgtt 2100
tttggtgatc accgtggatt ctttacggaa tcatattcag atcgtgactt taaagaagct 2160
gggattgata tcgattttat tcaagacaat cattcattat caactcaagc aggtgtctta 2220
cgtggcttac atttccaacg tggcaaagct gctcaaacga aactaatccg agttgtgaca 2280
ggtgctgttt tagacgtcat cgtggatgtg cgtgccggtt caccaactta caagcaatgg 2340
gaaggctata ttctttcagc aagtaatcac cgtcaattat tagtaccaaa gggcttcgcc 2400

cacggttttg tgacgttaac cgataacgtt aacttcttat ataaatgtga tggttactac 2460
gatgccgaag ctgatggtgg gatttcattt aagaccccag aattaaacat tgactggcca 2520
atcgattttg accaagcaat tacatcagaa aaagatgcaa accaaccaac ctttatagaa 2580
ttcgaaaaag acaacccatt tgtttacggt gaaatttaag gagactctaa cttatgaaaa 2640
atattattgt taccggcggc gccggtttta tcggttctaa ttttgtccac tatgttgtta 2700
acaatcatcc agaggttcac gtaacggttt tggataagtt aacttatgca gggaatcgcg 2760
ctaacttagc tggtttacca gcagaccgtg tagaattagt ggttggcgat atctgtgacg 2820
ctgaattagt cgataggttg gttcaaaaga cggacgcggt tgtgcattac gctgcagaaa 2880
gtcacaatga taactcctta aaggatccat cacctttcat tcaaactaat attattggct 2940
catatacttt aattgaagct tgtcgtaaat ataacgtgcg ttatcatcac gcttcaactg 3000
atgaagttta tggtgattta ccattgcggg aagacttacc aggtcagggt gaaggtgctg 3060
gtgaaaaatt tacacctgaa acacgttaca atccaagtag cccttattca tcatcaaaag 3120
ccagttcaga tttattagtg cgtgcttggg tccgttcgtt cggtctacaa gcaactattt 3180
caaactgttc aaacaactat gggccttacc aacacattga gaaatttatt ccccgtcaaa 3240
ttactaacgt gttaagtggc attcgtccta agctttacgg ttcaggtcaa aacgtgcgtg 3300
attggattca tacgaatgac cactcaagcg ctgtttgggc catcttaact aaaggtaaga 3360
ttggtgaaac ttatttaatc ggtgccgacg gcgaacaaaa caacaaagat gtcttggaat 3420
tactcttaga attaatgggt caacctaagg atacttacga ccaagttaaa gatcgtcccg 3480
gtcatgattt acgttatgcg attgattcaa cgaagttacg tgaagaatta ggctggcaac 3540
cagaatttac tgatttcaaa tcaggattac aacatacgat tgactggtat cgtgataacg 3600
aagactggtg gcaagcagaa aaagcagcag ttgaagccaa ctatgctaaa aatggtcaat 3660
aaatataact aaatggaagt aggcgagcgc ctactttttt taaggagaag agttaacaat 3720
gaaaatttta atcacgggtg ccaatggtca attaggaacc gaattacgtc atttattaga 3780
tgaaaatcaa atcgaatatg taggaactga tgccgctgaa ttggatatca cgaatgcaga 3840
agcagttaat gcttattttg aagctaacaa accggaatta gtttatcact gtgctgctta 3900
tacagccgtt gatgcagccg aagaagaacc tggcaagtca atcaactaca aggtcaacgt 3960
cgttggtaca caaaatattg ctaatgccgc agaagtagtt ggcgcaacct tggtttacat 4020
cagtacagat tatgtcttcg atggcactaa cgaccaaatg tatacagaag aaacacctgc 4080
tgcacctaag aacgaatatg gtcggacaaa attagctggt gaagaagctg ttgctaacac 4140
gatgagcaag tattatatta tccggacatc atgggtcttt ggtgaatatg gtaagaactt 4200
tgtttatacg atgttgaacc tggcgaagac acacgatcat ttaacagttg ttagtgatca 4260
agtcggtcgg ccaacttgga ctcggacgtt ggctgaattc atgttatatt cagttaaaaa 4320
taatgtacct tacggtttat atcaactttc aaacgacaat tcatgtactt ggtatgaatt 4380
cgcgagtgaa atcttgaagg ataaagacgt agaagtaagt ccagttacat cagatgaata 4440
cccacaaaaa gctttccgtc caagacattc aatcatggat ttgacgaaga ttaaagcaac 4500

cggctttgaa gtaccaacat ggcaagaagc attacaaaaa tttatggcaa agattgacta 4560

atatagatag tatataaaaa ggataaagtc gactgacttt atccttcttt attagattaa 4620

caatttacta aactttttct gcaccctaaa tttaatatta ttagccgttc gccgtgtgat 4680

gtttaagtcc gcggcgat 4698

<210> 51
<211> 2549
<212> DNA
<213> Lactobacillus sakei

<400> 51
atgaagggta ttattttagc aggtgggtca ggtactcgct tgtatccaat tacaaaggca 60

gtttcaaagc aacttattcc agtttatgat aaaccaatga tttattatcc aatgtcaact 120

ttaatgttag ctggcattca ggacattatg attatttcca caccaactga tacaccacgt 180

ttcgaagaat tatttggtga tggtcatgat ctgggtttaa atattgaata tgccgtgcag 240

gataatccca acggtttggc cgaagcattc attattgggg ccgattttat cggtgatgat 300

tcagtctgtt taatcctagg tgataacatc tattatggtg gtggcctatc agaaatgcta 360

caaagagcgg ctcaaaagga atctggtgcg acggtctttg gctaccatgt taatgaccca 420

gaacgttttg gggtcgttga ttttgatgaa aatatgcatg cgaaatcaat tatcgaaaaa 480

ccggaacatc cagcaagtaa ctacgcggtc actggtttat atttctacga caaccaagtt 540

gtcgagattg ctaaaaatat caaaccatca ccacgaggtg aactcgaaat taccgatatt 600

aacaaggctt atctcgaaaa aggtcaatta gatgttgaat taatgggccg gggctttgct 660

tggttagata ctggcactca cgattcattg catgaggcca gcagttttat tgcaaccgtt 720

caaaagcggc aaaatttaaa agttgcttgt ttggaagaaa ttgcttaccg aatgaactat 780

atcgataaag caaaagtgat tgaattggca caaccgctta agaaaaatga ttatggccaa 840

tatttattga gattagttgg tgaaaaaaat gagagattag taaaagaaga ttaggggtaa 900

gcaattatgg gaaaattaaa agtaacaaca actaaactac aagacgttaa aattattgaa 960

ccagccgttt ttggtgatca ccgtgggttc tttacggaat catattcaga tcgtgacttt 1020

aaagaagctg ggattgatat cgattttatt caagacaatc attcattatc aactcaagca 1080

ggtgtcttac gtggcttaca tttccaacgt ggcaaagccg ctcaaacgaa actaattcgg 1140

gttgttactg gcgctgtttt agacgtcatt gtagatgtgc gtgctggctc cccaacttat 1200

aagcaatggg aaggttatat cctttcagca agtaatcacc gtcaattatt agtaccaaag 1260

ggctttgccc acggttttgt gacgttaacc gataacgtta acttcttata caaatgtgat 1320

ggctattatg atgcagaagc tgatggtggg atttcattta aaaccccaga attaaacatt 1380

gactggccaa tcgattttga ccaagcaatt acatcagaaa aagatgcaaa ccaaccaacc 1440

tttatagaat tcgaaaaaga caacccattt gtttacggtg aaatttaagg agactctaac 1500

ttatgaaaaa tattattgtt accggcggcg ccggttttat cggttctaat tgaaggagaa 1560

taataaatta tgaaaatttt aatcaccggt gctaatggtc aattagggac tgaattacgt 1620

cacctattag atgaaaatca aattgaatac gtgggcacgg atgctgctga attggatatt 1680

accaatgcag aagcagttaa tacttatttt gaagctaaca aaccagaatt agtttatcac 1740

tgtgctgctt atacagccgt ggatgctgct gaagaagaac ctggcaagtc aatcaattac 1800

aaggtcaacg tggttgggac gcaaaatatt gctaacgctg ctgaagcagt tggggcaaca 1860

ttggtttaca tcagtacaga ctatgtcttc gatggcacta ataatcaaat gtatacagaa 1920

gaaacaccag cagcacctaa gaacgaatat ggtcggacaa aattgactgg tgaagaagcc 1980

gtggctaata cgatgagtaa gtattacatc atccggacat catgggtctt tggtgaatat 2040

ggtaagaact ttgtttatac catgttaaac cttgctaaaa cacatgatca tttaacggtt 2100

gttagtgatc aagtcggtcg gccaacttgg acccggacat tggctgaatt catgttatat 2160

tcagttaaaa acgacgtccc ttatggttta taccaacttt caaatgacaa ttcatgtact 2220

tggtatgaat ttgcgagtga aatcttgaag gacaaagctg ttgaagtgag tcctgttaca 2280

tcagacgaat atccccaaaa agccttccgt ccaagacatt caattatgga tttgacgaag 2340

attaaagcaa ctggttttga agtaccaaca tggcaagaag cattacaaaa atttatggca 2400

aagattgact aatatagata gtatataaaa aggataaagt cgactgactt tatccttctt 2460

tattagatta acaacttact aaactttttc tgcaccctaa atttaatatt attagctgtt 2520

cgccgtgtga tgtttaagtc cgcggcgat 2549

<210> 52
<211> 2549
<212> DNA
<213> Lactobacillus sakei

<400> 52
atgaagggta ttattttagc aggtgggtca ggtactcgct tgtatccaat tacaaaggca 60

gtttcaaagc aacttattcc agtttatgat aaaccaatga tttattatcc aatgtcaact 120

ttaatgttag ctggcattca ggacattatg attatttcca caccaactga tacaccacgt 180

ttcgaagaat tatttggtga tggccatgat ttgggcttaa atattgaata tgccgtgcag 240

gataatccca acggtttggc cgaagcattc attattgggg ccgattttat cggtgatgat 300

tcagtctgtt taatcctagg tgataacatc tattatggtg gtggcctatc agaaatgcta 360

caaagagcgg ctcaaaagga atctggtgcg acggtctttg gttatcatgt taatgaccca 420

gaacgttttg gggtcgttga ttttgatgaa aatatgcatg cgaaatcaat tatcgaaaaa 480

ccagaacatc cagcaagtaa ctacgcggtc actggtttat atttctacga caaccaagtt 540

gtcgagattg ctaaaaatat caaaccatca ccacggggtg aactcgaaat tacagatatt 600

aacaaggctt atcttgaaaa aggccaatta gatgttgaat taatgggccg gggctttgcg 660

tggttagata ctggcactca cgattcatta catgaggcta gcagttttat tgcaaccgtt 720

caaaagcgcc aaaacttaaa agttgcttgt ttggaagaaa ttgcttaccg aatgaactat 780

atcgataaag caaaagtgat tgaattggca caaccgctta agaaaaatga ttatggccaa 840

tatttattga gattagttgg tgaaaaaaat gagagattag taaacgaaga ttaggggtaa 900

gcaatcatgg gaaaattaaa agtaacaaca actaaactac aagacgttaa aattattgaa 960

ccagccgttt ttggtgatca ccgaggcttc tttacggaat catattcaga ccgtgacttt 1020

aaagaagccg gaattgatat caattttatt caagataacc attcactttc aacgcaagcc 1080

ggcgttttac gtggtttgca tttccaacgg ggcaaagctg ctcaaacgaa attaattcgg 1140

gttgttactg gtgccgtttt agacgtcatc gtggatgtgc gtgccggttc accaacttat 1200

aagcaatggg aaggttatat tctttcagca agtaaccacc gtcaattatt agtgccaaag 1260

ggttttgctc acggctttgt aactttaaca gataatgtta atttcttata taaatgtgac 1320

ggctattacg atgccgaagc tgatggtggg atttcattta aaaccccaga attaaatatt 1380

gactggccaa ttgattttga ccaagcgatt acatcagaaa aagatgcgaa ccaaccaacc 1440

tttacagaat tcgaaaaaga caacccattt gtttacggtg aaatttaagg agactctaac 1500

ttatgaaaaa tattattgtt accggcggcg ccggttttat cggttctaat ttaaggagaa 1560

gagttaacga tgaaaatttt aattactggt gccaatggtc aattaggaac cgaactacgt 1620

cacttattag atgaaaatca aattgagtat gtgggaacag atgctgctga attagatatt 1680

acgaatgcag aagcggttaa tacttatttt gaagccaaca aaccggaatt agtttatcac 1740

tgtgctgctt atacagccgt ggatgctgct gaagaggaac ctggcaagtc aatcaactat 1800

aaggtaaacg tggttgggac acaaaatatt gctaatgctg ctgaagcagt tggcgcaacc 1860

ttggtttaca tcagtacaga ttatgtcttc gatggcacta acgatcaaat gtacacagaa 1920

gaaacacctt ctgcaccgaa gaacgaatat ggtcggacaa aattagcggg tgaagaagct 1980

gttgctaaca cgatgagcaa gtattacatc atccggacat catgggtttt tggtgaatac 2040

ggtaagaact ttgtttacac catgttgaac ctggcgaaga cacacgatca tttaacagtt 2100

gttagtgatc aagtcggtcg gccaacttgg actcggacgt tggctgaatt catgttatat 2160

tcagttaaaa atgatgtccc ttacggttta tatctacttt caaatgataa ttcatgtact 2220

tggtatgaat ttgcgagcga aattttgagg gataaggatg tagaagtaag tcccgttacg 2280

tcagacgaat atccacaaaa agcattccgt ccaagacatt caatcatgga tttgacgaag 2340

attaaagcaa ccggctttga agtaccaaca tggcaagaag cattacaaaa atttatggca 2400

aagattgact aatatagata gtatataaaa aggataaagt cgactgactt tatccttttt 2460

tattagatta acaatctact aaactctttc tgcaccctaa atttaatatt attagccgtt 2520

cgccgtgtga tgtttaagtc cgcggcgat 2549

<210> 53
<211> 2582
<212> DNA
<213> Lactobacillus sakei

<400> 53
gttaatgcgg cgagtcgtcc taaatctttt tatgaattag catttgatgc gcaagatttt 60

gaaccaatca cagtacccca acatatcgaa ttggcgggtt atgagcaatt gcactacatc 120

aacacaatgt atccgtggga aggtcattat tatcggcgtc ccgctttttc aacgtccgat 180

gacaagcaac atctcggcat gtttagcgaa gccgattata atcccgtcgg ttcatacctg 240
catcattttg atttaacgcc agctttgaga aatcaacggg tcatcattcg ctttgaaggg 300
gtcgaacaag cgatgtatgt ttggctcaat ggtcaattta tcggctatgc tgaagatagt 360
tttacaccgt ctgaatttga cttaacccct tatcttaaag aaactgataa ttgcttggcg 420
gttgaagtgc ataagcgcag tagtgcggct ttcatcgaag atcaagattt cttccgcttc 480
ttcgggattt tccgggatgt taagttatta gctaaacccc gcacgcattt ggaagattta 540
tgggtaattc ccgaatatga cgtggtacaa caaacgggac aggtgaaact tcgccttcaa 600
ttttcaggag atgaaaaccg agtccatcta cgcattaggg accaacatca aatcatctta 660
acggccgatt taacaagtgc cgctcaggta aacggccttt ataagatgcc cgaattggtt 720
caagcatggt ccaaccaaac tcccaactta tacacattag agctagaagt tgtggatcaa 780
gctggggaaa cgattgaaat tagtcagcaa ccatttggct tccgtaaaat tgagattaaa 840
gataaagtga tgttgctcaa cggaaaacgg ttagtcatta acggcgttaa tcggcatgaa 900
tggcaccctg aaactggtcg gacaattacg gccgaagatg aagcgtggga tattgcctgc 960
atgcagcgca accatatcaa tgccgtccga acatcgcatt atccagatcg gttatccttt 1020
tataatggtt gcgatcaagc tggcatctat atgatggctg aaaccaattt ggaatcgcat 1080
ggttcttggc aaaaaatggg ggcggtcgaa ccttcttgga acgtcccagg ttcatatgat 1140
gaatgggaag cggcgacgct cgatcgagca cgaaccaatt ttgaaacgtt taaaaatcac 1200
gtctcaattc tcttttggtc attagggaat gaatcttacg ctggtagtgt tcttgagaag 1260
atgaacgcct attacaagca acaagatcca acgcgcttag tgcattatga aggggtcttc 1320
cgggcgcccg agtataaggc gacgatttcg gatgttgaaa gtcggatgta tgcgacaccg 1380
gctgagatta aggcttattt agataacgcc ccacaaaaac catttattct ctgcgaatat 1440
atgcatgata tggggaattc gctgggtggt atgcaatcgt atatcgactt attgtcacaa 1500
tatgacatgt accaaggcgg ctttatctgg gatttcattg atcaagcact cttggtgaca 1560
gatccagtga ctggtcaacg tgaattacgc tacggtggcg attttgatga ccgcccatca 1620
gactatgaat tttcggggga tgggttagtc tttgctaccc gcgatgaaaa accagcaatg 1680
caggaggtta gatactatta tggcgaacac aaataaacgc ttagcagtga tcttcggaga 1740
tgtgacttta ggacttaaag gccctgactt tcactacctc ttttcgtacc aaacgggtgg 1800
gccagaatca ttgcggattc aaggtaaaga atggctatat cgctcaccaa aaccgacttt 1860
ttggcgggca actaccgata atgatcgcgg caaccaattc ccattgaaat cgggaatgtg 1920
gctagctgct gatcagttca ttgcttgtca gtcgattaca gtggcgattg acgggcaaac 1980
gattccgctg ccaattgcgc ccgagaacaa ccgctatagt ggtcaggaaa cggcccaaga 2040
agtgacggtt acgtatacct atcaaacaat tacgacgccg cagacgacgg tagaagtcag 2100
ttatacgatt caagccagtg gtaaaatccg tgtagcggtg acttatcatg gtcaagctgg 2160
attaccatca ttaccagtct ttggtttacg ctttgtcatg ccaaccccag cgacgcgctt 2220

tatttatcaa gggttgtcag gggaaactta tccagatcga atggctggtg gaatgggctg 2280

gtgagtacga agtcaccggg ctacctgtga cgccttatct ggtgccacaa gattgtggtg 2340

tgcacatggc aactgattgg gtaacgattt atcggcaagc ggttctggat aatcgtttgc 2400

gcgaaccagt cgaaaccggc ttgaagttta agatggtcga tcaaccattt gccttttcat 2460

gcttgccata taccgctgaa gaattggaaa atgcgaccca tcattcagaa ctacccgcac 2520

cacaccgaac agtcttaaat ctcttagggg ccgttcgtgg cgtgggcggt atcgatagtt 2580

gg 2582

<210> 54
<211> 2582
<212> DNA
<213> Lactobacillus sakei

<400> 54
gttaatgcgg cgagtcgtcc taaatctttt tatgaattag catttgatgc gcaagatttt 60

gaaccaatca cagtacccca acatatcgaa ttggcgggtt atgagcaatt gcactacatc 120

aacacaatgt atccgtggga aggtcattat tatcggcgtc ccgctttttc aacgtccgat 180

gacaagcaac atctcggcat gtttagcgaa gccgattata atcccgtcgg ttcatacctg 240

catcattttg atttaacgcc agctttgaga aatcaacggg tcatcattcg ctttgaaggg 300

gtcgaacaag cgatgtatgt ttggctcaat ggtcaattta tcggctatgc tgaagatagt 360

tttacaccgt ctgaatttga cttaacccct tatcttaaag aaactgataa ttgcttggcg 420

gttgaagtgc ataagcgcag tagtgcggct ttcatcgaag atcaagattt cttccgcttc 480

ttcgggattt tccgggatgt taagttatta gctaaacccc gcacgcattt ggaagattta 540

tgggtaattc ccgaatatga cgtggtacaa caaacgggac aggtgaaact tcgccttcaa 600

ttttcaggag atgaaaaccg agtccatcta cgcattaggg accaacatca aatcatctta 660

acggccgatt taacaagtgc cgctcaggta aacggccttt ataagatgcc cgaattggtt 720

caagcatggt ccaaccaaac tcccaactta tacacattag agctagaagt tgtggatcaa 780

gctggggaaa cgattgaaat tagtcagcaa ccatttggct tccgtaaaat tgagattaaa 840

gataaagtga tgttgctcaa cggaaaacgg ttagtcatta acggcgttaa tcggcatgaa 900

tggcaccctg aaactggtcg gacaattacg gccgaagatg aagcgtggga tattgcctgc 960

atgcagcgca accatatcaa tgccgtccga acatcgcatt atccagatcg gttatccttt 1020

tataatggtt gcgatcaagc tggcatctat atgatggctg aaaccaattt ggaatcgcat 1080

ggttcttggc aaaaaatggg ggcggtcgaa ccttcttgga acgtcccagg ttcatatgat 1140

gaatgggaag cggcgacgct cgatcgagca cgaaccaatt ttgaaacgtt taaaaatcac 1200

gtctcaattc tcttttggtc attagggaat gaatcttacg ctggtagtgt tcttgagaag 1260

atgaacgcct attacaagca acaagatcca acgcgcttag tgcattatga aggggtcttc 1320

cgggcgcccg agtataaggc gacgatttcg gatgttgaaa gtcggatgta tgcgacaccg 1380

gctgagatta aggcttattt agataacgcc ccacaaaaac catttattct ctgcgaatat 1440

atgcatgata tggggaattc gctgggtggt atgcaatcgt atatcgactt attgtcacaa 1500

tatgacatgt accaaggcgg ctttatctgg gatttcattg atcaagcact cttggtgaca 1560

gatccagtga ctggtcaacg tgaattacgc tacggtggcg attttgatga ccgcccatca 1620

gactatgaat tttcggggga tgggttagtc tttgctaccc gcgatgaaaa accagcaatg 1680

caggaggtta gatactatta tggcgaacac aaataaacgc ttagcagtga tcttcggaga 1740

tgtgacttta ggacttaaag gccctgactt tcactacctc ttttcgtacc aaacgggtgg 1800

gccagaatca ttgcggattc aaggtaaaga atggctatat cgctcaccaa aaccgacttt 1860

ttggcgggca actaccgata atgatcgcgg caaccaattc ccattgaaat cgggaatgtg 1920

gctagctgct gatcagttca ttgcttgtca gtcgattaca gtggcgattg acgggcaaac 1980

gattccgctg ccaattgcgc ccgagaacaa ccgctatagt ggtcaggaaa cggcccaaga 2040

agtgacggct acgtatacct atcaaacaat tacgacgccg cagacgacgg tagaagtcag 2100

ttatacgatt caagccagtg gtaaaatccg tgtagcggtg acttatcatg gtcaagctgg 2160

attaccatca ttaccagtct ttggtttacg ctttgtcatg ccgaccccag cgacgcgctt 2220

tatttatcaa gggttgtcag gggaaactta tccagatcga atggctggtg gaatgggctg 2280

gtgagtacga agtcaccggg ctacctgtga cgccttatct ggtgccacaa gattgtggtg 2340

tgcacatggc aactgattgg gtaacgattt atcggcaagc ggttctggat aatcgtttgc 2400

gcgaaccagt cgaaaccggc ttgaagttta agatggtcga tcaaccattt gccttttcat 2460

gcttgccata taccgctgaa gaattggaaa atgcgaccca tcattcagaa ctacccgcac 2520

cacaccgaac agtcttaaat ctcttagggg ccgttcgtgg cgtgggcggt atcgatagtt 2580

gg 2582

<210> 55
<211> 2157
<212> DNA
<213> Lactobacillus sakei

<400> 55
atgatagatt gtaaaatata tattccgcag gttgatgaga tggattgtgg cgtagcagcc 60

ttagcgatga ttcttaagca ttatggttct tcagtttcgc tggcttattt acgtaatgag 120

gctaaaactg atttagaagg aacaacggcg cttggactag tgaagacagc tgagaattta 180

ggtttcgaaa cgaaggcaat tcaagctgac atgagtttgt ttgaagtcgc agatttacca 240

tttcctttta ttgcacatgt tctaaaaaat ggcgagctat tacattatta tgttgtacta 300

gggataaaaa aagatgccat tataattgct gatcctgatc caacagttgg tgttactaaa 360

gtggctcaat cacagtttaa agcggaatgg acaggtgttg ctttatttat tgcaccaaag 420

ccgaactatc aaccagttaa gcaacaaaca aaagcgagtt tattctcatt tgtcccaacc 480

ttgataaaac aaaagcggtt ggtgattaat attacattag cagctttact catcacgata 540

attagtattt tgggttcgta cttcttacaa actgtaattg atacgtatat tccaaataat 600

atgcatagta cattagcgat agtcgcgatt gggttaatca tattttatgg ttttcagtca 660

atttttacat atgcacagac ctttttacta acaatcttgg ggcaacgatt atcaatagaa 720

attattttga gttacttgag gcacgttttt gaattaccaa tgtcattttt tgcaacacga 780

aagacagggg aaataatttc aagattcaat gatgctaata aaattattga tgcactagcg 840

agtgcgatta tttcaatttt tttagatatt actgtggtcg ttattatggg cagtatcttg 900

atgattcaaa atacaacttt attttggtta actttagcgg ttattccaat ttatgttgca 960

atcattactt tatttgcgaa gccatttgaa cggttgaatc aaaaggaaat ggaaagtaat 1020

gcagtattga attcagcgat tattgaagat ctacatgata ttgaaacaat taaagcatta 1080

acaagtgaac aggaacgtta tcaaaaaatt gatacggcgt ttattgatta cttaaaaaag 1140

atttttagct atgttaaggc taatacactg cagcaggcga ttaagttatt tattcaatta 1200

ggattaaacg tctttgtttt gtgggttggt gcgctattag ttacccataa tcggttatca 1260

atcggccagt taatgacata taatgcgcta ttggcttatt ttgtaaatcc attacagaat 1320

attattaatc tacaaactaa attacaaagt gcgcgggttg ctaacaatcg tttgaacgaa 1380

gtgtatctag tagactctga atttaaggta gatcgaccgg ttaaatctga gacacaattg 1440

ataggaccaa ttgaattaca gaatgtgagt tatcaatacg gttatggtca aaaggtgtta 1500

gaagcggtta atctaagaat tatgccaaat gaaaaactga caattgttgg catgagtgga 1560

tcggggaaat caacattagt taagttatta gttgattttt ttgaaccaac ggagggaaag 1620

gttcttttta atcaacaaga tacgcagaca attgataaaa agacattgcg gcagtttttt 1680

aattatattc cgcagactcc ttatattttt tcaggaacca tctgggataa tcttaagtta 1740

ggtaatcgtc ccgggattac acaagctgat atcattcatg catgtgaatt agcagaaatt 1800

aagaccgata ttgaaaaaat gcctctccaa tttgaaacat tattggatga agatggtaat 1860

actttatcag gtggtcaaaa acagcgatta acgattgcgc gggcactcct gtcaccagct 1920

caagtattaa tatttgatga gtcaactagt gggttagata cgataacaga gggactattg 1980

gttgatcatt taatggcatt agaagaaaaa acaatcattt tcatagcgca tcgattagcg 2040

attgctaaga aaacgaataa tattctggtt ttagaaaatg ggagagtggt tgaaactgga 2100

acacatgaaa tgttagtgtc agcacaggga cggtattatc aattgcttaa tcattag 2157

<210> 56
<211> 2157
<212> DNA
<213> Lactobacillus sakei

<400> 56
atgatagatc gtaaaattta tataccacaa gtagatgaaa tggattgtgg cgtagcagct 60

ttagcgatga ttcttaaaca ttatggttct tcagcttcgc tggcttattt acgtaatgag 120

gctaaaactg atttagaagg aacaacggcg cttggactag tgaagacagc tgagaattta 180

ggtttcgaaa cgaaggcgat tcaagctgat atgagtttgt ttgaagtcgc agatttacca 240

tttcctttta ttgcacatgt tctaaaaaat ggcgagctat tacattatta tgttgtacta 300

gggataaaaa aagatgccat tataatcgcc gatcctgatc caacagtggg catcgctaag 360

atgacgcaat cacagtttaa atcggaatgg acgggcgttg ctttatttat tgctccaaaa 420
ccgagctatc aaccagttaa gcagcaaaca aaagcgagtt tattttcatt tgttccaact 480
ttgatgcagc aaaagcgatt ggtaattaat attactttgg cggcgctact cataaccatt 540
attagtattc taggatctta tttcttacag actgtcattg atacctatat cccaaataac 600
atgcagagca cgctggcgat tgttgcaatt ggtttaatta tcttttatgg cttccagtca 660
atctttacat atgctcaaaa ttttttattg gcggttttag gacagcggtt atcaattgag 720
attattttaa gttacttaag acatgttttt gaattaccaa tgtctttttt tgcaacacgt 780
aaaacaggtg aaataatttc acgatttaat gacgcgaata aaattattga tgcgctagca 840
agcgcaatta tttcaatttt tttagatatt acagtcgttg ttattatggg gattattctg 900
atcattcaaa atacaacttt attctggtta accttagcag tgattccaat ctatattgcc 960
gtgattactt tatttgcaaa accttttgaa aaattaaatc aaaaggaaat ggaaagtaat 1020
gcagtattga attcggcgat cattgaagat ttacatggca ttgaaacgat taaagcgtta 1080
actagtgaaa gtgaacgata tcaaaaaata gatacagcat ttgttgatta tctgaagaag 1140
agttttgact atgtaaaagc tgatacattg caacaagcaa ttaagttgtt tgtccaatta 1200
gggctgaatg tctttgtgct gtgggtaggc gctattttag tcacgcataa cgagttatca 1260
attgggcaat tgatgaccta caatgccttg ctggcgtatt ttgtaaatcc gttacagaat 1320
atcatcaatt tacagactaa gttacagagc gcgagggtag ccaataatcg gttgaatgaa 1380
gtttatctgg ttgattctga gttcaagaca gaacgaccaa ttaaatccga gtcacagttg 1440
gtaggcccaa ttgagttaca aaatgtgagt taccaatatg gttatgggca gagagtgctt 1500
gaatcgatta gtttaaagat tgagccgaat gaaaaattaa caattgtggg catgagcggt 1560
tcggggaaat caacattggt taagttactc gttaattttt ttgaaccaac ggagggaaag 1620
gttcttttta atcaacaaga tacacagaca attgataaaa agacattgcg gcagtttgtt 1680
aactatattg cacagacacc gtacattttc tcaggaagta tttgggataa tcttaagtta 1740
ggtaatcggc caggaatcac acaagcggac atcatgcatg cgtgtgagct agcagagata 1800
aagaatgata ttgaaaaaat gccgctccaa tttgaaacat tattggatga agatggaaac 1860
acattgtcag gtggtcaaaa acagcgatta acaattgcgc gagcactatt atcaccagct 1920
cgggtactaa tttttgatga gtctactagt gggttagata cgataacaga ggggctatta 1980
gttgatcatc taatggcgtt agaagaaaga acaatcattt tcatagcgca tcgtttagcg 2040
attgctaaga aaacgaacaa tattcttgtt ttagaaaatg gtagtgttgt tgaaactggg 2100
acacatgatg cgttggtgga tacacaaggg cgatattacc aattgctcaa tcattag 2157

<210> 57
<211> 2202
<212> DNA
<213> Lactobacillus sakei

<400> 57
ttgaatgcgt ctaatgcgca aggaaaagcg ctggctggcg ttcaactaaa gctcaaactg 60

atgggttcta aagaaagtta tgagcgaaca gttacgacaa cggcaacagg taaagctact 120
ttggcagctg tcccagtagg cgactatcaa gtgacgcaag aatcaacggt gatgggttat 180
cagccagcca ctgctgttca gtcattaaca attagtaacg accatcagaa taccgtagat 240
tggcaattgc aacgaatggt agcggatgtt acttttagag ttcgcgatgc cgcaacgcat 300
caaccgctag ccaaagctgc ttttacttta acgacagcaa cgccggctga taacggtcaa 360
actgtttttg tttcgcaaaa gacgaatcaa gatggtgaag tgaccattaa gcaattacca 420
acaggcaaaa taacttacga gcaaatggcc aatgctgcgg gctacgagcc actagcagcg 480
attcaaacgg cgattgtggg tgctgatggg gcacaaaaca ctgaagtaac agttgataat 540
cagcggctaa aagtgactga aaaacagcaa attatcgtgc ataaaaccaa tcaacaaggg 600
caagggttag acgatgcggc ctttaaatta actaatctgg caactgggca gacacaaacg 660
caaaagacgg tgtccgggca actacacttt actgagttga acccaggacg gtatgagatt 720
caggaaacaa aagcaccaac tggctatcag ttggatcaaa cacctcagtt tgtgacgatt 780
aaagtccatg aaaaacggtt ataccaagtc cgctttgctg ataaacgtga agtgactgcg 840
ccaatcagtc cattgcgcat ccagatttta gataatcact tgcaaggcgt tgctggtgtt 900
ttactgcgac taaccgccga tcaaccggat gaccaaggtc agaccgtttg ggaattgaca 960
actgatcgat tcggacaggc tatactcccc aatgcaagta cggggcacta tcgggttgag 1020
gttttacaag taccgaccgg gtatcaattg tcgtttgatc aaagtcaatt agatgtcagc 1080
cgctatggtg aaaatcagtt acaattacaa gctaatcgaa ttgagatgcc attgcaaacc 1140
ttaacgatta ataaaaccaa cttaaaaggg caacctttat caggtgctgt ctttaaagtc 1200
gagtcactga aaactggtca gatgactaaa gttgaaacgg atcaaaccgg tcgggctgag 1260
ttgcgggacc aaaaaccagg gcgctatcgc gtaactgaag tggaagcacc acttggttac 1320
cggttagcaa ctgaacctag aattgtagcg ttgtctgaaa aatcaccgcg agcaacacaa 1380
ctaacggtaa cagatgaagc acaaatgggg cagttactga ttaagcatac aactaaaaag 1440
ggcgcaccga ttgcaaaggc ctactttgag gttaaggatc aaagtggtcg agaagtcggt 1500
tattatcaaa ctgatagtca aggacagatt aaattaaccc agctagcagt ggggcaatat 1560
actgttcaag aaatcaaggc accaactggg tatgaaatta atccggcagt gacaaaggtg 1620
gccattacgg atcgcaaaac ggcaacggtc gccattaagg ctgagcaaca agcacccgat 1680
gttaaattgg ggagttttgt attgatcgat agggatcaga aaacgcagtt agcgattgct 1740
agtgcaactt atcgattgga aacgttagct ggtcaggcgg ttcggccaga aattgtagtg 1800
ggtgcaactg gtcaagtcgt agttaacgac ctcacacctg gtcaatatcg actcgtacaa 1860
ttgacggcgg cacctagcta tcaaaaacaa actaacgatc aaatcatcga gattaaacag 1920
gatgcgcaac ttaaacaagt gacgattgaa agtcatcagt cacaagggac agtcattgta 1980
aatcaagtag atggcaatac gaaccaagct ttggttggag cgaaatatga attacaaaat 2040
caaagtggta aagtgcttgt cgaaaacttg aagagtgatg aacatggtca agttcgcatc 2100
acacatctcg atcctgatac gtaccgtttg gtgcaagtat cggcaactaa gggctatgac 2160

ccgcttaaaa agccgattgt ctttacgata accaatcgct aa 2202

<210> 58
<211> 2202
<212> DNA
<213> Lactobacillus sakei

<400> 58
ttgaatgcgt ctaatgcgca aggaaaagcg ctagctggcg ttcaactaaa gctcaaactg 60

atgggttcta aagaaagtta tgagcgaaca gttacgacaa cggcaacagg taaagctact 120

ttggcagctg ttccagtagg cgactatcaa gtgacgcaag aatcaacggt ggcgggttat 180

cagccagcca ctgctgttca gtcattaaca attagtaacg accatcagaa tactgtagat 240

tggcaattgc aacgaacggt agcggatgtt acttttagag ttcgcgatgc cgcaacgcat 300

caaccgttag ccaatgctgc ttttacttta acgacagcaa cgccggctga taacggtcaa 360

actgtttttg tttctcaaaa gacgaatcaa gatggtgaag ggaccattaa gcaagtacca 420

acaggcgaaa taacttacga gcaaattgcc aatgctgcgg gctacgagcc actagcagcg 480

attcaaacgg cgattgtggg tgctgatggg gcacaaaaca ctgaagtaac agttgataat 540

cagcggctaa aagtgactga aaaacagcaa attatcgtgc ataaaaccaa tcaacaaggg 600

caagggctag acgatgcggc cattaaatta actaatctgg caactgggcg gacacaaacg 660

caaaagacgg tgtccgggca actacacttt actgagttga acccaggacg gtatcagatt 720

caggaaacaa aagccccaac tggctatcag ttggatcaaa cacctcagtt tgtgacggtt 780

aaagcccatg aaaaacggtt ataccaagtc cgctttgctg ataaacgtga agtgactgcg 840

ccaatcagtc cattgtgcat ccagatttta gataatcact tgcaaggcgt tgctggtgtt 900

ttactgcgac taactgccga tcaaccagat gaccaaggtc agaccgtttg ggaattgaca 960

actgatcgat tcggacaggc tatactcccc aatgcaagta cggggcacta tcgggttgag 1020

gttttacaag taccgaccgg gtatcaattg tcgtttgatc aaagtcaatt agatgtcagc 1080

cgctatggtg aaaatcagtt acaattacaa gctaatcgaa ttgagatgcc attgcaaacc 1140

ttaacgatta acaaaaccaa cttaaaaggg caacctttat caggtgctgt ctttaaagtc 1200

gagtcactga aaactggtca gatgactaaa gttgaaacgg atcaaaccgg tcgggctgag 1260

ttgccggacc aaaaaccagg gcgctatcgc gtaactgaag tggaagcacc acttggttac 1320

cggttagcaa ctgaacctag aattgtagcg ttgtctgaaa aatcaccgcg agcaacacaa 1380

ctaacggtaa cagatgaagc acaaatgggg cagttactga ttaagcatac aactaaaaag 1440

ggcgcaccga ttgcaaaggc ctactttgag gttaaggatc aaagtggtcg agaagtcggt 1500

tattatcaaa ctgatagtca aggacagatt aaattaaccc agctagcagt ggggcaatat 1560

actgttcaag aaatcaaggc accaactggg tatgaaatta atccggcagt gacaaaggtg 1620

gccattacgg atcgcaaaac agcaacggtc gccattaagg ctgagcaaca agcacccgat 1680

gttaaattgg ggagttttgt attgatcgat agggatcaga aaacgcagtt agcgattgct 1740

ggtgcaactt atcgattggg aacgttagct ggtcaggtgg ttcggccaga aattgtagtg 1800

ggtgcagctg gtcaagtcgt agttgacgac ctcacacctg gtcaatatcg actcgtacaa 1860

ttgacggcgg cacctggcta tcaaaaacaa actaacaatc aaatcatcga aattaaacag 1920

gatgcgcaac ttaaacaggt gacgattgaa agtcatcagt cacaagggac agtcattgta 1980

aatcaagtag atggcaatac gaaccaagct ttggttggag cgaaatatga attacaaaat 2040

caaagtggta aggtgcttgt cgaaaacttg aagagcgatg aacatggtca agttcgcatc 2100

acacatctcg atcctgatac gtaccgtttg gtgcaagtat cggcaactaa gggctatgac 2160

ccgcttaaaa agccgattgt ctttacgata accaatcgct aa 2202

<210> 59
<211> 3912
<212> DNA
<213> Lactobacillus sakei

<400> 59
atgttcagtt tggcgttact agcagtaatg ggtatacaac tagttgttac taccggaacg 60

atccttgctg aaaatagtat caaagtagat tcaaatcttg cgttaacatc ggatcaggaa 120

ttatctaatg aaaaacaaat tgtaattaga gcgagcgatc agtcaaaagc gcaacgtgat 180

ttagcgctaa ctataccaga gggggccaca tttgatcgca caactagtaa tcaattaaac 240

aaaaaatcag cgtatacggc tacccttgat caaaaaaata attccaatca agttatttta 300

aaacaaacag cacaaacagc tgaaaatatt gagaatgatt tggtaaatag acaagcaaca 360

acgttcaaaa aagaatactt attagttttt aaactaaaag atactggtat atccgctaaa 420

caacagttga aagtgacaac aataatgaac gggattcgct atgaatctaa accactattg 480

ctgaaagagg tcgttggaac agatggtgtt caacgacaaa ctagtttgac gaaggagggt 540

gaacctcaaa taacctttaa tctaaaccaa acagaatata tcgttggaga taccatccag 600

ttaaaagttg atcttgatgc tagcgaaatt aatgaacctt tctatattaa tcttggcaat 660

aaagcaatac taacgccgat aaaagtatct agcgataaca ggaatattaa ctttgaatca 720

caaaataatc agaatacagg gaagaacatt ttaaagattt ccagtaacga accgataaag 780

aaagatttta cgctactagt aaaagcgaca acagttggga aggtcgatat tactgcttct 840

accgaaactg atcatttaat ttcgaagaca gctactgtta aagtggttga taacccaaca 900

tcaaaagtat tagcaattaa tcccaatact tttgatgacg gtattcctaa ttgggttcaa 960

aagacaaaaa cagaggtcgg aagtaagtct tttcaaagaa gtacaagtcg atttcttaat 1020

tataccttcg gtggtgtgaa tggtggaatt gatgcagaag cagattcaaa aatttctagc 1080

tacaatattc agggtagcca gattaatatc tttattaaag ataacaataa aacaatcggc 1140

gcttttttta agggagaaga cactagtagt aaagcagtaa gaacatcaac cggttttggg 1200

attgttttcg aaccaaatga aagtaatgat ggacctaaac aagctatctt agctaacaag 1260

ttaacaaata aggcatattg tgtcggacac gacagcgagg gtaaccttgt tagcaaaata 1320

atagggcagt ttgaacgtaa tgggaagaca ttaattgctg aaattctatt gcggccttca 1380

ctatcaggta ctactgcggt tcaacaagag ctttatttga aaaatgacac aactcaatcc 1440

gtttcttatg ggacctttat tggtcaagat acaatgttaa acggtaatga taaagtaccg 1500
atgtcatcaa tgggcaataa cgcaggatta tatatcactc aaaatcccta taaattggcc 1560
atcaatatga aggttcctga tggaccaatt aattatgcgg cacaaacttg gactaggaga 1620
aatccttggt ttgatgggtt cacaccacgt aatttttctg gaacgggctt ggaacaaaag 1680
aatttagaag agggttacac agttttaaaa aattccgata cttcatatac agccaagtgg 1740
ccgtttgcta ccttggcccc tggtgaatca caacattatc gtcaagatat agggattact 1800
aaagcccctg acgttgcacc agaagcttat aaagaatatc aaaatgaaac tagtactgat 1860
gggagtaata gaccaggcga taatataaag tttacattac gtgctcataa ttcgggattg 1920
gattcttctt ggagtgatgt ttcgtttagt gatattattc cgtccgaatt tcaaattaat 1980
acaaattcaa taaggttaat aaataaaagc ggtcaagaaa taaccatttc accgtcagct 2040
tacaatgaag taactcgaga gttgaaagtt acggttccta acagtgttaa agataaccag 2100
tgggttagtg tcacatttga agcgaaggct ttaactacag cttctgggcg aactgttcgt 2160
aacactgtca atgttgttgg tatagacagt aatgttaatc aacaagaacg ttcagcaagt 2220
gcgatagtcg atgtaccgtt tatcaaagta caattaccag aattaacgaa aaaagtaaaa 2280
aatatttcta gattagatgc gaagtatgct actgaaacag aagcaagtat tggcgatgaa 2340
attggttata aattagtttt cactaacact aattctgagg aagtaccgga ggccattatt 2400
gaagatccgc tcgattctga tctagatgaa ccaagcgctg ttcatgttag ttataaagat 2460
tcaaacggga gtgaggtgag aagcgaagac ttaaccttta ctggtaatca attaatttta 2520
aaagctatcc cagcggatgg aagtgtggtg ctaacttttg agactattct caaagacact 2580
aaaaagaccg ttattaataa tatagctatg gttgctgatg ttaaatcaaa tgtcgctaaa 2640
gtcaatgtta tcaaaaaaac accaccaacc ctaattaagg aagttaaaaa tataacgaag 2700
tccgacgcag attatatgac agaaacaacg gctgaagttg acgatgagat tgaatatcgg 2760
atacaaattg ttaacactgc ctcaggcgat attaaaccgg gtgcaatatt aaaggatgtt 2820
tttgatgctg acttaggtga tattcaacaa gttagaattg attatttgga caaagatgaa 2880
aaaattattg cgaagcaaac aactgattgg actgataacc aggttatctt ggatcatggt 2940
attccgattg cgggtcaagg tatggcgatt gcttatgtca aagccaaagt aaaagagact 3000
aataaatcag tgattaataa tagtgttagt ctggacaccg attttggagc tggggcttct 3060
gaaaaagcca aattaaatat taaacagtct aaaggccgga ttgttgttcg ctatcgggac 3120
cgaaaagatg aaagccacaa gttggccgaa gatgagacct ttgatggcaa aattgggaac 3180
actagactag tgcaacccaa agttattcca gaaacagacg gcaattggac cgtggttgat 3240
tcttctaata tggtagatcc agattggggc tcaaccacga aacctgattg gacattggct 3300
catgatcata ctgtcactta cgccaaagat gaacaagtca ttacttatcg atatgaagaa 3360
tcccacattg gcattattgc cgataaacgc tgggattttg gtaaacatga tacgactggc 3420
actgaccgaa actattatct aaaagctaaa actaaagaca atcaaaaaca gccatacgcc 3480

gtcagcgttg aggattacta cacctctaag ggttggactt taaatgttaa acaagatgat 3540

cagttccata ccaatgctaa tgaaaagatt gctggagatc aaaaattctt agataatgcc 3600

gtcttaaatt tccataatgg gcagattgtc ttaaaagaaa gtgatgacgt aggggcaacc 3660

gccccagttt ctaaggtaac ttctgagttt gagttaacac caaaaggcgc ggcagttaat 3720

ttaatgacgc acaccaataa gacacctaat cctggttatt atgccgcaca tggctttggg 3780

atttgggctt atcaatttgg ggatgcccaa caggctgatt acagcattgg tttgaaagtg 3840

cctaaagcaa caaaacgatt cccaagacaa tatacaagcc aattaacttg gtcattagta 3900

atcgcagaat aa 3912

<210> 60
<211> 3921
<212> DNA
<213> Lactobacillus sakei

<400> 60
atgttcaggt tgttgttgtt agcagtaatg ggtatacaac tgattgttac taccggaacg 60

agtcttgctg aaagtagtat caaagtagat tcagatcttg cattaacatc ggatcaggaa 120

ttatctaatg aaaaacaaat tgtaattagg gtgagcgatc agtcaaaagc gcaacgtgat 180

ttagggttaa ctataccaga gggggccgca tttgatcgca caactagtaa tcaattaaac 240

aaaaaatcag cgtatacggc tatccttgat caaaaaaata attccaatca agttatttta 300

aaacaaacag cacaaacagc acaaacagct gaaaatattg agaacgattc ggtaaataga 360

caagcaacaa cgttcaaaaa agaatactta ttagtcttta aactaaaaga tactagtata 420

ttcgctaaac aacagttgaa agtgacaaca ataatgaacg gcgttcacta tgaatctaaa 480

ccactattgc tgaaagatat cgttggaaca gatggtgttc aacgacaaac tagtttgtcg 540

aaggaggata cacctcaaat aacctttaat ctaaatcaaa cagaatatat cgttggagat 600

accatccagt taaaagttga tcttaatgct agtgaaatca atgagcctct ctatattaat 660

cttggcaata aagcaatact aacgccgata aaaatatcta gcgataacgg gggtattaac 720

tttgaatcac aaaataatca gaatacaggg aagaacattt taaagatttc cagtaacgaa 780

ccgataaaga aagattttac gctactagta aaagcgacaa cagttgggaa ggtcgatatt 840

acggcttcta ccgaaattga tcatttaatt tcgaagacag ctactgttaa agtgattgat 900

aacccaacat caaaagcatt agcaattaat cccaatactt ttgatgacgg tattcctaat 960

tgggttcaaa agacaaaaac agaggtcgga agtaaggctt ttcaaggaag tacaagtcga 1020

tttcttaatt ataccttcgg tggtgtgaat ggcggaattg acgcagaagc agattcaaaa 1080

atttctagct acaatgttca gggtagccag attaatatct ttattaaaga taacaataaa 1140

acaatcggcg ctttttttaa gggagaagac actagtagta aagcagtaag aacatcaacc 1200

ggttttggga ttgttttcga accaaatgaa agtaatgatg gacctaaaca agctatctta 1260

gctaacaagt taacaaataa ggcatatttt gtcgggcacg acagcgaggg taaccttgtt 1320

agcaaaataa tggggcagtt tgaacgtaat gggaagacat taattgctga aattctattg 1380

cggccttcac tatcaggtac tactgcggtt caacaagagc tttatttgaa aaatgacaca 1440
actcaatccg tttcttatgg gacctttatt ggtcaagata caatgttaaa cggtaatgat 1500
aaagtaccga tgtcatcaat gggcaataac gcaggattat atatcactca aaatccctat 1560
aaattggcca tcaatatgaa ggttcctgat ggaccaatta attatgcggc acaaacttgg 1620
actaggagaa atccttggtt tgatgggttc acaccacgta atttttctgg aacgggcttg 1680
gaacaaaaga atttagaaga gggttacaca gttttaaaaa attccgatac ttcatataca 1740
gccaagtggc cgtttgctac cttggcccct ggtgaatcac aacattatcg tcaagatata 1800
gggattacta aagcccctga cgttgcacca gaagcttata aagaatatca aaatgaaact 1860
agtactgatg ggagtaatag accaggcgat aatataaagt ttacattacg tgctcataat 1920
tcgggattgg attcttcttg gagtgatgtt tcgtttagtg atattattcc gtccgaattt 1980
caaattaata caaattcaat aaggttaata aatcaaagcg gtcaagaaat aaccatttca 2040
ccgtcagctt acaatgaagt aactcgagag ttgaaagtta cggttcctaa cagtgttaaa 2100
gataaccagt gggttagtgt cacatttgaa gcgaaggctt taactacagc ttctgggcga 2160
actgttcgta acactgtcaa tgttgttggt atagacagta atgttaatca acaagaacgt 2220
tcagcaagtg cgatagtcga tgtaccgttt atcaaagtac aattaccaga attaacgaaa 2280
aaagtaaaaa atatttctag attagatgcg aagtatgcta ctgaaacaga agcaagtatt 2340
ggcgatgaaa ttggttataa attagttttc actaacacta attctgagga agtaccggag 2400
gccattattg aagatccgct cgattctgat ctagatgaac caagcgctgt tcatgttagt 2460
tataaagatt caaacgggag tgaggtgaga agcgaagact taacctttac tggtaatcaa 2520
ttaattttaa aagctatccc agcggatgga agtgtggtgc taacttttga gactattctc 2580
aaagacacta aaaagaccgt tattaataat atagctatgg ttgctgatgt taaatcaaat 2640
gtcgctaaag tcaatgttat caaaaaaaca ccaccaaccc taattaagga agttaaaaat 2700
ataacgaagt ccgacgcaga ttatatgaca gaaacaacgg ctgaagttga cgatgagatt 2760
gaatatcgga tacaaattgt taacactgcc tcaggcgata ttaaaccggg tgcaatatta 2820
aaggatgttt ttgatgctga cttaggtgat attcaacaag ttagaattga ttatttggac 2880
aaagatgaaa aaattattgc gaagcaaaca actgattgga ctgataacca ggttatcttg 2940
gatcatggta ttccgattgc gggtcaaggt atggcgattg cttatgtcaa agccaaagta 3000
aaagagacta ataaatcagt gattaataat agtgttagtc tggacaccga ttttggagct 3060
ggggcttctg aaaaagccaa attaaatatt aaacagtcta aaggccggat tgttgttcgc 3120
tatcgggacc gaaaagatga aagccacaag ttggccgaag atgagacctt tgatggcaaa 3180
attgggaaca ctagactagt gcaacccaaa gttattccag aaacagacgg caattggacc 3240
gtggttgatt cttctaatat ggtagatcca gattggggct caaccacgaa acctgattgg 3300
acattggctc atgatcatac tgtcacttac gccaaagatg aacaagtcat tacttatcga 3360
tatgaagaat cccacattgg cattattgcc gataaacgct gggattttgg taaacatgat 3420
acgactggca ctgaccgaaa ctattatcta aaagctaaaa ctaaagacaa tcaaaaacag 3480

ccatacgccg tcagcgttga ggattactac acctctaagg gttggacttt aaatgttaaa 3540

caagatgatc agttccatac caatgctaat gaaaagattg ctggagatca aaaattctta 3600

gataatgccg tcttaaattt ccataatggg cagattgtct taaaagaaag tgatgacgta 3660

ggggcaaccg ccccagtttc taaggtaact tctgagtttg agttaacacc aaaaggcgcg 3720

gcagttaatt taatgacgca caccaataag acacctaatc ctggttatta tgccgcacat 3780

ggctttggga tttgggctta tcaatttggg gatgcccaac aggctgatta cagcattggt 3840

ttgaaagtgc ctaaagcaac aaaacgattc ccaagacaat atacaagcca attaacttgg 3900

tcattagtaa tcgcagaata a 3921

<210> 61
<211> 5938
<212> DNA
<213> Lactobacillus sakei

<400> 61
tacaagcgct tgaatggatt gtaatcggca acggtgttgg ttttggcaaa caccctggcg 60

acttggtcga tgaatcaaag attgaacgcc gttttatcgc aaccggttca aaggggttcg 120

atgcaaccca agttcaggca ctaacagaaa tcaatgcaaa aatgctaacc attatgtccg 180

aaatcgttac gttagttgag ccagcattag atactaagat taacgatcgc caatatctga 240

ttctagcgga ccatctccaa ttcgccgtta aacgcatcaa cgcccatgtc acgttaaacg 300

atcaatcatt actctgggaa ttacgtaatt tatttccaac tgaatataaa actgcgcagc 360

agactattca actgatcaat caaaaattag acttcaaact acctgatggt gaagcagtgc 420

ccctcactta tcattttgta aatatcaaaa acggcgacag tgaaatcgaa gacacgatga 480

ccatggccaa gttggtcgct aacatcattg gtatcgtcca aaaaaactac ggcattgtgc 540

tagacgaaga gtcgttcaat tacagtcgct tcatctccca tcttcgctac ttcatcattc 600

gacgtctcaa aaagacgcaa gaaacagaca cacaacttga tccggcacta cttgctatga 660

tgatcacacg ttatccacaa gcgtatcaag ttgtccaaca aattacggca tttttgaaac 720

ttgagataga ttgggcctta caatccgacg aagaagttta cttattattg catatctggc 780

gtgtcaccca gcatcaacaa actgaataaa atggtgtgtt actgattcga tcaggcatta 840

acccagttaa cgaatgcgac ctatttttag gctgtattca ttagctgggt ttttgtttta 900

acaattatta atctaaggag gtactatcat ggcttacgaa caacttgcaa cagacatctt 960

ggcaaacgtg ggcggacccg aaaatatccg cagcgtcgtc cactgtacga cgcgcttgcg 1020

cttcaaattg aaggacgaaa aaaaggcgca cactgatatt ttaaaaaata tggacggcgt 1080

tgtgacggtt gtccaaagtg gtggtcaata ccaagtcgtt atcggtaatc aagtcgctga 1140

cgtttatgac gcacttttga aaatcggcaa tattaccggt gaaggcatcg tccctgatga 1200

tgacgaccaa caacctaaaa tgagtttact cgataaattc atcgatttaa tttctggcat 1260

cttcgcacca acgcttggcg tcttagcggc aaccgggatg attaaaggcc tcgtcgccct 1320

ctttgtcgca gtcggttggc tcaccatcaa atctggcact tatcaaattc tatttgcgct 1380

cggcgattca ttcttctatt tctttccaat tatgttaggc tacagtgcca gcaagaaatt 1440
caaagtcaat ccgttcctcg gtatggtgat tggggcctcc ctcgtttacc cgtcaatcgt 1500
gggacgagtc ccttcagcgc tggcgcaaac aggcgctaaa ccactttaca ctttgtttgc 1560
cggtacgcta ttcgaatcac caatttacac aacattcctt ggtattccag tgattatgat 1620
gagttacgcg tcatccgtca tcccaatcgt ggttgctgtt tggttcacct caaaagttga 1680
acccttcttc aaaaaagtct tacccgaagt gattcggaca ttcttagtac cgttttgtac 1740
ggccctcgtc gttgtgccgg tgaccttctt agtgattggt ccactgtcaa cttggcttgg 1800
tgatctatta ggcgctggta gtaatgcgct ctatcaattc agtccagtag ttactggggc 1860
agtgcttggt ggcctctggc aagtgctcgt tatcttcggc cttcattggg ggattgtccc 1920
actggcaatt ctcaacattt ccacactggg ttacgacacg attttaacac ttgtctttgc 1980
cgcatccttt gcacaaatcg gatctgtttt agccatcaca ttacgaacga aaaataaaaa 2040
gctcaaaggc ttaggctggt catctttcat ctctgggatt ttcggtgtca cagaacccgt 2100
aatttacggg attacactgc cacgtaaaaa gccattcatc ttcagttgta tcggtggtgc 2160
agtcggcgga ttgctactcg gcttatttgg cactcgcggc tacctcgtgg gtggactcgg 2220
tgtcttcggt attccgaact ttatcaatcc aaagaccggt ctcgatatga ccgtttacgg 2280
tgcgctaatt gctgccgctg ttgccttcgt aattgcatta ttattaacac ttttcttcgg 2340
catttctaac gaagatcaag tggcaaccgt tgaacccgct gctcctatgg cacctactcc 2400
tccgacagaa gcgcctgcca aagtaacacc tgaaatcatt actagtcccc ttagtggcac 2460
cgtaatacct ctttcagaac taaaagatcc tgttttctca tcagctgtca tgggcaaagg 2520
ggtggcaatc actccttctg atggccaact tttcgcaccc gttgccggaa cagtcagcct 2580
cgtctttcca actggacacg caattggtat taattctgaa cacggtgctg aaattttaat 2640
gcatgtcggg atggataccg tccaacttga gggcaaacca tttacggtac atatgaaaca 2700
ggggcaagca gtcctccccg gtgatttatt attaaccttc gatattgaag ccattcaagc 2760
ggccggttac gaaatcgcga caccggtcat cattactaat agtaatttgt acgattcagt 2820
ggcacctctg atttcaaccg gaactgtgtc tactggcgat caactcttaa ctttaaaata 2880
acattcaaag gagactaaag atgactaatc acaccccttt cccaaaagat tttctctggg 2940
gcggcgcaac tgccgctaac caatatgaag gtgcttacca aaccgacggc aaaggtttgt 3000
cgctcgttga tattttgcca gatgctcacc atggccgctg ggaagcccta atgaatccta 3060
aaaaagcgca agcgactcaa tacgacttct atccaagtca caaatccgtt gatttctatc 3120
atcattatga agaagacctg cgcttggtgg ctgagatggg ctttaaaacc tatcggctat 3180
caattagctg gcctcgtatt ttcccaaatg gtgatgattt aacacctaac gaagccggtc 3240
tcgcctttta cgatcgcgtg attgatctgt gtcgcgagct caacattgaa ccactcgtga 3300
ccatcaacca tttcgatacc ccactagcga tgatggataa gtgtaacggc tgggccaatc 3360
gccagatgat cgaccactac ttgcgctttt gttcggtgct ttttaaacgc tatagaggta 3420

aagttcacta ttggttaacg tttaacgaaa tcaacatgat tctccacatt ccgttcttag 3480
gcggtggcat ggacatttcc gatgctgctc atccagatca aattaaatac caagcttccc 3540
atcaccaatt agtggcttca gcgttagcaa ccaagcttgc acatgaaatc gatccagata 3600
atcaaattgg ctgcatgcta gccgctggcg aaacttatcc gaatacgcca aatccagcgg 3660
atgtcttcaa agcacttgaa accaatcgcg aaggctactt cttcatcgat gtgcaatcac 3720
gtggtgctta tccgaactat gccaagcggt tatttaaagc caaaaatatc gtattagcaa 3780
tgggacctga tgatgaggcg attctcaaag ccaatacagt cgacttcatt tcatttagtt 3840
attactcatc tcgtttagtt agtgctgatc cagtggtgaa tgaacaaact gaaggtaacg 3900
tcttcgcctc gcttaaaaat ccatatctca aaacaagcga atggggctgg caaactgacg 3960
gtcttggctt acggacgaca atgaatcaac tctatgatcg atatcaaaaa ccactgttta 4020
tcgttgaaaa cggcctaggt gcacgtgata cagtcactgc tgatggtcaa attcacgacg 4080
actaccgtat cagttatctg caagaaaata tcaccgctat gggccaagca attgccgacg 4140
gcgtaccctg tatcggttac acttcctggg gctgtatcga tttagtcagc gccggttcag 4200
gtgaaatgga caagcgttac ggttacgtat acgttgaccg cgacaatgat ggtaacggca 4260
cattagcccg tacaccaaag gattctttct actggtatca aaatgtcatt aaaaccaacg 4320
gtagcgtgtt atataacggc caaacatttt aatttttgga gggacaatta tgtattcaaa 4380
acaaccaatt accggcttta aaccagattt cttatggggt ggtgcaaccg cggctaatca 4440
aattgaaggt gcctggaata tcgacggcaa ggggctcacg accgccgaag tcgtccaaaa 4500
agcgaccgat cgccagaatt tttcaatgaa caccgtgact aaagacagca ttcaagtcgc 4560
aattgacgat ccaaccgata ccctttatcc aaaacggcac ggaatcgatt tttaccacca 4620
ctacaaagaa gacatcgcat tattcgccga aatgggcttc aaggcttttc gaataagtat 4680
cgcatgggct cgtatttttc caaacggcga tgacgcgcaa cccaatgaaa taggcttagc 4740
cttctacgat aacgtcttcg ctgaactcaa aaaatacaat atcgagccag tcgtgaccct 4800
ctcccattac gaaatgccgc ttgcactgac actgaaatat aacggttgga ccagtcgcga 4860
aaccatcact gcgtttaacc gctataccga aacggtcttc aaacgttatc aacatcaagt 4920
taaatactgg atgactttta acgaaattaa tgccgcaact tggggattca ctggtaccgg 4980
cgcggttgac gataccctct ctccaaatga gcaattacaa ctgcgctatc aagcgctgca 5040
tcatcaattt gttgcgagta gcatcgccgt taaacaatgt catgaattga tgcccgatgc 5100
gaaaattggt tcaatgttgg cgcggatgca aagttatcct aaaacaccga accccaccga 5160
tgtccgtcaa gcgcaagaag atgatgaatt gaatctcttt ttcacagatg ttcaggtccg 5220
tggcgaatat cccaactata tgaaccgtta cttcaacgac cacgacatcg aacttgaaat 5280
ggcacctgac gacttacaaa taatcaaaga ttatccggtc gattatttaa gcttcagcta 5340
ttacatgtca atggtcagct cagctaaacc cgccggtgaa aaaacagctg gcaacttaat 5400
cctcggtgaa aagaatccgt atctcgaatc aagcgactgg ggttggcaaa ttgatcccgt 5460
cggtttacgg attaccctca agaatctctg ggaacgctat ggtgtaccac tattcatcgt 5520

cgaaaatggc ttaggcgcaa tcgataaggt tgcagctgac ggtcaaattc atgaccctta 5580

tcgtattgac tacatgcgca aacacatcgc acagatgaaa gaggccgttc aagacggcgt 5640

ggatctaatg ggctacacca tgtggggacc aatcgactta attagtgcct ctacctctga 5700

aatgtccaag cgctacggct ttatttacgt cgaccaagat gatgacggca acggtacgct 5760

cgaacgacgc cgtaaagact cgttctactg gttcaaaaac gttattgcca acaatggtga 5820

agacctctaa aacatagaaa aagcccccga cttttgtccg ggcttttttc ttatactgta 5880

aaaataaatc gatcgaatgc ttgcgcaatc ccatcagaat cgttatcggc ggtaataa 5938

<210> 62
<211> 5935
<212> DNA
<213> Lactobacillus sakei

<400> 62
tacaagcgct tgaatggatt gtaattggta acggtgtcgg ttttggcaaa caccctggcg 60

acttggtcga tgaatcaaag attgaacgcc gttttatcgc aaccggttca aaggggttcg 120

atgcaaccca ggttcaggca ctaacagaaa tcaatgcaaa aatgctaacc attatgtccg 180

aaatcgttac gttagttgag ccagcattag atactaagat taacgatcgc caatatctga 240

ttctagcgga ccatctccaa ttcgccgtta aacgcatcaa cgcccatgtc acgttaaacg 300

atcaatcatt actctgggaa ttacgtaatt tatttccaac tgaatataaa actgcgcagc 360

agactattca actgatcaat caaaaattag gcttcaaact acccgatggt gaagcagtgc 420

ctctcactta tcattttgtg aatatcaaaa atggcgacag tgaaatcgaa gatacgatga 480

ccatggccaa gttggtcgct aacatcatta gtatcgtcca aaagaactac ggcattgtgt 540

tagatgaaga gtcgttcaat tacagtcgtt tcatctccca tcttcgctac ttcatcattc 600

ggcgtctcaa aaagacgcaa gaaacagaca cacaacttga ccccgcactg cttgccatga 660

tgatcacacg ttatccacag gcgtatcaag ttgtccaaca aatcacggcc tttttaaaac 720

ttgagatgga ttggacctta caatctgacg aagaagttta cttattattg catatctggc 780

gtgtcaccca gcatcaacaa actgaataaa atggtgtgtt actgattcga tcaggcatta 840

acccagttaa cgaatgcgac ctatttttag gctgtattca ttagctgggt ttttgtttta 900

acaattatta atctaaggag gtactatcat ggcttacgaa caacttgcaa cagacatctt 960

ggcaaacgtg ggcggacccg aaaatatccg cagcgtcgtc cactgtacga cgcgcttgcg 1020

cttcaaattg aaggacgaaa aaaaggcgca cactgatatt ctaaaaaata tggacggcgt 1080

tgtgacggtt gtccaaagtg gtggtcaata ccaagtcgtt atcggtaatc aagtcgctga 1140

cgtttatgac gcacttttga aaatcggtaa tatcgctggt gaaggcatcg tccctgatga 1200

tgacgaccaa caacctaaga tgagtttact cgataaattc atcgatttaa tttctggcat 1260

cttcgcacca acccttggtg tcttagccgc aactgggatg attaaaggac tcgtcgcgct 1320

ctttgtcgta gtgggctggc tcacgactaa atctggcact tatcaaattt tattcgcact 1380

cggtgattca ttcttctatt tctttccaat tatgttaggc tacagcgcta gcaagaagtt 1440

caaggtcaat ccgtttctcg gtatggcgat tggcgcctcc ctcatttacc catcaatcgt 1500
gggcttagtc ccttcagcgc tggcgcaaac tggcgctaaa ccactttaca ctttgtttgc 1560
cggtacgcta tttgagtcac caatctacac aacattcctc ggtattccag taattatgat 1620
gagttacgcg tcatccgtca tcccaatcgt ggttgccgtt tggttcacct caaaagtcga 1680
acccttcttc aaaaaagtct tacccgaagt gattcggaca ttcttagtac cgttttgtac 1740
ggccctcgtc gttgtgccgg tgaccttctt agtgattggt ccactgtcaa cttggcttgg 1800
tgatctatta ggcgctggta gtaatgcgct ctatcaattc agtccagtag ttactggggc 1860
agtgcttggt ggcctctggc aagtgctcgt tatcttcggc cttcattggg ggattgtccc 1920
actggcaatt ctcaatattt ccacattagg ttacgacacg atcttaacgc ttgtctttgc 1980
cgcatccttt gcacaaatcg gatctgtttt agccatcaca ttacgaacga aaaataaaaa 2040
gctcaaaggc ttaggccggt catccttcat ctctgggatt tttggtgtca cagaacccgc 2100
aatttacggg attacactgc cgcgtaaaaa gccattcatc ttcagttgta tcggtggtgc 2160
agtcggtgga ttgctactcg gcttattcgg cactcgcggt tacctcgtgg gtggacttgg 2220
tgtcttcggt attccgaact ttatcaatcc aaagaccggt ctcgatatga ccgtttatgg 2280
tgcgttaatt gctgccactg ttgccttcgt aattgcatta ttattaacac ttttcttcgg 2340
catttctaaa gaagatcaag tggcaacggt tgaacccgca actcctatga caccaactcc 2400
ggcagaaacc cctgccaaag taacgcccga aatcattact agtcccctta gcggcaccct 2460
agcacctctt tcagaactta aagatcctgt tttctcatca gcagtcatgg gtaaaggagt 2520
ggcaatcatt ccttctgatg gccaactttt cgcacccgtt gccggaacag tcagtctcgt 2580
ctttccaact ggacacgcaa ttggtattaa ttctgaacac ggtgctgaaa ttttaatgca 2640
tgtcgggatg gataccgtcc aacttgaggg caaaccattt acggtacatg tgaaacaggg 2700
tcaagcagtc ctccccggtg atttattatt aaccttcgat attgaagcca ttcaagcggc 2760
cggttacgaa atcgcgacac cggtcatcat tactaatagt aatttgtacg attcagtggc 2820
acctctgatt tcaaccggaa ctgtgtctac tggcgatcaa ctcttaactt taaaataaca 2880
ttcaaaggag actaaagatg actaatcaca cccctttccc aaaagatttt ctctggggcg 2940
gcgcaactgc cgctaaccaa tatgaaggtg cttaccaaac cgacggcaaa ggtttgtcgc 3000
tcgttgatat tttgccagat gctcaccatg gccgctggga agccctaatg aatcctaaaa 3060
aagcgcaagc gactcaatac gacttctatc caagtcacaa atccgttgat ttctatcatc 3120
attatgaaga agacctgcgc ttgatggctg agatgggctt taaaacctat cggctatcaa 3180
ttagctggcc tcgtattttc ccaaatggtg atgatttaac acctaacgaa gccggtctcg 3240
ccttttacga tcgcgtgatt gatctgtgtc gcgagctcaa cattgaacca ctcgtgacta 3300
tcaaccattt cgatactcca ctagcgatga tggataagtg taacggctgg gccgatcgcc 3360
ggatgatcga ccactatctg cgcttttgtt cggtgctttt taaacgctat agaggtaaag 3420
ttcactattg gttaacgttt aacgaaatca acatgattct ccacattccg ttcttaggcg 3480

gtggcatgga cgtttccgat accgctcatc cagatcaaat taaataccaa gcttcccatc 3540
accaattagt ggcttcagcg ttagcaacca agcttgcaca tgaaatcgat ccagataatc 3600
aaattggctg catgctagcc gctggcgaaa cttatccgaa tacgccaaat ccagcggatg 3660
tcttcaaagc acttgaaacc aatcgcgaag gctacttctt catcgatgtg caatcacgta 3720
gtgcttatcc gaactatgcc aagcggttat ttaaagccaa aaatatcgta ttagcaatgg 3780
gacctgatga tgaggcgatt ctcaaagcca atacagttga cttcatttca tttagctatt 3840
actcatctcg tttagttagt gctgatccag cggtgaatga acaaactgaa ggtaacgtct 3900
tcgcctcgct taaaaatcca tatctcaaaa caagcgaatg gggctggcaa actgacggtc 3960
ttggcttacg gacgacaatg aatcaactct atgatcgata tcaaaaacca ctgttcatcg 4020
ttgaaaacgg cctaggtgca cgtgatacag tcactgctga tggccaagtt cacgacgact 4080
accgtatcag ttatctgcaa gaaaatatca ccgctatggg ccaagcaatt gccgacggcg 4140
taccttgtat cggttacact tcctggggct gtatcgattt agtcagcgcc ggttcaggtg 4200
aaatggacaa gcgttacggt tacgtatacg ttgaccgcga caatgatggt aacggcacat 4260
tagcccgtac accaaaggat tctttctact ggtatcaaaa tgtcattaaa accaatggta 4320
gcgtgttata taacggccaa acattttaat ttttggaggg acaattatgt attcaaaaca 4380
accaattacc ggctttaaac cagatttctt atggggtggt gcaaccgcgg ctaatcaaat 4440
tgaaggtgcc tggaatatcg acggcaaggg gctcacgacc gccgaagtcg tccaaaaagc 4500
gaccgatcgc cagaattttt caatgaacac cgtgactaaa gacagcattc aagtcgcaat 4560
tgacgatcca accgatacgc tttatccaaa acggcgcgga atcgattttt atcaccacta 4620
caaagaagac atcgcattat tcgccgaaat gggcttcaag gctttccggg tgagtatcgc 4680
gtgggctcgt atttttccaa acggcgatga cgcgcacccc aatgaaatag gcttagcctt 4740
ctacgataac gtcttcgctg aactcaaaaa atacaatatc gagccagtcg tgaccctctc 4800
ccattatgaa atgccgcttg cactgacact gaaatataac ggttgggcca gtcgcgaaac 4860
catcactgcg tttaaccgtt ataccgaaac ggtcttcaaa cgttatcaac atcaagttaa 4920
atactggatg acttttaacg aaattaatgc cgcaacttgg ggattcactg gtaccggcgc 4980
ggttgacgat accctctccc caaatgagca attacaactg cgctatcaag cgctgcatca 5040
tcaatttgtt gcgagcagta tcgccgttaa acaatgtcac gaattaatgc caaacgccaa 5100
aattggttca atgttggcgc ggatgcaaag ttatcctaaa acatcgaacc cagtcgatgt 5160
ccgtcaagcg caagaagatg atgaattgaa tctctttttc acagatgttc aggtccgtgg 5220
cgaatatccc aactatatga accgttactt caacgaccac ggcatcgaac ttgaaatggc 5280
acctgacgac ttacaattaa tcaaagatta tccggtcgat tatttaagct tcagctatta 5340
catgtcaatg gtcagctcag ctagacccgc cggtgaaaaa acagctggca acttaattct 5400
cggtgaaaaa aatccgtatc tcgaatcaag cgactggggt tggcaaattg atcccgtcgg 5460
tttacggatt accctcaaga atctctggga acgctatggt gtaccactat tcatcgtcga 5520
aaatggctta ggcgcaatcg ataaggttgc agctgacggt caaattcatg acccttatcg 5580

tattgactac atgcgcaaac acatcgcaca gatgaaagag gccgttcaag acggcgtgga 5640

tctaatgggc tacaccatgt ggggcccaat cgacttaatt agtgcctcta cctctgaaat 5700

gtcaaaacgc tacggcttca tctacgtcga ccaagatgat gacggtaacg gtacgctcga 5760

acggagccgt aaagactcgt tctactggtt caaaaatgtc attgctagca atggcgaaga 5820

tctctagaac atagaaaaag cccccgactt ttatcagggg ctttttctta tactgtaaaa 5880

ataaatcggt cgaatgcttg cgtaatccca acataattgt tatcggcagt aataa 5935

<210> 63
<211> 1840
<212> DNA
<213> Lactobacillus sakei

<400> 63
ttatggccaa tttgctcgta ttgtttctag aatggaggcg atttggcgtt tattatgggt 60

taaatagttt gcgtaaattg gcatcttggc ctgttcatca ctaattggaa ccgtaattct 120

atctaattga tgtggtgata attcgttgaa gaaagcatcc ttttgtgaga ggttggaagt 180

gaaaaatggt aggcttgagt gcttaataat ctcaatgaag gcggcgcggt cgtcttgata 240

cataaattgc gcgttgggaa tatcttgttt aatcatatcg ctccaggggc cgattgcgcg 300

catgacgacg aaactcatgc cagctaaatc ttgaaagcta acggttgatt ggctagcaaa 360

cgtcgtgaat tgatcgagat taatttgaag ctgttcttga ccgaggtatt gagattcgat 420

ggtcgtatca aggatttctt gatctgaaaa gattagggca acttggtctg tctttagtgc 480

attactaatt tgatccaccg caatgaattc tttggtgatt tgcacatcat cacggttagg 540

cacgagggtt agcggtccgg gaatcgttgc gccaattatc gtaaagtgct gaccgcgatc 600

gaaattttga attttagtca gcatcgtttg actagtcgtc aaaagttgtt cagcttctcg 660

agcggcaagc tcgcctgttg gtgtcagttt aatgcgatta gcttgacggt caaacagctg 720

gacaccaaaa tcctcttcca acttttgcat gccgcgtgtg acggatggct gggtgatgtt 780

caaagcagca gcagttttcg tgagggtgcc agtcttggcg aaggtcgtca attgttctaa 840

taaatattaa tctaacattc gctgttctcc ttagtatacg tttaatgtat actagcatgt 900

tttttaagta attttcaagt gagccactgc gcactatact aaagtcatca attagaaaaa 960

gaggttatca acgatggata tggtgcaatt aaacaatggc gtacaaatgc cacaattagg 1020

ttttggtgtt ttccaaatga cggatttagc agaatgtgaa caagcagtgg ttgatgcaat 1080

tgcatcaggg taccgtttga ttgatacagc ggcagcttac cataatgaag ccgcagttgg 1140

ccgggccatt aaacgcagcg gcgtggcgcg cgaagattta ttcattacct ctaaactctg 1200

ggtatcagat gccagttatg aacgtgctaa aaaagggatt gaccaatcac tccaaaacat 1260

aggggttgat tatttagacc tctacttact tcaccaacca ttcggcgatg tgattggggc 1320

atggcgggct ctagaagaag catacaaagc cggcaagatt cgcgcaatcg gcgtttctaa 1380

tttctatgcc gatcaactta aaaatttaat tttatcaacc aatgtcaaac cagtgattaa 1440

ccaaattgaa gttaatcctt ggtatcaaga acattcagaa gttgcttttg cacaagcgga 1500

agagattcaa gttgaagctt gggcaccatt tgcagaaggc aaacacaata tctttactga 1560

tgaacgaatt gccgcaattg gtagtcgtta tggtaaatca aacggacaag tgatcttaag 1620

atggttactt caacgcggca ttgtggtgat tccgaagtct gtgcaccaaa atcggatggc 1680

cgaaaatatc aatgtctttg attttgaatt aaccacagct gaaatggcaa cgatgaatga 1740

tttggacaaa ggcgaaagcc aattcttcga tcaccgtgat ccagcagcaa tcgaacaaat 1800

ttttgggacg agcttagcgg ctttaaagga gaataaataa 1840

<210> 64
<211> 1840
<212> DNA
<213> Lactobacillus sakei

<400> 64
ttatggccaa tttgctcgta ttgtttctag aatggaggcg atttggcgtt tattatgggt 60

taaatagttt gcgtaaattg gcatcttggc ctgttcatca ctaattggga ccgtgattct 120

gtctaattga tgtggtgata gttcgttgaa gaaagcatcc ttttgtgaga gattggaagt 180

gaaaaacggt aggcttgaat gtttgatgat ctcaatgaag gcggcgcggt cgtcttgata 240

cataaattgt gcgttgggaa tatcttgttt aatcatatcg ctccaggggc cgattgcgcg 300

catgacgacg aaactcatgc cagctaaatc ttgaaaacta accgttgatt ggctagcaaa 360

cgtcgtgaat tgatcgagat taatttggag ctgttcctga ccgaggtatt gggattcgat 420

ggctgtatca aggatttctt gatctggaga gattagggca acttggtctg tctttagtgc 480

attactaatt tgatccaccg cgatgaattc tttggtgatt tgcacatcat cacggttagg 540

cacgagggtt agcggtccgg gaatcgttgc gccaattatc gtaaagtgtt gaccgcggtc 600

gaaattttga attttagtca gcatcgtttg gctagctgtc aaaagttgtt cagcttctcg 660

agccgcaagc tcgccagttg gtgtcagttt aatgcgatta gcctgacggt caaacagctg 720

gacaccaaaa tcctcttcca acttttgcat gccgcgtgtg acggatggct gggtgatgtt 780

caaagcagca gcagttttcg tgagggggcc agtcttggcg aaggtcgtca attgttctaa 840

taaatattaa tctaacattc gctgttctcc ttagtatacg tttaatgtat actagcatgt 900

tttttaagta attttcaagt gagccactgc gcactatact aaagtcatca attagaaaaa 960

aaggttatca acgatggata tggtgcaatt aaacaatggc gtacaaatgc cacaattagg 1020

ttttggtgtt ttccaaatga cggatttagc agaatgtgaa caagcagtgg ttgatgcgat 1080

tgcatcaggg taccgtttga ttgatacagc ggcagcttac cataatgaag ccgcagttgg 1140

ccgggccatt aaacgtagcg gcgtggcgcg cgaagaacta ttcattactt cgaaactctg 1200

ggtatcagat gcgagctatg aacgtgctaa aaaagggatt gaccaatcac tccaaaattt 1260

aggcgttgat tatttagatc tttacttact tcaccaacct tttggcgatg tgattggggc 1320

atggcgggct ctagaagaag catacaaagc cggcaagatt cgcgcaatcg gcgtttctaa 1380

tttctatgcc gatcaactta aaaatttaat cttatcaacc gatgtcaaac cagtgattaa 1440

ccaaattgaa gttaaccctt ggtatcaaga acactcagaa gtggcttttg cacaagcgga 1500

agagattcaa gttgaagctt gggcaccatt tgcagaaggc aaatacaata tttttactga 1560

tgaacgaatt gcggcaattg gcgagcgtta cggtaaatca aacggacaag tcatctcaag 1620

atggttactt caacgcggca ttgtggtgat tccgaagtct gtgcaccaaa atcggatggc 1680

cgaaaatatc aatgtctttg attttgaatt aaccacagcc gaaatggcaa cgatgaatga 1740

tttggacaaa ggcgaaagtc aatttttcga tcatcgtgat ccagcggcaa ttgaacaaat 1800

ttttgggacg agcttagcgg ctttaaagga gaataaataa 1840

<210> 65
<211> 930
<212> DNA
<213> Lactobacillus sakei

<400> 65
accacttata acgatgcctt atattgcccg tgttttaggc ccaacgggtg ttgggattaa 60

ctcatttacc aattcaaata cacagtattt tattttaatt ggtagtatcg gtgtttcatt 120

gtatggtaat cgccagattg cttattatcg agatgataaa gcgaaaactt ctcaaatttt 180

ttgggaagtt tttctaatga gaatgattac gattcttgta gcactagcag cattctttat 240

tttcttaggg ttcgagaaat cttgtcacca agcatatctg atgcaagcaa ttttaattgt 300

tgctgcagct tttgatattt catggttctt tatggggttt gagaatttca aggtaacggt 360

tcttagaaat attatcgtta agttaatatc gttagcttgt attttcatat ttgttcgtga 420

taaaggtgat ttgacattgt acattgctgt tttgtcaatt tctcaattaa tcggtaatat 480

tacgttattt ccttatttaa aacgatacat taacttacca aattggcgag aattaaaaat 540

ttggcgtcat tttaaacctt ctttagtttt atttgttccc caaattgcaa cccaatttta 600

cttgattctt aataaaacaa tggttgggaa gatggtatca gttgaagcag caggcttcta 660

cgataattcc gataaaatta tcaagatggt attagcgatt gtaaccgcga caggaacagt 720

gatgttacct cgagtcgcta atacatttgc taagggagat catgaacagg ttaaaaaata 780

cttatatcaa tcttttgatt ttgtgagcgc catttcgata ccgatgatgt ttgggatggc 840

ggccatcgct cctaaatttg ctacgatgtt ttttggacaa gcatttgatg cagtaggacc 900

actaatgatg gttgaatcgt tagtaattat 930

<210> 66
<211> 931
<212> DNA
<213> Lactobacillus sakei

<400> 66
accacttata acgatgcctt atattgcccg tgttttaggc ccaacgggtg ttgggattaa 60

ctcatttacc aattcaaata cacagtattt tattttagca ggtagtattg gagtttcact 120

atatggtaat cgtcaaattg catattaccg cgataataaa tccaaagctt ctcaaatttt 180

ttgggaagtt tttctaatgc gaatgattac cattcttgct tcactaattg ctttttttgt 240

gtttttaggg tttgaaaaag aatatcatca agcttatctc atgcaatcta tcttaattat 300

tgcggctgca tttgacatct catggttttt tatgggtttt gaaaacttta aagtaactgt 360

attaagaaac atgattatta aattgatatc attggcatgt atttttattt ttgtgcgagg 420

taagggagat ttaacattat atattgctgt attatctatt tcacaattaa ttgggaatat 480

tacgttattt ccacatttac gtcgatatat taatttatct gattggcgag aatttaagaa 540

tatggcgtca ttttaaaccc tcattagtat tatttgtgcc gcaaatagca acacaagttt 600

atttgattct caataaaacg atggttggga agatggtttc agttgaagca gctggattct 660

atgataattc ggataaggtt gtaaaaatgg tattagcaat tgtaacagct acaggtactg 720

tcatgctacc acgagttgcc aatacattcg ctaaggggga tcatgaacag gttaagaagt 780

atttatatca atcgtttgat tttgtgagtg cggtatcaat tccaatgatg tttggcattg 840

ccgcgattgc gcctcaattt tcaattatgt tctttggaaa agcttttagt tccgctggag 900

aattgatgat ggttgagtct attgttatta t 931

<210> 67
<211> 930
<212> DNA
<213> Lactobacillus sakei

<400> 67
accacttata acgatgcctt atattgcccg tgttttaggc ccaacgggtg ttgggattaa 60

ctcatttacc aattcaaata cacagtattt tattttaatt ggtagtatcg gggtttcatt 120

gtatggtaat cgccagattg cttattatcg agatgataaa gcgaaaactt ctcaaatttt 180

ttgggaagtt tttctaatga gaatgattac gattttagta gcattattag cattttttat 240

ctttttaggg ttcgagaaat cctatcacca agcatatcta atgcaagcaa ttttaatcgt 300

tgcggcagct tttgatattt catggttctt tatgggcttt gagaacttca aggtaacggt 360

tcttagaaat attattgtta agttaatatc attagcttgt attttcatat ttgtccgtgg 420

taaaggtgat ttgacattat acattgctgt tttgtcaatt tctcaattaa tcggcaatgt 480

tacgttattc ccttatttga aacggtatat taatttaccc aattggcgag aattaaaaat 540

ttggcgccat ttcaaacctt ctctagtttt gtttgttccc caaatcgcaa cgcaagttta 600

tttaatactt aataaaacga tggttgggaa aatggtatcg gttgaagcag caggcttcta 660

cgataattct gataaaattg ttaagatggt cttggcaatt gtaacggcga cgggcacggt 720

aatgttacca cgagtggcca atacgtttgc taagggtgat cacgaacaag ttaagaagta 780

tttatatcaa tccttcgatt ttgtgagtgc tgtttcaatc ccaatgatgt ttggaattgc 840

agcgattgcg cctaaatttg ctacgatgtt ttttggacaa gcatttgatg cagtaggccc 900

attaatgatg gttgaatcgt tagtaattat 930

<210> 68
<211> 3260
<212> DNA
<213> Lactobacillus sakei

<400> 68
ccgctattaa acgaatgaat ttaactgcca agattaagtt aattaaattt gatttggttg 60

ttgatgggca gcgggatgag gatgagcgac aactcttaaa tttagccgga gatgactatt 120
tttatcaact gcaatataaa tattatggtg acggtgaatt aatggctttc caaaaagtat 180
caatattatt tgatcaattt ccactaatta gagtatccga gataaaagat aagtcgttat 240
atccgatgct ggccaaaaaa tatgggttta gtaatgtaga ttttgtacag gagacgacaa 300
cagggttagt tacaaataag gaacggaaat ttttagagat aaatacggga tcaccgtttg 360
taaaaattga aaaacaaggt atctcagaac atcgcacagt attgtttgat gaaatctata 420
tgattgattc attaccaatg tatttaattg ataactagaa aattggagga attagatatg 480
tcagaaccta atattgtatt aacaagaatt gataacagat tggtccatgg acaggtaggg 540
gttgtttgga cgtcatcaat tggcgctaat ttgttaattg ttgctaatga cgaaacagcg 600
gaaaatgaat tagaacaaca attaatgtca gcaactgcgg aaaattcggg tgttggtata 660
cgctttttta acttacaaaa aacaattgat gttatccaca aagcagcagc tcgtcaaaaa 720
atatttttag ttgttcgcaa tccacaagat gtattgatgt tagttaaggg cggggttcct 780
gttaaagatt taaatatcgg taacatgcac ttttcagaag gcaagactga aattactaaa 840
aaagtttata tggatgataa tgataaagaa acattaaagg aaattgctga tttaggggtt 900
aacgtcttta ttcaagacgt accagaagat aagcgattaa tgttgggaga tgctattaaa 960
tgacagtaac cttaactcaa gggattctaa ttgctgtatt tgccttgatt gcaggttttg 1020
atttttggtt agaaggcctt tatatcttca gaccaatgat tgtttgtaca gtaacaggat 1080
ttcttttagg cgacttaaca atgggaatta tggctggtgg tttaactgaa ctagcctttg 1140
ctggtttaac accggtcggc ggaacacagc cacctaaccc tgtgatggct ggtattatga 1200
ctgttgtgat tgcccataca acaggacaca gtccagctgc agcaattggt ttggctttac 1260
catttagtat tttaatgcaa tatattatct tgttttatta ttcaattttc cctgttttca 1320
tgacaaaatt agaccggtat gccaaggaag ctaatacaag tgcttatacg cgggtagctt 1380
tattgccaac aggaattgtt atgatcacgt acgctgtttt cacattcctt tgtgtttatg 1440
cagctcaagg accaatgcgc gatttagtga atgcaatgcc tatttggttg agtcatgggt 1500
ttgaaattgc cgggggtatc ttgccagcag tcggatttgg tttattactt aagagcatgc 1560
tgaagagcga tttgattcca tatctgttga tcggttttac ggcagcttgc tttattaaat 1620
tcggcaactt aatgccagtt gctgttattg gtgttgcatt tgctttaatc gaatttaata 1680
gaactaagaa acaaaatgat aatgatcaaa aaatcaagaa aatgatcgaa gaatcaagat 1740
taggaggtga cgaagaagat ggcatctaat aaagaaaaag aaacgacaag tacgattact 1800
aaaaaagata tcaacaaaat ggcactccgt tcaattttta tgcaaacatc aatgaactat 1860
gaacgtatgc agtctgcggg ttggttattg gcaatgctac ctgtattaaa gaaaatctat 1920
aaagatgata aagaaggttt atctagcgca atgcaagata atctagaatt tatcaacact 1980
aataatacgg tagcaccttt attgatggga ttattgacat ccttagaaga aaaccatgaa 2040
aagcgttcaa ctatcaaagg tttgaggatt gccttatttg gtcctttagc tggtattggg 2100

gatgccattt tctggttcac cgttttacca atcgttgcgg ggataacggc ttcttttgct 2160

aaacaaggta gtatattagg tccattagtc ctcttcttag tgtatgtcgg gattttcttt 2220

agtcgaattg ggttcgctag attagggtac tatactggtt caaaagcgat tactaaaatt 2280

cgagacaact caagtatgat ttcgaaggca gctagcatct taggtgtcac agtaattggt 2340

ggtttaattg caacttacgt taacatcaat gtactttcta aaattaaagt ggcggctggt 2400

catacaatct caatccaaac acaattctta gataagatat tccccaatat tttagcttta 2460

ggctatacct tcttattgta ttggttattg aaaaagaaaa atgttagtcc agttgtactc 2520

attttgataa cgtttgtctt agcaattggt ctatcatggt tgggagtgtt gtaacatgga 2580

tattattgtg acaggacatg ggcattttgc gacaggttta tcatcaacta ttcaattatt 2640

agctggtgaa ttaccgcatg ttcattatgt tgattttact aaagaaatgt cagaagaaaa 2700

tttggtggat cagttaaaat caataatggt taaggataaa tcttatgtat tcttttgcga 2760

tttagttggt ggaacaccct ataaggaggc cgctcgattg agtgccgctg atgacaaaag 2820

aataggtgta gttgctgggt gcaatctagc atctttatta gaggtcggaa tttcattagc 2880

agatagtgat aatgatgtgg gttatttaac tgaagaatta gtaactatca gtcaaaaagg 2940

aacccaggta tttaaggtta aaccaatcaa accggaagtt attgaagatg gtatttagag 3000

tttaaaaagg attctattgt gaatatatta ttcacaatag ggtccttttt tatgtaatat 3060

aatcggtagc aatcaataat taaagcgctt agactaccga aaagaggggc atcaatggta 3120

gaattgaatt agaatctgat tgaggaggat gacaatgaca aacgttcgaa tgcaagatga 3180

tttttatgcg gcagtgaatc aggaatggtt aaaaacggcc gaaattccag ctgataaacc 3240

agcaactggt ggctttgtgg 3260

<210> 69
<211> 3260
<212> DNA
<213> Lactobacillus sakei

<400> 69
ccgctattaa acggatgaat ttaactgcca agattaagtt aattaaattt gatttgggtg 60

ttgatgggca gcgggatgag gatgaacgac aactcttaaa tttagccgga gatgactatt 120

tttatcaact gcaatataaa tattacggtg acggtgaatt aatggctttc caaaaggtat 180

caatattatt tgatcaattt ccattaatta gagtatccga gataaaagat aagtcgttat 240

atccgatgct ggccaaaaaa tatgggttta gtaatgtaga ttttgtacag gagacgacaa 300

cagggttagt tacaaataag gaacggaaat ttttagagat aaatacggga tcaccgtttg 360

taaaaattga aaaacaaggt atctcagaac atcgcacagt attgtttgat gaaatctata 420

tgattgattc attaccaatg tacttaattg ataattagaa aaccggagga attagatatg 480

tcagaaccta atattgtatt aacaagaatt gataacagat tggtccacgg acaggtaggg 540

gttgtttgga cgtcatcaat tggcgctaat ttgttaattg ttgccaatga tgaaacagcg 600

gaaaatgaat tagaacaaca attaatgtca gcaactgcgg aaaattcggg tgttggtatt 660

cgctttttta acttacaaaa aacaattgat gttatccaca aagcagcagc tcgtcaaaaa 720
atattcttag ttgttcgcaa tccacaagat gtattgacgt tagttaaggg cggagttcct 780
gttaaagatt taaatatcgg taacatgcac ttttcagaag gcaagactga aattactaaa 840
aaggtttata tggatgataa tgataaagaa acattaaagg aaattgctga tttaggggtt 900
aacgtcttta ttcaagacgt accagaagat aagcgattaa tgttgggaga tgctattaaa 960
tgacagtaac cttaactcaa gggattctaa ttgctgtatt tgccttgatt gcaggttttg 1020
atttttggtt agaaggcctt tatatcttca gaccaatgat tgtttgtaca gtaacaggat 1080
ttcttttagg cgacttaaca atgggaatta tggctggtgg tttaactgaa ctagcctttg 1140
caggtttaac accggtcggc gggacacagc cacctaaccc tgtgatggct ggtattatga 1200
ctgttgtgat tgctcataca acagggcaca gtccagctgc agcaattggt ttggctttac 1260
catttagtat tttaatgcaa tatattatct tgttttatta ttcaattttc cctgttttca 1320
tgacaaaatt agaccggtat gccaaggaag ctaatacaag tgcttatacg cgggtagctt 1380
tattgccaac aggaattgtt atgatcacgt acgctgtttt cacattcctt tgtgtttatg 1440
cagctcaagg accaatgcgc gatttagtga atgcaatgcc tgtctggttg agtcatgggt 1500
ttgaaattgc cggcggtatt ttgccagcag tcggatttgg tttattactt aagagcatgt 1560
tgaagagcga tttgattcca tatcttttaa tcggttttac ggcagcttgc tttattaaat 1620
tcggcaactt aatgccagtt gccgtcatcg gtgttgcatt tgctttaatc gaatttaata 1680
gaactaagaa acaaaatgat aatgatcaaa aaattaagaa aatgatcgaa gaatcaagat 1740
taggaggtga cgaagaagat ggcatctaat aaagaaaaag aaacgacaag tacgattact 1800
aaaaaagata tcaacaaaat ggcactgcgt tcaattttta tgcaaacatc aatgaactat 1860
gaacgtatgc agtctgcggg ttggttattg gcaatgctac ctgtattaaa gaaaatctat 1920
aaagatgata aagaaggttt atctagcgca atgcaagata atctagaatt tatcaatact 1980
aataatacgg tagcgccttt attgatggga ctattaacat ccctagaaga aaatcatgaa 2040
aaacgttcaa ctatcaaagg tttgagaatt gccttatttg gtcctttggc tggtattggg 2100
gatgccattt tctggttcac cgttttacca atcgttgcgg ggataactgc ttcttttgct 2160
aaacaaggta gtatactagg gccattaatt ttcttcttag tatatgtcgg gattttcttt 2220
agtcgaattg ggtttgctag attagggtac tatactggtt caaaagcgat tactaaaatt 2280
cgagacaact caagtatgat ttcaaaggca gctagcatct taggtgtcac agtaattggt 2340
ggtttaattg caacttatgt taacatcaat gtactttcta aaattaaagt ggcggctggt 2400
catacaatct caattcaaac acaattctta gataaaatat tccccaatat tttagcttta 2460
ggatatacat tcttattgta ttggttattg aaaaagaaaa atgttagtcc agttgttctc 2520
attttgataa cgtttgtctt agcaattggt ctatcatggt tgggagtgtt gtaacatgga 2580
tattattgtg acaggacatg ggcattttgc gacaggttta tcgtcaacca ttcaattgtt 2640
agctggtgaa ttatcgcatg ttcattacgt tgattttact aaagaaatgt cagaagaaaa 2700
tttggtggat cagttaaaat caataatggt taaggataaa tcgtatgtat tcttttgcga 2760

tttgattggt gggacaccct ataaagaggc cgctcgattg attgccgctg atgccaaaag 2820

aataggtgta gttgctggtt gtaatctagc gtctttatta gaggtcggaa tttcattaac 2880

agatagcgat aatgatgtag actatttaac cgaagaatta gtaactatca gtcaaaaagg 2940

aacccagata tttaaggtta aaccaattaa acaggaagtt attgaagacg gtatttagat 3000

attaaaaagg attccgctgt ggatgatata ttcacaatag aatctttttt tgtacaatat 3060

tgtcgttagt gattaataat taaagcgctt agactaccgg aaagtagggc atcaatggta 3120

gaattgaatt agaatctgat tgaggaggat gacaatgaca aacgttcgaa tgcaagatga 3180

tttttacacg gcagtgaatc aagaatggtt aaaaacagcc gaaattccag ctgataaacc 3240

agcaaccggt ggctttgtgg 3260

<210> 70
<211> 269
<212> DNA
<213> Lactobacillus sakei

<400> 70
tttatgggtt cacgatttag aagcaatgaa ggctttttat caaacgatgt ttcaagcgac 60

tgcgggtgag ttgtatgaga atcacaaaaa aggattccaa tcatactttt taacttttgc 120

agatggtgtg cgtttagaaa ttatgcacca agatagtgaa ctagccggct tagcaactag 180

gggccaattt gcgcacttag cgatggctgt tggcagtgaa actgcggttg atcaattagt 240

gactaagttt caagcacaag gcttaccat 269

<210> 71
<211> 269
<212> DNA
<213> Lactobacillus sakei

<400> 71
tttatgggtt cacgatttag aagcaatgaa ggctttttat caaacgatgt ttcaagcgac 60

tgcgggtgag ttgtatgaga accacaaaaa agggttccaa tcatactttt taactttcgc 120

agatggtgcg cgtttagaaa ttatgcacca agatagtgaa ctagtcggct tagcaactag 180

gggccaattt gcgcacttag cgatggctgt tggtagtgaa actgcagttg accaattagt 240

gactaagttt caagcacaag gcttaccat 269

<210> 72
<211> 18
<212> DNA
<213> Artificial Sequence

<220>
<223> forward primer for SEQ ID NO: 1

<400> 72
gcggtctatg cggatatt 18

<210> 73
<211> 18
<212> DNA

<213>  Artificial Sequence

<220>
<223>  reverse primer for SEQ ID NO: 1

<400>  73
gtggcactgc catgaaat                                                    18


<210>  74
<211>  18
<212>  DNA
<213>  Artificial Sequence

<220>
<223>  forward primer for SEQ ID NO: 2

<400>  74
ggcgcaatcg ataaggtt                                                    18


<210>  75
<211>  18
<212>  DNA
<213>  Artificial Sequence

<220>
<223>  reverse primer for SEQ ID NO: 2

<400>  75
ccgtcatcat cttggtcg                                                    18


<210>  76
<211>  20
<212>  DNA
<213>  Artificial Sequence

<220>
<223>  forward primer for SEQ ID NO: 3

<400>  76
cagttttatt ggtgtggagg                                                  20


<210>  77
<211>  20
<212>  DNA
<213>  Artificial Sequence

<220>
<223>  reverse primer for SEQ ID NO: 3

<400>  77
cggtcgtgat tgatgtttgt                                                  20


<210>  78
<211>  21
<212>  DNA
<213>  Artificial Sequence

<220>
<223>  forward primer for SEQ ID NO: 4

<400>  78
gtggtaaaga aagtgttgcg a                                                21

<210>  79
<211>  24
<212>  DNA
<213>  Artificial Sequence

<220>
<223>  reverse primer for SEQ ID NO: 4

<400>  79
aatgccacgg cgccattcat cagc                                              24


<210>  80
<211>  20
<212>  DNA
<213>  Artificial Sequence

<220>
<223>  forward primer for SEQ ID NO: 5

<400>  80
gcgattgctg gtaacatagg                                                   20


<210>  81
<211>  20
<212>  DNA
<213>  Artificial Sequence

<220>
<223>  reverse primer for SEQ ID NO: 5

<400>  81
aaaatcttgc aaccgaactc                                                   20


<210>  82
<211>  24
<212>  DNA
<213>  Artificial Sequence

<220>
<223>  forward primer for SEQ ID NO: 6

<400>  82
ccgagaacaa ccgctatagt ggtc                                              24


<210>  83
<211>  24
<212>  DNA
<213>  Artificial Sequence

<220>
<223>  reverse primer for SEQ ID NO: 6

<400>  83
ccaactatcg ataccgccca cgcc                                              24


<210>  84
<211>  24
<212>  DNA
<213>  Artificial Sequence

<220>
<223>  forward primer for SEQ ID NO: 7

<400>  84

attgcacatg ttctaaaaaa tggc                                              24

<210>  85
<211>  25
<212>  DNA
<213>  Artificial Sequence

<220>
<223>  reverse primer for SEQ ID NO: 7

<400>  85
taaaaagaac ctttccctcc gttgg                                             25

<210>  86
<211>  21
<212>  DNA
<213>  Artificial Sequence

<220>
<223>  forward primer for SEQ ID NO: 8

<400>  86
cgtctcctaa aatgacagtg c                                                 21

<210>  87
<211>  21
<212>  DNA
<213>  Artificial Sequence

<220>
<223>  reverse primer for SEQ ID NO: 8

<400>  87
atatcgtccg gaattcttta c                                                 21

<210>  88
<211>  22
<212>  DNA
<213>  Artificial Sequence

<220>
<223>  forward primer for SEQ ID NO: 9

<400>  88
tttggccttt tagccggttt ag                                                22

<210>  89
<211>  22
<212>  DNA
<213>  Artificial Sequence

<220>
<223>  reverse primer for SEQ ID NO: 9

<400>  89
tttcaagctt tcatccaaac cc                                                22

<210>  90
<211>  23
<212>  DNA
<213>  Artificial Sequence

<220>

<223>  forward primer for SEQ ID NO: 10

<400>  90
agttttggta aaggatttat tgg                                              23


<210>  91
<211>  20
<212>  DNA
<213>  Artificial Sequence

<220>
<223>  reverse primer for SEQ ID NO: 10

<400>  91
cgacccataa ttcttcacct                                                  20


<210>  92
<211>  23
<212>  DNA
<213>  Artificial Sequence

<220>
<223>  forward primer for SEQ ID NO: 11

<400>  92
tgacatttgt gatattcgtg ata                                              23


<210>  93
<211>  24
<212>  DNA
<213>  Artificial Sequence

<220>
<223>  reverse primer for SEQ ID NO: 11

<400>  93
tgttcttatc ttcaagccac ttag                                             24


<210>  94
<211>  23
<212>  DNA
<213>  Artificial Sequence

<220>
<223>  forward primer for SEQ ID NO: 12

<400>  94
taatttaatt ttagagtagt gat                                              23


<210>  95
<211>  22
<212>  DNA
<213>  Artificial Sequence

<220>
<223>  reverse primer for SEQ ID NO: 12

<400>  95
ttatatagtt tatttattat gg                                               22


<210>  96
<211>  20
<212>  DNA

<213>  Artificial Sequence

<220>
<223>  forward primer for SEQ ID NO: 13

<400>  96
caattgccat gtgtaacgta                                                   20


<210>  97
<211>  21
<212>  DNA
<213>  Artificial Sequence

<220>
<223>  reverse primer for SEQ ID NO: 13

<400>  97
ccaattgcag ctaacaagta a                                                 21


<210>  98
<211>  22
<212>  DNA
<213>  Artificial Sequence

<220>
<223>  forward primer for SEQ ID NO: 14

<400>  98
aagaaagtta tgagcgaaca gt                                                22


<210>  99
<211>  20
<212>  DNA
<213>  Artificial Sequence

<220>
<223>  reverse primer for SEQ ID NO: 14

<400>  99
accaaagctt ggttcgtatt                                                   20


<210>  100
<211>  22
<212>  DNA
<213>  Artificial Sequence

<220>
<223>  forward primer for SEQ ID NO: 15

<400>  100
tgtaattaat gtaatatatt tg                                                22


<210>  101
<211>  22
<212>  DNA
<213>  Artificial Sequence

<220>
<223>  reverse primer for SEQ ID NO: 15

<400>  101
gtgtattgtg tatatgttat tg                                                22

<210>  102
<211>  20
<212>  DNA
<213>  Artificial Sequence

<220>
<223>  forward primer for SEQ ID NO: 16

<400>  102
actgataaat cagcgtccaa                                                    20


<210>  103
<211>  21
<212>  DNA
<213>  Artificial Sequence

<220>
<223>  reverse primer for SEQ ID NO: 16

<400>  103
ctaaatatcg caacgattgt g                                                  21


<210>  104
<211>  20
<212>  DNA
<213>  Artificial Sequence

<220>
<223>  forward primer for SEQ ID NO: 17

<400>  104
ttaatttatt ttgaatttgt                                                    20


<210>  105
<211>  20
<212>  DNA
<213>  Artificial Sequence

<220>
<223>  reverse primer for SEQ ID NO: 17

<400>  105
taatgatgat gtaataattg                                                    20


<210>  106
<211>  23
<212>  DNA
<213>  Artificial Sequence

<220>
<223>  forward primer for SEQ ID NO: 18

<400>  106
tggtgaaatt acagctaaag atg                                                23


<210>  107
<211>  20
<212>  DNA
<213>  Artificial Sequence

<220>
<223>  reverse primer for SEQ ID NO: 18

<400>  107

acgccataat tatcggtgac                                                    20

<210>  108
<211>  24
<212>  DNA
<213>  Artificial Sequence

<220>
<223>  forward primer for SEQ ID NO: 19

<400>  108
aatttctagc tacaatattc aggg                                               24

<210>  109
<211>  22
<212>  DNA
<213>  Artificial Sequence

<220>
<223>  reverse primer for SEQ ID NO: 19

<400>  109
tatcacctaa gtcagcatca aa                                                 22

<210>  110
<211>  22
<212>  DNA
<213>  Artificial Sequence

<220>
<223>  forward primer for SEQ ID NO: 20

<400>  110
tccttgttgg tgcattataa gt                                                 22

<210>  111
<211>  23
<212>  DNA
<213>  Artificial Sequence

<220>
<223>  reverse primer for SEQ ID NO: 20

<400>  111
cctgagtttt actcaagaat caa                                                23

<210>  112
<211>  18
<212>  DNA
<213>  Artificial Sequence

<220>
<223>  forward primer for SEQ ID NO: 21

<400>  112
ggcgcaatcg ataaggtt                                                      18

<210>  113
<211>  18
<212>  DNA
<213>  Artificial Sequence

<220>

<223>  reverse primer for SEQ ID NO: 21

<400>  113
ccgtcatcat cttggtcg                                                    18


<210>  114
<211>  21
<212>  DNA
<213>  Artificial Sequence

<220>
<223>  forward primer for SEQ ID NO: 22

<400>  114
acagcttata ctgatggcac a                                                21


<210>  115
<211>  20
<212>  DNA
<213>  Artificial Sequence

<220>
<223>  reverse primer for SEQ ID NO: 22

<400>  115
tgttgtgcac tgaagacaat                                                  20


<210>  116
<211>  21
<212>  DNA
<213>  Artificial Sequence

<220>
<223>  forward primer for SEQ ID NO: 23

<400>  116
gatgacgcca ctaactgttt a                                                21


<210>  117
<211>  19
<212>  DNA
<213>  Artificial Sequence

<220>
<223>  reverse primer for SEQ ID NO: 23

<400>  117
gtcaaggagg cgattagaa                                                   19


<210>  118
<211>  16
<212>  DNA
<213>  Artificial Sequence

<220>
<223>  forward primer for SEQ ID NO: 24

<400>  118
ccacgttggt caaccg                                                      16


<210>  119
<211>  19
<212>  DNA

<213>  Artificial Sequence

<220>
<223>  reverse primer for SEQ ID NO: 24

<400>  119
ttgaccttgt ttcctggtt                                                    19


<210>  120
<211>  25
<212>  DNA
<213>  Artificial Sequence

<220>
<223>  forward primer for SEQ ID NO: 25

<400>  120
tcaggaatta gatgaatatg tgatt                                             25


<210>  121
<211>  23
<212>  DNA
<213>  Artificial Sequence

<220>
<223>  reverse primer for SEQ ID NO: 25

<400>  121
tcttcaacaa ccatctgtaa atc                                               23


<210>  122
<211>  22
<212>  DNA
<213>  Artificial Sequence

<220>
<223>  forward primer for SEQ ID NO: 26

<400>  122
gctaaatctt gaaagctaac gg                                                22


<210>  123
<211>  24
<212>  DNA
<213>  Artificial Sequence

<220>
<223>  reverse primer for SEQ ID NO: 26

<400>  123
tttattagaa caattgacga cctt                                              24


<210>  124
<211>  20
<212>  DNA
<213>  Artificial Sequence

<220>
<223>  forward primer for SEQ ID NO: 27

<400>  124
aagcaatcgt tgtctcaaag                                                   20

<210>  125
<211>  18
<212>  DNA
<213>  Artificial Sequence

<220>
<223>  reverse primer for SEQ ID NO: 27

<400>  125
gccggctgct aataattg                                                    18


<210>  126
<211>  18
<212>  DNA
<213>  Artificial Sequence

<220>
<223>  forward primer for SEQ ID NO: 28

<400>  126
cttgagatcg atcgggtt                                                    18


<210>  127
<211>  18
<212>  DNA
<213>  Artificial Sequence

<220>
<223>  reverse primer for SEQ ID NO: 28

<400>  127
ggacaccatg tgcaggat                                                    18


<210>  128
<211>  21
<212>  DNA
<213>  Artificial Sequence

<220>
<223>  forward primer for SEQ ID NO: 29

<400>  128
atgaattatt ggcgcaaagt g                                                21


<210>  129
<211>  24
<212>  DNA
<213>  Artificial Sequence

<220>
<223>  reverse primer for SEQ ID NO: 29

<400>  129
cctgtcgtca agatttcatg gaag                                             24


<210>  130
<211>  25
<212>  DNA
<213>  Artificial Sequence

<220>
<223>  forward primer for SEQ ID NO: 30

<400>  130

aataaaacga tggttgggaa gatgg                                          25

<210>  131
<211>  23
<212>  DNA
<213>  Artificial Sequence

<220>
<223>  reverse primer for SEQ ID NO: 30

<400>  131
gattgatata aatacttctt aac                                            23

<210>  132
<211>  21
<212>  DNA
<213>  Artificial Sequence

<220>
<223>  forward primer for SEQ ID NO: 31

<400>  132
cgagctttag cgatgattct t                                              21

<210>  133
<211>  22
<212>  DNA
<213>  Artificial Sequence

<220>
<223>  reverse primer for SEQ ID NO: 31

<400>  133
tgcatgttat ttgggatata gg                                             22

<210>  134
<211>  23
<212>  DNA
<213>  Artificial Sequence

<220>
<223>  forward primer for SEQ ID NO: 32

<400>  134
ctggtggttt aactgaacta gcc                                            23

<210>  135
<211>  23
<212>  DNA
<213>  Artificial Sequence

<220>
<223>  reverse primer for SEQ ID NO: 32

<400>  135
gcaactggca ttaagttgcc gaa                                            23

<210>  136
<211>  22
<212>  DNA
<213>  Artificial Sequence

<220>

<223>  forward primer for SEQ ID NO: 33

<400>  136
agggcaaggc agtcaacaag tc                                                22


<210>  137
<211>  24
<212>  DNA
<213>  Artificial Sequence

<220>
<223>  reverse primer for SEQ ID NO: 33

<400>  137
cacacagcat catcaaacga taac                                              24


<210>  138
<211>  22
<212>  DNA
<213>  Artificial Sequence

<220>
<223>  forward primer for SEQ ID NO: 34

<400>  138
ttagtgggca acatgaggaa gc                                                22


<210>  139
<211>  21
<212>  DNA
<213>  Artificial Sequence

<220>
<223>  reverse primer for SEQ ID NO: 34

<400>  139
acatgctctc attcttatct a                                                 21


<210>  140
<211>  24
<212>  DNA
<213>  Artificial Sequence

<220>
<223>  forward primer for SEQ ID NO: 35

<400>  140
atgaactatc gatgagttgg caag                                              24


<210>  141
<211>  24
<212>  DNA
<213>  Artificial Sequence

<220>
<223>  reverse primer for SEQ ID NO: 35

<400>  141
atatcacctt cttgtaattc taaa                                              24


<210>  142
<211>  25
<212>  DNA

<213>  Artificial Sequence

<220>
<223>  forward primer for SEQ ID NO: 36

<400>  142
aaaatgcgta aaataccctc ggtgg                                          25


<210>  143
<211>  22
<212>  DNA
<213>  Artificial Sequence

<220>
<223>  reverse primer for SEQ ID NO: 36

<400>  143
aggttatcac gtccgtgcca cc                                             22


<210>  144
<211>  21
<212>  DNA
<213>  Artificial Sequence

<220>
<223>  forward primer for SEQ ID NO: 37

<400>  144
actgtggttg gcgaaccggc c                                              21


<210>  145
<211>  24
<212>  DNA
<213>  Artificial Sequence

<220>
<223>  reverse primer for SEQ ID NO: 37

<400>  145
ccaagaagtg attcggtcaa aatc                                           24


<210>  146
<211>  21
<212>  DNA
<213>  Artificial Sequence

<220>
<223>  forward primer for SEQ ID NO: 38

<400>  146
gtggtaaaga aagtgttgcg a                                              21


<210>  147
<211>  22
<212>  DNA
<213>  Artificial Sequence

<220>
<223>  reverse primer for SEQ ID NO: 38

<400>  147
cgataacatg cgtcaaaacc gc                                             22

<210>  148
<211>  20
<212>  DNA
<213>  Artificial Sequence

<220>
<223>  forward primer for SEQ ID NO: 39

<400>  148
tacgaataac cctttagtcc                                                    20


<210>  149
<211>  23
<212>  DNA
<213>  Artificial Sequence

<220>
<223>  reverse primer for SEQ ID NO: 39

<400>  149
ctgtgtctca atggattgcc caa                                                23


<210>  150
<211>  20
<212>  DNA
<213>  Artificial Sequence

<220>
<223>  forward primer for SEQ ID NO: 40

<400>  150
atcatgaata agagcgcgaa                                                    20


<210>  151
<211>  24
<212>  DNA
<213>  Artificial Sequence

<220>
<223>  reverse primer for SEQ ID NO: 40

<400>  151
caatggcaac actcatgatt taaa                                               24


<210>  152
<211>  21
<212>  DNA
<213>  Artificial Sequence

<220>
<223>  forward primer for SEQ ID NO: 41

<400>  152
gactgcgggt gagttgtatg a                                                  21


<210>  153
<211>  24
<212>  DNA
<213>  Artificial Sequence

<220>
<223>  reverse primer for SEQ ID NO: 41

<400>  153

aagccttgtg cttgaaactt agtc                                              24

<210>  154
<211>  20
<212>  DNA
<213>  Artificial Sequence

<220>
<223>  forward primer for SEQ ID NO: 42

<400>  154
cgcgagaaag tccaaattat                                                    20

<210>  155
<211>  20
<212>  DNA
<213>  Artificial Sequence

<220>
<223>  reverse primer for SEQ ID NO: 42

<400>  155
ctgttttcat ggctaagtgc                                                    20

<210>  156
<211>  23
<212>  DNA
<213>  Artificial Sequence

<220>
<223>  forward primer for SEQ ID NO: 43

<400>  156
ttatattgca gttgatattg ggg                                                23

<210>  157
<211>  24
<212>  DNA
<213>  Artificial Sequence

<220>
<223>  reverse primer for SEQ ID NO: 43

<400>  157
agaatactca gatgttaatt cttg                                               24

<210>  158
<211>  21
<212>  DNA
<213>  Artificial Sequence

<220>
<223>  forward primer for SEQ ID NO: 44

<400>  158
tttgcagcaa ccgatacgac c                                                  21

<210>  159
<211>  21
<212>  DNA
<213>  Artificial Sequence

<220>

<223>  reverse primer for SEQ ID NO: 44

<400>  159
tcataaagtg ggtcaccttt g                                                21


<210>  160
<211>  21
<212>  DNA
<213>  Artificial Sequence

<220>
<223>  forward primer for SEQ ID NO: 45

<400>  160
cacctaaggc caatatcttt a                                                21


<210>  161
<211>  22
<212>  DNA
<213>  Artificial Sequence

<220>
<223>  reverse primer for SEQ ID NO: 45

<400>  161
accctaaccg tcacgccttg cc                                               22


<210>  162
<211>  20
<212>  DNA
<213>  Artificial Sequence

<220>
<223>  forward primer for SEQ ID NO: 46

<400>  162
gcttaacacg cgacggtatc                                                  20


<210>  163
<211>  23
<212>  DNA
<213>  Artificial Sequence

<220>
<223>  reverse primer for SEQ ID NO: 46

<400>  163
ccgaagtagc ggacttgttc gag                                              23


<210>  164
<211>  28
<212>  DNA
<213>  Artificial Sequence

<220>
<223>  forward primer for SEQ ID NO: 47

<400>  164
tatcgaaatg aaagcgcata atttaggc                                         28


<210>  165
<211>  20
<212>  DNA

<213>  Artificial Sequence

<220>
<223>  reverse primer for SEQ ID NO: 47

<400>  165
taccactcat tttcggacaa                                                    20


<210>  166
<211>  25
<212>  DNA
<213>  Artificial Sequence

<220>
<223>  forward primer for SEQ ID NO: 48

<400>  166
ggtcatcgat cagtttcgat gccaa                                              25


<210>  167
<211>  26
<212>  DNA
<213>  Artificial Sequence

<220>
<223>  reverse primer for SEQ ID NO: 48

<400>  167
ggacagctca actacttgtt ccttta                                             26


<210>  168
<211>  23
<212>  DNA
<213>  Artificial Sequence

<220>
<223>  forward primer for SEQ ID NO: 50

<400>  168
tcgaatatgt aggaactgat gcc                                                23


<210>  169
<211>  23
<212>  DNA
<213>  Artificial Sequence

<220>
<223>  reverse primer for SEQ ID NO: 50

<400>  169
agacccatga tgtccggata ata                                                23


<210>  170
<211>  24
<212>  DNA
<213>  Artificial Sequence

<220>
<223>  forward primer for SEQ ID NO: 51

<400>  170
aattagggac tgaattacgt cacc                                               24

<210>  171
<211>  27
<212>  DNA
<213>  Artificial Sequence

<220>
<223>  reverse primer for SEQ ID NO: 51

<400>  171
taatacttac tcatcgtatt agccacg                                          27


<210>  172
<211>  21
<212>  DNA
<213>  Artificial Sequence

<220>
<223>  forward primer for SEQ ID NO: 53

<400>  172
ggcccaagaa gtgacggctg t                                                21


<210>  173
<211>  25
<212>  DNA
<213>  Artificial Sequence

<220>
<223>  reverse primer for SEQ ID NO: 53

<400>  173
ggcaaatggt tgatcgaccg ccttg                                            25


<210>  174
<211>  16
<212>  DNA
<213>  Artificial Sequence

<220>
<223>  forward primer for SEQ ID NO: 54

<400>  174
ggctggtgga atgggc                                                      16


<210>  175
<211>  25
<212>  DNA
<213>  Artificial Sequence

<220>
<223>  reverse primer for SEQ ID NO: 54

<400>  175
ggcaaatggt tgatcgacca tctta                                            25


<210>  176
<211>  20
<212>  DNA
<213>  Artificial Sequence

<220>
<223>  forward primer for SEQ ID NO: 55

<400>  176

atttaaggta gatcgaccgg                                                    20

<210>  177
<211>  19
<212>  DNA
<213>  Artificial Sequence

<220>
<223>  reverse primer for SEQ ID NO: 55

<400>  177
ggtcttaatt tctgctaat                                                     19

<210>  178
<211>  25
<212>  DNA
<213>  Artificial Sequence

<220>
<223>  forward primer for SEQ ID NO: 57

<400>  178
aagtgacgca agaatcaacg gtgat                                              25

<210>  179
<211>  23
<212>  DNA
<213>  Artificial Sequence

<220>
<223>  reverse primer for SEQ ID NO: 57

<400>  179
actgatgact ttcaatcgtc act                                                23

<210>  180
<211>  24
<212>  DNA
<213>  Artificial Sequence

<220>
<223>  forward primer for SEQ ID NO: 59

<400>  180
caaaagcgca acgtgattta gcgc                                               24

<210>  181
<211>  25
<212>  DNA
<213>  Artificial Sequence

<220>
<223>  reverse primer for SEQ ID NO: 59

<400>  181
aaggttcatt aatttcgcta gcatc                                              25

<210>  182
<211>  24
<212>  DNA
<213>  Artificial Sequence

<220>

<223>  forward primer for SEQ ID NO: 60

<400>  182
caaaagcgca acgtgattta gggt                                              24


<210>  183
<211>  25
<212>  DNA
<213>  Artificial Sequence

<220>
<223>  reverse primer for SEQ ID NO: 60

<400>  183
gaggctcatt gatttcacta gcatt                                             25


<210>  184
<211>  20
<212>  DNA
<213>  Artificial Sequence

<220>
<223>  forward primer for SEQ ID NO: 61

<400>  184
ggcgcaatcg ataaggttgc                                                   20


<210>  185
<211>  21
<212>  DNA
<213>  Artificial Sequence

<220>
<223>  reverse primer for SEQ ID NO: 61

<400>  185
taaataaagc cgtagcgctt g                                                 21


<210>  186
<211>  20
<212>  DNA
<213>  Artificial Sequence

<220>
<223>  forward primer for SEQ ID NO: 62

<400>  186
ggcgcaatcg ataaggttga                                                   20


<210>  187
<211>  21
<212>  DNA
<213>  Artificial Sequence

<220>
<223>  reverse primer for SEQ ID NO: 62

<400>  187
tagatgaagc cgtagcgttt t                                                 21


<210>  188
<211>  25
<212>  DNA

<213>  Artificial Sequence

<220>
<223>  forward primer for SEQ ID NO: 63

<400>  188
cctctactta cttcaccaac cattc                                          25


<210>  189
<211>  28
<212>  DNA
<213>  Artificial Sequence

<220>
<223>  reverse primer for SEQ ID NO: 63

<400>  189
tgctggatca cggtgatcga agaattgg                                       28


<210>  190
<211>  26
<212>  DNA
<213>  Artificial Sequence

<220>
<223>  forward primer for SEQ ID NO: 65

<400>  190
gcttgtattt tcatatttgt tcgtga                                         26


<210>  191
<211>  22
<212>  DNA
<213>  Artificial Sequence

<220>
<223>  reverse primer for SEQ ID NO: 65

<400>  191
aaacatcatc ggtatcgaaa tg                                             22


<210>  192
<211>  25
<212>  DNA
<213>  Artificial Sequence

<220>
<223>  forward primer for SEQ ID NO: 68

<400>  192
tggtttaaca ccggtcggcg gaaca                                          25


<210>  193
<211>  21
<212>  DNA
<213>  Artificial Sequence

<220>
<223>  reverse primer for SEQ ID NO: 68

<400>  193
gcaaatgcaa caccaataac a                                              21

<210>  194
<211>  25
<212>  DNA
<213>  Artificial Sequence

<220>
<223>  forward primer for SEQ ID NO: 69

<400>  194
aggtttaaca ccggtcggcg ggaca                                          25


<210>  195
<211>  21
<212>  DNA
<213>  Artificial Sequence

<220>
<223>  reverse primer for SEQ ID NO: 69

<400>  195
gcaaatgcaa caccgatgac g                                              21


<210>  196
<211>  25
<212>  DNA
<213>  Artificial Sequence

<220>
<223>  forward primer for SEQ ID NO: 70

<400>  196
gttgtatgag aatcacaaaa aagga                                          25


<210>  197
<211>  22
<212>  DNA
<213>  Artificial Sequence

<220>
<223>  reverse primer for SEQ ID NO: 70

<400>  197
ttgaaactta gtcactaatt ga                                             22


<210>  198
<211>  28
<212>  DNA
<213>  Artificial Sequence

<220>
<223>  forward primer for SEQ ID NO: 49

<400>  198
ttactatcga aatgaaagcg cataattc                                       28


<210>  199
<211>  27
<212>  DNA
<213>  Artificial Sequence

<220>
<223>  reverse primer for SEQ ID NO: 49

<400>  199

tcgtattcgc tgtagctgta tcgaaat                                          27

<210>  200
<211>  31
<212>  DNA
<213>  Artificial Sequence

<220>
<223>  forward primer for SEQ ID NO: 52

<400>  200
ttcaacgcaa gccggcgttt tacgtggttt g                                    31

<210>  201
<211>  27
<212>  DNA
<213>  Artificial Sequence

<220>
<223>  reverse primer for SEQ ID NO: 52

<400>  201
ccatcagctt cggcatcgta atagccg                                          27

<210>  202
<211>  20
<212>  DNA
<213>  Artificial Sequence

<220>
<223>  forward primer for SEQ ID NO: 56

<400>  202
gttcaagaca gaacgaccaa                                                  20

<210>  203
<211>  19
<212>  DNA
<213>  Artificial Sequence

<220>
<223>  reverse primer for SEQ ID NO: 56

<400>  203
attctttatc tctgctagc                                                   19

<210>  204
<211>  25
<212>  DNA
<213>  Artificial Sequence

<220>
<223>  forward primer for SEQ ID NO: 58

<400>  204
aagtgacgca agaatcaacg gtggc                                            25

<210>  205
<211>  23
<212>  DNA
<213>  Artificial Sequence

<220>

<223>  reverse primer for SEQ ID NO: 58

<400>  205
actgatgact ttcaatcgtc acc                                              23


<210>  206
<211>  25
<212>  DNA
<213>  Artificial Sequence

<220>
<223>  forward primer for SEQ ID NO: 64

<400>  206
tctttactta cttcaccaac ctttt                                            25


<210>  207
<211>  28
<212>  DNA
<213>  Artificial Sequence

<220>
<223>  reverse primer for SEQ ID NO: 64

<400>  207
cgctggatca cgatgatcga aaaattga                                         28


<210>  208
<211>  22
<212>  DNA
<213>  Artificial Sequence

<220>
<223>  forward primer for SEQ ID NO: 66

<400>  208
ctcattagta ttatttgtgc cg                                               22


<210>  209
<211>  25
<212>  DNA
<213>  Artificial Sequence

<220>
<223>  reverse primer for SEQ ID NO: 66

<400>  209
tgtattggca actcgtggta gcatg                                            25


<210>  210
<211>  27
<212>  DNA
<213>  Artificial Sequence

<220>
<223>  forward primer for SEQ ID NO: 67

<400>  210
gccaatacgt ttgctaaggg tgatcac                                          27


<210>  211
<211>  22
<212>  DNA

<213>  Artificial Sequence

<220>
<223>  reverse primer for SEQ ID NO: 67

<400>  211
aaacatcatt gggattgaaa ca                                                22


<210>  212
<211>  25
<212>  DNA
<213>  Artificial Sequence

<220>
<223>  forward primer for SEQ ID NO: 71

<400>  212
gttgtatgag aagcacaaaa aaggg                                             25


<210>  213
<211>  25
<212>  DNA
<213>  Artificial Sequence

<220>
<223>  reverse primer for SEQ ID NO: 71

<400>  213
taattggtca actgcagttt cacta                                             25

## Description

### Field of the invention

The present invention relates to a combination of DNA markers for characterizing a *Lactobacillus sakei* isolate. In particular, the present invention concerns the use of a pattern of presence or absence of DNA markers in the genome of the isolate to be characterized for identifying said isolate.

### Background of the invention

Fresh meat and fish are nutritious but highly perishable foods. During production and storage they are exposed to unavoidable microbial contamination from the processing environment. Such contamination may include spoilage organisms and pathogens. It is therefore a priority for food processors to restrict the growth of contaminants so that they do not develop to potentially dangerous levels. One of the methods employed is the use of safe bacteria to curb growth of spoilage and disease-causing micro-organisms. The meat-borne lactic acid bacterium *Lactobacillus sakei* shows in this view excellent properties.

*L. sakei* has the ability to survive and grow on fresh meat, forming the dominant population when selective techniques are applied. Some strains are widely used in Europe for artisanal and larger scale manufacture of fermented sausages because of their useful preservative properties. Besides being a natural meat/fish-borne lactic acid bacterium, *L*. *sakei* is also a promising candidate for being used as natural bio-preservative agent to curb the growth of spoilage and disease-causing microorganisms, (Vermeiren et al. 2004 Int. J. Food Microbiol. 96:149-164) without a fermentation process of these foodstuffs. However, it may be difficult to distinguish the natural *L. sakei* population from that of bio-preservative cultures. This emerging technology thus requires a robust molecular tool to ensure follow up of the *L. sakei* microbial development in the product. Indeed, bio-protective cultures need to be monitored, at the isolate level, to ensure an efficient ecological activity in large industrial settings. Accordingly, there is a need for a technological tool for such measurement and for following the cultures up to the consumers, as well as for protecting industrial use of the cultures.

Strains of *L. sakei* can display an important variability in phenotypic traits and have for long been considered difficult to classify. In particular, the inventors have demonstrated the broad intra-species diversity of *L. sakei* isolates sourced from various food products and from worldwide laboratory collections (Chaillou et al. (2009) Appl. Environ. Microbiol. 75:970-980). This study demonstrated that the relatedness between isolates could be determined using a PCR-based method that detects the possession of chromosomal genes belonging to the flexible gene pool (genes poorly conserved between isolates). Therefore *L. sakei* isolates may be enormously diverse in gene content and it is possible to classify isolates in discrete variations of isolate clusters using comparative genomic content. Nevertheless, genes characterized from only few strains may not provide sufficient accuracy to profile isolates at the strain level. Accordingly, there is a need for a tool with an increased discriminating power.

The present inventors have designed such a tool by characterizing the variable gene pools in a wider pool of strains. The present invention thus provides a tool using a combination of 46 DNA markers, enabling obtaining rapidly, reproducibly and easily, highly accurate PCR fingerprinting profiles of any *L. sakei* isolates.

### Detailed description of the invention

The present inventors have identified a combination of DNA markers, and allele-specific-polymorphic sites (ASP) and specific insertion/deletion sites (INDEL) within these DNA markers, enabling characterizing a highly specific PCR fingerprinting profile of any *L. sakei* isolate. To obtain this combination of DNA markers, they conducted a large-scale substractive suppressive genomic hybridization experiment (SSH) on various strains chosen specifically from the diversity scale *of L. sakei* species. They thus generated 20,000 SSH clones of few hundreds of genomic loci inferred to the flexible gene pool of the *L*. *sakei* species and investigated the pattern of variation (absence or presence of many of these islands and the degree of polymorphism in these genomic islands) between the chosen isolates. This analysis resulted in the selection of 46 DNA regions offering the best minimal set of markers for giving a unique PCR fingerprinting profile of any *L. sakei* isolate. These 46 DNA regions are summarized in **Table 1.**

**Table 1: Description of the 46 DNA markers**

| **Strain** | **SEQ ID NO** | **length (bp)** | **Function** | **Locus ID** | **ASP** | **INDEL** |
|---|---|---|---|---|---|---|
| - | 1 | 2928 | CSC-type putative cell-surface protein | FGP112-0001 | YES | YES |
| - | 2 | 4700 | dTTP-rhamnose metabolism | FGP160-0001 | YES | |
| J160K | 3 | 1081 | hypothetical protein | FGP160-0002 | NO | |
| J160K | 4 | 3534 | CSC-type putative cell-surface protein | FGP160-0003 | NO | |
| J160K | 5 | 1260 | putative cytochrome P450 | FGP160-0004 | NO | |
| - | 6 | 2582 | β-galactosidase LacLM | LacLM | YES | YES |
| - | 7 | 2157 | putative bacteriocin transporter | FGPJG3-0002 | YES | |
| CTC163 | 8 | 2455 | putative Lon protease | FGP163-0001 | YES | |
| CTC163 | 9 | 777 | CSC-type putative cell-surface protein | FGP163-0003 | NO | |
| 23K | 10 | 1024 | putative bacteriocin production cluster | LSA0564-ac | NO | |
| 23K | 11 | 1041 | threonine deaminase | LSA0572 | NO | |
| 23K | 12 | 7441 | sialic acid catabolism | LSA1639-1645 | NO | |
| 23K | 13 | 3162 | thiosulfate reduction | LSA0217-0219c | NO | |
| - | 14 | 2202 | putative cell-surface precursor | LSA0118 | YES | |
| 23K | 15 | 3047 | putative teichoic acid export complex | LSA1579-1580 | NO | |
| 23K | 16 | 849 | putative lipase/esterase precursor | LSA0439 | NO | |
| 23K | 17 | 5307 | CSC-type putative cell-surface protein | LSA1731 | NO | |
| 23K | 18 | 3398 | CSC-type putative cell-surface protein | LSA0211-0212 | NO | |
| | 19 | 3921 | CSC-type putative cell-surface protein | LSA0172 | YES | YES |
| 23K | 20 | 918 | putative cell-surface precursor | LSA0727 | NO | |
| - | 21 | 5938 | putative phosphotransferase transport system | FGP332-0001 | YES | |
| 332F | 22 | 1327 | putative autotransport protein | FGP332-0007 | NO | |
| 332F | 23 | 500 | hypothetical protein | FGP332-0008 | NO | |
| 332F | 24 | 141 | hypothetical protein | FGP332-0009 | NO | |
| 332F | 25 | 1476 | putative cell-surface protein | FGP332-0010 | NO | |
| - | 26 | 1840 | glucose 6-phosphate catabolism | FGP332-0011 | YES | |
| 332F | 27 | 866 | putative quinone oxidoreductase | FGP332-0012 | NO | |
| 332F | 28 | 1587 | putative asparagine synthase | FGP332-0013 | NO | |
| LTH673 | 29 | 2462 | putative 2-hydroxyacid dehydrogenase cluster | FGP673-0001 | NO | |
| - | 30 | 931 | putative exopolysaccharide transporter | FGP673-0003 | YES | |
| LTH673 | 31 | 576 | pre-sakacin P and immunity protein | FGP673-0002 | NO | |
| - | 32 | 3260 | putative phosphotransferase transport system | FGP18-0001 | YES | |
| J18 | 33 | 8265 | malonate decarboxylation | FGP18-0002 | NO | |
| J18 | 34 | 1609 | putative choline kinase | FGP18-0003 | NO | |
| J21 | 35 | 336 | putative bacteriocin immunityprotein | FGP21-0001 | NO | |
| LTH677 | 36 | 482 | putative short-chain dehydrogenase/reductase | FGP677-0001 | NO | |
| LTH677 | 37 | 3291 | electron-transferring flavoprotein complex fixAB | FGP677-0002 | NO | |
| LTH677 | 38 | 4520 | phosphonate metabolism | FGP677-0003 | NO | |
| J64 | 39 | 390 | putative phosphotransferase transport system | FGP64-0001 | NO | |
| J64 | 40 | 1851 | putative branch-chain amino acid transporter | FGP64-0004 | NO | |
| | 41 | 270 | putative lactoylglutathione lyase | FGP64-0005 | YES | YES |
| LTH675 | 42 | 5790 | lactose catabolism | FGP675-0001 | NO | |
| LTH675 | 43 | 6346 | rhamnose catabolism | FGP675-0002 | NO | |
| LTH675 | 44 | 1984 | putative β-xylosidase | FGP675-0006 | NO | |
| LTH675 | 45 | 2511 | CSC-type putative cell-surface protein | FGP675-0005 | NO | |
| LTH675 | 46 | 2757 | putative β-galactosidase (LacZ-like) | FGP675-0004 | NO | |

### Combination of DNA markers

Accordingly, the present invention relates to a combination of markers enabling for characterizing a *Lactobacillus sakei* strain which comprises DNA markers of sequence SEQ ID NO: 1 to SEQ ID NO: 46.

As used herein, the terms "characterizing" and "identifying" are used indifferently and mean distinguishing a bacterial isolate or strain from another bacterial isolate or strain or from a mixture of bacterial isolates or strains.

In the context of the invention, the expression "DNA marker" refers to a chromosomal region or locus, having a property of variability between *L. sakei* isolates. Preferably, the size of DNA markers according to the invention ranges from about 150 bp to about 7.5 kb. The DNA markers according to the invention may comprise several encoding genes. Additionally, the DNA markers according to the invention may correspond to different allelic versions according to the strain of interest. More specifically, the allelic versions of the DNA markers according to the invention consist in ASP sites and specific INDEL sites. In particular, among the 46 DNA markers according to the invention, 11 DNA markers display allelic versions. These allelic variations are represented in the degenerated sequences SEQ ID NO: 1, SEQ ID NO: 2, SEQ ID NO: 6, SEQ ID NO: 7, SEQ ID NO: 14, SEQ ID NO: 19, SEQ ID NO: 21, SEQ ID NO: 26, SEQ ID NO: 30, SEQ ID NO: 32 and SEQ ID NO: 41. The 46 DNA markers according to the invention are thus most preferably of sequences SEQ ID NO: 1 to SEQ ID NO: 46. The genes encoded by the 46 DNA markers according to the invention are listed in Table 1.

As described in Example 1, the inventors have demonstrated that these 46 DNA markers could be used to characterize a *L. sakei* strain among other *L*. *sakei* strains. More precisely, the following correspondence between the pattern of presence or absence of the 46 DNA markers defined above and 11 *L. sakei* strains has been obtained, as illustrated in

### Figure 1:

- in strain J64, the DNA markers SEQ ID NO: 1, SEQ ID NO: 7, SEQ ID NO: 11, SEQ ID NO: 14, SEQ ID NO: 18, SEQ ID NO: 19, SEQ ID NO: 22, SEQ ID NO: 23, SEQ ID NO: 26, SEQ ID NO: 29, SEQ ID NO: 35, SEQ ID NO: 36, SEQ ID NO: 39, SEQ ID NO: 40 SEQ ID NO: 41 and SEQ ID NO: 43 are present and the other DNA markers are absent;
- in strain T332, the DNA markers SEQ ID NO: 1, SEQ ID NO: 7, SEQ ID NO: 10, SEQ ID NO: 11, SEQ ID NO: 12, SEQ ID NO: 13, SEQ ID NO: 14, SEQ ID NO: 15, SEQ ID NO: 18, SEQ ID NO: 19, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 30, SEQ ID NO: 35 and SEQ ID NO: 41 are present and the other DNA markers are absent;
- in strain LTH677, the DNA markers SEQ ID NO: 1, SEQ ID NO: 7, SEQ ID NO: 10, SEQ ID NO: 11, SEQ ID NO: 13, SEQ ID NO: 14, SEQ ID NO: 15, SEQ ID NO: 16, SEQ ID NO: 21, SEQ ID NO: 22, SEQ ID NO: 23, SEQ ID NO: 26, SEQ ID NO: 27, SEQ ID NO: 28, SEQ ID NO: 29, SEQ ID NO: 35, SEQ ID NO: 36, SEQ ID NO: 37, SEQ ID NO: 38, SEQ ID NO: 40, SEQ ID NO: 41, SEQ ID NO: 42, SEQ ID NO: 43 and SEQ ID NO: 45 are present and the other DNA markers are absent;
- in strain LTH675, the DNA markers SEQ ID NO: 7, SEQ ID NO: 9, SEQ ID NO: 11, SEQ ID NO: 13, SEQ ID NO: 14, SEQ ID NO: 18, SEQ ID NO: 21, SEQ ID NO: 22, SEQ ID NO: 35, SEQ ID NO: 38, SEQ ID NO: 41, SEQ ID NO: 42, SEQ ID NO: 43, SEQ ID NO: 44, SEQ ID NO: 45 and SEQ ID NO: 46 are present and the other DNA markers are absent;
- in strain J18, the DNA markers SEQ ID NO: 1, SEQ ID NO: 5, SEQ ID NO: 7, SEQ ID NO: 12, SEQ ID NO: 13, SEQ ID NO: 14, SEQ ID NO: 15, SEQ ID NO: 16, SEQ ID NO: 17, SEQ ID NO: 19, SEQ ID NO: 21, SEQ ID NO: 23, SEQ ID NO: 24, SEQ ID NO: 25, SEQ ID NO: 26, SEQ ID NO: 28, SEQ ID NO: 31, SEQ ID NO: 32, SEQ ID NO: 33, SEQ ID NO: 34 and SEQ ID NO: 41 are present and the other DNA markers are absent;
- in strain LTH673, the DNA markers SEQ ID NO: 1, SEQ ID NO: 5, SEQ ID NO: 7, SEQ ID NO: 8, SEQ ID NO: 12, SEQ ID NO: 14, SEQ ID NO: 16, SEQ ID NO: 19, SEQ ID NO: 21, SEQ ID NO: 24, SEQ ID NO: 25, SEQ ID NO: 26, SEQ ID NO: 29, SEQ ID NO: 30, SEQ ID NO: 31, SEQ ID NO: 32 and SEQ ID NO: 34 are present and the other DNA markers are absent;
- in strain 23K, the DNA markers SEQ ID NO: 5, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 and SEQ ID NO: 20 are present and the other DNA markers are absent;
- in strain CTC 163, the DNA markers SEQ ID NO: 1, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 14, SEQ ID NO: 15, SEQ ID NO: 17, SEQ ID NO: 18, SEQ ID NO: 19, SEQ ID NO: 21, SEQ ID NO: 23, SEQ ID NO: 27, SEQ ID NO: 29, SEQ ID NO: 30 and SEQ ID NO: 32 are present and the other DNA markers are absent;
- in strain JG3, the DNA markers SEQ ID NO: 1, SEQ ID NO: 2, SEQ ID NO: 3, SEQ ID NO: 5, SEQ ID NO: 6, SEQ ID NO: 7, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 18, SEQ ID NO: 19, SEQ ID NO: 23, SEQ ID NO: 26 and SEQ ID NO: 28 are present and the other DNA markers are absent;
- in strain J160K, the DNA markers SEQ ID NO: 1, SEQ ID NO: 2, SEQ ID NO: 3, SEQ ID NO: 4, SEQ ID NO: 5, SEQ ID NO: 10, SEQ ID NO: 11, SEQ ID NO: 12, SEQ ID NO: 14, SEQ ID NO: 15, SEQ ID NO: 16, SEQ ID NO: 17, SEQ ID NO: 18, SEQ ID NO: 22, SEQ ID NO: 23, SEQ ID NO: 24, SEQ ID NO: 26 and SEQ ID NO: 27 are present and the other DNA markers are absent;
- in strain J112, the DNA markers SEQ ID NO: 1, SEQ NO: 2, SEQ NO: 5, SEQ NO: 10, SEQ NO: 11, SEQ NO: 12, SEQ NO: 14, SEQ NO: 15, SEQ ID NO: 16, SEQ NO: 17, SEQ NO: 19, SEQ NO: 21, SEQ NO: 22, SEQ NO: 23, SEQ NO: 26, SEQ NO: 28 and SEQ NO: 30 are present and the other DNA markers are absent.

The present inventors have shown that a better characterization could be obtained when allele variations of some of the 46 DNA markers defined above were further detected. Such allele variations include allele-specific-polymorphic sites (ASP) and specific insertion/deletion sites (INDEL) and constitute additional DNA markers according to the invention. The allele-specific-polymorphic sites and specific insertion/deletion sites of interest according to the invention are summarized in Table 2.

**Table 2: Additional DNA markers**

| **Strain** | **SEQ ID NO** | **length (bp)** | **Allele variation from SEQ ID NO:** | **ASP** | **INDEL** |
|---|---|---|---|---|---|
| J112 | 47 | 2928 | 1 | X | X |
| T332 | 48 | 2927 | 1 | X | X |
| J160K | 49 | 2842 | 1 | X | X |
| J160K | 50 | 4700 | 2 | X | |
| JG3 | 51 | 2549 | 2 | X | |
| LTH675 | 52 | 2549 | 2 | X | |
| BMG.115 | 53 | 2582 | 6 | X | X |
| BMG.37 | 54 | 2582 | 6 | X | X |
| JG3 | 55 | 2157 | 7 | X | |
| LTH673 | 56 | 2157 | 7 | X | |
| 23K | 57 | 2202 | 14 | X | |
| LTH675 | 58 | 2202 | 14 | X | |
| 23K | 59 | 3921 | 19 | X | |
| J112 | 60 | 3921 | 19 | X | X |
| 332F | 61 | 5938 | 21 | X | |
| CTC163 | 62 | 5935 | 21 | X | |
| 332F | 63 | 1840 | 26 | X | |
| J160K | 64 | 1840 | 26 | X | |
| LTH673 | 65 | 931 | 30 | X | |
| CTC163 | 66 | 931 | 30 | X | |
| 332F | 67 | 930 | 30 | X | |
| J18 | 68 | 3260 | 32 | X | |
| CTC163 | 69 | 3260 | 32 | X | |
| J64 | 70 | 270 | 41 | X | X |
| T332 | 71 | 269 | 41 | X | X |

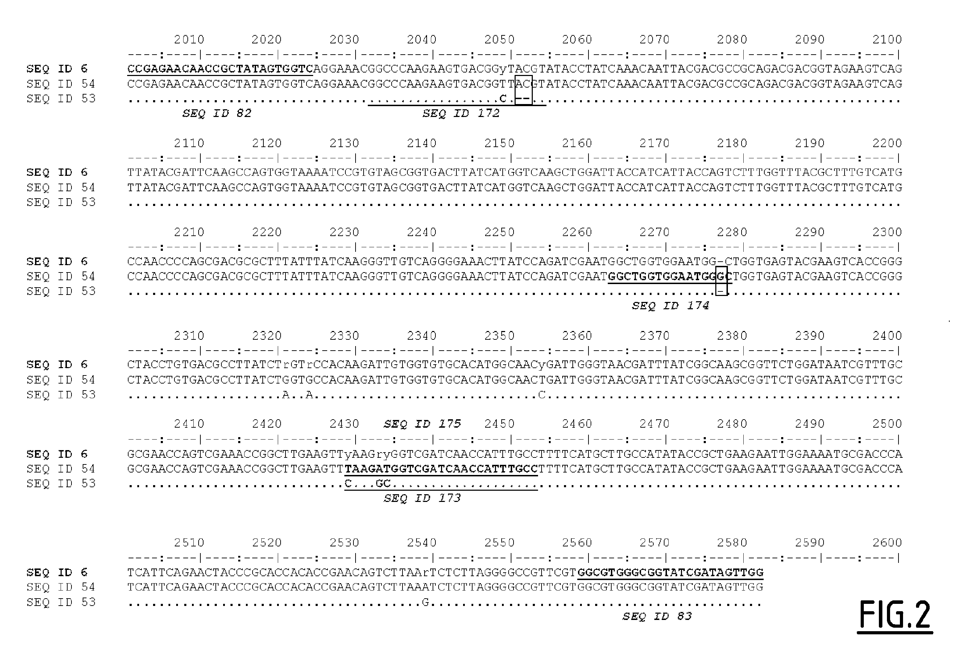

For a purpose of clarity, **Figure 2** represents allelic variations of the DNA marker of sequence SEQ ID NO: 6 identified by the inventors.

The present inventors have shown that by increasing the number of DNA markers to 60, it was possible obtain the best accuracy for the characterization. These 60 DNA markers are the 35 DNA markers defined above that do not display allelic versions (*i. e*. DNA markers of sequence SEQ ID NO: 3 to SEQ ID NO: 5, SEQ ID NO: 8 to SEQ ID NO: 13, SEQ ID NO: 15 to SEQ ID NO: 18, SEQ ID NO: 20, SEQ ID NO: 22 to SEQ ID NO: 25, SEQ ID NO: 27 to SEQ ID NO: 29, SEQ ID NO: 31, SEQ ID NO: 33 to SEQ ID NO: 40 and SEQ ID NO: 42 to SEQ ID NO: 46) and the allelic versions of the 11 DNA markers defined above that display allelic versions, said allelic versions being of sequence SEQ ID NO: 47 to SEQ ID NO: 71. Accordingly, in another preferred embodiment, the combination of markers defined above further comprises DNA markers of sequence SEQ ID NO: 47 to SEQ ID NO: 71.

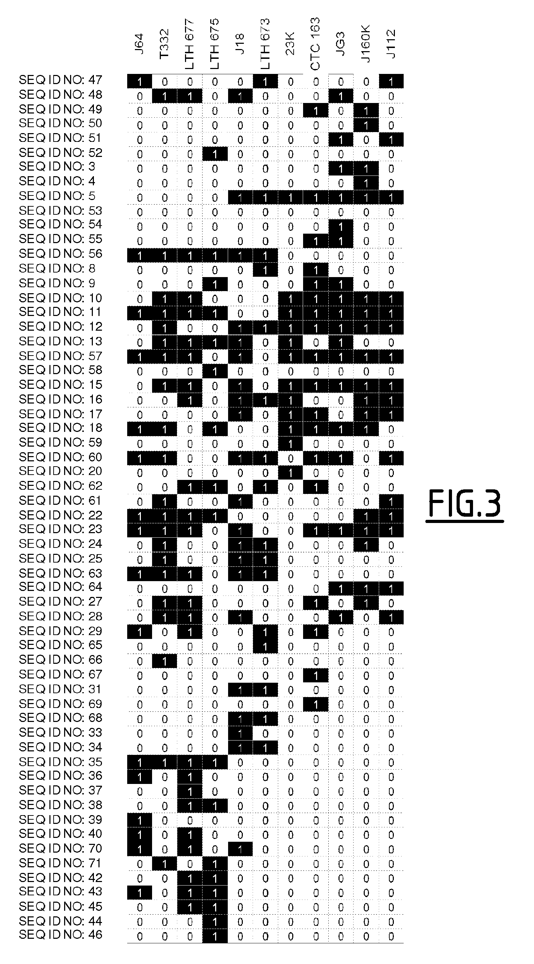

More precisely, the following correspondence between the pattern of presence or absence of the 60 DNA markers defined above and 11 *L. sakei* strains has been obtained, as illustrated in **Figure 3****:**
- in strain J64, the DNA markers SEQ ID NO: 47, SEQ ID NO: 56, SEQ ID NO: 11, SEQ ID NO: 57, SEQ ID NO: 18, SEQ ID NO: 60, SEQ ID NO: 22, SEQ ID NO: 23, SEQ ID NO: 63, SEQ ID NO: 29, SEQ ID NO: 35, SEQ ID NO: 36, SEQ ID NO: 39, SEQ ID NO: 40, SEQ ID NO: 70 and SEQ ID NO: 43 are present and the other DNA markers are absent;
- in strain T332, the DNA markers SEQ ID NO: 48, SEQ ID NO: 56, SEQ ID NO: 10, SEQ ID NO: 11, SEQ ID NO: 12, SEQ ID NO: 13, SEQ ID NO: 57, SEQ ID NO: 15, SEQ ID NO: 18, SEQ ID NO: 60, SEQ ID NO: 61, SEQ ID NO: 22, SEQ ID NO: 23, SEQ ID NO: 24, SEQ ID NO: 25, SEQ ID NO: 63, SEQ ID NO: 27, SEQ ID NO: 28, SEQ ID NO: 66, SEQ ID NO: 35 and SEQ ID NO: 71 are present and the other DNA markers are absent;
- in strain LTH677, the DNA markers SEQ ID NO: 48, SEQ ID NO: 56, SEQ ID NO: 10, SEQ ID NO: 11, SEQ ID NO: 13, SEQ ID NO: 57, SEQ ID NO: 15, SEQ ID NO: 16, SEQ ID NO: 62, SEQ ID NO: 22, SEQ ID NO: 23, SEQ ID NO: 63, SEQ ID NO: 27, SEQ ID NO: 8, SEQ ID NO: 29, SEQ ID NO: 35, SEQ ID NO: 36, SEQ ID NO: 37, SEQ ID NO: 38, SEQ ID NO: 40, SEQ ID NO: 70, SEQ ID NO: 42, SEQ ID NO: 43 and SEQ ID NO: 45 are present and the other DNA markers are absent;
- in strain LTH675, the DNA markers SEQ ID NO: 52, SEQ ID NO: 56, SEQ ID NO: 9, SEQ ID NO: 11, SEQ ID NO: 13, SEQ ID NO: 58, SEQ ID NO: 18, SEQ ID NO: 62, SEQ ID NO: 22, SEQ ID NO: 35, SEQ ID NO: 38, SEQ ID NO: 71, SEQ ID NO: 42, SEQ ID NO: 43, SEQ ID NO: 45, SEQ ID NO: 44 and SEQ ID NO: 46 are present and the other DNA markers are absent;
- in strain J18, the DNA markers SEQ ID NO: 48, SEQ ID NO: 5, SEQ ID NO 56, SEQ ID NO: 12, SEQ ID NO: 13, SEQ ID NO: 57, SEQ ID NO: 15, SEQ ID NO: 16, SEQ ID NO: 17, SEQ ID NO: 60, SEQ ID NO: 61, SEQ ID NO: 23, SEQ ID NO: 24, SEQ ID NO: 25, SEQ ID NO: 63, SEQ ID NO: 28, SEQ ID NO: 31, SEQ ID NO: 68, SEQ ID NO: 33, SEQ ID NO: 34 and SEQ ID NO: 70 are present and the other DNA markers are absent;
- in strain LTH673; the DNA markers SEQ ID NO: 47, SEQ ID NO: 5, SEQ ID NO: 56, SEQ ID NO: 8, SEQ ID NO: 12, SEQ ID NO: 16, SEQ ID NO: 60, SEQ ID NO: 62, SEQ ID NO: 24, SEQ ID NO: 25, SEQ ID NO: 63, SEQ ID NO: 29, SEQ ID NO: 65, SEQ ID NO: 31, SEQ ID NO: 68 and SEQ ID NO: 34 are present and the other DNA markers are absent;
- in strain 23K, the DNA markers SEQ ID NO: 5, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 17, SEQ ID NO: 18, SEQ ID NO: 59 and SEQ ID NO: 20 are present and the other DNA markers are absent;
- in strain CTC 163, the DNA markers SEQ ID NO: 49, SEQ ID NO: 5, SEQ ID NO: 55, SEQ ID NO: 8, SEQ ID NO: 9, SEQ ID NO: 10, SEQ ID NO: 11, SEQ ID NO: 12, SEQ ID NO: 57, SEQ ID NO: 15, SEQ ID NO: 17, SEQ ID NO: 18, SEQ ID NO: 60, SEQ ID NO: 62, SEQ ID NO: 23, SEQ ID NO: 27, SEQ ID NO: 29, SEQ ID NO: 67 and SEQ ID NO: 69 are present and the other DNA markers are absent;
- in strain JG3, the DNA markers SEQ ID NO: 48, SEQ ID NO: 51, SEQ ID NO: 3, SEQ ID NO: 5, SEQ ID NO: 53, SEQ ID NO: 55, SEQ ID NO: 9, SEQ ID NO: 10, SEQ ID NO: 11, SEQ ID NO: 12, SEQ ID NO: 13, SEQ ID NO: 57, SEQ ID NO: 15, SEQ ID NO: 18, SEQ ID NO: 60, SEQ ID NO: 23, SEQ ID NO: 64 and SEQ ID NO: 28 are present and the other DNA markers are absent;
- in strain J160K, the DNA markers SEQ ID NO: 49, SEQ ID NO: 50, SEQ ID NO: 4, SEQ ID NO: 5, SEQ ID NO: 54, SEQ ID NO: 10, SEQ ID NO: 11, SEQ ID NO: 12, SEQ ID NO: 57, SEQ ID NO: 15, SEQ ID NO: 16, SEQ ID NO: 17, SEQ ID NO: 18, SEQ ID NO: 22, SEQ ID NO: 23, SEQ ID NO: 24, SEQ ID NO: 64 and SEQ ID NO: 27 are present and the other DNA markers are absent;
- in strain J112, the DNA markers SEQ ID NO: 47, SEQ ID NO: 51, SEQ ID NO: 5, SEQ ID NO: 10, SEQ ID NO: 11, SEQ ID NO: 12, SEQ ID NO: 57, SEQ ID NO: 15, SEQ ID NO: 16, SEQ ID NO: 17, SEQ ID NO: 60, SEQ ID NO: 61, SEQ ID NO: 22, SEQ ID NO: 23, SEQ ID NO: 64 and SEQ ID NO: 28 are present and the other DNA markers are absent.

Specifically, the present inventors have shown that the accuracy of the characterization was dependent of the number of DNA markers used. The minimum set of DNA markers to be used is composed of the 46 DNA markers defined above. The accuracy of the characterization can then be raised by increasing the number of DNA markers used, the best accuracy being obtain with the 60 DNA markers defined above. Depending on the number of *L. sakei* strains to differentiate, the minimum set of 46 DNA markers may prove sufficient, or the number of DNA markers to be used to characterize the strains may be raised up to 60 if some *L. sakei* strains cannot be discriminated.

For example, as shown in Example 1, the present inventors have demonstrated that the determination of the presence or absence of 52 DNA markers including an allelic version of each of the 46 DNA markers defined above and 6 additional DNA markers consisting in ASP sites of 6 of the first 46 DNA markers, was sufficient to characterize any *L. sakei* isolate among 129 tested. Said 6 additional DNA markers are DNA markers of sequence SEQ ID NO: 48, SEQ ID NO: 51, SEQ ID NO: 54, SEQ ID NO: 60, SEQ ID NO: 62 and SEQ ID NO: 69. Accordingly, in a preferred embodiment, the combination of markers according to the invention comprises DNA markers of sequence SEQ ID NO: 47, SEQ ID NO: 48, SEQ ID NO: 50, SEQ ID NO: 51, SEQ ID NO:3 to SEQ ID NO: 5, SEQ ID NO: 53 to SEQ ID NO: 55, SEQ ID NO: 8 to SEQ ID NO: 13, SEQ ID NO: 57, SEQ ID N0:15 to SEQ ID NO:18, SEQ ID NO: SEQ ID NO: 59 to SEQ ID NO: 63, SEQ ID NO:20, SEQ ID NO:22 to SEQ ID NO:25, SEQ ID NO:27 to SEQ ID NO:29, SEQ ID NO:65, SEQ ID NO: 31, SEQ ID NO:68 to SEQ ID NO: 70, SEQ ID NO: 33 to SEQ ID NO:40 and SEQ ID NO: 42 to SEQ ID NO: 46.

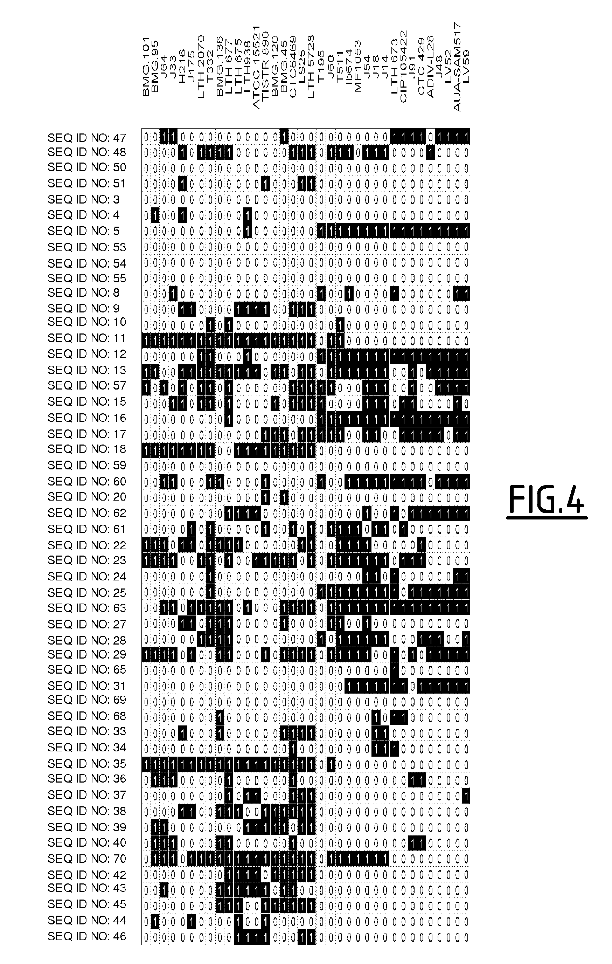

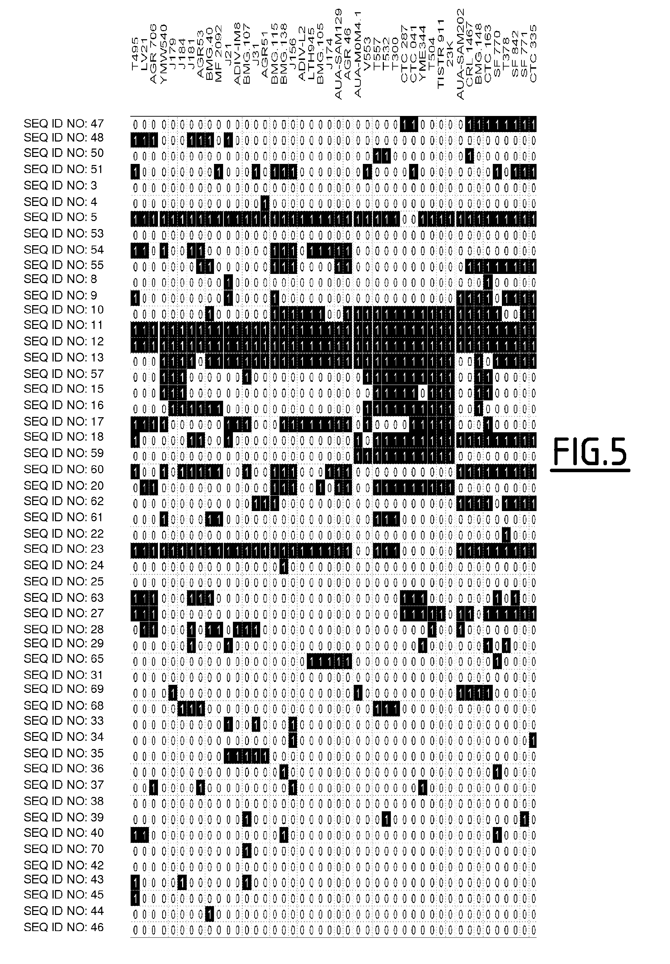

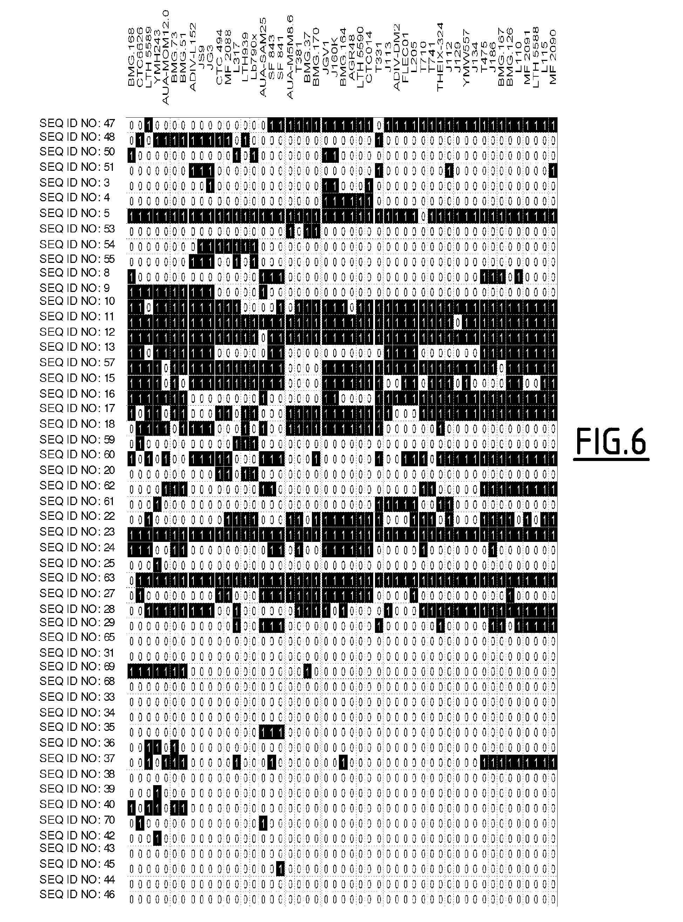

More precisely, the following correspondence between the pattern of presence or absence of the 52 DNA markers defined above and 129 *L. sakei* strains has been obtained, as illustrated in **Figures 4 to 6****:**
- in strain BMG.101, the DNA markers SEQ ID NO: 11, SEQ ID NO: 13, SEQ ID NO: 57, SEQ ID NO: 18, SEQ ID NO: 22, SEQ ID NO: 23, SEQ ID NO: 29 and SEQ ID NO: 35 are present and the 44 other DNA markers are absent;
- in strain BMG.95, the DNA markers SEQ ID NO: 4, SEQ ID NO: 11, SEQ ID NO: 13, SEQ ID NO: 18, SEQ ID NO: 22, SEQ ID No: 23, SEQ ID NO: 29, SEQ ID NO: 35, SEQ ID NO: 36, SEQ ID NO: 39, SEQ ID NO: 40, SEQ ID NO: 70 and SEQ ID NO: 44 are present and the 39 other DNA markers are absent;
- in strain J64, the DNA markers SEQ ID NO: 47, SEQ ID NO: 11, SEQ ID NO: 57, SEQ ID NO: 18, SEQ ID NO: 60, SEQ ID NO: 22, SEQ ID NO: 23, SEQ ID NO: 63, SEQ ID NO: 29, SEQ ID NO: 35, SEQ ID NO: 36, SEQ ID NO: 39, SEQ ID NO: 40, SEQ ID NO: 70 and SEQ ID NO: 43 are present and the 37 other DNA markers are absent;
- in strain J33, the DNA markers SEQ ID NO: 47, SEQ ID NO: 8, SEQ ID NO: 11, SEQ ID NO: 15, SEQ ID NO: 18, SEQ ID NO: 60, SEQ ID NO: 23, SEQ ID NO: 63, SEQ ID NO: 29, SEQ ID NO: 35, SEQ ID NO: 36, SEQ ID NO: 40 and SEQ ID NO: 70 are present and the 39 other DNA markers are absent;
- in strain H216, the DNA markers SEQ ID NO: 48, SEQ ID NO: 51, SEQ ID NO: 4, SEQ ID NO: 9, SEQ ID NO: 11, SEQ ID NO: 13, SEQ ID NO: 57, SEQ ID NO: 15, SEQ ID NO: 18, SEQ ID NO: 22, SEQ ID NO: 27, SEQ ID NO: 33, SEQ ID NO: 35 and SEQ ID NO: 38 are present and the 38 other DNA markers are absent;
- in strain J175, the DNA markers SEQ ID NO: 9, SEQ ID NO: 11, SEQ ID NO: 13, SEQ ID NO: 18, SEQ ID NO: 61, SEQ ID NO: 22, SEQ ID NO: 63, SEQ ID NO: 27, SEQ ID NO: 29, SEQ ID NO: 35, SEQ ID NO: 38, SEQ ID NO: 70 and SEQ ID NO: 44 are present and the 39 other DNA markers are absent;
- in strain LTH2070, the DNA markers SEQ ID NO: 48, SEQ ID NO: 11, SEQ ID NO: 12, SEQ ID NO: 13, SEQ ID NO: 57, SEQ ID NO: 15, SEQ ID NO: 18, SEQ ID NO: 23, SEQ ID NO: 63, SEQ ID NO: 28, SEQ ID NO: 35 and SEQ ID NO: 70 are present and the 40 other DNA markers are absent;
- in strain T332, the DNA markers SEQ ID NO: 48, SEQ ID NO: 10, SEQ ID NO: 11, SEQ ID NO: 12, SEQ ID NO: 13, SEQ ID NO: 57, SEQ ID NO: 15, SEQ ID NO: 18, SEQ ID NO: 60, SEQ ID NO: 61, SEQ ID NO: 22, SEQ ID NO: 23, SEQ ID NO: 24, SEQ ID NO: 25, SEQ ID NO: 63, SEQ ID NO: 27, SEQ ID NO: 28, SEQ ID NO: 35 and SEQ ID NO: 70 are present and the 33 other DNA markers are absent;
- in strain BMG.136, the DNA markers SEQ ID NO: 48, SEQ ID NO: 11, SEQ ID NO: 13, SEQ ID NO: 60, SEQ ID NO: 22, SEQ ID NO: 63, SEQ ID NO: 27, SEQ ID NO: 28, SEQ ID NO: 29, SEQ ID NO: 68, SEQ ID NO: 33, SEQ ID NO: 35, SEQ ID NO: 38, SEQ ID NO: 40, SEQ ID NO: 70, SEQ ID NO: 43 and SEQ ID NO: 45 are present and the 35 other DNA markers are absent;
- in strain LTH677, the DNA markers SEQ ID NO: 48, SEQ ID NO: 10, SEQ ID NO: 11, SEQ ID NO: 13, SEQ ID NO: 57, SEQ ID NO: 15, SEQ ID NO: 16, SEQ ID NO: 62, SEQ ID NO: 22, SEQ ID NO: 23, SEQ ID NO: 63, SEQ ID NO: 27, SEQ ID NO: 28, SEQ ID NO: 29, SEQ ID NO: 35, SEQ ID NO: 36, SEQ ID NO: 37, SEQ ID NO: 38, SEQ ID NO: 40, SEQ ID NO: 70, SEQ ID NO: 42, SEQ ID NO: 43 and SEQ ID NO: 45 are present and the 29 other DNA markers are absent;
- in strain LTH675, the DNA markers SEQ ID NO: 9, SEQ ID NO: 11, SEQ ID NO: 13, SEQ ID NO: 18, SEQ ID NO: 62, SEQ ID NO: 22, SEQ ID NO: 35, SEQ ID NO: 38, SEQ ID NO: 70, SEQ ID NO: 42, SEQ ID NO: 43, SEQ ID NO: 45, SEQ ID NO: 44 and SEQ ID NO: 46 are present and the 38 other DNA markers are absent;
- in strain LTH938, the DNA markers SEQ ID NO: 4, SEQ ID NO: 5, SEQ ID NO: 9, SEQ ID NO: 11, SEQ ID NO: 12, SEQ ID NO: 13, SEQ ID NO: 18, SEQ ID NO: 62, SEQ ID NO: 63, SEQ ID NO: 35, SEQ ID NO: 37, SEQ ID NO: 39, SEQ ID NO: 70, SEQ ID NO: 42, SEQ ID NO: 43 and SEQ ID NO: 46 are present and the 36 other DNA markers are absent;
- in strain ATCC15521, the DNA markers SEQ ID NO: 9, SEQ ID NO: 11, SEQ ID NO: 13, SEQ ID NO: 18, SEQ ID NO: 62, SEQ ID NO: 23, SEQ ID NO: 35, SEQ ID NO: 37, SEQ ID NO: 39, SEQ ID NO: 70, SEQ ID NO: 42, SEQ ID NO: 43 and SEQ ID NO: 46 are present and the 39 other DNA markers are absent;
- in strain TISTR890, the DNA markers SEQ ID NO: 51, SEQ ID NO: 9, SEQ ID NO: 11, SEQ ID NO: 17, SEQ ID NO: 18, SEQ ID NO: 60, SEQ ID NO: 20, SEQ ID NO: 61, SEQ ID NO: 23, SEQ ID NO: 29, SEQ ID NO: 35, SEQ ID NO: 38, SEQ ID NO: 39, SEQ ID NO: 70, SEQ ID NO: 43, SEQ ID NO: 45, SEQ ID NO: 44 and SEQ ID NO: 46 are present and the 34 other DNA markers are absent;
- in strain BMG.120, the DNA markers SEQ ID NO: 11, SEQ ID NO: 13, SEQ ID NO: 15, SEQ ID NO: 17, SEQ ID NO: 18, SEQ ID NO: 23, SEQ ID NO: 35, SEQ ID NO: 38, SEQ ID NO: 39, SEQ ID NO: 70, SEQ ID NO: 42 and SEQ ID NO: 45 are present and the 40 other DNA markers are absent;
- in strain BMG.45, the DNA markers SEQ NO: 47, SEQ NO: 11, SEQ NO: 13, SEQ ID NO: 17, SEQ NO: 18, SEQ NO: 20, SEQ ID NO: 23, SEQ NO: 63, SEQ ID NO: 27, SEQ NO: 29, SEQ NO: 33, SEQ ID NO: 35, SEQ NO: 38, SEQ NO: 39, SEQ NO: 70, SEQ NO: 42, SEQ NO: 43 and SEQ NO: 45 are present and the 34 other DNA markers are absent;
- in strain CTC6469, the DNA markers SEQ NO: 48, SEQ NO: 9, SEQ NO: 11, SEQ ID NO: 57, SEQ NO: 15, SEQ NO: 18, SEQ ID NO: 61, SEQ NO: 23, SEQ ID NO: 63, SEQ NO: 29, SEQ NO: 33, SEQ ID NO: 34, SEQ NO: 35, SEQ NO: 36, SEQ NO: 37, SEQ NO: 38, SEQ NO: 40, SEQ NO: 70, SEQ ID NO: 42, SEQ NO: 43 and SEQ NO: 45 are present and the 31 other DNA markers are absent;
- in strain LS25, the DNA markers SEQ NO: 48, SEQ NO: 51, SEQ NO: 9, SEQ NO: 11, SEQ NO: 13, SEQ NO: 57, SEQ NO: 15, SEQ NO: 17, SEQ NO: 18, SEQ NO: 22, SEQ NO: 63, SEQ NO: 29, SEQ NO: 33, SEQ ID NO: 35, SEQ NO: 37, SEQ NO: 38, SEQ NO: 39, SEQ NO: 70, SEQ NO: 42, SEQ NO: 45 and SEQ NO: 46 are present and the 31 other DNA markers are absent;
- in strain LTH5728, the DNA markers SEQ NO: 48, SEQ ID NO: 51, SEQ NO: 9, SEQ NO: 11, SEQ NO: 13, SEQ NO: 57, SEQ NO: 15, SEQ NO: 17, SEQ ID NO: 18, SEQ NO: 61, SEQ NO: 22, SEQ ID NO: 23, SEQ NO: 63, SEQ NO: 29, SEQ NO: 33, SEQ NO: 35, SEQ NO: 37, SEQ NO: 38, SEQ ID NO: 39, SEQ NO: 70, SEQ NO: 42, SEQ NO: 45 and SEQ NO: 46 are present and the 29 other DNA markers are absent;
- in strain T195, the DNA markers SEQ NO: 5, SEQ NO: 8, SEQ NO: 12, SEQ NO: 57, SEQ NO: 15, SEQ NO: 16, SEQ NO: 17, SEQ NO: 60, SEQ ID NO: 25 and SEQ NO: 28 are present and the 42 other DNA markers are absent;
- in strain J60, the DNA markers SEQ NO: 48, SEQ ID NO: 5, SEQ NO: 11, SEQ NO: 12, SEQ NO: 13, SEQ NO: 57, SEQ NO: 16, SEQ NO: 17, SEQ ID NO: 61, SEQ NO: 23, SEQ NO: 25, SEQ NO: 63, SEQ NO: 27, SEQ NO: 29, SEQ NO: 35 and SEQ NO: 70 are present and the 36 other DNA markers are absent;
- in strain T511, the DNA markers SEQ NO: 48, SEQ NO: 5, SEQ NO: 10, SEQ ID NO: 11, SEQ NO: 12, SEQ NO: 13, SEQ ID NO: 16, SEQ NO: 17, SEQ ID NO: 61, SEQ ID NO: 22, SEQ ID NO: 23, SEQ ID NO: 25, SEQ ID NO: 63, SEQ ID NO: 27, SEQ ID NO: 28, SEQ ID NO: 29 and SEQ ID NO: 70 are present and the 35 other DNA markers are absent;
- in strain Ib674, the DNA markers SEQ ID NO: 48, SEQ ID NO: 5, SEQ ID NO: 8, SEQ ID NO: 12, SEQ ID NO: 13, SEQ ID NO: 16, SEQ ID NO: 60, SEQ ID NO: 61, SEQ ID NO: 22, SEQ ID NO: 23, SEQ ID NO: 25, SEQ ID NO: 63, SEQ ID NO: 28, SEQ ID NO: 29, SEQ ID NO: 31 and SEQ ID NO: 70 are present and the 36 other DNA markers are absent;
- in strain MF1053, the DNA markers SEQ ID NO: 5, SEQ ID NO: 12, SEQ ID NO: 13, SEQ ID NO: 16, SEQ ID NO: 60, SEQ ID NO: 61, SEQ ID NO: 22, SEQ ID NO: 23, SEQ ID NO: 25, SEQ ID NO: 63, SEQ ID NO: 28, SEQ ID NO: 29, SEQ ID NO: 31 and SEQ ID NO: 70 are present and the 38 other DNA markers are absent;
- in strain J54, the DNA markers SEQ ID NO: 48, SEQ ID NO: 5, SEQ ID NO: 12, SEQ ID NO: 13, SEQ ID NO: 57, SEQ ID NO: 15, SEQ ID NO: 16, SEQ ID NO: 17, SEQ ID NO: 60, SEQ ID NO: 62, SEQ ID NO: 22, SEQ ID NO: 23, SEQ ID NO: 24, SEQ ID NO: 25, SEQ ID NO: 63, SEQ ID NO: 27, SEQ ID NO: 28, SEQ ID NO: 29, SEQ ID NO: 31 and SEQ ID NO: 70 are present and the 32 other DNA markers are absent;
- in strain J18, the DNA markers SEQ ID NO: 48, SEQ ID NO: 5, SEQ ID NO: 12, SEQ ID NO: 13, SEQ ID NO: 57, SEQ ID NO: 15, SEQ ID NO: 16, SEQ ID NO: 17, SEQ ID NO: 60, SEQ ID NO: 61, SEQ ID NO: 23, SEQ ID NO: 24, SEQ ID NO: 25, SEQ ID NO: 63, SEQ ID NO: 28, SEQ ID NO: 31, SEQ ID NO: 68, SEQ ID NO: 33, SEQ ID NO: 34 and SEQ ID NO: 70 are present and the 32 other DNA markers are absent;
- in strain J14, the DNA markers SEQ ID NO: 48, SEQ ID NO: 5, SEQ ID NO: 12, SEQ ID NO: 13, SEQ ID NO: 57, SEQ ID NO: 15, SEQ ID NO: 16, SEQ ID NO: 60, SEQ ID NO: 61, SEQ ID NO: 23, SEQ ID NO: 25, SEQ ID NO: 63, SEQ ID NO: 28, SEQ ID NO: 31, SEQ ID NO: 33, SEQ ID NO: 34 and SEQ ID NO: 70 are present and the 35 other DNA markers are absent;
- in strain LTH673, the DNA markers SEQ ID NO: 47, SEQ ID NO: 5, SEQ ID NO: 8, SEQ ID NO: 12, SEQ ID NO: 16, SEQ ID NO: 60, SEQ ID NO: 62, SEQ ID NO: 24, SEQ ID NO: 25, SEQ ID NO: 63, SEQ ID NO: 29, SEQ ID NO: 65, SEQ ID NO: 31, SEQ ID NO: 68 and SEQ ID NO: 34 are present and the 37 other DNA markers are absent;
- in strain CIP105422, the DNA markers SEQ ID NO: 47, SEQ ID NO: 5, SEQ ID NO: 12, SEQ ID NO: 15, SEQ ID NO: 16, SEQ ID NO: 17, SEQ ID NO: 60, SEQ ID NO: 61, SEQ ID NO: 23, SEQ ID NO: 63, SEQ ID NO: 31 and SEQ ID NO: 68 are present and the 40 other DNA markers are absent;
- in strain J91, the DNA markers SEQ ID NO: 47, SEQ ID NO: 5, SEQ ID NO: 12, SEQ ID NO: 13, SEQ ID NO: 57, SEQ ID NO: 15, SEQ ID NO: 16, SEQ ID NO: 17, SEQ ID NO: 60, SEQ ID NO: 62, SEQ ID NO: 23, SEQ ID NO: 25, SEQ ID NO: 63, SEQ ID NO: 29, SEQ ID NO: 36 and SEQ ID NO: 40 are present and the 36 other DNA markers are absent;
- in strain CTC429, the DNA markers SEQ ID NO: 47, SEQ ID NO: 5, SEQ ID NO: 12, SEQ ID NO: 16, SEQ ID NO: 17, SEQ ID NO: 60, SEQ ID NO: 62, SEQ ID NO: 22, SEQ ID NO: 23, SEQ ID NO: 25, SEQ ID NO: 63, SEQ ID NO: 28, SEQ ID NO: 31, SEQ ID NO: 36 and SEQ ID NO: 40 are present and the 37 other DNA markers are absent;
- in strain ADIV-L28, the DNA markers SEQ ID NO: 48, SEQ ID NO: 5, SEQ ID NO: 12, SEQ ID NO: 13, SEQ ID NO: 16, SEQ ID NO: 17, SEQ ID NO: 62, SEQ ID NO: 25, SEQ ID NO: 63, SEQ ID NO: 28, SEQ ID NO: 29 and SEQ ID NO: 31 are present and the 40 other DNA markers are absent;
- in strain J48, the DNA markers SEQ ID NO: 47, SEQ ID NO: 5, SEQ ID NO: 12, SEQ ID NO: 13, SEQ ID NO: 57, SEQ ID NO: 16, SEQ ID NO: 17, SEQ ID NO: 60, SEQ ID NO: 62, SEQ ID NO: 25, SEQ ID NO: 63, SEQ ID NO: 28, SEQ ID NO: 29 and SEQ ID NO: 31 are present and the 38 other DNA markers are absent;
- in strain LV52, the DNA markers SEQ ID NO: 47, SEQ ID NO: 5, SEQ ID NO: 12, SEQ ID NO: 13, SEQ ID NO: 57, SEQ ID NO: 16, SEQ ID NO: 60, SEQ ID NO: 62, SEQ ID NO: 25, SEQ ID NO: 63, SEQ ID NO: 29 and SEQ ID NO: 31 are present and the 40 other DNA markers are absent;
- in strain AUA-SAM517, the DNA markers SEQ ID NO: 47, SEQ ID NO: 5, SEQ ID NO: 8, SEQ ID NO: 12, SEQ ID NO: 13, SEQ ID NO: 57, SEQ ID NO: 15, SEQ ID NO: 16, SEQ ID NO: 17, SEQ ID NO: 60, SEQ ID NO: 62, SEQ ID NO: 24, SEQ ID NO: 25, SEQ ID NO: 63, SEQ ID NO: 29 and SEQ ID NO: 31 are present and the 36 other DNA markers are absent;
- in strain LV59, the DNA markers SEQ ID NO: 47, SEQ ID NO: 5, SEQ ID NO: 8, SEQ ID NO: 12, SEQ ID NO: 13, SEQ ID NO: 57, SEQ ID NO: 16, SEQ ID NO: 17, SEQ ID NO: 60, SEQ ID NO: 62, SEQ ID NO: 24, SEQ ID NO: 25, SEQ ID NO: 63, SEQ ID NO: 28, SEQ ID NO: 29, SEQ ID NO: 31 and SEQ ID NO: 37 are present and the 35 other DNA markers are absent;
- in strain T495, the DNA markers SEQ ID NO: 48, SEQ ID NO: 51, SEQ ID NO: 5, SEQ ID NO: 53, SEQ ID NO: 9, SEQ ID NO: 11, SEQ ID NO: 12, SEQ ID NO: 17, SEQ ID NO: 18, SEQ ID NO: 60, SEQ ID NO: 23, SEQ ID NO: 63, SEQ ID NO: 27, SEQ ID NO: 40, SEQ ID NO: 43 and SEQ ID NO: 45 are present and the 36 other DNA markers are absent;
- in strain LV21, the DNA markers SEQ ID NO: 48, SEQ ID NO: 5, SEQ ID NO: 53, SEQ ID NO: 11, SEQ ID NO: 12, SEQ ID NO: 17, SEQ ID NO: 20, SEQ ID NO: 23, SEQ ID NO: 63, SEQ ID NO: 27, SEQ ID NO: 28 and SEQ ID NO: 40 are present and the 40 other DNA markers are absent;
- in strain AGR706, the DNA markers SEQ ID NO: 48, SEQ ID NO: 5, SEQ ID NO: 11, SEQ ID NO: 12, SEQ ID NO: 17, SEQ ID NO: 20, SEQ ID NO: 23, SEQ ID NO: 63, SEQ ID NO: 27, SEQ ID NO: 28 and SEQ ID NO: 37 are present and the 41 other DNA markers are absent;
- in strain YMW540, the DNA markers SEQ ID NO: 5, SEQ ID NO: 53, SEQ ID NO: 11, SEQ ID NO: 12, SEQ ID NO: 13, SEQ ID NO: 57, SEQ ID NO: 15, SEQ ID NO: 17, SEQ ID NO: 60, SEQ ID NO: 61 and SEQ ID NO: 23 are present and the 41 other DNA markers are absent;
- in strain J179, the DNA markers SEQ ID NO: 5, SEQ ID NO: 11, SEQ ID NO: 12, SEQ ID NO: 13, SEQ ID NO: 57, SEQ ID NO: 15, SEQ ID NO: 16, SEQ ID NO: 23 and SEQ ID NO: 69 are present and the 43 other DNA markers are absent;
- in strain J184, the DNA markers SEQ ID NO: 5, SEQ ID NO: 11, SEQ ID NO: 12, SEQ ID NO: 13, SEQ ID NO: 57, SEQ ID NO: 15, SEQ ID NO: 16, SEQ ID NO: 60, SEQ ID NO: 23, SEQ ID NO: 68 and SEQ ID NO: 43 are present and the 41 other DNA markers are absent;
- in strain J181, the DNA markers SEQ ID NO: 48, SEQ ID NO: 5, SEQ ID NO: 53, SEQ ID NO: 11, SEQ ID NO: 12, SEQ ID NO: 13, SEQ ID NO: 16, SEQ ID NO: 18, SEQ ID NO: 60, SEQ ID NO: 23, SEQ ID NO: 63, SEQ ID NO: 28, SEQ ID NO: 29 and SEQ ID NO: 68 are present and the 38 other DNA markers are absent;
- in strain AGR53, the DNA markers SEQ ID NO: 48, SEQ ID NO: 5, SEQ ID NO: 53, SEQ ID NO: 55, SEQ ID NO: 11, SEQ ID NO: 12, SEQ ID NO: 16, SEQ ID NO: 18, SEQ ID NO: 60, SEQ ID NO: 23, SEQ ID NO: 63, SEQ ID NO: 68 and SEQ ID NO: 37 are present and the 39 other DNA markers are absent;
- in strain BMG.40, the DNA markers SEQ ID NO: 48, SEQ ID NO: 5, SEQ ID NO: 55, SEQ ID NO: 10, SEQ ID NO: 11, SEQ ID NO: 12, SEQ ID NO: 13, SEQ ID NO: 16, SEQ ID NO: 60, SEQ ID NO: 61, SEQ ID NO: 23, SEQ ID NO: 63, SEQ ID NO: 28 and SEQ ID NO: 44 are present and the 38 other DNA markers are absent;
- in strain MF 2092, the DNA markers SEQ ID NO: 51, SEQ ID NO: 5, SEQ ID NO: 11, SEQ ID NO: 12, SEQ ID NO: 13, SEQ ID NO: 16, SEQ ID NO: 60, SEQ ID NO: 61, SEQ ID NO: 23 and SEQ ID NO: 28 are present and the 42 other DNA markers are absent;
- in strain J21, the DNA markers SEQ ID NO: 48, SEQ ID NO: 5, SEQ ID NO: 8, SEQ ID NO: 9, SEQ ID NO: 11, SEQ ID NO: 12, SEQ ID NO: 13, SEQ ID NO: 17, SEQ ID NO: 18, SEQ ID NO: 23, SEQ ID NO: 29, SEQ ID NO: 33 and SEQ ID NO: 35 are present and the 39 other DNA markers are absent;
- in strain ADIV-IM8, the DNA markers SEQ ID NO: 5, SEQ ID NO: 11, SEQ ID NO: 12, SEQ ID NO: 13, SEQ ID NO: 17, SEQ ID NO: 23, SEQ ID NO: 28 and SEQ ID NO: 35 are present and the 44 other DNA markers are absent;
- in strain BMG.107, the DNA markers SEQ ID NO: 5, SEQ ID NO: 11, SEQ ID NO: 12, SEQ ID NO: 13, SEQ ID NO: 57, SEQ ID NO: 17, SEQ ID NO: 60, SEQ ID NO: 23, SEQ ID NO: 28, SEQ ID NO: 35, SEQ ID NO: 39, SEQ ID NO: 70 and SEQ ID NO: 43 are present and the 39 other DNA markers are absent;
- in strain J31, the DNA markers SEQ ID NO: 51, SEQ ID NO: 5, SEQ ID NO: 11, SEQ ID NO: 12, SEQ ID NO: 13, SEQ ID NO: 62, SEQ ID NO: 23, SEQ ID NO: 28, SEQ ID NO: 33 and SEQ ID NO: 35 are present and the 42 other DNA markers are absent;
- in strain AGR51, the DNA markers SEQ ID NO: 4, SEQ ID NO: 5, SEQ ID NO: 11, SEQ ID NO: 12, SEQ ID NO: 13, SEQ ID NO: 62, SEQ ID NO: 23 and SEQ ID NO: 35 are present and the 44 other DNA markers are absent;
- in strain BMG.115, the DNA markers SEQ ID NO: 51, SEQ ID NO: 5, SEQ ID NO: 53, SEQ ID NO: 55, SEQ ID NO: 9, SEQ ID NO: 10, SEQ ID NO: 11, SEQ ID NO: 12, SEQ ID NO: 13, SEQ ID NO: 60, SEQ ID NO: 20, SEQ ID NO: 62 and SEQ ID NO: 23 are present and the 39 other DNA markers are absent;
- in strain BMG.138, the DNA markers SEQ ID NO: 51, SEQ ID NO: 5, SEQ ID NO: 53, SEQ ID NO: 55, SEQ ID NO: 10, SEQ ID NO: 11, SEQ ID NO: 12, SEQ ID NO: 13, SEQ ID NO: 17, SEQ ID NO: 60, SEQ ID NO: 20, SEQ ID NO: 23, SEQ ID NO: 24, SEQ ID NO: 36 and SEQ ID NO: 40 are present and the 37 other DNA markers are absent;
- in strain J156, the DNA markers SEQ ID NO: 51, SEQ ID NO: 5, SEQ ID NO: 53, SEQ ID NO: 55, SEQ ID NO: 10, SEQ ID NO: 11, SEQ ID NO: 12, SEQ ID NO: 13, SEQ ID NO: 17, SEQ ID NO: 60, SEQ ID NO: 20, SEQ ID NO: 23, SEQ ID NO: 33, SEQ ID NO: 34 and SEQ ID NO: 37 are present and the 37 other DNA markers are absent;
- in strain ADIV-L2, the DNA markers SEQ ID NO: 5, SEQ ID NO: 10, SEQ ID NO: 11, SEQ ID NO: 12, SEQ ID NO: 13, SEQ ID NO: 17 and SEQ ID NO: 23 are present and the 45 other DNA markers are absent;
- in strain LTH945, the DNA markers SEQ ID NO: 5, SEQ ID NO: 53, SEQ ID NO: 10, SEQ ID NO: 11, SEQ ID NO: 12, SEQ ID NO: 13, SEQ ID NO: 17, SEQ ID NO: 23 and SEQ ID NO: 65 are present and the 43 other DNA markers are absent;
- in strain BMG.105, the DNA markers SEQ ID NO: 5, SEQ ID NO: 53, SEQ ID NO: 10, SEQ ID NO: 11, SEQ ID NO: 12, SEQ ID NO: 13, SEQ ID NO: 17, SEQ ID NO: 20, SEQ ID NO: 23 and SEQ ID NO: 65 are present and the 42 other DNA markers are absent;
- in strain J174, the DNA markers SEQ ID NO: 5, SEQ ID NO: 53, SEQ ID NO: 11, SEQ ID NO: 12, SEQ ID NO: 13, SEQ ID NO: 17, SEQ ID NO: 60, SEQ ID NO: 23 and SEQ ID NO: 65 are present and the 43 other DNA markers are absent;
- in strain AUA-SAM129, the DNA markers SEQ ID NO: 5, SEQ ID NO: 53, SEQ ID NO: 55, SEQ ID NO: 11, SEQ ID NO: 12, SEQ ID NO: 13, SEQ ID NO: 17, SEQ ID NO: 60, SEQ ID NO: 20, SEQ ID NO: 23 and SEQ ID NO: 65 are present and the 41 other DNA markers are absent;
- in strain AGR 46, the DNA markers SEQ ID NO: 5, SEQ ID NO: 53, SEQ ID NO: 55, SEQ ID NO: 10, SEQ ID NO: 11, SEQ ID NO: 12, SEQ ID NO: 13, SEQ ID NO: 17, SEQ ID NO: 60, SEQ ID NO: 20, SEQ ID NO: 23 and SEQ ID NO: 65 are present and the 40 other DNA markers are absent;
- in strain AUA-MOM4.1, the DNA markers SEQ ID NO: 5, SEQ ID NO: 10, SEQ ID NO: 11, SEQ ID NO: 12, SEQ ID NO: 13, SEQ ID NO: 18, SEQ ID NO: 59 and SEQ ID NO: 69 are present and the 44 other DNA markers are absent;
- in strain V553, the DNA markers SEQ ID NO: 51, SEQ ID NO: 5, SEQ ID NO: 10, SEQ ID NO: 11, SEQ ID NO: 12, SEQ ID NO: 13, SEQ ID NO: 57, SEQ ID NO: 16, SEQ ID NO: 17 and SEQ ID NO: 59 are present and the 42 other DNA markers are absent;
- in strain T557, the DNA markers SEQ ID NO: 50, SEQ ID NO: 5, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 18, SEQ ID NO: 59, SEQ ID NO: 20, SEQ ID NO: 61, SEQ ID NO: 23 and SEQ ID NO: 68 are present and the 37 other DNA markers are absent;
- in strain T532, the DNA markers SEQ ID NO: 50, SEQ ID NO: 5, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 18, SEQ ID NO: 59, SEQ ID NO: 20, SEQ ID NO: 61, SEQ ID NO: 23, SEQ ID NO: 68 and SEQ ID NO: 39 are present and the 36 other DNA markers are absent;
- in strain T300, the DNA markers SEQ ID NO: 5, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 18, SEQ ID NO: 59, SEQ ID NO: 20, SEQ ID NO: 61, SEQ ID NO: 23 and SEQ ID NO: 68 are present and the 38 other DNA markers are absent;
- in strain CTC287, the DNA markers SEQ ID NO: 47, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 18, SEQ ID NO: 59, SEQ ID NO: 20, SEQ ID NO: 63 and SEQ ID NO: 27 are present and the 39 other DNA markers are absent;
- in strain CTC041, the DNA markers SEQ ID NO: 47, SEQ ID NO: 51, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 17, SEQ ID NO: 18, SEQ ID NO: 59, SEQ ID NO: 20, SEQ ID NO: 63 and SEQ ID NO: 27 are present and the 37 other DNA markers are absent;
- in strain YME344, the DNA markers SEQ ID NO: 5, SEQ ID NO: 10, SEQ ID NO: 11, SEQ ID NO: 12, SEQ ID NO: 13, SEQ ID NO: 57, SEQ ID NO: 16, SEQ ID NO: 17, SEQ ID NO: 18, SEQ ID NO: 59, SEQ ID NO: 20, SEQ ID NO: 63, SEQ ID NO: 27, SEQ ID NO: 29 and SEQ ID NO: 37 are present and the 37 other DNA markers are absent;
- in strain T504, the DNA markers SEQ ID NO: 5, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 17, SEQ ID NO: 18, SEQ ID NO: 59, SEQ ID NO: 20, SEQ ID NO: 27 and SEQ ID NO: 28 are present and the 38 other DNA markers are absent;
- in strain TISTR911, the DNA markers SEQ ID NO: 5, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 17, SEQ ID NO: 18, SEQ ID NO: 59, SEQ ID NO: 20 and SEQ ID NO: 27 are present and the 39 other DNA markers are absent;
- in strain 23K, the DNA markers SEQ ID NO: 5, SEQ ID NO: 10, SEQ ID NO: 11, SEQ ID NO: 12, SEQ ID NO: 13, SEQ ID NO: 57, SEQ ID NO: 15, SEQ ID NO: 16, SEQ NO: 17, SEQ NO: 18, SEQ NO: 59 and SEQ ID NO: 20 are present and the 40 other DNA markers are absent;
- in strain AUA-SAM202, the DNA markers SEQ NO: 5, SEQ NO: 9, SEQ ID NO: 10, SEQ NO: 11, SEQ NO: 12, SEQ NO: 18, SEQ NO: 60, SEQ NO: 62, SEQ NO: 23, SEQ NO: 27, SEQ NO: 28 and SEQ NO: 69 are present and the 40 other DNA markers are absent;
- in strain CRL1467, the DNA markers SEQ NO: 47, SEQ ID NO: 50, SEQ NO: 5, SEQ NO: 55, SEQ NO: 9, SEQ NO: 10, SEQ NO: 11, SEQ NO: 12, SEQ NO: 18, SEQ NO: 60, SEQ NO: 62, SEQ ID NO: 23, SEQ NO: 27 and SEQ NO: 69 are present and the 38 other DNA markers are absent;
- in strain BMG.148, the DNA markers SEQ NO: 47, SEQ NO: 5, SEQ NO: 55, SEQ NO: 9, SEQ NO: 10, SEQ NO: 11, SEQ NO: 12, SEQ NO: 13, SEQ ID NO: 57, SEQ NO: 15, SEQ NO: 16, SEQ ID NO: 18, SEQ NO: 60, SEQ NO: 62, SEQ NO: 23 and SEQ NO: 69 are present and the 36 other DNA markers are absent;
- in strain CTC163, the DNA markers SEQ NO: 47, SEQ ID NO: 5, SEQ NO: 55, SEQ NO: 8, SEQ NO: 9, SEQ NO: 10, SEQ ID NO: 11, SEQ NO: 12, SEQ ID NO: 57, SEQ NO: 15, SEQ NO: 17, SEQ ID NO: 18, SEQ NO: 60, SEQ ID NO: 62, SEQ NO: 23, SEQ NO: 27, SEQ NO: 29 and SEQ NO: 69 are present and the 34 other DNA markers are absent;
- in strain SF770, the DNA markers SEQ NO: 47, SEQ ID NO: 51, SEQ ID NO: 5, SEQ NO: 55, SEQ NO: 10, SEQ NO: 11, SEQ NO: 12, SEQ NO: 13, SEQ ID NO: 18, SEQ NO: 60, SEQ NO: 23, SEQ NO: 63, SEQ NO: 27, SEQ ID NO: 65, SEQ NO: 36 and SEQ NO: 40 are present and the 36 other DNA markers are absent;
- in strain T378, the DNA markers SEQ NO: 47, SEQ NO: 5, SEQ NO: 55, SEQ NO: 9, SEQ NO: 11, SEQ NO: 12, SEQ NO: 13, SEQ NO: 18, SEQ NO: 60, SEQ NO: 62, SEQ NO: 22, SEQ NO: 23, SEQ NO: 27 and SEQ ID NO: 29 are present and the 38 other DNA markers are absent;
- in strain SF842, the DNA markers SEQ NO: 47, SEQ ID NO: 51, SEQ ID NO: 5, SEQ ID NO: 55, SEQ NO: 9, SEQ NO: 11, SEQ NO: 12, SEQ NO: 13, SEQ NO: 18, SEQ NO: 60, SEQ NO: 62, SEQ NO: 23, SEQ NO: 63 and SEQ ID NO: 27 are present and the 38 other DNA markers are absent;
- in strain SF771, the DNA markers SEQ NO: 47, SEQ ID NO: 51, SEQ ID NO: 5, SEQ ID NO: 55, SEQ NO: 9, SEQ NO: 10, SEQ ID NO: 11, SEQ NO: 12, SEQ NO: 13, SEQ NO: 18, SEQ NO: 60, SEQ NO: 62, SEQ NO: 23, SEQ ID NO: 27 and SEQ NO: 39 are present and the 37 other DNA markers are absent;
- in strain CTC335, the DNA markers SEQ NO: 47, SEQ NO: 51, SEQ NO: 5, SEQ NO: 55, SEQ NO: 9, SEQ NO: 10, SEQ NO: 11, SEQ NO: 12, SEQ ID NO: 13, SEQ NO: 18, SEQ NO: 60, SEQ ID NO: 62, SEQ NO: 23, SEQ ID NO: 27 and SEQ NO: 34 are present and the 37 other DNA markers are absent;
- in strain BMG.168, the DNA markers SEQ NO: 50, SEQ NO: 5, SEQ NO: 8, SEQ NO: 9, SEQ NO: 10, SEQ NO: 11, SEQ NO: 12, SEQ NO: 13, SEQ ID NO: 57, SEQ NO: 15, SEQ NO: 16, SEQ ID NO: 17, SEQ NO: 60, SEQ NO: 23, SEQ NO: 24, SEQ NO: 69 and SEQ ID NO: 40 are present and the 35 other DNA markers are absent;
- in strain CTC6626, the DNA markers SEQ NO: 48, SEQ NO: 5, SEQ NO: 9, SEQ NO: 10, SEQ NO: 11, SEQ NO: 12, SEQ NO: 13, SEQ NO: 57, SEQ ID NO: 15, SEQ NO: 16, SEQ NO: 18, SEQ ID NO: 59, SEQ NO: 23, SEQ NO: 24, SEQ NO: 63, SEQ NO: 27, SEQ NO: 69 and SEQ NO: 70 are present and the 34 other DNA markers are absent;
- in strain LTH5589, the DNA markers SEQ NO: 47, SEQ NO: 5, SEQ NO: 9, SEQ NO: 11, SEQ NO: 12, SEQ NO: 57, SEQ NO: 15, SEQ NO: 16, SEQ ID NO: 17, SEQ NO: 18, SEQ NO: 60, SEQ ID NO: 22, SEQ NO: 23, SEQ NO: 24, SEQ NO: 63, SEQ NO: 28, SEQ NO: 69, SEQ NO: 36, SEQ ID NO: 37 and SEQ NO: 40 are present and the 32 other DNA markers are absent;
- in strain YMH243, the DNA markers SEQ NO: 48, SEQ NO: 5, SEQ NO: 9, SEQ NO: 10, SEQ NO: 11, SEQ NO: 12, SEQ NO: 13, SEQ NO: 57, SEQ ID NO: 15, SEQ NO: 16, SEQ NO: 17, SEQ ID NO: 18, SEQ NO: 61, SEQ NO: 23, SEQ NO: 25, SEQ NO: 63, SEQ NO: 28, SEQ NO: 69, SEQ ID NO: 36, SEQ NO: 39, SEQ ID NO: 40 and SEQ NO: 42 are present and the 30 other DNA markers are absent;
- in strain AUA-MOM12.0, the DNA markers SEQ NO: 48, SEQ NO: 5, SEQ NO: 9, SEQ NO: 10, SEQ NO: 11, SEQ NO: 12, SEQ NO: 13, SEQ ID NO: 16, SEQ NO: 18, SEQ NO: 60, SEQ NO: 62, SEQ NO: 23, SEQ NO: 63, SEQ ID NO: 28, SEQ ID NO: 69 and SEQ ID NO: 37 are present and the 36 other DNA markers are absent;
- in strain BMG.73, the DNA markers SEQ ID NO: 48, SEQ ID NO: 5, SEQ ID NO: 9, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 17, SEQ ID NO: 62, SEQ ID NO: 23, SEQ ID NO: 24, SEQ ID NO: 63, SEQ ID NO: 28, SEQ ID NO: 69, SEQ ID NO: 36, SEQ ID NO: 37 and SEQ ID NO: 40 are present and the 32 other DNA markers are absent;
- in strain BMG.51, the DNA markers SEQ ID NO: 48, SEQ ID NO: 5, SEQ ID NO: 9, SEQ ID NO: 10, SEQ ID NO: 11, SEQ ID NO: 12, SEQ ID NO: 13, SEQ ID NO: 57, SEQ ID NO: 16, SEQ ID NO: 17, SEQ ID NO: 18, SEQ ID NO: 62, SEQ ID NO: 23, SEQ ID NO: 24, SEQ ID NO: 63, SEQ ID NO: 28, SEQ ID NO: 69, SEQ ID NO: 37 and SEQ ID NO: 40 are present and the 33 other DNA markers are absent;
- in strain ADIV-L152, the DNA markers SEQ ID NO: 48, SEQ ID NO: 51, SEQ ID NO: 5, SEQ ID NO: 55, SEQ ID NO: 9, SEQ ID NO: 10, SEQ ID NO: 11, SEQ ID NO: 12, SEQ ID NO: 13, SEQ ID NO: 57, SEQ ID NO: 15, SEQ ID NO: 18, SEQ ID NO: 60, SEQ ID NO: 23, SEQ ID NO: 63 and SEQ ID NO: 28 are present and the 36 other DNA markers are absent;
- in strain JS9, the DNA markers SEQ ID NO: 48, SEQ ID NO: 51, SEQ ID NO: 5, SEQ ID NO: 53, SEQ ID NO: 55, SEQ ID NO: 9, SEQ ID NO: 10, SEQ ID NO: 11, SEQ ID NO: 12, SEQ ID NO: 13, SEQ ID NO: 57, SEQ ID NO: 15, SEQ ID NO: 18, SEQ ID NO: 60, SEQ ID NO: 23, SEQ ID NO: 63 and SEQ ID NO: 28 are present and the 35 other DNA markers are absent;
- in strain JG3, the DNA markers SEQ ID NO: 48, SEQ ID NO: 51, SEQ ID NO: 3, SEQ ID NO: 5, SEQ ID NO: 53, SEQ ID NO: 55, SEQ ID NO: 9, SEQ ID NO: 10, SEQ ID NO: 11, SEQ ID NO: 12, SEQ ID NO: 13, SEQ ID NO: 57, SEQ ID NO: 15, SEQ ID NO: 18, SEQ ID NO: 60, SEQ ID NO: 23, SEQ ID NO: 63 and SEQ ID NO: 28 are present and the 34 other DNA markers are absent;
- in strain CTC494, the DNA markers SEQ ID NO: 48, SEQ ID NO: 5, SEQ ID NO: 53, SEQ ID NO: 10, SEQ ID NO: 11, SEQ ID NO: 12, SEQ ID NO: 57, SEQ ID NO: 15, SEQ ID NO: 17, SEQ ID NO: 60, SEQ ID NO: 20, SEQ ID NO: 23, SEQ ID NO: 63 and SEQ ID NO: 27 are present and the 38 other DNA markers are absent;
- in strain MF2088, the DNA markers SEQ ID NO: 48, SEQ ID NO: 5, SEQ ID NO: 53, SEQ ID NO: 10, SEQ ID NO: 11, SEQ ID NO: 12, SEQ ID NO: 57, SEQ ID NO: 15, SEQ ID NO: 17, SEQ ID NO: 60, SEQ ID NO: 20, SEQ ID NO: 22, SEQ ID NO: 23, SEQ ID NO: 63 and SEQ ID NO: 27 are present and the 37 other DNA markers are absent;
- in strain L317, the DNA markers SEQ ID NO: 50, SEQ ID NO: 5, SEQ ID NO: 53, SEQ ID NO: 55, SEQ ID NO: 10, SEQ ID NO: 11, SEQ ID NO: 12, SEQ ID NO: 57, SEQ ID NO: 15, SEQ ID NO: 59, SEQ ID NO: 22, SEQ ID NO: 23, SEQ ID NO: 63, SEQ ID NO: 28, SEQ ID NO: 29 and SEQ ID NO: 37 are present and the 36 other DNA markers are absent;
- in strain LTH939, the DNA markers SEQ ID NO: 48, SEQ ID NO: 5, SEQ ID NO: 53, SEQ ID NO: 11, SEQ ID NO: 12, SEQ ID NO: 57, SEQ ID NO: 15, SEQ ID NO: 17, SEQ ID NO: 18, SEQ ID NO: 59, SEQ ID NO: 20, SEQ ID NO: 22, SEQ ID NO: 23 and SEQ ID NO: 63 are present and the 38 other DNA markers are absent;
- in strain Lb790X, the DNA markers SEQ ID NO: 50, SEQ ID NO: 5, SEQ ID NO: 53, SEQ ID NO: 55, SEQ ID NO: 11, SEQ ID NO: 12, SEQ ID NO: 57, SEQ ID NO: 15, SEQ ID NO: 17, SEQ ID NO: 59, SEQ ID NO: 20, SEQ ID NO: 22, SEQ ID NO: 23 and SEQ ID NO: 63 are present and the 38 other DNA markers are absent;
- in strain AUA-SAM25, the DNA markers SEQ ID NO: 5, SEQ ID NO: 8, SEQ ID NO: 9, SEQ ID NO: 11, SEQ ID NO: 57, SEQ ID NO: 15, SEQ ID NO: 16, SEQ ID NO: 18, SEQ ID NO: 60, SEQ ID NO: 62, SEQ ID NO: 23, SEQ ID NO: 63, SEQ ID NO: 27, SEQ ID NO: 29, SEQ ID NO: 35 and SEQ ID NO: 70 are present and the 36 other DNA markers are absent;
- in strain SF843, the DNA markers SEQ ID NO: 47, SEQ ID NO: 5, SEQ ID NO: 8, SEQ ID NO: 11, SEQ ID NO: 12, SEQ ID NO: 13, SEQ ID NO: 57, SEQ ID NO: 15, SEQ ID NO: 60, SEQ ID NO: 62, SEQ ID NO: 23, SEQ ID NO: 24, SEQ ID NO: 63, SEQ ID NO: 27, SEQ ID NO: 29, SEQ ID NO: 35 and SEQ ID NO: 37 are present and the 35 other DNA markers are absent;
- in strain SF841, the DNA markers SEQ ID NO: 47, SEQ ID NO: 5, SEQ ID NO: 8, SEQ ID NO: 10, SEQ ID NO: 11, SEQ ID NO: 12, SEQ ID NO: 13, SEQ ID NO: 57, SEQ ID NO: 15, SEQ ID NO: 60, SEQ ID NO: 23, SEQ ID NO: 24, SEQ ID NO: 63, SEQ ID NO: 27, SEQ ID NO: 29, SEQ ID NO: 35 and SEQ ID NO: 45 are present and the 35 other DNA markers are absent;
- in strain AUA-M5M8.6, the DNA markers SEQ ID NO: 47, SEQ ID NO: 5, SEQ ID NO: 54, SEQ ID NO: 11, SEQ ID NO: 12, SEQ ID NO: 17, SEQ ID NO: 18, SEQ ID NO: 22, SEQ NO: 23, SEQ NO: 63 and SEQ ID NO: 27 are present and the 41 other DNA markers are absent;
- in strain T381, the DNA markers SEQ NO: 47, SEQ ID NO: 5, SEQ NO: 10, SEQ ID NO: 11, SEQ NO: 12, SEQ NO: 17, SEQ ID NO: 18, SEQ NO: 22, SEQ NO: 23, SEQ NO: 24, SEQ NO: 63, SEQ NO: 27 and SEQ NO: 28 are present and the 39 other DNA markers are absent;
- in strain BMG.37, the DNA markers SEQ NO: 47, SEQ NO: 5, SEQ NO: 54, SEQ ID NO: 10, SEQ NO: 11, SEQ NO: 12, SEQ ID NO: 17, SEQ NO: 18, SEQ NO: 23, SEQ NO: 63, SEQ NO: 27, SEQ ID NO: 28 and SEQ NO: 69 are present and the 39 other DNA markers are absent;
- in strain BMG.164, the DNA markers SEQ NO: 47, SEQ NO: 5, SEQ NO: 54, SEQ ID NO: 10, SEQ NO: 11, SEQ NO: 12, SEQ ID NO: 17, SEQ NO: 18, SEQ NO: 60, SEQ NO: 22, SEQ NO: 23, SEQ NO: 63, SEQ NO: 27 and SEQ NO: 28 are present and the 38 other DNA markers are absent;
- in strain JGV1, the DNA markers SEQ NO: 47, SEQ NO: 50, SEQ NO: 3, SEQ ID NO: 4, SEQ NO: 5, SEQ NO: 10, SEQ NO: 11, SEQ NO: 12, SEQ ID NO: 57, SEQ NO: 15, SEQ NO: 16, SEQ NO: 17, SEQ NO: 18, SEQ NO: 22, SEQ NO: 23, SEQ NO: 24, SEQ NO: 63, SEQ NO: 27 and SEQ NO: 28 are present and the 33 other DNA markers are absent;
- in strain J160K, the DNA markers SEQ NO: 47, SEQ ID NO: 50, SEQ ID NO: 3, SEQ ID NO: 4, SEQ NO: 5, SEQ NO: 10, SEQ NO: 11, SEQ NO: 12, SEQ ID NO: 57, SEQ NO: 15, SEQ NO: 16, SEQ NO: 17, SEQ NO: 18, SEQ NO: 22, SEQ NO: 23, SEQ NO: 24, SEQ NO: 63 and SEQ NO: 27 are present and the 34 other DNA markers are absent;
- in strain BMG.164, the DNA markers SEQ NO: 47, SEQ NO: 4, SEQ NO: 5, SEQ NO: 10, SEQ NO: 11, SEQ NO: 12, SEQ NO: 57, SEQ NO: 15, SEQ ID NO: 17, SEQ NO: 18, SEQ NO: 22, SEQ NO: 23, SEQ NO: 24, SEQ NO: 63, SEQ NO: 27, SEQ NO: 28 and SEQ NO: 37 are present and the 35 other DNA markers are absent;
- in strain AGR48, the DNA markers SEQ NO: 47, SEQ ID NO: 4, SEQ ID NO: 5, SEQ NO: 11, SEQ NO: 12, SEQ NO: 57, SEQ NO: 15, SEQ NO: 17, SEQ NO: 18, SEQ NO: 22, SEQ NO: 23, SEQ NO: 24, SEQ NO: 63 and SEQ ID NO: 27 are present and the 38 other DNA markers are absent;
- in strain LTH5590, the DNA markers SEQ NO: 47, SEQ NO: 4, SEQ NO: 5, SEQ NO: 10, SEQ NO: 11, SEQ NO: 12, SEQ NO: 57, SEQ NO: 15, SEQ ID NO: 17, SEQ NO: 18, SEQ NO: 22, SEQ ID NO: 23, SEQ NO: 24, SEQ ID NO: 63 and SEQ NO: 27 are present and the 37 other DNA markers are absent;
- in strain CTC014, the DNA markers SEQ NO: 47, SEQ NO: 3, SEQ NO: 4, SEQ NO: 5, SEQ NO: 10, SEQ NO: 11, SEQ NO: 12, SEQ NO: 57, SEQ ID NO: 15, SEQ NO: 17, SEQ NO: 18, SEQ ID NO: 22, SEQ NO: 23, SEQ NO: 24, SEQ NO: 63 and SEQ NO: 27 are present and the 36 other DNA markers are absent;
- in strain T331, the DNA markers SEQ NO: 48, SEQ NO: 51, SEQ NO: 5, SEQ NO: 10, SEQ NO: 11, SEQ NO: 12, SEQ NO: 57, SEQ NO: 15, SEQ NO: 16, SEQ NO: 17, SEQ NO: 18, SEQ NO: 60, SEQ NO: 61, SEQ ID NO: 22, SEQ NO: 23, SEQ ID NO: 63 and SEQ NO: 29 are present and the 35 other DNA markers are absent;
- in strain J113, the DNA markers SEQ NO: 47, SEQ ID NO: 5, SEQ NO: 10, SEQ ID NO: 11, SEQ NO: 12, SEQ NO: 13, SEQ ID NO: 57, SEQ NO: 16, SEQ NO: 17, SEQ NO: 61, SEQ NO: 23, SEQ NO: 63 and SEQ NO: 28 are present and the 39 other DNA markers are absent;
- in strain ADIV-DM2, the DNA markers SEQ NO: 47, SEQ NO: 5, SEQ NO: 10, SEQ ID NO: 11, SEQ NO: 12, SEQ NO: 13, SEQ ID NO: 57, SEQ NO: 16, SEQ ID NO: 61, SEQ NO: 23 and SEQ NO: 63 are present and the 41 other DNA markers are absent;
- in strain FLEC01, the DNA markers SEQ NO: 47, SEQ NO: 5, SEQ NO: 10, SEQ ID NO: 11, SEQ NO: 12, SEQ NO: 13, SEQ ID NO: 57, SEQ NO: 15, SEQ NO: 16, SEQ NO: 60, SEQ NO: 61, SEQ ID NO: 23 and SEQ NO: 63 are present and the 39 other DNA markers are absent;
- in strain L205, the DNA markers SEQ NO: 47, SEQ ID NO: 5, SEQ NO: 10, SEQ ID NO: 11, SEQ NO: 12, SEQ NO: 13, SEQ ID NO: 57, SEQ NO: 15, SEQ ID NO: 16, SEQ NO: 60, SEQ NO: 61, SEQ NO: 22, SEQ NO: 23, SEQ ID NO: 63 and SEQ NO: 27 are present and the 37 other DNA markers are absent;
- in strain T710, the DNA markers SEQ NO: 47, SEQ NO: 10, SEQ NO: 11, SEQ ID NO: 12, SEQ NO: 57, SEQ NO: 16, SEQ ID NO: 17, SEQ NO: 60, SEQ ID NO: 62, SEQ ID NO: 22, SEQ ID NO: 23, SEQ ID NO: 24, SEQ ID NO: 63 and SEQ ID NO: 28 are present and the 38 other DNA markers are absent;
- in strain T741, the DNA markers SEQ ID NO: 47, SEQ ID NO: 5, SEQ ID NO: 10, SEQ ID NO: 11, SEQ ID NO: 12, SEQ ID NO: 57, SEQ ID NO: 15, SEQ ID NO: 16, SEQ ID NO: 17, SEQ ID NO: 62, SEQ ID NO: 22, SEQ ID NO: 23, SEQ ID NO: 63 and SEQ ID NO: 28 are present and the 38 other DNA markers are absent;
- in strain THEIX-324, the DNA markers SEQ ID NO: 47, SEQ ID NO: 5, SEQ ID NO: 10, SEQ ID NO: 11, SEQ ID NO: 12, SEQ ID NO: 57, SEQ ID NO: 15, SEQ ID NO: 16, SEQ ID NO: 17, SEQ ID NO: 18, SEQ ID NO: 60, SEQ ID NO: 61, SEQ ID NO: 23, SEQ ID NO: 63, SEQ ID NO: 28 and SEQ ID NO: 29 are present and the 36 other DNA markers are absent;
- in strain J112, the DNA markers SEQ ID NO: 47, SEQ ID NO: 51, SEQ ID NO: 5, SEQ ID NO: 10, SEQ ID NO: 11, SEQ ID NO: 12, SEQ ID NO: 57, SEQ ID NO: 15, SEQ ID NO: 16, SEQ ID NO: 17, SEQ ID NO: 60, SEQ ID NO: 61, SEQ ID NO: 22, SEQ ID NO: 23, SEQ ID NO: 63 and SEQ ID NO: 28 are present and the 36 other DNA markers are absent;
- in strain J129, the DNA markers SEQ ID NO: 47, SEQ ID NO: 5, SEQ ID NO: 10, SEQ ID NO: 12, SEQ ID NO: 57, SEQ ID NO: 16, SEQ ID NO: 17, SEQ ID NO: 60, SEQ ID NO: 23, SEQ ID NO: 63 and SEQ ID NO: 28 are present and the 41 other DNA markers are absent;
- in strain YMW557, the DNA markers SEQ ID NO: 47, SEQ ID NO: 5, SEQ ID NO: 10, SEQ ID NO: 11, SEQ ID NO: 12, SEQ ID NO: 57, SEQ ID NO: 15, SEQ ID NO: 16, SEQ ID NO: 17, SEQ ID NO: 60, SEQ ID NO: 23, SEQ ID NO: 63 and SEQ ID NO: 28 are present and the 39 other DNA markers are absent;
- in strain J134, the DNA markers SEQ ID NO: 47, SEQ ID NO: 5, SEQ ID NO: 10, SEQ ID NO: 11, SEQ ID NO: 12, SEQ ID NO: 57, SEQ ID NO: 16, SEQ ID NO: 17, SEQ ID NO: 60, SEQ ID NO: 23, SEQ ID NO: 63 and SEQ ID NO: 28 are present and the 40 other DNA markers are absent;
- in strain T475, the DNA markers SEQ ID NO: 47, SEQ ID NO: 5, SEQ ID NO: 8, SEQ ID NO: 10, SEQ ID NO: 11, SEQ ID NO: 12, SEQ ID NO: 13, SEQ ID NO: 57, SEQ ID NO: 16, SEQ ID NO: 17, SEQ ID NO: 60, SEQ ID NO: 62, SEQ ID NO: 22, SEQ ID NO: 23, SEQ ID NO: 63, SEQ ID NO: 28 and SEQ ID NO: 37 are present and the 35 other DNA markers are absent;
- in strain J186, the DNA markers SEQ NO: 47, SEQ ID NO: 5, SEQ NO: 8, SEQ NO: 10, SEQ NO: 11, SEQ NO: 12, SEQ NO: 13, SEQ NO: 57, SEQ ID NO: 16, SEQ NO: 17, SEQ NO: 60, SEQ NO: 62, SEQ NO: 22, SEQ NO: 23, SEQ NO: 24, SEQ NO: 63, SEQ NO: 28, SEQ NO: 29 and SEQ NO: 37 are present and the 33 other DNA markers are absent;
- in strain BMG.167, the DNA markers SEQ NO: 47, SEQ NO: 5, SEQ NO: 8, SEQ NO: 10, SEQ NO: 11, SEQ NO: 12, SEQ NO: 13, SEQ NO: 16, SEQ ID NO: 17, SEQ NO: 60, SEQ NO: 62, SEQ ID NO: 22, SEQ NO: 23, SEQ NO: 63, SEQ NO: 28, SEQ NO: 29 and SEQ NO: 37 are present and the 35 other DNA markers are absent;
- in strain BMG.126, the DNA markers SEQ NO: 47, SEQ NO: 5, SEQ NO: 10, SEQ ID NO: 11, SEQ NO: 12, SEQ NO: 13, SEQ ID NO: 57, SEQ NO: 15, SEQ ID NO: 16, SEQ NO: 17, SEQ NO: 60, SEQ ID NO: 62, SEQ NO: 22, SEQ NO: 23, SEQ NO: 63, SEQ NO: 27, SEQ NO: 28 and SEQ NO: 37 are present and the 34 other DNA markers are absent;
- in strain L110, the DNA markers SEQ NO: 47, SEQ NO: 5, SEQ NO: 8, SEQ NO: 10, SEQ NO: 11, SEQ NO: 12, SEQ NO: 13, SEQ NO: 57, SEQ ID NO: 15, SEQ NO: 16, SEQ NO: 17, SEQ NO: 60, SEQ NO: 62, SEQ NO: 23, SEQ NO: 63, SEQ NO: 28, SEQ NO: 29 and SEQ NO: 37 are present and the 34 other DNA markers are absent;
- in strain MF2091, the DNA markers SEQ NO: 47, SEQ NO: 5, SEQ NO: 10, SEQ ID NO: 11, SEQ NO: 12, SEQ NO: 13, SEQ ID NO: 57, SEQ NO: 16, SEQ ID NO: 17, SEQ NO: 60, SEQ NO: 62, SEQ ID NO: 22, SEQ NO: 23, SEQ NO: 63, SEQ NO: 28, SEQ NO: 29 and SEQ NO: 37 are present and the 35 other DNA markers are absent;
- in strain LTH5588, the DNA markers SEQ NO: 47, SEQ NO: 5, SEQ NO: 10, SEQ ID NO: 11, SEQ NO: 12, SEQ NO: 13, SEQ ID NO: 57, SEQ NO: 16, SEQ ID NO: 17, SEQ NO: 60, SEQ NO: 62, SEQ ID NO: 23, SEQ NO: 63, SEQ NO: 28, SEQ NO: 29 and SEQ NO: 37 are present and the 36 other DNA markers are absent;
- in strain L115, the DNA markers SEQ NO: 47, SEQ NO: 5, SEQ NO: 10, SEQ ID NO: 11, SEQ NO: 12, SEQ NO: 13, SEQ ID NO: 57, SEQ NO: 15, SEQ NO: 16, SEQ NO: 17, SEQ NO: 60, SEQ NO: 62, SEQ NO: 22, SEQ ID NO: 23, SEQ ID NO: 63, SEQ ID NO: 28, SEQ ID NO: 29 and SEQ ID NO: 37 are present and the 34 other DNA markers are absent;
- in strain MF2090, the DNA markers SEQ ID NO: 47, SEQ ID NO: 51, SEQ ID NO: 5, SEQ ID NO: 10, SEQ ID NO: 11, SEQ ID NO: 12, SEQ ID NO: 13, SEQ ID NO: 57, SEQ ID NO: 15, SEQ ID NO: 16, SEQ ID NO: 17, SEQ ID NO: 60, SEQ ID NO: 62, SEQ ID NO: 22, SEQ ID NO: 23, SEQ ID NO: 63, SEQ ID NO: 28, SEQ ID NO: 29 and SEQ ID NO: 37 are present and the 33 other DNA markers are absent.

### Methods for characterizing a L. sakei isolate or a food sample likely to contain a L. sakei strain

The present invention also relates to a method for characterizing a *Lactobacillus sakei* isolate which comprises the step consisting of determining the presence or absence of 46 DNA markers of sequence SEQ ID NO: 1 to SEQ ID NO: 46 as defined above in said *Lactobacillus sakei* isolate.

As used herein, the term "isolate" refers to an essentially homogenous population constituted by a single bacterial strain. Preferably, the *L. sakei* isolate according to the invention corresponds to a *L. sakei* strain. Techniques to obtain isolates from a non-homogenous population are well-known from the one skilled in the art and include for example streak plate technique, pour plate technique, serial dilution. Such techniques are in particular described in Najjari *et al.* 2008. (Najjari et al. (2008). Int J Food Microbiod. 121(3):342-51).

*L. sakei* belongs to the natural flora of many food products and is widely used in food manufacturing. Accordingly, the present invention also relates to a method for characterizing a food sample likely to contain a *Lactobacillus sakei* strain which comprises the step consisting of determining the presence or absence of 46 DNA markers of sequence SEQ ID NO: 1 to SEQ ID NO: 46 defined above in said food sample.

As used herein, a "food sample" refers to a portion of a food preparation. Food preparations encompass in particular fresh preparations, preparations stored under modified atmosphere, vacuum-packed preparations, fermented preparations, smoked preparations and dry preparations. Example of food preparations include meat preparations (including beef meat, calf meat, pork meat, horse meat, lamb meat, mutton meat, goat meat, rabbit meat, turkey meat, chicken meat, duck meat, guinea fowl meat, as sausages, minced, or ground), fish preparations (including salmon, trout) vegetable preparations (including cabbage, gherkin, bean, grape leaf, lemons, garlic, Swiss chard, beet, carrot, celery, mushroom, cucumber, courgette, broad bean, turnip, onion, peas, sweet pepper, pumpkin, radish, escarole, tomato, orange, olives, or mixes such as pickles), cereals preparations (including rice, soy, wheat) and milk (yogurt, cheese, kefir).

In a preferred embodiment, the above defined methods comprise the step of determining the presence or absence of the 52 DNA markers defined above. In another preferred embodiment, they comprise the step of determining the presence or absence of the 60 DNA defined above.

Numerous methods allowing determining the presence or absence of a DNA marker in a bacterial strain are well known from one skilled in the art. These methods include, without being limited, the use of an antibody specifically binding to an antigen which is constituted by the expression product of said DNA marker(s), the detection of mRNA, cDNA or polypeptide from said DNA marker(s), or of fragments thereof, the detection of said DNA markers by amplification or hybridization with DNA probes or primers specific of said DNA markers or of fragments thereof.

Preferably, the presence or absence of said DNA marker(s) is determined according to the invention by amplification, and/or by hybridization with DNA probes specific of said DNA marker(s).

In the context of the invention, the term "amplification" encompasses amplification by polymerase chain reaction (PCR), in particular quantitative PCR (qPCR), or real-time quantitative PCT (RT-qPCR).

As used herein, "real-time quantitative PCR", "real-time polymerase chain reaction", or "kinetic polymerase chain reaction" refers to a laboratory technique based on the polymerase chain reaction, which is used to amplify and simultaneously quantify a targeted DNA molecule. It enables both detection and quantification (as absolute number of copies or relative amount when normalized to DNA input or additional normalizing genes) of a specific sequence in a sample. Two common methods of quantification are the use of fluorescent dyes that intercalate with double-stranded DNA, and modified DNA oligonucleotide probes that fluoresce when hybridized with a complementary DNA.

In the context of the invention, the terms "hybridize" or "hybridization," as is known to those skilled in the art, refer to the binding of a nucleic acid molecule to a particular nucleotide sequence under suitable conditions, namely under stringent conditions.

The term "stringent conditions" or "high stringency conditions" as used herein corresponds to conditions that are suitable to produce binding pairs between nucleic acids having a determined level of complementarity, while being unsuitable to the formation of binding pairs between nucleic acids displaying a complementarity inferior to said determined level. Stringent conditions are the combination of both hybridization and wash conditions and are sequence dependent. These conditions may be modified according to methods known from those skilled in the art (Tijssen, 1993, Laboratory Techniques in Biochemistry and Molecular Biology - Hybridization with Nucleic Acid Probes, Part I, Chapter 2 "Overview of principles of hybridization and the strategy of nucleic acid probe assays", Elsevier, New York). Generally, high stringency conditions are selected to be about 5°C lower than the thermal melting point (Tm), preferably at a temperature close to the Tm of perfectly base-paired duplexes (Andersen, Nucleic acid Hybridization, Springer, 1999, p. 54). Hybridization procedures are well known in the art and are described for example in Ausubel, F.M., Brent, R., Kingston, R.E., Moore, D.D.,Seidman, J.G., Smith, J. A., Struhl, K. eds. (1998) Current protocols in molecular biology. V.B. Chanda, series ed. New York: John Wiley & Sons.

High stringency conditions typically involve hybridizing at about 50°C to about 68°C in 5x SSC/5x Denhardt's solution/1.0% SDS, and washing in 0.2x SSC/0.1% SDS at about 60°C to about 68°C.

### Primers, probes and DNA arrays

In a preferred embodiment, said amplification is performed using primers or probes, as defined herein below, having sequences that specifically hybridize under high stringency conditions to at least one sequence selected from group consisting of the sequences SEQ ID NO: 1 to SEQ ID NO: 46, and/or a complementary sequence thereof.

As used herein, the expression "hybridizes specifically" indicates that the nucleic acid of a determined sequence displays a sufficient degree of complementarity with a target sequence to form a stable binding between the nucleic acid and the target sequence and to avoid non-specific binding with a non-target sequence, under high stringency conditions. The degree of complementarity is calculated by comparing the sequence of said nucleic acid optimally aligned with the complementary target sequence, determining the number of positions at which the nucleic acid bases are complementary to yield the number of complementary positions, dividing the number of complementary positions by the total number of positions of the target sequence, and multiplying the result by 100 to yield the degree of complementarity. The degree of complementarity may be determined routinely using BLAST programs (basic local alignment search tools) and PowerBLAST programs known in the art (Altschul et al., (1990) J. Mol. Biol. 215: 403-410; Zhang and Madden (1997) Genome Res. 7:649-656).

As used herein, a "probe" refers to an oligonucleotide capable of binding in a base-specific manner to a complementary strand of nucleic acid. Probes according to the invention may be purified or recombinant. They may be labelled with a detectable moiety, *i.e.* a moiety capable of generating a detectable signal, such as radioactive, calorimetric, fluorescent, chemiluminescent or electrochemiluminescent signal. Numerous such detectable moieties are known in the art. By way of example, the moiety may be a radioactive compound or a detectable enzyme (*e.g*. horseradish peroxidase (HRP)). Preferably, the probe according to the invention comprises or is constituted of from about 10 to about 1000 nucleotides. More preferably, the probe according to the invention has a minimal size of more than 10 nucleotides, more than 20 nucleotides, more than 30 nucleotides, or more than 40 nucleotides, and a maximal size of less than 1000 nucleotides, less than 900 nucleotides, less than 800 nucleotides, less than 700 nucleotides, less than 600 nucleotides, less than 500 nucleotides, less than 400 nucleotides, less than 300 nucleotides, less than 200 nucleotides, less than 100 nucleotides, less than 90 nucleotides, less than 80 nucleotides, less than 70 nucleotides, less than 60 nucleotides, or less than 50 nucleotides. Most preferably, the probe according to the invention has a size of 60 to 70 nucleotides.

As used herein, the term "primer" refers to an oligonucleotide which is capable of annealing to a target sequence and serving as a point of initiation of DNA synthesis under conditions suitable for amplification of the primer extension product which is complementary to said target sequence. The primer is preferably single stranded for maximum efficiency in amplification. Preferably, the primer is an oligodeoxyribonucleotide. The length of the primer depends on several factors, including temperature and sequence of the primer, but must be long enough to initiate the synthesis of amplification products. Preferably the primer is from 10 to 100 nucleotides in length. More preferably, the primer is from 25 to 80 nucleotides in length, still preferably from 50 to 75 nucleotides in length. Most preferably, the primer is from 60 to 70 nucleotides in length. A primer can further contain additional features which allow for detection, immobilization, or manipulation of the amplified product. The primer may furthermore comprise covalently-bound fluorescent dyes, which confer specific fluorescence properties to the hybrid consisting of the primer and the target sequence or non covalently-bound fluorescent dyes which can interact with the double-stranded DNA/RNA to change the fluorescence properties. Fluorescent dyes which can be used are for example SYBR-green or ethidium bromide.

Preferably, the probes or primers according to the invention have sequences that specifically hybridize under high stringency conditions to at least one sequence selected from the group consisting of the sequences SEQ NO: 1 to SEQ NO: 46 and complementary sequences thereof independently of the allelic version of said sequences. Examples of suitable probes or primers according to the invention include the probes or primers having the sequence SEQ NO: 72, that specifically hybridizes to SEQ ID NO: 1, SEQ NO: 73 that specifically hybridizes to the complementary sequence of SEQ NO: 1, SEQ NO: 74 that specifically hybridizes to SEQ NO: 2, SEQ ID NO: 75 that specifically hybridizes to the complementary sequence of SEQ NO: 2, SEQ ID NO: 76 that specifically hybridizes to SEQ NO: 3, SEQ NO: 77 that specifically hybridizes to the complementary sequence of SEQ NO: 3, SEQ ID NO: 78 that specifically hybridizes to SEQ NO: 4, SEQ NO: 79 that specifically hybridizes to the complementary sequence of SEQ ID NO: 4, SEQ NO: 80 that specifically hybridizes to SEQ NO: 5, SEQ NO: 81 that specifically hybridizes to the complementary sequence of SEQ NO: 5, SEQ NO: 82 that specifically hybridizes to SEQ NO: 6, SEQ ID NO: 83 that specifically hybridizes to the complementary sequence of SEQ NO: 6, SEQ NO: 84 that specifically hybridizes to SEQ NO: 7, SEQ ID NO: 85 that specifically hybridizes to the complementary sequence of SEQ NO: 7, SEQ ID NO: 86 that specifically hybridizes to SEQ NO: 8, SEQ NO: 87 that specifically hybridizes to the complementary sequence of SEQ NO: 8, SEQ ID NO: 88 that specifically hybridizes to SEQ NO: 9, SEQ NO: 89 that specifically hybridizes to the complementary sequence of SEQ ID NO: 9, SEQ NO: 90 that specifically hybridizes to SEQ NO: 10, SEQ NO: 91 that specifically hybridizes to the complementary sequence of SEQ NO: 10, SEQ NO: 92 that specifically hybridizes to SEQ NO: 11, SEQ ID NO: 93 that specifically hybridizes to the complementary sequence of SEQ NO: 11, SEQ NO: 94 that specifically hybridizes to SEQ NO: 12, SEQ NO: 95 that specifically hybridizes to the complementary sequence of SEQ NO: 12, SEQ NO: 96 that specifically hybridizes to SEQ ID NO: 13, SEQ ID NO: 97 that specifically hybridizes to the complementary sequence of SEQ ID NO: 13, SEQ ID NO: 98 that specifically hybridizes to SEQ ID NO: 14, SEQ ID NO: 99 that specifically hybridizes to the complementary sequence of SEQ ID NO: 14, SEQ ID NO: 100 that specifically hybridizes to SEQ ID NO: 15, SEQ ID NO: 101 that specifically hybridizes to the complementary sequence of SEQ ID NO: 15, SEQ ID NO: 102 that specifically hybridizes to SEQ ID NO: 16, SEQ ID NO: 103 that specifically hybridizes to the complementary sequence of SEQ ID NO: 16, SEQ ID NO: 104 that specifically hybridizes to SEQ ID NO: 17, SEQ ID NO: 105 that specifically hybridizes to the complementary sequence of SEQ ID NO: 17, SEQ ID NO: 106 that specifically hybridizes to SEQ ID NO: 18, SEQ ID NO: 107 that specifically hybridizes to the complementary sequence of SEQ ID NO: 18, SEQ ID NO: 108 that specifically hybridizes to SEQ ID NO: 19, SEQ ID NO: 109 that specifically hybridizes to the complementary sequence of SEQ ID NO: 19, SEQ ID NO: 110 that specifically hybridizes to SEQ ID NO: 20, SEQ ID NO: 111 that specifically hybridizes to the complementary sequence of SEQ ID NO: 20, SEQ ID NO: 112 that specifically hybridizes to SEQ ID NO: 21, SEQ ID NO: 113 that specifically hybridizes to the complementary sequence of SEQ ID NO: 21, SEQ ID NO: 114 that specifically hybridizes to SEQ ID NO: 22, SEQ ID NO: 115 that specifically hybridizes to the complementary sequence of SEQ ID NO: 22, SEQ ID NO: 116 that specifically hybridizes to SEQ ID NO: 23, SEQ ID NO: 117 that specifically hybridizes to the complementary sequence of SEQ ID NO: 23, SEQ ID NO: 118 that specifically hybridizes to SEQ ID NO: 24, SEQ ID NO: 119 that specifically hybridizes to the complementary sequence of SEQ ID NO: 24, SEQ ID NO: 120 that specifically hybridizes to SEQ ID NO: 25, SEQ ID NO: 121 that specifically hybridizes to the complementary sequence of SEQ ID NO: 25, SEQ ID NO: 122 that specifically hybridizes to SEQ ID NO: 26, SEQ ID NO: 123 that specifically hybridizes to the complementary sequence of SEQ ID NO: 26, SEQ ID NO: 124 that specifically hybridizes to SEQ ID NO: 27, SEQ ID NO: 125 that specifically hybridizes to the complementary sequence of SEQ ID NO: 27, SEQ ID NO: 126 that specifically hybridizes to SEQ ID NO: 28, SEQ ID NO: 127 that specifically hybridizes to the complementary sequence of SEQ ID NO: 28, SEQ ID NO: 128 that specifically hybridizes to SEQ ID NO: 29, SEQ ID NO: 129 that specifically hybridizes to the complementary sequence of SEQ ID NO: 29, SEQ ID NO: 130 that specifically hybridizes to SEQ ID NO: 30, SEQ ID NO: 131 that specifically hybridizes to the complementary sequence of SEQ ID NO: 30, SEQ ID NO: 132 that specifically hybridizes to SEQ ID NO: 31, SEQ ID NO: 133 that specifically hybridizes to the complementary sequence of SEQ ID NO: 31, SEQ ID NO: 134 that specifically hybridizes to SEQ ID NO: 32, SEQ ID NO: 135 that specifically hybridizes to the complementary sequence of SEQ ID NO: 32, SEQ ID NO: 136 that specifically hybridizes to SEQ ID NO: 33, SEQ ID NO: 137 that specifically hybridizes to the complementary sequence of SEQ ID NO: 33, SEQ ID NO: 138 that specifically hybridizes to SEQ ID NO: 34, SEQ ID NO: 139 that specifically hybridizes to the complementary sequence of SEQ ID NO: 34, SEQ ID NO: 140 that specifically hybridizes to SEQ ID NO: 35, SEQ ID NO: 141 that specifically hybridizes to the complementary sequence of SEQ ID NO: 35, SEQ ID NO: 142 that specifically hybridizes to SEQ ID NO: 36, SEQ ID NO: 143 that specifically hybridizes to the complementary sequence of SEQ ID NO: 36, SEQ ID NO: 144 that specifically hybridizes to SEQ ID NO: 37, SEQ ID NO: 145 that specifically hybridizes to the complementary sequence of SEQ ID NO: 37, SEQ ID NO: 146 that specifically hybridizes to SEQ ID NO: 38, SEQ ID NO: 147 that specifically hybridizes to the complementary sequence of SEQ ID NO: 38, SEQ ID NO: 148 that specifically hybridizes to SEQ ID NO: 39, SEQ ID NO: 149 that specifically hybridizes to the complementary sequence of SEQ ID NO: 39, SEQ ID NO: 150 that specifically hybridizes to SEQ ID NO: 40, SEQ ID NO: 151 that specifically hybridizes to the complementary sequence of SEQ ID NO: 40, SEQ ID NO: 152 that specifically hybridizes to SEQ ID NO: 41, SEQ ID NO: 153 that specifically hybridizes to the complementary sequence of SEQ ID NO: 41, SEQ ID NO: 154 that specifically hybridizes to SEQ ID NO: 42, SEQ ID NO: 155 that specifically hybridizes to the complementary sequence of SEQ ID NO: 42, SEQ ID NO: 156 that specifically hybridizes to SEQ ID NO: 43, SEQ ID NO: 157 that specifically hybridizes to the complementary sequence of SEQ ID NO: 43, SEQ ID NO: 158 that specifically hybridizes to SEQ ID NO: 44, SEQ ID NO: 159 that specifically hybridizes to the complementary sequence of SEQ ID NO: 44, SEQ ID NO: 160 that specifically hybridizes to SEQ ID NO: 45, SEQ ID NO: 161 that specifically hybridizes to the complementary sequence of SEQ ID NO: 45, SEQ ID NO: 162 that specifically hybridizes to SEQ ID NO: 46, SEQ ID NO: 163 that specifically hybridizes to the complementary sequence of SEQ ID NO: 46. Accordingly, preferably, said amplification is performed using primers or probes having sequences SEQ ID NO: 72 to SEQ ID NO: 163.

The probes or primers according to the invention may also have sequences that specifically hybridize under high stringency conditions to at least one sequence selected from the group consisting of the sequences SEQ ID NO: 1 to SEQ ID NO: 46 and complementary sequences thereof, in an allelic version-specific way. Examples of suitable probes or primers according to the invention that specifically hybridize to an allelic version of the DNA markers according to the invention include probes or primers having the sequence SEQ ID NO: 164 that specifically hybridizes to SEQ ID NO: 47, SEQ ID NO: 165 that specifically hybridizes to the complementary sequence of SEQ ID NO: 47, SEQ ID NO: 166 that specifically hybridizes to SEQ ID NO: 48, SEQ ID NO: 167 that specifically hybridizes to the complementary sequence of SEQ ID NO: 48, SEQ ID NO: 168 that specifically hybridizes to SEQ ID NO: 50, SEQ ID NO: 169 that specifically hybridizes to the complementary sequence of SEQ ID NO: 50, SEQ ID NO: 170 that specifically hybridizes to SEQ ID NO: 51, SEQ ID NO: 171 that specifically hybridizes to the complementary sequence of SEQ ID NO: 51, SEQ ID NO: 172 that specifically hybridizes to SEQ ID NO: 53, SEQ ID NO: 173 that specifically hybridizes to the complementary sequence of SEQ ID NO: 53, SEQ ID NO: 174 that specifically hybridizes to SEQ ID NO: 54, SEQ ID NO: 175 that specifically hybridizes to the complementary sequence of SEQ ID NO: 54, SEQ ID NO: 176 that specifically hybridizes to SEQ ID NO: 55, SEQ ID NO: 177 that specifically hybridizes to the complementary sequence of SEQ ID NO: 55, SEQ ID NO: 178 that specifically hybridizes to SEQ ID NO: 57, SEQ ID NO: 179 that specifically hybridizes to the complementary sequence of SEQ ID NO: 57, SEQ ID NO: 180 that specifically hybridizes to SEQ ID NO: 59, SEQ ID NO: 181 that specifically hybridizes to the complementary sequence of SEQ ID NO: 59, SEQ ID NO: 182 that specifically hybridizes to SEQ ID NO: 60, SEQ ID NO: 183 that specifically hybridizes to the complementary sequence of SEQ ID NO: 60, SEQ ID NO: 184 that specifically hybridizes to SEQ ID NO: 61, SEQ ID NO: 185 that specifically hybridizes to the complementary sequence of SEQ ID NO: 61, SEQ ID NO: 186 that specifically hybridizes to SEQ ID NO: 62, SEQ ID NO: 187 that specifically hybridizes to the complementary sequence of SEQ ID NO: 62, SEQ ID NO: 188 that specifically hybridizes to SEQ ID NO: 63, SEQ ID NO: 189 that specifically hybridizes to the complementary sequence of SEQ ID NO: 64, SEQ ID NO: 190 that specifically hybridizes to SEQ ID NO: 65, SEQ ID NO: 191 that specifically hybridizes to the complementary sequence of SEQ ID NO: 65, SEQ ID NO: 192 that specifically hybridizes to SEQ ID NO: 68, SEQ ID NO: 193 that specifically hybridizes to the complementary sequence of SEQ ID NO: 68, SEQ ID NO: 194 that specifically hybridizes to SEQ ID NO: 69, SEQ ID NO: 195 that specifically hybridizes to the complementary sequence of SEQ ID NO: 69, SEQ ID NO: 196 that specifically hybridizes to SEQ ID NO: 70, SEQ ID NO: 197 that specifically hybridizes to the complementary sequence of SEQ ID NO: 70, SEQ ID NO: 198 that specifically hybridizes to SEQ ID NO: 49, SEQ ID NO: 199 that specifically hybridizes to the complementary sequence of SEQ ID NO: 49, SEQ ID NO: 200 that specifically hybridizes to SEQ ID NO: 52, SEQ ID NO: 201 that specifically hybridizes to the complementary sequence of SEQ ID NO: 52, SEQ ID NO: 202 that specifically hybridizes to SEQ ID NO: 56, SEQ ID NO: 203 that specifically hybridizes to the complementary sequence of SEQ ID NO: 56, SEQ ID NO: 204 that specifically hybridizes to SEQ ID NO: 58, SEQ ID NO: 205 that specifically hybridizes to the complementary sequence of SEQ ID NO: 58, SEQ ID NO: 206 that specifically hybridizes to SEQ ID NO: 64, SEQ ID NO: 207 that specifically hybridizes to the complementary sequence of SEQ ID NO: 64, SEQ ID NO: 208 that specifically hybridizes to SEQ ID NO: 66, SEQ ID NO: 209 that specifically hybridizes to the complementary sequence of SEQ ID NO: 66, SEQ ID NO: 210 that specifically hybridizes to SEQ ID NO: 67, SEQ ID NO: 211 that specifically hybridizes to the complementary sequence of SEQ ID NO: 67, SEQ ID NO: 212 that specifically hybridizes to SEQ ID NO: 71, SEQ ID NO: 213 that specifically hybridizes to the complementary sequence of SEQ ID NO: 71.

In a particular embodiment, said probes specific of these DNA markers are assembled on a same support, preferably a standardized support. These supports are known from one skilled in the art. Their size can vary according to the apparatuses used to detect the presence or absence of said marker gene(s).

Advantageously, the combination of DNA markers according to the invention is in form of a DNA matrix, comprising a support on which nucleic acids fragments likely to hybridize to target genes are deposed, preferably in a standardized way. The size of such supports varies according to the preparation and detection methods used. Such small supports are also referred to DNA array.

Another aspect of the present invention thus concerns a DNA array which comprises at least one probe comprising or consisting of a sequence that specifically hybridizes under high stringency conditions to a sequence selected from the sequences SEQ ID NO: 1 to SEQ ID NO: 71 and a complementary sequence thereof. Preferably, said at least one probe comprises or consists of a sequence selected from the group consisting of sequences SEQ ID NO: 72 to SEQ ID NO: 213.

Preferably, the DNA array according to the invention comprises at least 46 probes comprising or consisting of sequences that specifically hybridize under high stringency conditions to the 46 sequences SEQ ID NO: 1 to SEQ ID NO: 46 and a complementary sequence thereof. Specifically, each of the 46 probes defined above specifically hybridizes to a sequence different from the other probes, said sequence being selected from the group consisting of sequences SEQ ID NO: 1 to SEQ ID NO: 46 and a complementary sequence thereof. Preferably, said at least 46 probes specifically hybridizes to the 46 sequences defined above independently of the allelic version of said sequences. Preferably, said at least 46 probes comprise or consist of the sequences selected from the group consisting of sequences SEQ ID NO: 72 to SEQ ID NO: 163. Said at least 46 probes may in another embodiment specifically hybridizes to specific allelic versions of the 46 sequences defined above.

More preferably, the DNA array according to the invention comprises at least 52 probes comprising or consisting of sequences that specifically hybridize under high stringency conditions to the 52 sequences of the 52 DNA markers defined above and a complementary sequence thereof. Specifically, each of the 52 probes defined above specifically hybridizes to a sequence different from the other probes, said sequence being selected from the group consisting of sequences SEQ ID NO: 47, SEQ ID NO: 48, SEQ ID NO: 50, SEQ ID NO: 51,SEQ ID NO: 3 to SEQ ID NO: 5, SEQ ID NO: 53 to SEQ ID NO: 55, SEQ ID NO: 8 to SEQ ID NO: 13, SEQ ID NO: 57, SEQ ID NO: 15 to SEQ ID NO: 18, SEQ ID NO: 59 to SEQ ID NO: 63, SEQ ID NO: 20, SEQ ID NO: 22 to SEQ ID NO: 25, SEQ ID NO: 27 to SEQ ID NO: 29, SEQ ID NO: 65, SEQ ID NO: 31, SEQ ID NO: 68 to SEQ ID NO: 70, SEQ ID NO: 33 to SEQ ID NO: 40 and SEQ ID NO: 42 to SEQ ID NO: 46 and a complementary sequence thereof. Preferably, said at least 52 probes comprise or consist of the sequences selected from the group consisting of sequences SEQ ID NO: 76 to SEQ ID NO: 81, SEQ ID NO: 86 to SEQ ID NO: 97, SEQ ID NO: 100 to SEQ ID NO: 107, SEQ ID NO: 110 to SEQ ID NO: 111, SEQ ID NO: 114 to SEQ ID NO: 121, SEQ ID NO: 124 to SEQ ID NO: 129, SEQ ID NO: 132 to SEQ ID NO: 133, SEQ ID NO: 136 to SEQ ID NO: 151, and SEQ ID NO: 154 to SEQ ID NO: 197.

Still preferably, the DNA array according to the invention comprises at least 60 probes comprising or consisting of sequences that specifically hybridize under high stringency conditions to the 60 sequences SEQ ID NO: 3 to SEQ ID NO: 5, SEQ ID NO: 8 to SEQ ID NO: 13, SEQ ID NO: 15 to SEQ ID NO: 18, SEQ ID NO: 20, SEQ ID NO: 22 to SEQ ID NO: 25, SEQ ID NO: 27 to SEQ ID NO: 29, SEQ ID NO: 31, SEQ ID NO: 33 to SEQ ID NO: 40, SEQ ID NO: 42 to SEQ ID NO: 46 and SEQ ID NO: 47 to SEQ ID NO: 71 and a complementary sequence thereof. Specifically, each of the 60 probes defined above specifically hybridizes to a sequence different from the other probes, said sequence being selected from the group consisting of the 60 sequences defined above and a complementary sequence thereof. Preferably, said at least 60 probes comprise or consist of the sequences selected from the group consisting of sequences SEQ ID NO: 76 to SEQ ID NO: 81, SEQ ID NO: 86 to SEQ ID NO: 97, SEQ ID NO: 100 to SEQ ID NO: 107, SEQ ID NO: 110 to SEQ ID NO: 111, SEQ ID NO: 114 to SEQ ID NO: 121, SEQ ID NO: 124 to SEQ ID NO: 129, SEQ ID NO: 132 to SEQ ID NO: 133, SEQ ID NO: 136 to SEQ ID NO: 151, and SEQ ID NO: 154 to SEQ ID NO: 213.

As used herein, the term "DNA array" refers to a set of genes, fragment of genes, oligonucleotides deposited on a support (glass slide, nylon membrane...) with a high density. Numerous scientific publications about the preparation and the use of DNA arrays are available.

### Kits

Another object of the present invention is therefore a kit for characterizing a *Lactobacillus sakei* isolate or for characterizing a food sample likely to contain a *L. sakei* strain, which comprises 46 pairs of primers, wherein each primer has a sequence that specifically hybridizes under high stringency conditions to one sequence selected from the group consisting of sequences SEQ ID NO: 1 to SEQ ID NO: 46 and a complementary sequence thereof. Specifically, each of the 46 pairs of primers defined above specifically hybridizes to a sequence different from the other pairs of primers, said sequence being selected from the group consisting of sequences SEQ ID NO: 1 to SEQ ID NO: 46 and a complementary sequence thereof. Preferably, said at least 46 pairs of primers specifically hybridizes to the 46 sequences defined above independently of the allelic version of said sequences. Said at least 46 pairs of primers may in another embodiment specifically hybridizes to specific allelic versions of the 46 sequences defined above.

In a preferred embodiment, the kit according to the invention comprises 52 pairs of primers, wherein each primer has a sequence that specifically hybridizes under high stringency conditions to one sequence selected from the group consisting of sequences SEQ ID NO: 47, SEQ ID NO: 48, SEQ ID NO: 50, SEQ ID NO: 51,SEQ ID NO: 3 to SEQ ID NO: 5, SEQ ID NO: 53 to SEQ ID NO: 55, SEQ ID NO: 8 to SEQ ID NO: 13, SEQ ID NO: 57, SEQ ID NO: 15 to SEQ ID NO: 18, SEQ ID NO: 59 to SEQ ID NO: 63, SEQ ID NO: 20, SEQ ID NO: 22 to SEQ ID NO: 25, SEQ ID NO: 27 to SEQ ID NO: 29, SEQ ID NO: 65, SEQ ID NO: 31, SEQ ID NO: 68 to SEQ ID NO: 70, SEQ ID NO: 33 to SEQ ID NO: 40 and SEQ ID NO: 42 to SEQ ID NO: 46 and a complementary sequence thereof. Specifically, each of the 52 pairs of primers defined above specifically hybridizes to a sequence different from the other pairs of primers, said sequence being selected from the group consisting of the 52 sequences defined above and a complementary sequence thereof.

In another preferred embodiment, the kit according to the invention comprises 60 pairs of primers, wherein each primer has a sequence that specifically hybridizes under high stringency conditions to one sequence selected from the group consisting of sequences SEQ ID NO: 3 to SEQ ID NO: 5, SEQ ID NO: 8 to SEQ ID NO: 13, SEQ ID NO: 15 to SEQ ID NO: 18, SEQ ID NO: 20, SEQ ID NO: 22 to SEQ ID NO: 25, SEQ ID NO: 27 to SEQ ID NO: 29, SEQ ID NO: 31, SEQ ID NO: 33 to SEQ ID NO: 40, SEQ ID NO: 42 to SEQ ID NO: 46 and SEQ ID NO: 47 to SEQ ID NO: 71 and a complementary sequence thereof. Specifically, each of the 60 pairs of primers defined above specifically hybridizes to a sequence different from the other pairs of primers, said sequence being selected from the group consisting of the 60 sequences defined above and a complementary sequence thereof.

Preferably, where the kit according to the invention comprises 46 pairs of primers, it comprises (1) a pair of primers that specifically hybridizes under high stringency conditions to the sequence SEQ ID NO: 1 and/or a complementary sequence thereof, (2) a pair of primers that specifically hybridizes under high stringency conditions to the sequence SEQ ID NO: 2 and/or a complementary sequence thereof, (3) a pair of primers that specifically hybridizes under high stringency conditions to the sequence SEQ ID NO: 3 and/or a complementary sequence thereof, (4) a pair of primers that specifically hybridizes under high stringency conditions to the sequence SEQ ID NO: 4 and/or a complementary sequence thereof, (5) a pair of primers that specifically hybridizes under high stringency conditions to the sequence SEQ ID NO: 5 and/or a complementary sequence thereof, (6) a pair of primers that specifically hybridizes under high stringency conditions to the sequence SEQ ID NO: 6 and/or a complementary sequence thereof, (7) a pair of primers that specifically hybridizes under high stringency conditions to the sequence SEQ ID NO: 7 and/or a complementary sequence thereof, (8) a pair of primers that specifically hybridizes under high stringency conditions to the sequence SEQ ID NO: 8 and/or a complementary sequence thereof, (9) a pair of primers that specifically hybridizes under high stringency conditions to the sequence SEQ ID NO: 9 and/or a complementary sequence thereof, (10) a pair of primers that specifically hybridizes under high stringency conditions to the sequence SEQ ID NO: 10 and/or a complementary sequence thereof, (11) a pair of primers that specifically hybridizes under high stringency conditions to the sequence SEQ ID NO: 11 and/or a complementary sequence thereof, (12) a pair of primers that specifically hybridizes under high stringency conditions to the sequence SEQ ID NO: 12 and/or a complementary sequence thereof, (13) a pair of primers that specifically hybridizes under high stringency conditions to the sequence SEQ ID NO: 13 and/or a complementary sequence thereof, (14) a pair of primers that specifically hybridizes under high stringency conditions to the sequence SEQ ID NO: 14 and/or a complementary sequence thereof, (15) a pair of primers that specifically hybridizes under high stringency conditions to the sequence SEQ ID NO: 15 and/or a complementary sequence thereof, (16) a pair of primers that specifically hybridizes under high stringency conditions to the sequence SEQ ID NO: 16 and/or a complementary sequence thereof, (17) a pair of primers that specifically hybridizes under high stringency conditions to the sequence SEQ ID NO: 17 and/or a complementary sequence thereof, (18) a pair of primers that specifically hybridizes under high stringency conditions to the sequence SEQ ID NO: 18 and/or a complementary sequence thereof, (19) a pair of primers that specifically hybridizes under high stringency conditions to the sequence SEQ ID NO: 19 and/or a complementary sequence thereof, (20) a pair of primers that specifically hybridizes under high stringency conditions to the sequence SEQ ID NO: 20 and/or a complementary sequence thereof, (21) a pair of primers that specifically hybridizes under high stringency conditions to the sequence SEQ ID NO: 21 and/or a complementary sequence thereof, (22) a pair of primers that specifically hybridizes under high stringency conditions to the sequence SEQ ID NO: 22 and/or a complementary sequence thereof, (23) a pair of primers that specifically hybridizes under high stringency conditions to the sequence SEQ ID NO: 23 and/or a complementary sequence thereof, (24) a pair of primers that specifically hybridizes under high stringency conditions to the sequence SEQ ID NO: 24 and/or a complementary sequence thereof, (25) a pair of primers that specifically hybridizes under high stringency conditions to the sequence SEQ ID NO: 25 and/or a complementary sequence thereof, (26) a pair of primers that specifically hybridizes under high stringency conditions to the sequence SEQ ID NO: 26 and/or a complementary sequence thereof, (27) a pair of primers that specifically hybridizes under high stringency conditions to the sequence SEQ ID NO: 27 and/or a complementary sequence thereof, (28) a pair of primers that specifically hybridizes under high stringency conditions to the sequence SEQ ID NO: 28 and/or a complementary sequence thereof, (29) a pair of primers that specifically hybridizes under high stringency conditions to the sequence SEQ ID NO: 29 and/or a complementary sequence thereof, (30) a pair of primers that specifically hybridizes under high stringency conditions to the sequence SEQ ID NO: 30 and/or a complementary sequence thereof, (31) a pair of primers that specifically hybridizes under high stringency conditions to the sequence SEQ ID NO: 31 and/or a complementary sequence thereof, (32) a pair of primers that specifically hybridizes under high stringency conditions to the sequence SEQ ID NO: 32 and/or a complementary sequence thereof, (33) a pair of primers that specifically hybridizes under high stringency conditions to the sequence SEQ ID NO: 33 and/or a complementary sequence thereof, (34) a pair of primers that specifically hybridizes under high stringency conditions to the sequence SEQ ID NO: 34 and/or a complementary sequence thereof, (35) a pair of primers that specifically hybridizes under high stringency conditions to the sequence SEQ ID NO: 35 and/or a complementary sequence thereof, (36) a pair of primers that specifically hybridizes under high stringency conditions to the sequence SEQ ID NO: 36 and/or a complementary sequence thereof, (37) a pair of primers that specifically hybridizes under high stringency conditions to the sequence SEQ ID NO: 37 and/or a complementary sequence thereof, (38) a pair of primers that specifically hybridizes under high stringency conditions to the sequence SEQ ID NO: 38 and/or a complementary sequence thereof, (39) a pair of primers that specifically hybridizes under high stringency conditions to the sequence SEQ ID NO: 39 and/or a complementary sequence thereof, (40) a pair of primers that specifically hybridizes under high stringency conditions to the sequence SEQ ID NO: 40 and/or a complementary sequence thereof, (41) a pair of primers that specifically hybridizes under high stringency conditions to the sequence SEQ ID NO: 41 and/or a complementary sequence thereof, (42) a pair of primers that specifically hybridizes under high stringency conditions to the sequence SEQ ID NO: 42 and/or a complementary sequence thereof, (43) a pair of primers that specifically hybridizes under high stringency conditions to the sequence SEQ ID NO: 43 and/or a complementary sequence thereof, (44) a pair of primers that specifically hybridizes under high stringency conditions to the sequence SEQ ID NO: 44 and/or a complementary sequence thereof, (45) a pair of primers that specifically hybridizes under high stringency conditions to the sequence SEQ ID NO: 45 and/or a complementary sequence thereof, and (46) a pair of primers that specifically hybridizes under high stringency conditions to the sequence SEQ ID NO: 46 and/or a complementary sequence thereof.

Where the kit according to the invention comprises 52 pairs of primers, it further comprises (47) a pair of primers that specifically hybridizes under high stringency conditions to the sequence SEQ ID NO: 47 and/or a complementary sequence thereof, (48) a pair of primers that specifically hybridizes under high stringency conditions to the sequence SEQ ID NO: 49 and/or a complementary sequence thereof, (49) a pair of primers that specifically hybridizes under high stringency conditions to the sequence SEQ ID NO: 51 and/or a complementary sequence thereof, (50) a pair of primers that specifically hybridizes under high stringency conditions to the sequence SEQ ID NO: 54 and/or a complementary sequence thereof, (51) a pair of primers that specifically hybridizes under high stringency conditions to the sequence SEQ ID NO: 55 and/or a complementary sequence thereof, and (52) a pair of primers that specifically hybridizes under high stringency conditions to the sequence SEQ ID NO: 59 and/or a complementary sequence thereof.

Where the kit according to the invention comprises 60 pairs of primers, it further comprises (53) a pair of primers that specifically hybridizes under high stringency conditions to the sequence SEQ ID NO: 48 and/or a complementary sequence thereof, (54) a pair of primers that specifically hybridizes under high stringency conditions to the sequence SEQ ID NO: 50 and/or a complementary sequence thereof, (55) a pair of primers that specifically hybridizes under high stringency conditions to the sequence SEQ ID NO: 52 and/or a complementary sequence thereof, (56) a pair of primers that specifically hybridizes under high stringency conditions to the sequence SEQ ID NO: 53 and/or a complementary sequence thereof, (57) a pair of primers that specifically hybridizes under high stringency conditions to the sequence SEQ ID NO: 56 and/or a complementary sequence thereof, (58) a pair of primers that specifically hybridizes under high stringency conditions to the sequence SEQ ID NO: 57 and/or a complementary sequence thereof, (59) a pair of primers that specifically hybridizes under high stringency conditions to the sequence SEQ NO: 58 and/or a complementary sequence thereof, and (60) a pair of primers that specifically hybridizes under high stringency conditions to the sequence SEQ ID NO: 60 and/or a complementary sequence thereof.

As well-known from the one skilled in the art, a pair of primers is composed of a forward primer that specifically hybridizes to a sequence of interest and of a reverse primer that specifically hybridizes to the complementary sequence of said sequence of interest. Examples of sequences of suitable primers according to the invention that specifically hybridizes to the DNA markers according to the invention and/or to a complementary sequence thereof are provided herein above. Examples of suitable pairs of primers according to the invention may thus be deduced easily by the one skilled in the art from the examples of suitable primers described above.

### The kit according to the invention may further comprise instructions for use.

"Instructions for use" typically include a tangible expression describing the technique to be employed in using the components of the kit and explaining how to correlate determination of the presence or absence of sequences SEQ NO: 1 to SEQ NO: 60 to the characterization of a *L. sakei* isolate.

The 46 pairs of primers according to the invention are localized within the DNA markers defined above and are sufficient for defining a unique PCR fingerprinting profile of any *L. sakei* isolate tested. Using up to the 60 pairs of primers according to the invention enables increasing the accuracy of said PCR fingerprinting profile.

### Methods for fingerprinting a L. sakei isolate or a food sample likely to contain a L. sakei isolate

Accordingly, the present invention also relates to a method for fingerprinting a *Lactobacillus sakei* isolate which comprises the amplification of 46 DNA markers of sequence SEQ NO: 1 to SEQ ID NO: 46 in said *Lactobacillus sakei* isolate, wherein the amplification profile of said 46 DNA markers is the fingerprint of said *Lactobacillus sakei* isolate. Preferably, the method for fingerprinting as defined above comprises the amplification of the 60 DNA markers of sequences defined above.

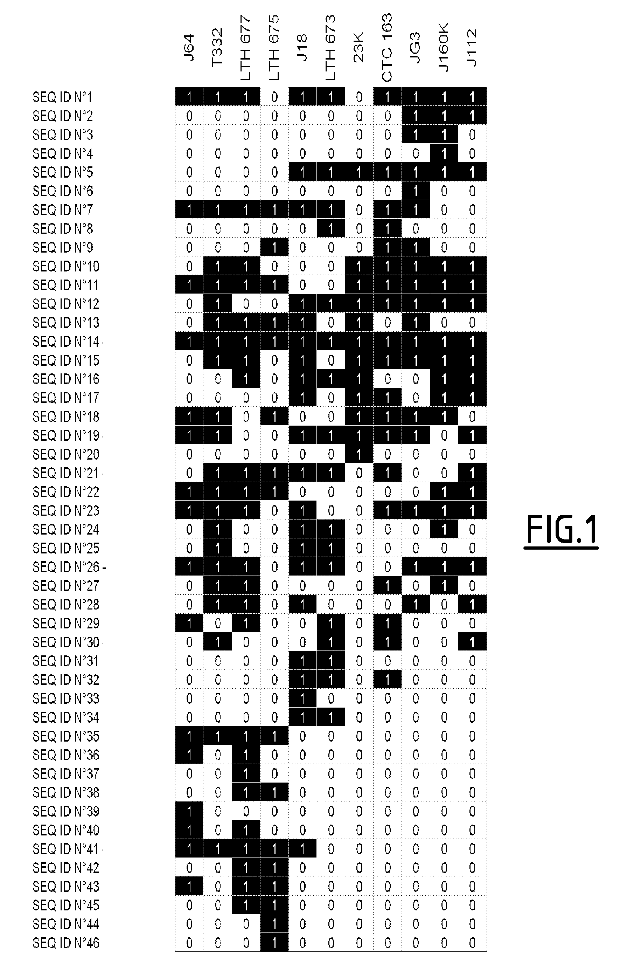

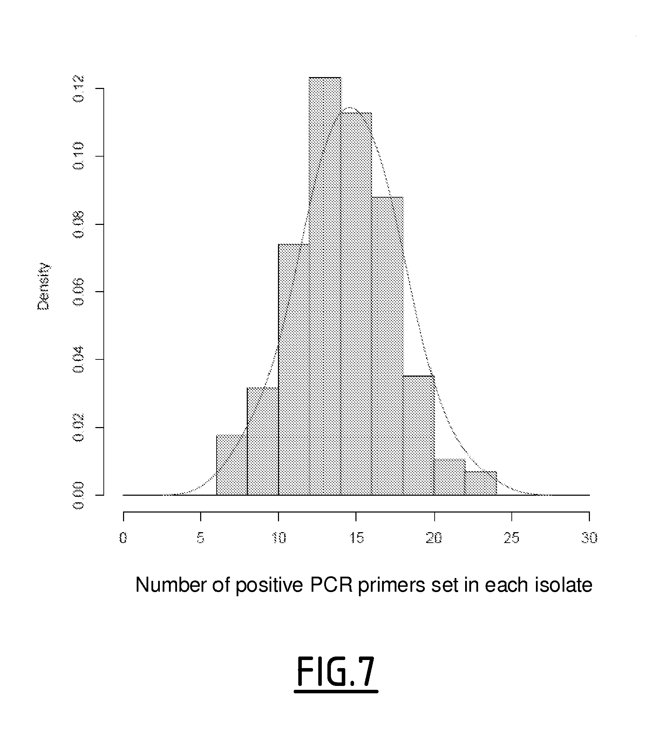

As used herein, the term "amplification profile" refers to the compilation of the results of the amplification of each DNA marker according to the invention, and corresponds to a table wherein the presence or absence of each DNA marker (detected by amplification) is summarized. In the context of the invention, a unique fingerprinting profile between two isolates is defined as two profiles differing by at least one DNA marker detection. Examples of amplification profiles are presented in **Figures 1 to 6****.** However, the number of fingerprints is not limited to those shown in these figures but is likely to be as large as the natural *L. sakei* strains diversity. Indeed, the number of DNA markers to which each isolate is responding positively is comprised between 8 and 25 (**Figure 7**), with an average of about 15 per isolate corresponding to a normal distribution. This is indicating that the invention offers a high degree of potential fingerprints (over 2.10¹² possibilities if 50 DNA markers and a fixed number of combination of 15 markers are chosen).

The herein defined DNA markers can also be used to establish the fingerprinting profile of the *L. sakei* population profile present in a food sample. Accordingly, the present invention also relates to a method for fingerprinting a food sample likely to contain a *Lactobacillus sakei* isolate which comprises the amplification of 46 DNA markers of sequence SEQ NO: 1 to SEQ ID NO: 46 in said food sample, wherein the amplification profile of said 46 DNA markers is the fingerprint of said food sample. Preferably, the method for fingerprinting as defined above comprises the amplification of the 60 DNA markers of sequences defined above.

According to the invention, the amplification of DNA markers may be carried out directly from chromosomal DNA of cells extracted from the food sample, or on *L. sakei* isolates prior individualized from other species and strains of the food sample. Techniques to individualize bacterial isolates are well-known from the one skilled in the art and include for example streak plate technique, pour plate technique, serial dilution.

Preferably, in the methods of fingerprinting defined above, the amplification is performed using primers as defined above.

### Quantifying methods

The above defined methods according to the present invention are of particular interest to identify *L. sakei* strains in a cocktail of strains that can be used as biopreservative on meat and/or fish. Another issue of characterizing such cocktail of strains is to quantify each strain present in said cocktail.

Accordingly, another aspect of the invention relates to a method of quantifying a specific *Lactobacillus sakei* isolate in a sample comprising the steps consisting of :
a) identifying and/or characterizing the *Lactobacillus sakei* strains present in said sample by a method as defined above,
b) determining the DNA marker(s) which is(are) differently present in said specific *Lactobacillus sakei* strain with respect to other strains present in said sample, and
c) amplifying said DNA marker(s) by quantitative PCR.
Methods of quantitative PCR are well-known in the art and include real-time PCR, competitive PCR and radioactive PCR.

As used herein the term "sample" encompasses samples in which one or several *L*. *sakei* strain(s) is/are present, optionally in combination with other bacterial species.

Accordingly, the above method of quantifying a specific *L. sakei* strain in a sample may include a prior step of individualizing said strain from other strains present in the sample.

### Comparing methods

Another aspect of the present invention concerns a method of comparing at least two *Lactobacillus sakei* isolates comprising the steps consisting of:
a) determining the presence or absence of the DNA markers of sequence SEQ ID NO: 1 to SEQ ID NO: 46, in a first *Lactobacillus sakei* isolate, and
b) determining the presence or absence of said DNA markers in a second *Lactobacillus sakei* isolate,
wherein, if the pattern of presence or absence of said DNA markers is different between said first and said second *Lactobacillus sakei* isolates, then the *Lactobacillus sakei* isolates are different.

Preferably, in the method of comparing defined above, step a) comprises the determination of the presence or absence of the DNA markers of sequence SEQ ID NO: 47, SEQ ID NO: 48, SEQ ID NO: 50, SEQ ID NO: 51,SEQ ID NO: 3 to SEQ ID NO: 5, SEQ ID NO: 53 to SEQ ID NO: 55, SEQ ID NO: 8 to SEQ ID NO: 13, SEQ ID NO: 57, SEQ ID NO: 15 to SEQ ID NO: 18, SEQ ID NO: 59 to SEQ ID NO: 63, SEQ ID NO: 20, SEQ ID NO: 22 to SEQ ID NO: 25, SEQ ID NO: 27 to SEQ ID NO: 29, SEQ ID NO: 65, SEQ ID NO: 31, SEQ ID NO: 68 to SEQ ID NO: 70, SEQ ID NO: 33 to SEQ ID NO: 40 and SEQ ID NO: 42 to SEQ ID NO: 46. More preferably, in the method of comparing defined above, step a) comprises the determination of the presence or absence of the DNA markers of sequence SEQ ID NO: 3 to SEQ ID NO: 5, SEQ ID NO: 8 to SEQ ID NO: 13, SEQ ID NO: 15 to SEQ ID NO: 18, SEQ ID NO: 20, SEQ ID NO: 22 to SEQ ID NO: 25, SEQ ID NO: 27 to SEQ ID NO: 29, SEQ ID NO: 31, SEQ ID NO: 33 to SEQ ID NO: 40, SEQ ID NO: 42 to SEQ ID NO: 46 and SEQ ID NO: 47 to SEQ ID NO: 71.

The following examples and figures further describe the way the present inventors have identified said marker genes. These examples are illustrative, without being limited, of the above defined methods.

### Description of the figures

**Figure 1****:** Rapid screening of fingerprinting profile with 46 DNA markers. PCR detection of the DNA marker is indicated by a black background with a white number 1. An absence of detection of the DNA marker is indicated by a white background with a black null. Names of isolates are indicated at the top of the matrix. DNA markers are indicated on the left of the matrix.
**Figure 2****:** Example of allelic variations and their use in defining marker's specific or non-specific pairs of primers. A Show-Align alignment of SEQ ID NO: 6, SEQ ID NO: 53 and SEQ ID NO: 54 is displayed. Differences in the sequences are indicated by letters, similarities by dots. Mutations corresponding to insertion or deletion sites are boxed. Sequences of pairs of primers are indicated underlined and in bold letters with the corresponding SEQ ID.
**Figure 3****:** Fingerprinting profiles obtained with 60 DNA markers. PCR detection of the DNA marker is indicated by a black background with a white number 1. An absence of detection of the DNA marker is indicated by a white background with a black null. Names of isolates are indicated at the top of the matrix. DNA markers are indicated on the left of the matrix.
**Figures 4 to 6****:** Fingerprinting profiles of 129 L. sakei isolates taken from the known diversity scale of the species. The profiles were obtained with 52 DNA markers. PCR detection of the DNA marker is indicated by a black background with a white number 1. An absence of detection of the DNA marker is indicated by a white background with a black null. Names of isolates are indicated at the top of the matrix. DNA markers are indicated on the left of the matrix.
**Figure 7****:** Histogram distribution of the number of DNA markers responding positively to establish the fingerprinting profiles of 129 *L. sakei* isolates. The Gaussian probability distribution of the number of DNA markers in the population (estimated density on the left axis) is shown by the black smooth line.

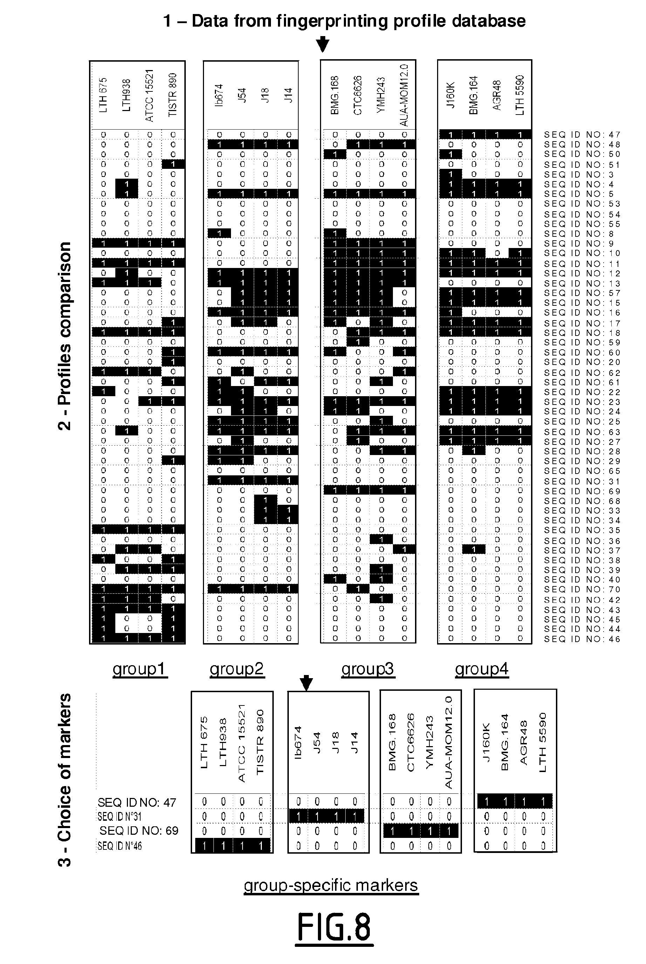

**Figure 8****:** Scheme representing the first strategy of a three steps protocol using the PCR fingerprinting profiles for designing specific markers for quantification of isolates.

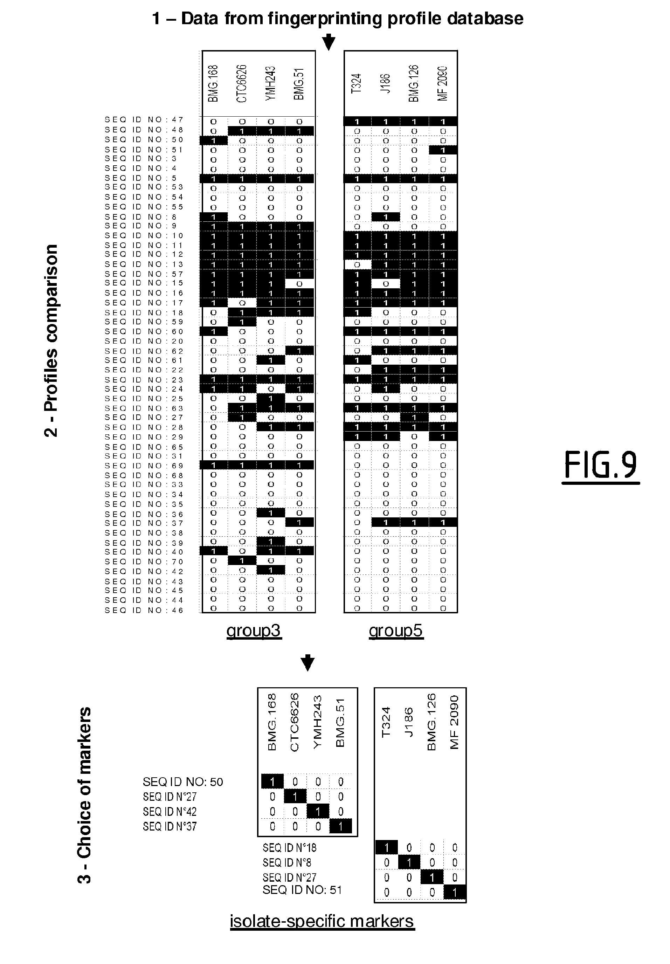

**Figure 9****:** Scheme representing the second strategy of a three steps protocol using the PCR fingerprinting profiles for designing specific markers for quantification of isolates.

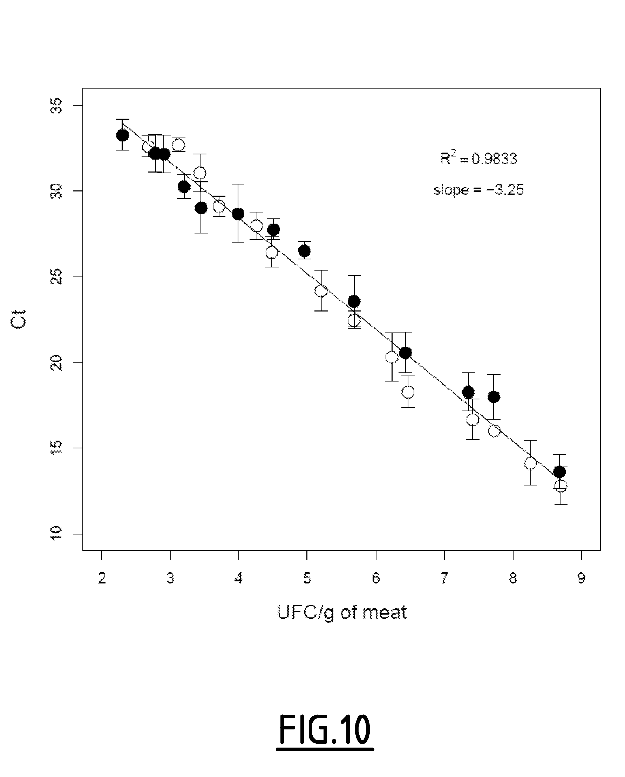

**Figure 10****:** Standard curves for the specific real-time PCR detection in ground beef inoculated with decreasing amount of two *L. sakei* strains. Strain 23K (white open circles) was detected using DNA marker SEQ NO: 20. Strain J112 (black circles) was detected using DNA marker SEQ NO: 28. Standard curves were plotted for the estimated log of cell number per g of meat versus Cₜ values. The Cₜ and UFC log values are the mean of six different measurements from duplicates.

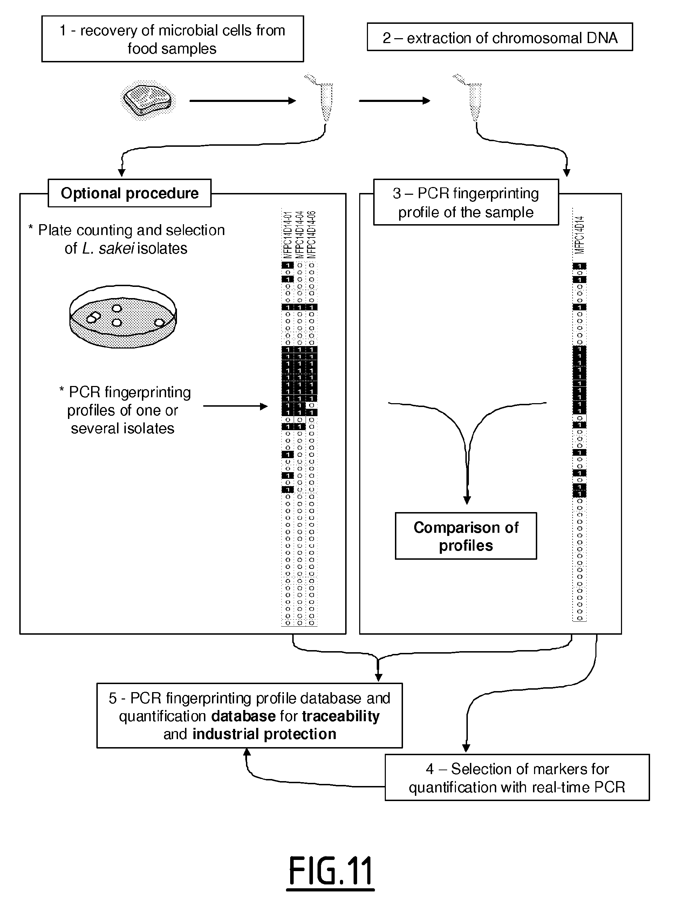

**Figure 11****:** Examples of experimental procedures making use of the present invention from a food sample to fingerprinting profile database. The fingerprinting profile may be obtained directly from isolates characterized from a food sample or directly from microbial DNA isolated from the same sample.

### EXAMPLES

### Example 1: PCR fingerprinting profile of any L. sakei isolates

For the purpose of constructing the present invention, the inventors conducted a large-scale substractive suppressive genomic hybridization experiment (SSH) on various strains chosen specifically from the diversity scale of *L. sakei species* (these strains are listed in **Table 1**). This experiment resulted in the generation of 20,000 SSH clones which were sequenced and assembled. The assembly resulted in the characterization of few hundreds of genomic loci inferred to the flexible gene pool of the *L. sakei* species. The pattern of variation (absence or presence of many of these islands and the degree of polymorphism in these genomic islands) was investigated between the chosen isolates described in **Table 1.**

This analysis resulted in the selection of 46 DNA regions (and associated ASP or INDEL) offering the best minimal set of markers for giving a unique PCR fingerprinting profile of any *L. sakei* isolates. As described in **Table 1**, the size of the DNA markers is ranging from 150 bp to about 7.5 kb and may comprise several encoding genes. These DNA markers may be associated with ASP or not.

These DNA markers are defined as a chromosomal region or loci (whose size are ranging from 150 bp to about 7.5 kb) and having a property of variability between *L. sakei* isolates. The design of only one PCR primer pair, anywhere within one of the DNA marker is sufficient to measure the variability property of the said marker. However ASP or INDEL sites offers the possibility to measure an additional level of variability by focusing also on the detection of specific alleles or INDEL mutations. The 46 DNA regions are described in SEQ NO: 1 to SEQ NO: 46.

The inventors have demonstrated that the combination of the 46 markers which can be tested in a minimal set of multiplex PCR allows the fingerprinting profile. Best fingerprinting profile is obtained when several set of primers are defined in ASP sites of the 46 DNA markers for detecting the alleles version of the said DNA marker, thus offering a highly specific PCR fingerprinting profile of any *L. sakei isolates.* As shown in **Figures 1** **and** **3**, several level of fingerprinting profile were tested on the reference strains used for characterizing the DNA markers. The inventors thus showed that fingerprinting profiles could be obtained from the minimal set of 46 DNA markers.

This minimal set is thus offering an easy procedure to rapidly discriminate different isolates. Increasing the fingerprinting profile to a set of 60 DNA markers, for instance, allowing the measurement of alleles within the 46 DNA markers, enables a highly accurate fingerprinting. The invention offers thus a flexible use of the DNA markers for either rapid fingerprinting (for screening groups of isolates for instance) to highly accurate fingerprinting isolates (to a level close to the strain-level).

To demonstrate this last use, the inventors carried out an assay among 130 *L. sakei* isolates taken from the known diversity scale of the species (70 isolates chosen from a large worldwide screening procedure as described by Chaillou et al. (2009) Appl. Environ. Microbiol. 75:970-980; and 60 new ones sourced with a similar strategy). As shown in **Figures 4 to 6**, the inventors demonstrated that a set of 52 pairs of primers localized within the 46 DNA markers and at whatsoever ASP site was sufficient for defining a unique PCR fingerprinting profile of any *L. sakei* isolates tested, describing thereby the 46 DNA markers and 6 of their ASP/INDEL sites as the best minimal set of markers for giving a unique PCR fingerprinting profile of any *L. sakei* isolates.

A unique profile between two isolates is defined as two profiles differing by at least one PCR detection. However, at present, the number of PCR fingerprint profiles is not limited to those shown in **Figures 4 to 6**, but is likely to be as large as the natural *L. sakei* strains diversity. Indeed, the number of DNA markers to which each isolate is responding positively is comprised between 8 and 25 (**Figure 7**), with an average of about 15 per isolate corresponding to a normal distribution. This is indicating that the invention offers a high degree of potential fingerprinting profiles (over 2.10¹² possibilities if 50 PCR primers set and a fixed number of combination of 15 markers are chosen).

### Example 2: Isolate- or cluster-specific markers for quantification of L. sakei isolates in a mixture

The inventors demonstrated that PCR fingerprint profiling as described above not only appears suitable for strain typing but can be apply for traceability (quantifying and/or monitoring) of any *L. sakei isolates* within a food reservoir. The PCR fingerprinting profiles can indeed be used as a strategy for comparison between isolates in order to design isolate-specific markers when several isolates are mixed together in a food sample and to achieve their accurate and specific quantification. Several level of specificity can be chosen from isolate-level specificity when several isolates with closely-related profiles are compared, or cluster-level when group of isolates are compared.

Typically, a three steps protocol using the PCR fingerprinting profiles was used for designing specific markers for quantification of isolates in a mixture. First, a PCR fingerprinting profile database was used for selecting isolates according to their profile in order to design mixtures of isolates for which markers could be chosen for their specific quantification. Several strategies were applied. Two examples of strategies are displayed in **Figures 8** **and** **9**. The first strategy was based on the selection of groups of isolates in which each of the said isolates have closely related fingerprinting profiles (**Figure 8**). The fingerprinting profiles of several groups were compared in order to choose group-specific markers for quantification and traceability of these groups in food matrices. The second strategy was based on the comparison of fingerprinting profiles within a group of related isolates for selecting isolate-specific markers (**Figure 9**).

As described in **Figure 10**, when the inventors performed a mixture of two isolates, they were able, by comparing their PCR fingerprinting profile, to design an isolate-specific marker for each isolate and to carry out their quantification after inoculation of the isolates in a fresh ground beef sample to which no natural *L. sakei* population was previously detected. Detection of each isolate revealed to be specific and linear from 10² to 10⁷ CFU/g. This strategy can therefore be used to design cocktail of isolates (*e.g*. protective cultures), for which a PCR fingerprinting profile can be used for monitoring the quantification of each isolate in the food samples.

### Example 3: PCR fingerprinting profile of food samples

The present inventors have demonstrated that the DNA markers defined above could be used for establishing the PCR fingerprinting profile of the *L. sakei* population present in a food sample.

As described in **Figure 11**, a PCR fingerprinting profile can be carried out directly from chromosomal DNA of microbial cells extracted from a food sample. In parallel, *L. sakei* isolates can be characterized by conventional plating methods and PCR fingerprinting profiles established for these isolates. The invention is thus suitable for comparative fingerprinting profile analysis, for profile database generation and for quantification of selected markers (monitoring of population at the isolate level in a food sample).

In food, the *L. sakei* population is likely to be influence by the "natural population" of these food samples as well as the types of food process and storage. Such population may also be influenced by the use of bio-preservative cultures. Therefore, the PCR fingerprinting profile of the *L. sakei* population can be applied for traceability of food processes.

## Claims

1. A kit for characterizing a *Lactobacillus sakei* isolate which comprises 46 pairs of primers, wherein each primer has a sequence that specifically hybridizes under high stringency conditions to one sequence selected from the group consisting of sequences SEQ ID NO: 1 to SEQ ID NO: 46 and a complementary sequence thereof.

2. A method for characterizing a *Lactobacillus sakei* isolate which comprises the step consisting of determining the presence or absence of 46 DNA markers of sequence SEQ ID NO: 1 to SEQ ID NO: 46 in said *Lactobacillus sakei* strain.

3. A method for characterizing a food sample likely to contain a *Lactobacillus sakei* strain which comprises the step consisting of determining the presence or absence of 46 DNA markers of sequence SEQ ID NO: 1 to SEQ ID NO: 46 in said food sample.

4. The method according to claim 2 or 3, which comprises the step of determining the presence or absence of 60 DNA markers of sequence SEQ ID NO: 3 to SEQ ID NO: 5, SEQ ID NO: 8 to SEQ ID NO: 13, SEQ ID NO: 15 to SEQ ID NO: 18, SEQ ID NO: 20, SEQ ID NO: 22 to SEQ ID NO: 25, SEQ ID NO: 27 to SEQ ID NO: 29, SEQ ID NO: 31, SEQ ID NO: 33 to SEQ ID NO:40, SEQ ID NO: 42 to SEQ ID NO: 46 and SEQ ID NO: 47 to SEQ ID NO: 71.

5. The method according to any one of claims 2 to 4, wherein the presence or absence of said DNA markers is determined by amplification.

6. The method according to any one of claims 2 to 5, wherein the presence or absence of said DNA markers is determined by hybridization with probes specific of said DNA markers.

7. A method for fingerprinting a *Lactobacillus sakei* isolate which comprises the amplification of 46 DNA markers of sequence SEQ ID NO: 1 to SEQ ID NO: 46 in said *Lactobacillus sakei* isolate, wherein the amplification profile of said 46 DNA markers is the fingerprint of said *Lactobacillus sakei* isolate.

8. A method for fingerprinting a food sample likely to contain a *Lactobacillus sakei* isolate which comprises the amplification of 46 DNA markers of sequence SEQ ID NO: 1 to SEQ ID NO: 46 in said food sample, wherein the amplification profile of said 46 DNA markers is the fingerprint of said food sample.

9. The method according to claim 7 or 8, which comprises the amplification of 60 DNA markers of sequence SEQ ID NO: 3 to SEQ ID NO: 5, SEQ ID NO: 8 to SEQ ID NO: 13, SEQ ID NO: 15 to SEQ ID NO: 18, SEQ ID NO: 20, SEQ ID NO: 22 to SEQ ID NO: 25, SEQ ID NO: 27 to SEQ ID NO: 29, SEQ ID NO: 31, SEQ ID NO: 33 to SEQ ID NO:40, SEQ ID NO: 42 to SEQ ID NO: 46 and SEQ ID NO: 47 to SEQ ID NO: 71.

10. A method of quantifying a specific *Lactobacillus sakei* isolate in a sample comprising the steps consisting of :
a) identifying and/or characterizing the *Lactobacillus sakei* strains present in said sample by a method as defined in any one of claims 2 and 4 to 6,
b) determining the DNA marker(s) which is(are) differently present in said specific *Lactobacillus sakei* strain with respect to other *L. sakei* strains present in said sample, and
c) amplifying said DNA marker(s) which are differently present by quantitative PCR.

11. The method according to claim 10, which comprises a prior step of individualizing said strain from other strains present in the sample.

12. A combination of markers enabling for characterizing a *Lactobacillus sakei* strain which comprises DNA markers of sequence SEQ ID NO: 1 to SEQ ID NO: 46.

13. The combination of markers according to claim 12, comprising DNA markers of sequence SEQ ID NO: 3 to SEQ ID NO: 5, SEQ ID NO: 8 to SEQ ID NO: 13, SEQ ID NO: 15 to SEQ ID NO: 18, SEQ ID NO: 20, SEQ ID NO: 22 to SEQ ID NO: 25, SEQ ID NO: 27 to SEQ ID NO: 29, SEQ ID NO: 31, SEQ ID NO: 33 to SEQ ID NO:40, SEQ ID NO: 42 to SEQ ID NO: 46 and SEQ ID NO: 47 to SEQ ID NO: 71.

14. A DNA array which comprises at least one probe comprising or consisting of a sequence that specifically hybridizes under high stringency conditions to a sequence selected from the sequences SEQ ID NO: 1 to SEQ ID NO: 60 and a complementary sequence thereof.

15. A DNA array according to claim 14, said at least one probe comprises or consists of a sequence selected from the group consisting of sequences SEQ ID NO: 76 to SEQ ID NO: 81, SEQ ID NO: 86 to SEQ ID NO: 97, SEQ ID NO: 100 to SEQ ID NO: 107, SEQ ID NO: 110 to SEQ ID NO: 111, SEQ ID NO: 114 to SEQ ID NO: 121, SEQ ID NO: 124 to SEQ ID NO: 129, SEQ ID NO: 132 to SEQ ID NO: 133, SEQ ID NO: 136 to SEQ ID NO: 151, and SEQ ID NO: 154 to SEQ ID NO: 213.

16. A method of comparing at least two *Lactobacillus sakei* isolates comprising the steps consisting of
a) determining the presence or absence of DNA markers of sequence SEQ ID NO: 1 to SEQ ID NO: 46, in a first *Lactobacillus sakei* isolate, and
b) determining the presence or absence of said DNA markers in a second *Lactobacillus sakei* isolate,
wherein, if the pattern of presence or absence of said DNA markers is different between said first and said second *Lactobacillus sakei* isolates, then the *Lactobacillus sakei* isolates are different.

17. The method according to claim 16, wherein step a) comprises the determination of the presence or absence of DNA markers of sequence SEQ ID NO: 3 to SEQ ID NO: 5, SEQ ID NO: 8 to SEQ ID NO: 13, SEQ ID NO: 15 to SEQ ID NO: 18, SEQ ID NO: 20, SEQ ID NO: 22 to SEQ ID NO: 25, SEQ ID NO: 27 to SEQ ID NO: 29, SEQ ID NO: 31, SEQ ID NO: 33 to SEQ ID NO: 40, SEQ ID NO: 42 to SEQ ID NO: 46 and SEQ ID NO: 47 to SEQ ID NO: 71.
